(19) **Europäisches Patentamt**
**European Patent Office**
**Office européen des brevets**

(11) **EP 3 622 519 B1**

(12) **EUROPEAN PATENT SPECIFICATION**

(45) Date of publication and mention
of the grant of the patent:
**13.09.2023 Bulletin 2023/37**

(51) International Patent Classification (IPC):
**G16B 20/00** (2019.01)   **G16B 40/20** (2019.01)

(52) Cooperative Patent Classification (CPC):
**G16B 20/00; G16B 40/20**

(21) Application number: **18796346.7**

(22) Date of filing: **15.10.2018**

(86) International application number:
**PCT/US2018/055919**

(87) International publication number:
**WO 2019/079200 (25.04.2019 Gazette 2019/17)**

(54) **DEEP LEARNING-BASED ABERRANT SPLICING DETECTION**

AUF TIEFENLERNEN BASIERENDE DETEKTION VON ABERRANTER SPLEISSUNG

DÉTECTION D'ÉPISSAGE ABERRANT BASÉE SUR UN APPRENTISSAGE PROFOND

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2017 US 201762573125 P**
**16.10.2017 US 201762573131 P**
**16.10.2017 US 201762573135 P**
**31.08.2018 US 201862726158 P**

(43) Date of publication of application:
**18.03.2020 Bulletin 2020/12**

(73) Proprietor: **Illumina, Inc.**
**San Diego, CA 92122 (US)**

(72) Inventors:
• **JAGANATHAN, Kishore**
**San Diego, CA 92122 (US)**
• **FARH, Kai-How**
**San Diego, CA 92122 (US)**
• **KYRIAZOPOULOU PANAGIOTOPOULOU, Sofia**
**San Diego, CA 92122 (US)**
• **MCRAE, Jeremy Francis**
**San Diego, CA 92122 (US)**

(74) Representative: **Robinson, David Edward Ashdown**
**Marks & Clerk LLP**
**1 New York Street**
**Manchester M1 4HD (GB)**

(56) References cited:
• XUEQIU JIAN ET AL: "In silico prediction of splice-altering single nucleotide variants in the human genome", NUCLEIC ACIDS RESEARCH, vol. 42, no. 22, 21 November 2014 (2014-11-21), pages 13534-13544, XP055549955, ISSN: 0305-1048, DOI: 10.1093/nar/gku1206
• CLAUDE HOUDAYER ET AL: "Guidelines for splicing analysis in molecular diagnosis derived from a set of 327 combined in silico/in vitro studies on BRCA1 and BRCA2 variants - Houdayer - 2012 - Human Mutation - Wiley Online Library", HUMAN MUTATION, vol. 33, 13 April 2012 (2012-04-13), pages 1228-1238, XP055551010,
• H. Y. XIONG ET AL: "The human splicing code reveals new insights into the genetic determinants of disease", SCIENCE, vol. 347, no. 6218, 18 December 2014 (2014-12-18), pages 1254806-1254806, XP055551008, US ISSN: 0036-8075, DOI: 10.1126/science.1254806
• YI ZHANG ET AL: "DeepSplice: Deep classification of novel splice junctions revealed by RNA-seq", 2016 IEEE INTERNATIONAL CONFERENCE ON BIOINFORMATICS AND BIOMEDICINE (BIBM), 1 December 2016 (2016-12-01), pages 330-333, XP055548206, DOI: 10.1109/BIBM.2016.7822541 ISBN: 978-1-5090-1611-2

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

<u>APPENDIX</u>

The Appendix includes a bibliography of potentially relevant references listed in a paper authored by the inventors. The subject matter of the paper is covered in the US Provisionals to which this Application claims priority to/benefit of. These references can be made available by the Counsel upon request or may be accessible via Global Dossier.

<u>PRIORITY APPLICATIONS</u>

This application claims priority to or the benefit of US Provisional Patent Application No. 62/573,125, titled, "Deep Learning-Based Splice Site Classification," by Kishore Jaganathan, Kai-How Farh, Sofia Kyriazopoulou Panagiotopoulou and Jeremy Francis McRae, filed October 16, 2017 (Attorney Docket No. ILLM 1001-1/IP-1610-PRV); US Provisional Patent Application No. 62/573,131, titled, "Deep Learning-Based Aberrant Splicing Detection," by Kishore Jaganathan, Kai-How Farh, Sofia Kyriazopoulou Panagiotopoulou and Jeremy Francis McRae, filed October 16, 2017 (Attorney Docket No. ILLM 1001-2/IP-1614-PRV); US Provisional Patent Application No. 62/573,135, titled, "Aberrant Splicing Detection Using Convolutional Neural Networks (CNNs)," by Kishore Jaganathan, Kai-How Farh, Sofia Kyriazopoulou Panagiotopoulou and Jeremy Francis McRae, filed October 16, 2017 (Attorney Docket No. ILLM 1001-3/IP-1615-PRV); and US Provisional Patent Application No. 62/726,158, titled, "Predicting Splicing from Primary Sequence with Deep Learning," by Kishore Jaganathan, Kai-How Farh, Sofia Kyriazopoulou Panagiotopoulou and Jeremy Francis McRae, filed August 31, 2018 (Attorney Docket No. ILLM 1001-10/IP-1749-PRV).

<u>FIELD OF THE TECHNOLOGY DISCLOSED</u>

The technology disclosed relates to artificial intelligence type computers and digital data processing systems and corresponding data processing methods and products for emulation of intelligence (i.e., knowledge based systems, reasoning systems, and knowledge acquisition systems); and including systems for reasoning with uncertainty (e.g., fuzzy logic systems), adaptive systems, machine learning systems, and artificial neural networks. In particular, the technology disclosed relates to using deep learning-based techniques for training deep convolutional neural networks. XUEQIU JIAN ET AL "In silico prediction of splice-altering single nucleotide variants in the human genome" describes a comparison of eight *in silico* tools on 2959 single nucleotide variants within splicing consensus regions (scSNVs) using receiver operating characteristic analysis. The Position Weight Matrix model and MaxEntScan outperformed other methods. Two ensemble learning methods, adaptive boosting and random forests, were used to construct models that take advantage of individual methods. Both models further improved prediction, with outputs of directly interpretable prediction scores.

<u>BACKGROUND</u>

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

**Machine Learning**

In machine learning input variables are used to predict an output variable. The input variables are often called features and are denoted by $X = (X_1, X_2, \ldots, X_k)$, where each $X_i$, $i \in 1, \ldots, k$ is a feature. The output variable is often called the response or dependent variable and is denoted by the variable $Y_i$. The relationship between $Y$ and the corresponding X can be written in a general form:

$$Y = f(X) + \in$$

In the equation above, $f$ is a function of the features $(X_1, X_2, \ldots, X_k)$ and $\in$ is the random error term. The error term is independent of X and has a mean value of zero.

In practice, the features X are available without having Y or knowing the exact relation between X and Y. Since the error term has a mean value of zero, the goal is to estimate *f*.

$$\hat{Y} = \hat{f} = (X)$$

In the equation above, $\hat{f}$ is the estimate of $\epsilon$, which is often considered a black box, meaning that only the relation between the input and output of $\hat{f}$ is known, but the question why it works remains unanswered.

The function $\hat{f}$ is found using learning. Supervised learning and unsupervised learning are two ways used in machine learning for this task. In supervised learning, labeled data is used for training. By showing the inputs and the corresponding outputs (=labels), the function $\hat{f}$ is optimized such that it approximates the output. In unsupervised learning, the goal is to find a hidden structure from unlabeled data. The algorithm has no measure of accuracy on the input data, which distinguishes it from supervised learning.

## Neural Networks

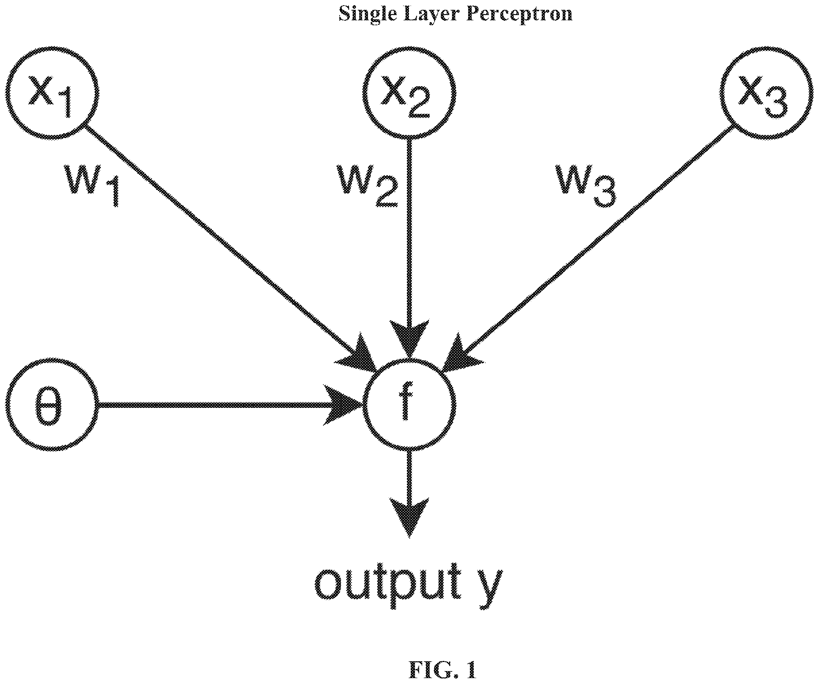

The single layer perceptron (SLP) is the simplest model of a neural network. It comprises one input layer and one activation function as shown in **FIG. 1.** The inputs are passed through the weighted graph. The function *f* uses the sum of the inputs as argument and compares this with a threshold $\theta$.

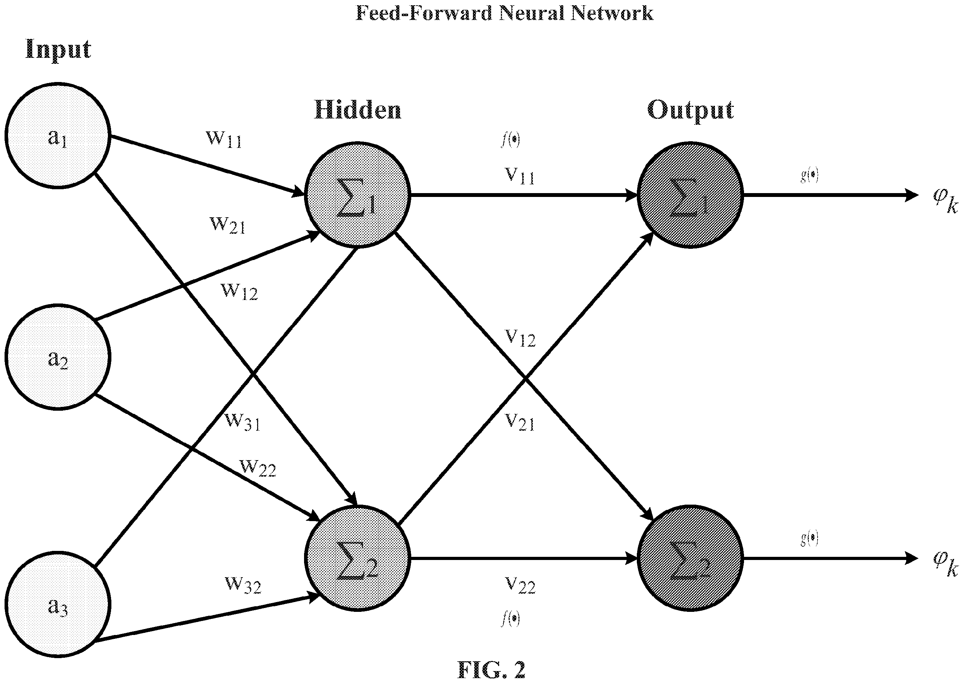

**FIG. 2** shows one implementation of a fully connected neural network with multiple layers. A neural network is a system of interconnected artificial neurons (*e.g.*, $a_1$, $a_2$, $a_3$) that exchange messages between each other. The illustrated neural network has three inputs, two neurons in the hidden layer and two neurons in the output layer. The hidden layer has an activation function $f(\bullet)$ and the output layer has an activation function $g(\bullet)$. The connections have numeric weights (*e.g.*, $w_{11}$, $w_{21}$, $w_{12}$, $w_{31}$, $w_{22}$, $w_{32}$, $v_{11}$, $v_{22}$) that are tuned during the training process, so that a properly trained network responds correctly when fed an image to recognize. The input layer processes the raw input, the hidden layer processes the output from the input layer based on the weights of the connections between the input layer and the hidden layer. The output layer takes the output from the hidden layer and processes it based on the weights of the connections between the hidden layer and the output layer. The network includes multiple layers of feature-detecting neurons. Each layer has many neurons that respond to different combinations of inputs from the previous layers. These layers are constructed so that the first layer detects a set of primitive patterns in the input image data, the second layer detects patterns of patterns and the third layer detects patterns of those patterns.

A survey of application of deep learning in genomics can be found in the following publications:

- T. Ching et al., Opportunities And Obstacles For Deep Learning In Biology And Medicine, www.biorxiv.org:142760, 2017:
- Angermueller C, Pärnamaa T, Parts L, Stegle O. Deep Learning For Computational Biology. Mol Syst Biol. 2016;12:878;
- Park Y, Kellis M. 2015 Deep Learning For Regulatory Genomics. Nat. Biotechnol. 33,825 826. (doi:10.1038/nbt.3313);
- Min, S., Lee, B. & Yoon, S. Deep Learning In Bioinformatics. Brief. Bioinform. bbw068 (2016);
- Leung MK, Delong A, Alipanahi B et al. Machine Learning In Genomic Medicine: A Review of Computational Problems and Data Sets 2016; and
- Libbrecht MW, Noble WS. Machine Learning Applications In Genetics and Genomics. Nature Reviews Genetics 2015;16(6):321-32.

## BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which:

**FIG. 1** shows a single layer perceptron (SLP).
**FIG. 2** shows one implementation of a feed-forward neural network with multiple layers.
**FIG. 3** depicts one implementation of workings of a convolutional neural network.
**FIG. 4** depicts a block diagram of training a convolutional neural network in accordance with one implementation of the technology disclosed.
**FIG. 5** shows one implementation of a ReLU non-linear layer in accordance with one implementation of the technology disclosed.

**FIG. 6** illustrates dilated convolutions.

**FIG. 7** is one implementation of sub-sampling layers (average/max pooling) in accordance with one implementation of the technology disclosed.

**FIG. 8** depicts one implementation of a two-layer convolution of the convolution layers.

**FIG. 9** depicts a residual connection that reinjects prior information downstream via feature-map addition.

**FIG. 10** depicts one implementation of residual blocks and skip-connections.

**FIG. 11** shows one implementation of stacked dilated convolutions.

**FIG. 12** shows the batch normalization forward pass.

**FIG. 13** illustrates the batch normalization transform at test time.

**FIG. 14** shows the batch normalization backward pass.

**FIG. 15** depicts use of a batch normalization layer with convolutional or densely connected layer.

**FIG. 16** shows one implementation of 1D convolution.

**FIG. 17** illustrates how global average pooling (GAP) works.

**FIG. 18** illustrates one implementation of a computing environment with training servers and production servers that can be used to implement the technology disclosed.

**FIG. 19** depicts one implementation of the architecture of an atrous convolutional neural network (abbreviated ACNN), referred to herein as "SpliceNet".

**FIG. 20** shows one implementation of a residual block that can used by the ACNN and a convolutional neural network (abbreviated CNN).

**FIG. 21** depicts another implementation of the architecture of the ACNN, referred to herein as "SpliceNet80".

**FIG. 22** depicts yet another implementation of the architecture of the ACNN, referred to herein as "SpliceNet400".

**FIG. 23** depicts yet further implementation of the architecture of the ACNN, referred to herein as "SpliceNet2000".

**FIG. 24** depicts yet another implementation of the architecture of the ACNN, referred to herein as "SpliceNet10000".

**FIGs. 25, 26,** and **27** show various types of inputs processed by the ACNN and the CNN.

**FIG. 28** shows that the ACNN can be trained on at least 800 million non-splicing sites and the CNN can be trained on at least **1** million non-splicing sites.

**FIG. 29** illustrates a one-hot encoder.

**FIG. 30** depicts training of the ACNN.

**FIG. 31** shows a CNN.

**FIG. 32** shows training, validation, and testing of the ACNN and the CNN.

**FIG. 33** depicts a reference sequence and an alternative sequence.

**FIG. 34** illustrates aberrant splicing detection.

**FIG. 35** illustrates processing pyramid of SpliceNet10000 for splice site classification.

**FIG. 36** depicts processing pyramid of SpliceNet10000 for aberrant splicing detection.

**FIGs. 37A, 37B, 37C, 37D, 37E, 37F, 37G,** and **37H** illustrates one implementation of predicting splicing from primary sequence with deep learning.

**FIGs. 38A, 38B, 38C, 38D, 38E, 38F,** and **38G** depict one implementation of validation of rare cryptic splice mutations in RNA-seq data.

**FIGs. 39A, 39B,** and **39C** show one implementation of cryptic splice variants frequently create tissue-specific alternative splicing.

**FIGs. 40A, 40B, 40C, 40D,** and **40E** depict one implementation of predicted cryptic splice variants are strongly deleterious in human populations.

**FIGs. 41A, 41B, 41C, 41D, 41E,** and **41F** show one implementation of de novo cryptic splice mutations in patients with rare genetic disease.

**FIGs. 42A** and **42B** depict evaluation of various splicing prediction algorithms on lincRNAs.

**FIGs. 43A** and **43B** illustrate position-dependent effects of the TACTAAC branch point and GAAGAA exonic-splice enhancer motifs.

**FIGs. 44A** and **44B** depict effects of nucleosome positioning on splicing.

**FIG. 45** illustrates example of calculating effect size for a splice-disrupting variant with complex effects.

**FIGs. 46A, 46B,** and **46C** show evaluation of the SpliceNet-10k model on singleton and common variants.

**FIGs. 47A** and **47B** depict validation rate and effect sizes of splice site-creating variants, split by the location of the variant.

**FIGs. 48A, 48B, 49C,** and 49D depict evaluation of the SpliceNet- 10k model on train and test chromosomes.

**FIG. 49A, 49B,** and **49C** illustrate de novo cryptic splice mutations in patients with rare genetic disease, only from synonymous, intronic or untranslated region sites.

**FIGs. 50A** and **50B** show cryptic splice de novo mutations in ASD and as a proportion of pathogenic DNMs.

**FIG. 51** depicts RNA-seq validation of predicted cryptic splice de novo mutations in ASD patients.

**FIGs. 52A** and **52B** illustrate validation rate and sensitivity on RNA-seq of a model trained on canonical transcripts only.

**FIGs. 53A, 53B,** and **53C** illustrate ensemble modeling improves SpliceNet-10k performance.

**FIGs. 54A** and **54B** show evaluation of SpliceNet-10k in regions of varying exon density.

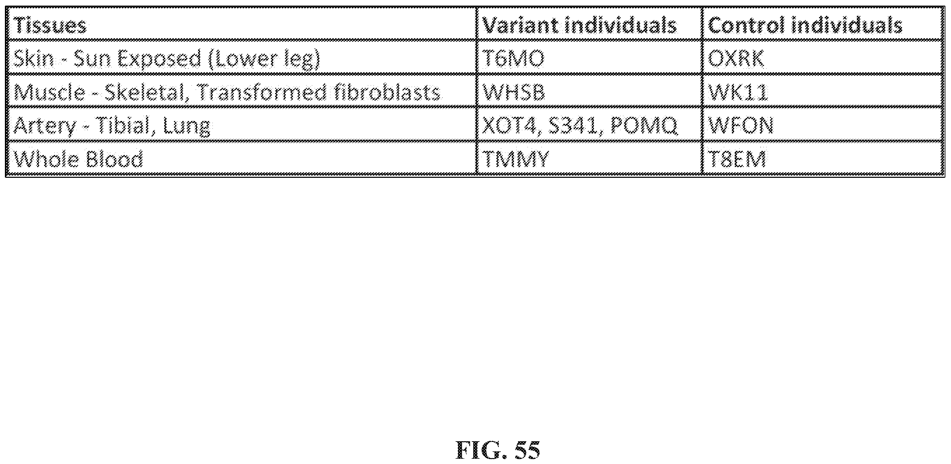

**FIG. 55** is Table S1 which depicts one implementation of GTEx samples used for demonstrating effect size calculations and tissue-specific splicing.

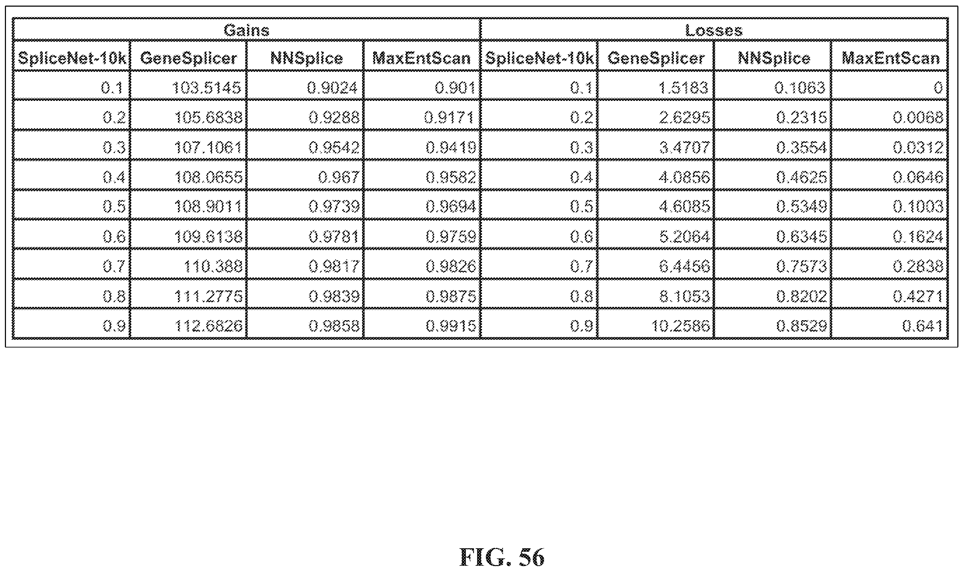

**FIG. 56** is Table S2 which depicts one implementation of cutoffs used for evaluating the validation rate and sensitivity of different algorithms.

**FIG. 57** shows one implementation of per-gene enrichment analysis.

**FIG. 58** shows one implementation of genome-wide enrichment analysis.

**FIG. 59** is a simplified block diagram of a computer system that can be used to implement the technology disclosed.

## DETAILED DESCRIPTION

The following discussion is presented to enable any person skilled in the art to make and use the technology disclosed, and is provided in the context of a particular application and its requirements. Uwiew

### Introduction

### Convolutional Neural Networks

A convolutional neural network is a special type of neural network. The fundamental difference between a densely connected layer and a convolution layer is this: Dense layers learn global patterns in their input feature space, whereas convolution layers learn local patters: in the case of images, patterns found in small 2D windows of the inputs. This key characteristic gives convolutional neural networks two interesting properties: (1) the patterns they learn are translation invariant and (2) they can learn spatial hierarchies of patterns.

Regarding the first, after learning a certain pattern in the lower-right corner of a picture, a convolution layer can recognize it anywhere: for example, in the upper-left corner. A densely connected network would have to learn the pattern anew if it appeared at a new location. This makes convolutional neural networks data efficient because they need fewer training samples to learn representations they have generalization power.

Regarding the second, a first convolution layer can learn small local patterns such as edges, a second convolution layer will learn larger patterns made of the features of the first layers, and so on. This allows convolutional neural networks to efficiently learn increasingly complex and abstract visual concepts.

A convolutional neural network learns highly non-linear mappings by interconnecting layers of artificial neurons arranged in many different layers with activation functions that make the layers dependent. It includes one or more convolutional layers, interspersed with one or more sub-sampling layers and non-linear layers, which are typically followed by one or more fully connected layers. Each element of the convolutional neural network receives inputs from a set of features in the previous layer. The convolutional neural network learns concurrently because the neurons in the same feature map have identical weights. These local shared weights reduce the complexity of the network such that when multi-dimensional input data enters the network, the convolutional neural network avoids the complexity of data reconstruction in feature extraction and regression or classification process.

Convolutions operate over 3D tensors, called feature maps, with two spatial axes (height and width) as well as a depth axis (also called the channels axis). For an RGB image, the dimension of the depth axis is 3, because the image has three color channels; red, green, and blue. For a black-and-white picture, the depth is 1 (levels of gray). The convolution operation extracts patches from its input feature map and applies the same transformation to all of these patches, producing an output feature map. This output feature map is still a 3D tensor: it has a width and a height. Its depth can be arbitrary, because the output depth is a parameter of the layer, and the different channels in that depth axis no longer stand for specific colors as in RGB input; rather, they stand for filters. Filters encode specific aspects of the input data: at a height level, a single filter could encode the concept "presence of a free in the input," for instance.

For example, the first convolution layer takes a feature map of size (28, 28, 1) and outputs a feature map of size (26, 26, 32): it computes 32 filters over its input. Each of these 32 output channels contains a $26 \times 26$ grid of values, which is a response map of the filter over the input, indicating the response of that filter pattern at different locations in the input. That is what the term feature map means: every dimension in the depth axis is a feature (or filter), and the 2D tensor output [:, :, n] is the 2D spatial map of the response of this filter over the input.

Convolutions are defined by two key parameters: (1) size of the patches extracted from the inputs-these are typically $1 \times 1$, $3 \times 3$ or $5 \times 5$ and (2) depth of the output feature map - the number of filters computed by the convolution. Often these start with a depth of 32, continue to a depth of 64, and terminate with a depth of 128 or 256.

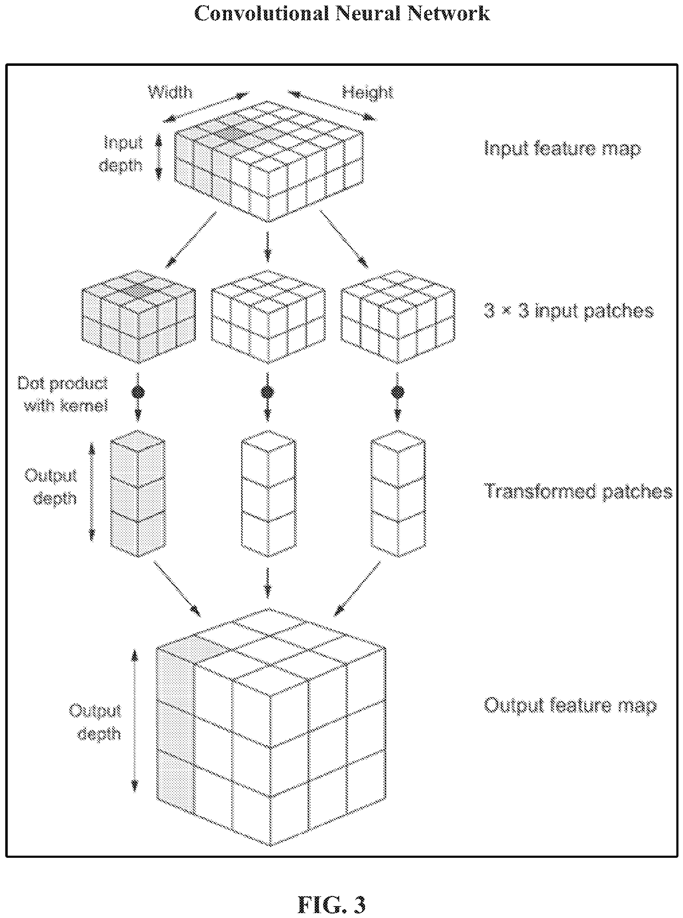

A convolution works by sliding these windows of size $3 \times 3$ or $5 \times 5$ over the 3D input feature map, stopping at every location, and extracting the 3D patch of surrounding features (shape (window_height, window_width, input_depth)). Each such 3D patch is ten transformed (via a tensor product with the same learned weight matrix, called the convolution kernel) into a 1D vector of shape (output_depth.). All of these vectors are then spatially reassembled into a 3D output map of shape (height, width, output_depth). Every spatial location in the output feature map corresponds to the same location in the input feature map (for example, the lower-right corner of the output contains information about the lower-right corner of the input). For instance, with $3 \times 3$ windows, the vector output [i,j,:] comes from the 3D patch input [i-1: i+1, j-1:J+-1,:]. The full process is detailed in **FIG. 3**.

The convolutional neural network comprises convolution layers which perform the convolution operation between the input values and convolution filters (matrix of weights) that are learned over many gradient update iterations during the training. Let $(m, n)$ be the filter size and $W$ be the matrix of weights, then a convolution layer performs a convolution of the $W$ with the input $X$ by calculating the dot product $W \cdot x + b$, where $x$ is an instance of $X$ and $b$ is the bias. The step size by which the convolution filters slide across the input is called the stride, and the filter area ($m \times n$) is called the receptive field. A same convolution filter is applied across different positions of the input, which reduces the number of weights learned. It also allows location invariant learning, i.e., if an important pattern exists in the input, the convolution filters learn it no matter where it is in the sequence.

## Training a Convolutional Neural Network

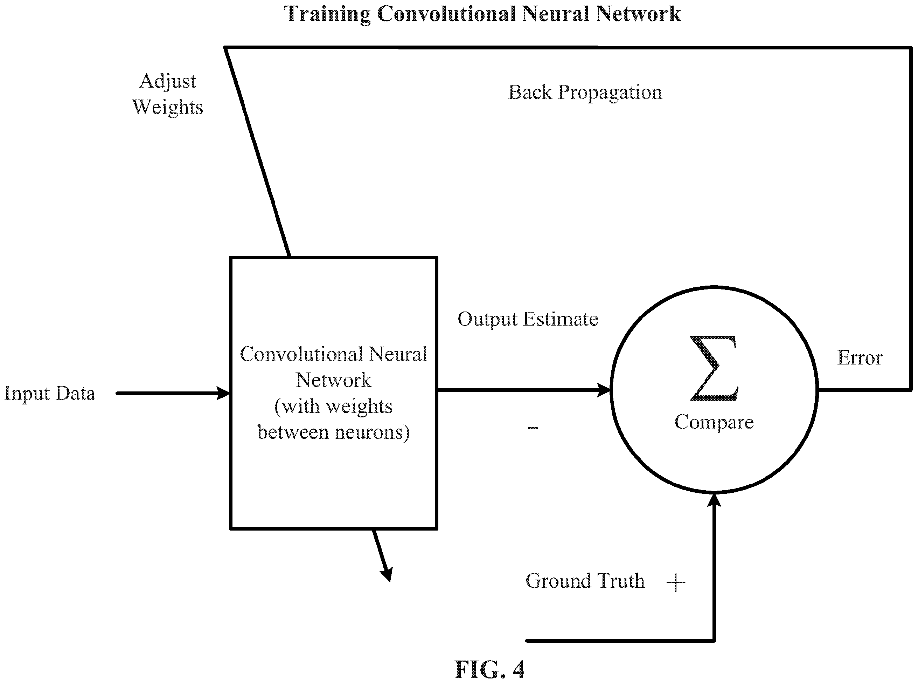

**FIG. 4** depicts a block diagram of training a convolutional neural network in accordance with one implementation of the technology disclosed. The convolutional neural network is adjusted or trained so that the input data leads to a specific output estimate. The convolutional neural network is adjusted using back propagation based on a comparison of the output estimate and the ground truth until the output estimate progressively matches or approaches the ground truth.

The convolutional neural network is trained by adjusting the weights between the neurons based on the difference between the ground truth and the actual output. This is mathematically described as:

$$\Delta w_i = x_i \delta$$

where $\delta$ = (*ground truth*)-(*actual output*)

In one implementation, the training rule is defined as:

$$W_{nm} \leftarrow W_{nm} + \alpha(t_m - \varphi_m)a_n$$

In the equation above: the arrow indicates an update of the value; $t_m$ is the target value of neuron $m$: $\varphi_m$ is the computed current output of neuron $m$ ; $a_n$ is input $n$; and $\alpha$ is the learning rate.

The intermediary step in the training includes generating a feature vector from the input data using the convolution layers. The gradient with respect to the weights in each layer, starting at the output, is calculated. This is referred to as the backward pass, or going backwards. The weights in the network are updated using a combination of the negative gradient and previous weights.

In one implementation, the convolutional neural network uses a stochastic gradient update algorithm (such as ADAM) that performs backward propagation of errors by means of gradient descent. One example of a sigmoid function based back propagation algorithm is described below:

$$\varphi = f(h) = \frac{1}{1 + e^{-h}}$$

In the sigmoid function above. $h$ is the weighted sum computed by a neuron. The sigmoid function has the following derivative:

$$\frac{\partial \varphi}{\partial h} = \varphi(1 - \varphi)$$

The algorithm includes computing the activation of all neurons in the network, yielding an output for the forward pass. The activation of neuron $m$ in the hidden layers is described as:

$$\varphi_m = \frac{1}{1+e^{-h_m}}$$

$$h_m = \sum_{n=1}^{N} a_n w_{nm}$$

This is done for all the hidden layers to get the activation described as:

$$\varphi_k = \frac{1}{1+e^{h_k}}$$

$$h_k = \sum_{m=1}^{M} \varphi_m v_{mk}$$

Then, the error and the correct weights are calculated per layer. The error at the output is computed as:

$$\delta_{ok} = (t_k - \varphi_k)\varphi_k(1-\varphi_k)$$

The error in the hidden layers is calculated as:

$$\delta_{hm} = \varphi_m(1-\varphi_m)\sum_{k=1}^{K} v_{mk}\delta_{ok}$$

The weights of the output layer are updated as:

$$v_{mk} \leftarrow v_{mk} + \alpha\delta_{ok}\varphi_m$$

The weights of the hidden layers are updated using the learning rate **a** as:

$$v_{nm} \leftarrow w_{nm} + \alpha\delta_{hm}a_n$$

In one implementation, the convolutional neural network uses a gradient descent optimization to compute the error across all the layers. In such an optimization, for an input feature vector $x$ and the predicted output $\hat{y}$, the loss function is defined as $l$ for the cost of predicting $\hat{y}$ when the target is $y$, i.e. $l(\hat{y}, y)$. The predicted output $\hat{y}$ is transformed from the input feature vector $x$ using function $f$. Function $f$ is parameterized by the weights of convolutional neural network, i.e. $\hat{y}=f_w(x)$. The loss function is described as $l(\hat{y},y)= l(f_w(x),y)$, or $Q(z,w)=l(f_w(x),y)$ where $z$ is an input and output data pair $(x, y)$. The gradient descent optimization is performed by updating the weights according to:

$$v_{t+1} = \mu v_t - \alpha \frac{1}{n}\sum_{i=1}^{N} \nabla_{w_t} Q(z_t, w_t)$$

$$w_{t+1} = w_t + v_{t+1}$$

In the equations above, $\alpha$ is the learning rate. Also, the loss is computed as the average over a set of **n** data pairs. The computation is terminated when the learning rate $\alpha$ is small enough upon linear convergence. In other implementations, the gradient is calculated using only selected data pairs fed to a Nesterov's accelerated gradient and an adaptive gradient to inject computation efficiency.

In one implementation, the convolutional neural network uses a stochastic gradient descent (SGD) to calculate the cost function. A SGD approximates the gradient with respect to the weights in the loss function by computing it from only one, randomized, data pair, $Z_t$, described as:

$$V_{t+1} = \mu V - \alpha \nabla_w Q(z_t, w_t)$$

$$W_{t+1} = W_t + V_{t+1}$$

In the equations above: $\alpha$ is the learning rate; $\mu$ is the momentum; and $t$ is the current weight state before updating. The convergence speed of SGD is approximately $O(1/t)$ when the learning rate $\alpha$ are reduced both fast and slow enough. In other implementations, the convolutional neural network uses different loss functions such as Euclidean loss and softmax loss. In a further implementation, an Adam stochastic optimizer is used by the convolutional neural network.

**Convolution Layers**

The convolution layers of the convolutional neural network serve as feature extractors. Convolution layers act as adaptive feature extractors capable of learning and decomposing the input data into hierarchical features. In one implementation, the convolution layers take two images as input and produce a third image as output. In such an implementation, convolution operates on two images in two-dimension (2D), with one image being the input image and the other image. called the "kernel", applied as a filter on the input image, producing an output image. Thus, for an input vector $f$ of length n and $a$ kernel $g$ of length $m$, the convolution $f*g$ of $f$ and $g$ is defined as:

$$(f * g)(i) = \sum_{j=1}^{m} g(j) \cdot f(i - j + m/2)$$

The convolution operation includes sliding the kernel over the input image. For each position of the kernel, the overlapping values of the kernel and the input image are multiplied and the results are added. The sum of products is the value of the output image at the point in the input image where the kernel is centered. The resulting different outputs from many kernels are called feature maps.
Once the convolutional layers are trained, they are applied to perform recognition tasks on new inference data. Since the convolutional layers learn from the training data. they avoid explicit feature extraction and implicitly learn from the training data. Convolution layers use convolution filter kernel weights, which are determined and updated as part of the training process. The convolution layers extract different features of the input, which are combined at higher layers. The convolutional neural network uses a various number of convolution layers, each with different convolving parameters such as kernel size, strides, padding, number of feature maps and weights.

**Non-Linear Layers**

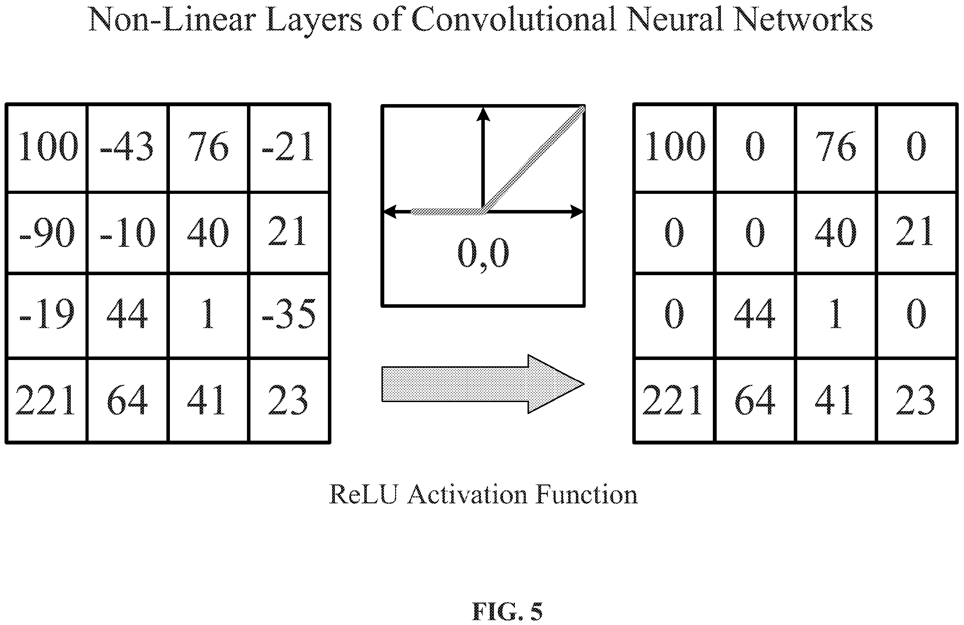

**FIG. 5** shows one implementation of non-linear layers in accordance with one implementation of the technology disclosed. Non-linear layers use different non-linear trigger functions to signal distinct identification of likely features on each hidden layer. Non-linear layers use a variety of specific functions to implement the non-linear triggering, including the rectified linear units (ReLUs), hyperbolic tangent, absolute of hyperbolic tangent, sigmoid and continuous trigger (non-linear) functions. In one implementation, a ReLU activation implements the function $y = max(x, 0)$ and keeps the input and output sizes of a layer the same. The advantage of using ReLU is that the convolutional neural network is trained many times faster. ReLU is a non-continuous, non-saturating activation function that is linear with respect to the input if the input values are larger than zero and zero otherwise. Mathematically, a ReLU activation function is described as:

$$\varphi(h) = \max(h, 0)$$

$$\varphi(h) = \begin{cases} h & \text{if } h > 0 \\ 0 & \text{if } h \leq 0 \end{cases}$$

In other implementations, the convolutional neural network uses a power unit activation function. which is a continuous, non-saturating function described by:

$$\varphi(h) = (a + bh)^C$$

In the equation above, *a, b* and c are parameters controlling the shift, scale and power respectively. The power activation function is able to yield *x* and *y*-antisymmetric activation if *c* is odd and *y*-symmetric activation if *c* is even. In some implementations, the unit yields a non-rectified linear activation.

In yet other implementations, the convolutional neural network uses a sigmoid unit activation function, which is a continuous, saturating function described by the following logistic function:

$$\varphi(h) = \frac{1}{1 + e^{-\beta h}}$$

In the equation above, $\beta$=**1.** The sigmoid unit activation function does not yield negative activation and is only antisymmetric with respect to the *y*-axis.

### Dilated Convolutions

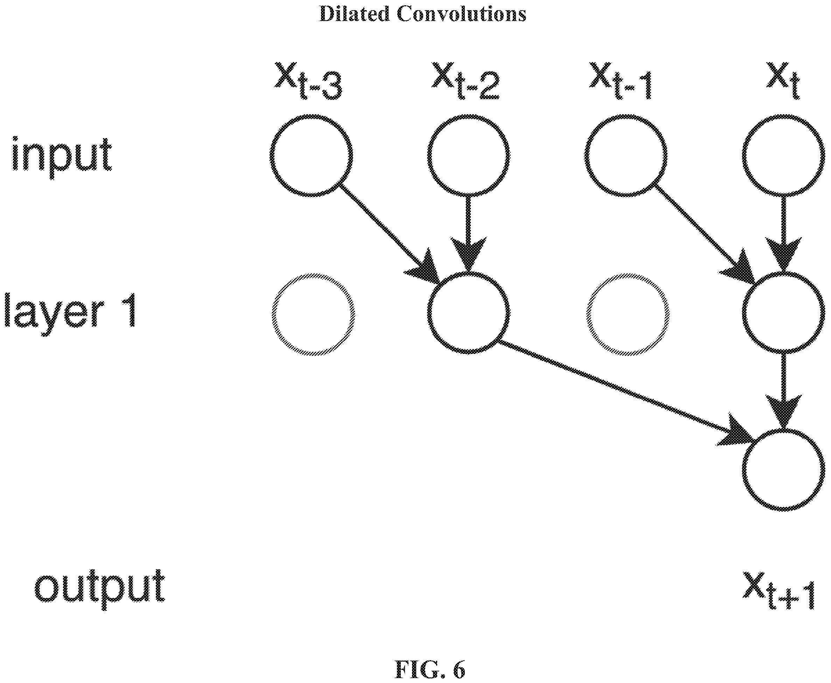

**FIG. 6** illustrates dilated convolutions. Dilated convolutions, sometimes called atrous convolutions, which literally means with holes. The French name has its origins in the algorithme a trous, which computes the fast dyadic wavelet transform. In these type of convolutional layers, the inputs corresponding to the receptive field of the filters are not neighboring points. This is illustrated in **FIG. 6.** The distance between the inputs is dependent on the dilation factor.

### Sub-Sampling Layers

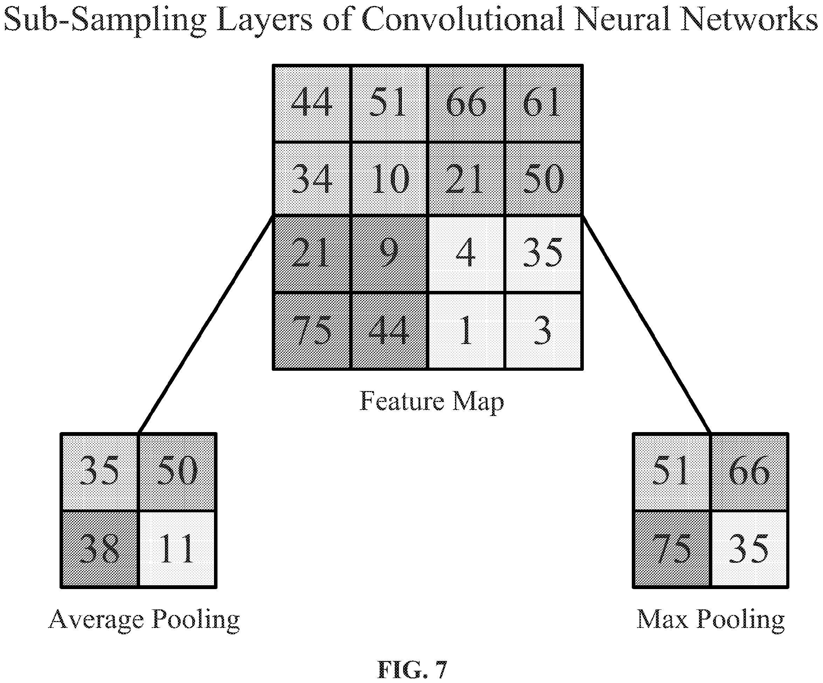

**FIG. 7** is one implementation of sub-sampling layers in accordance with one implementation of the technology disclosed. Sub-sampling layers reduce the resolution of the features extracted by the convolution layers to make the extracted features or feature maps- robust against noise and distortion. In one implementation, sub-sampling layers employ two types of pooling operations, average pooling and max pooling. The pooling operations divide the input into non-overlapping two-dimensional spaces. For average pooling, the average of the four values in the region is calculated. For max pooling, the maximum value of the four values is selected.

In one implementation, the sub-sampling layers include pooling operations on a set of neurons in the previous layer by mapping its output to only one of the inputs in max pooling and by mapping its output to the average of the input in average pooling. In max pooling. the output of the pooling neuron is the maximum value that resides within the input as described by:

$$\varphi_o = \max(\varphi_1, \varphi_2, ..., \varphi_N)$$

In the equation above, *N* is the total number of elements within a neuron set.

In average pooling, the output of the pooling neuron is the average value of the input values that reside with the input neuron set, as described by:

$$\varphi_o = \frac{1}{N} \sum_{n=1}^{N} \varphi_n$$

In the equation above, N is the total number of elements within input neuron set.

In **FIG. 7.** the input is of size 4 × 4. For 2 × 2 sub-sampling, a 4 × 4 image is divided into four non-overlapping matrices of size 2 × 2. For average pooling. the average of the four values is the whole-integer output. For max pooling. the maximum value of the four values in the 2 × 2 matrix is the whole-integer output,

### Convolution Examples

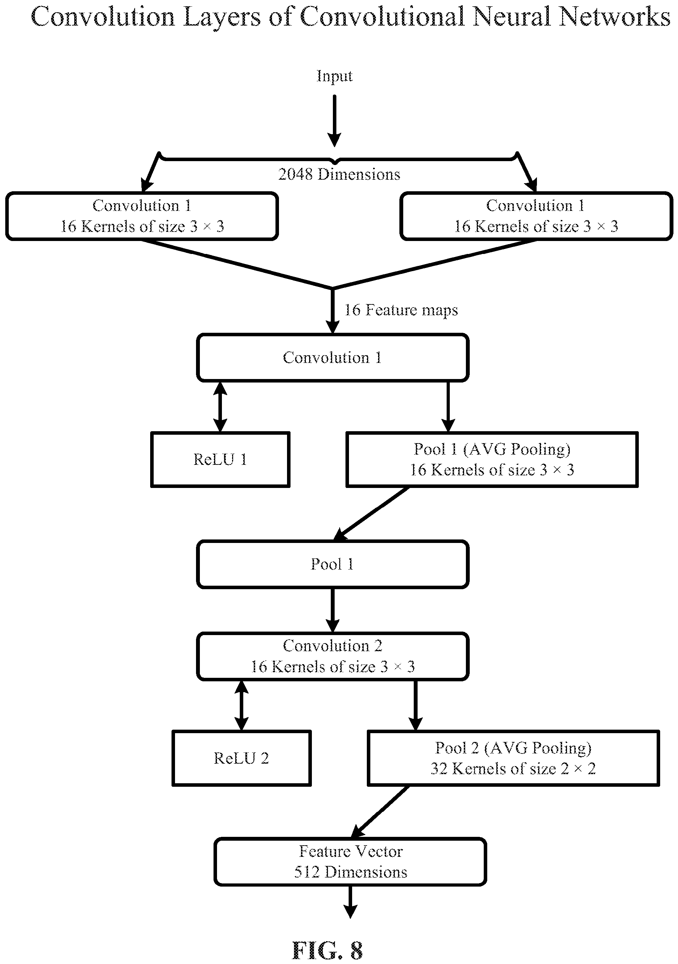

**FIG. 8** depicts one implementation of a two-layer convolution of the convolution layers. In **FIG. 8**, an input of size 2048 dimensions is convolved. At convolution 1, the input is convolved by a convolutional layer comprising of two channels of sixteen kernels of size 3 × 3. The resulting sixteen feature maps are then rectified by means of the ReLU activation function at ReLU1 and then pooled in Pool 1 by means of average pooling using a sixteen channel pooling layer with kernels of size 3 × 3. At convolution 2, the output of Pool 1 is then convolved by another convolutional layer comprising of sixteen channels of thirty kernels with a size of 3 × 3. This is followed by yet another ReLU2 and average pooling in Pool 2 with a kernel size of 2 × 2. The convolution layers use varying number of strides and padding, for example, zero, one, two and three. The resulting feature vector is five hundred and twelve (512) dimensions, according to one implementation.

In other implementations, the convolutional neural network uses different numbers of convolution layers, sub-sampling layers, non-linear layers and fully connected layers. In one implementation, the convolutional neural network is a shallow network with fewer layers and more neurons per layer, for example, one, two or three fully connected layers with hundred (100) to two hundred (200) neurons per layer. In another implementation, the convolutional neural network is a deep network with more layers and fewer neurons per layer, for example, five (5), six (6) or eight (8) fully connected layers with thirty (30) to fifty (50) neurons per layer.

## Forward Pass

The output of a neuron of row $x$, column y in the $l^{th}$ convolution layer and $k^{th}$ feature map for $f$ number of convolution cores in a feature map is determined by the following equation:

$$O_{x,y}^{(l,k)} = \tanh\left(\sum_{t=0}^{f-1} \sum_{r=0}^{k_h} \sum_{c=0}^{k_w} W_{(r,c)}^{(k,t)} O_{(x+r,x+c)}^{(l-1,t)} + Bias^{(l,k)}\right)$$

The output of a neuron of row $x$, column y in the $l^{th}$ sub-sample layer and $k^{th}$ feature map is determined by the following equation:

$$O_{x,y}^{(l,k)} = \tanh\left(W^{(k)} \sum_{r=0}^{S_h} \sum_{c=0}^{S_w} O_{(x\times S_h+r, y\times S_w+c)}^{(l-1,k)} + Bias^{(l,k)}\right)$$

The output of an $i^{th}$ neuron of the $l^{th}$ output layer is determined by the following equation:

$$O_{(l,i)} = \tanh\left(\sum_{j=0}^{H} O_{(l-1,j)} W_{(i,j)}^l + Bias^{(l,i)}\right)$$

## Backpropagation

The output deviation of a $k^{th}$ neuron in the output layer is determined by the following equation:

$$d(O_k^o) = y_k - t_k$$

The input deviation of a $k^{th}$ neuron in the output layer is determined by the following equation:

$$d(I_k^o) = (y_k - t_k)\varphi'(v_k) = \varphi'(v_k) d(O_k^o)$$

The weight and bias variation of a $k^{th}$ neuron in the output layer is determined by the following equation:

$$\Delta W_{k,x}^o) = d(I_k^o) y_{k,x}$$

$$\Delta Bias_k^o) = d(I_k^o)$$

The output bias of a $k^{th}$ neuron in the hidden layer is determined by the following equation:

$$d(O_k^H) = \sum_{i=0}^{i<84} d(I_i^o)W_{i,k}$$

The input bias of a $k^{th}$ neuron in the hidden layer is determined by the following equation:

$$d(I_k^H) = \varphi'(v_k)d(O_k^H)$$

The weight and bias variation in row $x$, column $y$ in a $m^{th}$ feature map of a prior layer receiving input from A neurons in the hidden layer is determined by the following equation:

$$\Delta W_{m,x,y}^{H,k}) = d(I_k^H)y_{x,y}^m$$

$$\Delta Bias_k^H) = d(I_k^H)$$

The output bias of row $x$, column $y$ in a $m^{th}$ feature map of sub-sample layer $S$ is determined by the following equation:

$$d(O_{x,y}^{S,m}) = \sum_{k}^{170} d(I_{m,x,y}^H)W_{m,x,y}^{H,k}$$

The input bias of row $x$, column $y$ in a $m^{th}$ feature map of sub-sample layer $S$ is determined by the following equation:

$$d(I_{x,y}^{S,m}) = \varphi'(v_k)d(O_{x,y}^{S,m})$$

The weight and bias variation in row $x$, column $y$ in a $m^{th}$ feature map of sub-sample layer $S$ and convolution layer C is determined by the following equation:

$$\Delta W^{S,m} = \sum_{x=0}^{fh} \sum_{y=0}^{fw} d(I_{[x/2],[y/2]}^{S,m})O_{x,y}^{C,m}$$

$$\Delta Bias^{S,m}) = \sum_{x=0}^{fh} \sum_{y=0}^{fw} d(O_{x,y}^{S,m})$$

The output bias of row $x$, column $y$ in a $k^{th}$ feature map of convolution layer C is determined by the following equation:

$$d(O_{x,y}^{C,k}) = d(I_{[x/2],[y/2]}^{S,k})W^k$$

The input bias of row $x$, column $y$ in a $k^{th}$ feature map of convolution layer C is determined by the following equation:

$$d(I_{x,y}^{C,k}) = \varphi'(v_k)d(O_{x,y}^{C,k})$$

The weight and bias variation in row $r$, column $c$ in an $m^{th}$ convolution core of a $k^{th}$ feature map of $l^{th}$ convolution layer $C$:

$$\Delta W_{r,c}^{k,m} = \sum_{x=0}^{fh} \sum_{y=0}^{fw} d(I_{x,y}^{C,k}) O_{x+r,y+c}^{l-1,m}$$

$$\Delta Bias^{C,k}) = \sum_{x=0}^{fh} \sum_{y=0}^{fw} d(I_{x,y}^{C,k})$$

**Residual Connections**

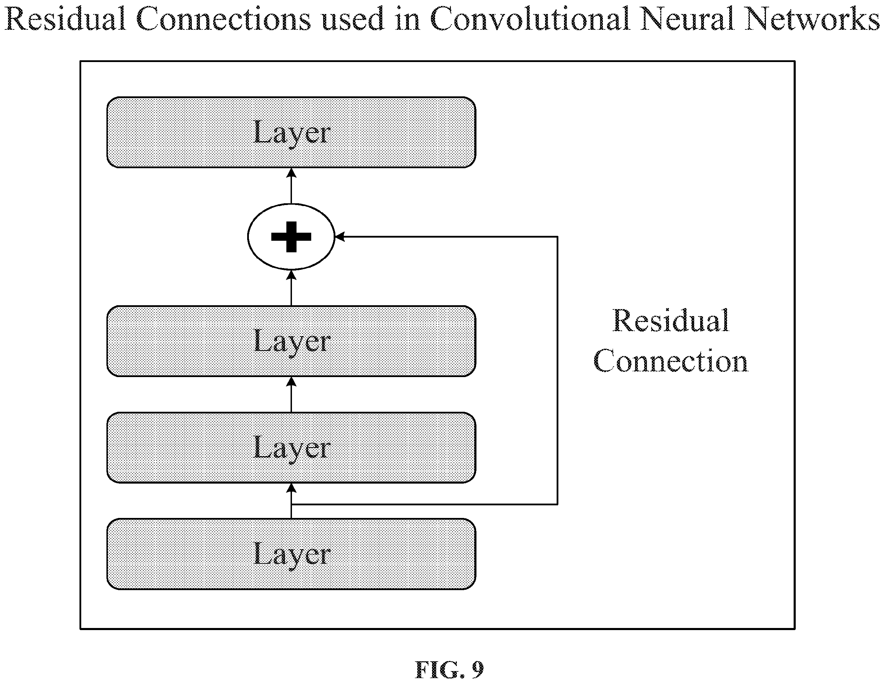

**FIG. 9** depicts a residual connection that reinjects prior information downstream via feature-map addition. A residual connection comprises reinjecting previous representations into the downstream flow of data by adding a past output tensor to a later output tensor, which helps prevent information loss along the data-processing flow. Residual connections tackle two common problems that plague any large-scale deep-learning model: vanishing gradients and representational bottlenecks. In general, adding residual connections to any model that has more than 10 layers is likely to be beneficial. As discussed above, a residual connection comprises making the output of an earlier layer available as input to a later layer, effectively creating a shortcut in a sequential network. Rather than being concatenated to the later activation, the earlier output is summed with the later activation, which assumes that both activations are the same size. If they are of different sizes, a linear transformation to reshape the earlier activation into the target shape can be used.

**Residual Learning and Skip-Connections**

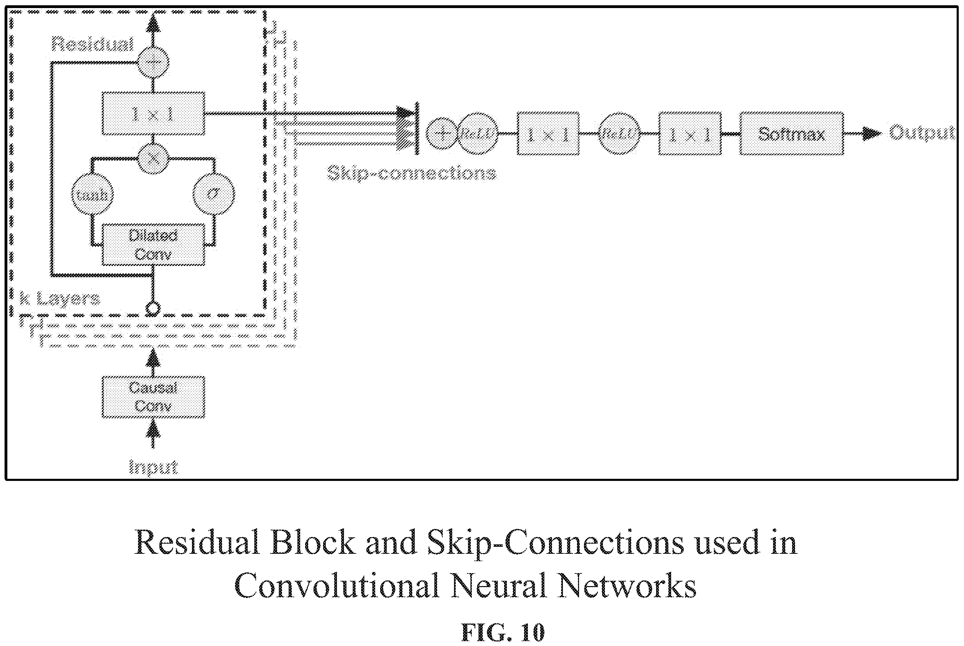

**FIG. 10** depicts one implementation of residual blocks and skip-connections. The main idea of residual learning is that the residual mapping is much easier to be learned than the original mapping. Residual network stacks a number of residual units to alleviate the degradation of training accuracy. Residual blocks make use of special additive skip connections to combat vanishing gradients in deep neural networks. At the beginning of a residual block, the data flow is separated into two streams: the first carries the unchanged input of the block, while the second applies weights and non-linearities. At the end of the block, the two streams are merged using an element-wise sum. The main advantage of such constructs is to allow the gradient to flow through the network more easily.

Benefited from residual network, deep convolutional neural networks (CNNs) can be easily trained and improved accuracy has been achieved for image classification and object detection. Convolutional feed-forward networks connect the output of the $l^{th}$ layer as input to the $(l+1)^{th}$ layer, which gives rise to the following layer transition: $x_l=H_l(x_{l-1})$. Residual blocks add a skip-connection that bypasses the non-linear transformations with an identify function: $x_l=H_l(x_{l-1})+x_{l-1}$. An advantage of residual blocks is that the gradient can flow directly through the identity function from later layers to the earlier layers. However, the identity function and the output of $H_l$ are combined by summation, which may impede the information flow in the network.

**WaveNet**

The WaveNet is a deep neural network for generating raw audio waveforms. The WaveNet distinguishes itself from other convolutional networks since it is able to take relatively large 'visual fields' at low cost. Moreover, it is able to add conditioning of the signals locally and globally, which allows the WaveNet to be used as a text to speech (TTS) engine with multiple voices, is the TTS gives local conditioning and the particular voice the global conditioning.

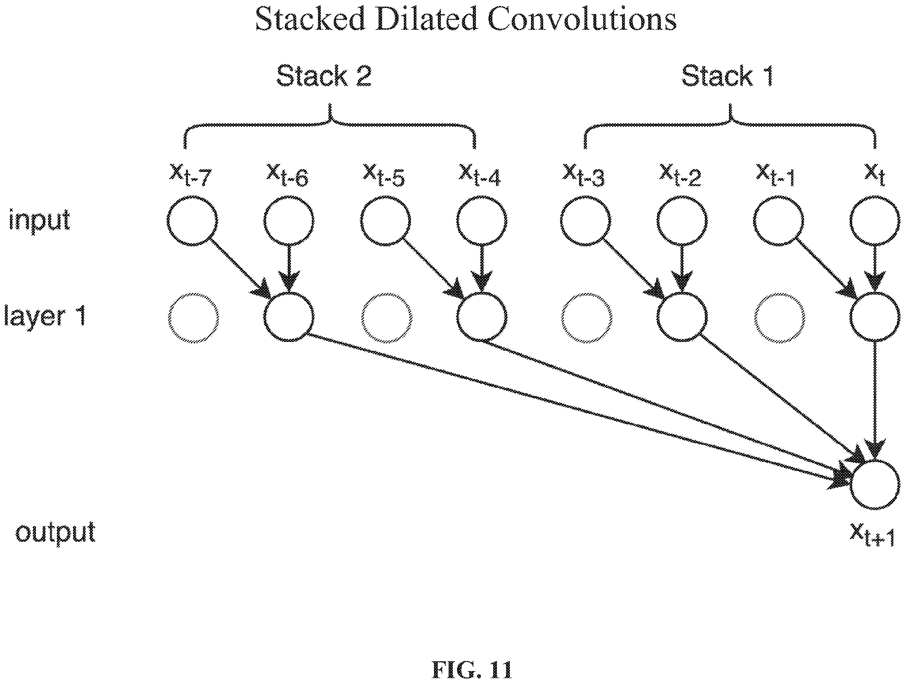

The main building blocks of the WaveNet are the causal dilated convolutions. As an extension on the causal dilated convolutions, theWaveNet also allows stacks of these convolutions, as shown in **FIG. 11.** To obtain the same receptive field with dilated convolutions in this figure, another dilation layer is required. The stacks are a repetition of the dilated convolutions, connecting the outputs of dilated convolution layer to a single output. This enables the WaveNet to get a large 'visual' field of one output node at a relatively low computational cost. For comparison, to get a visual field of 512 inputs, a fully convolutional network (FCN) would require 511 layers. In the case of a dilated convolutional network, we would need eight layers. The stacked dilated convolutions only need seven layers with two stacks or six layers with four stacks. To get an idea of the differences in computational power required for covering the same visual field, the following table shows the number of weights required in the network with the assumption of one filter per layer and a filter width of two. Furthermore, it is assumed that the network is using binary encoding of the 8 bits.

| Network type | No. stacks | No. weights per channel | Total No. of weights |
| --- | --- | --- | --- |
| FCN | 1 | $2.6. 10^5$ | $2.6. 10^6$ |
| WN | 1 | 1022 | 8176 |
| WN | 2 | 1022 | 8176 |
| WN | 4 | 508 | 4064 |

The WaveNet adds a skip connection before the residual connection is made, which bypasses all the following residual blocks. Each of these skip connections is summed before passing them through a series of activation functions and convolutions. Intuitively, this is the sum of the information extracted in each layer.

## Batch Normalization

Batch normalization is a method for accelerating deep network training by making data standardization an integral part of the network architecture. Batch normalization can adaptively normalize data even as the mean and variance change over time during training. It works by internally maintaining an exponential moving average of the batch-wise mean and variance of the data seen during training. The main effect of batch normalization is that it helps with gradient propagation - much like residual connections - and thus allows for deep networks. Some very deep networks can only be trained if they include multiple Batch Normalization layers.

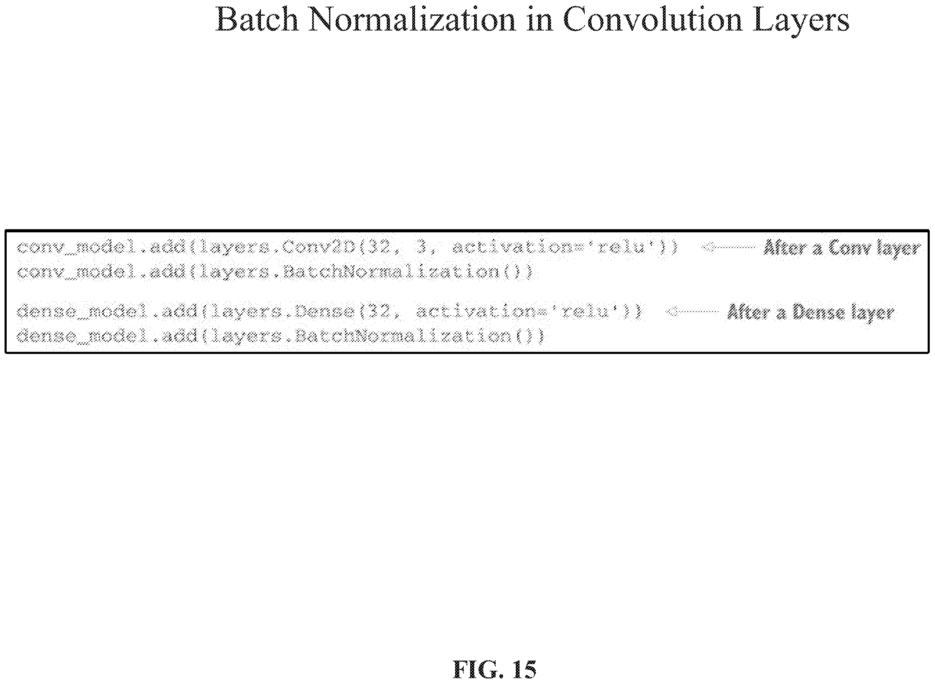

Batch normalization can be seen as yet another layer that can be inserted into the model architecture, just like the fully connected or convolutional layer. The BatchNormalization layer is typically used after a convolutional or densely connected layer. It can also be used before a convolutional or densely connected layer. Both implementations can be used by the technology disclosed and are shown in **FIG. 15.** The BatchNormalization layer takes an axis argument, which specifies the feature axis that should be normalized. This argument defaults to - 1, the last axis in the input tensor. This is the correct value when using Dense layers, Conv1D layers, RNN layers, and Conv2D layers with data format set to "channels_last". But in the niche use case of Conv2D layers with data_format set to "channels_first", the features axis is axis 1; the axis argument in BatchNormalization can be set to 1.

Batch normalization provides a definition for feed-forwarding the input and computing the gradients with respect to the parameters and its own input via a backward pass. In practice, batch normalization layers are inserted after a convolutional or fully connected layer, but before the outputs are fed into an activation function. For convolutional layers, the different elements of the same feature map - i.e. the activations - at different locations are normalized in the same way in order to obey the convolutional property. Thus, all activations in a mini-batch are normalized over all locations, rather than per activation.

The internal covariate shift is the major reason why deep architectures have been notoriously slow to train. This stems from the fact that deep networks do not only have to learn a new representation at each layer, but also have to account for the change in their distribution.

The covariate shift in general is a known problem in the deep learning domain and frequently occurs in real-world problems. A common covariate shift problem is the difference in the distribution of the training and test set which can lead to suboptimal generalization performance. This problem is usually handled with a standardization or whitening preprocessing step. However, especially the whitening operation is computationally expensive and thus impractical in an online setting, especially if the covariate shift occurs throughout different layers.

The internal covariate shift is the phenomenon where the distribution of network activations change across layers due to the change in network parameters during training. Ideally, each layer should be transformed into a space where they have the same distribution but the functional relationship stays the same. In order to avoid costly calculations of covariance matrices to decorrelate and whiten the data at every layer and step, we normalize the distribution of each input feature in each layer across each mini-batch to have zero mean and a standard deviation of one.

## Forward Pass

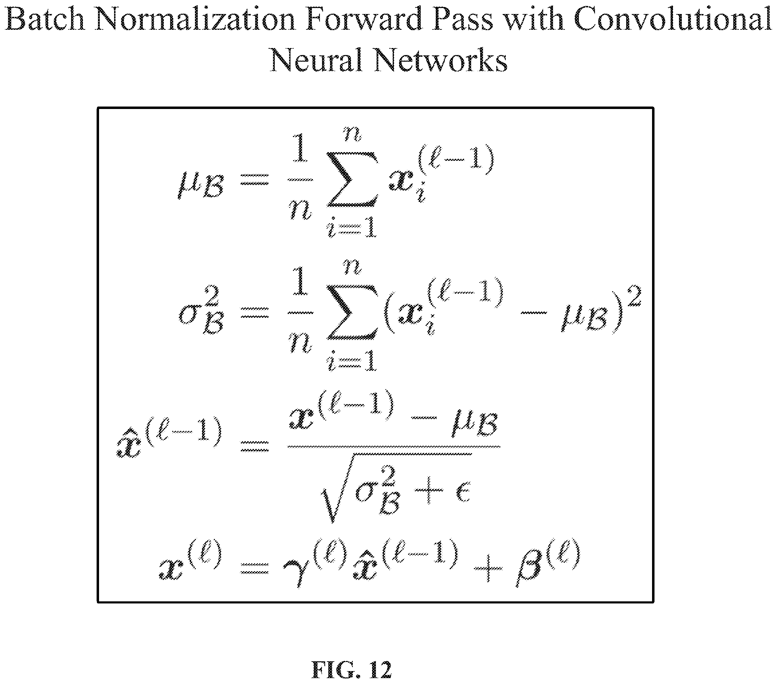

During the forward pass, the mini-batch mean and variance are calculated. With these mini-batch statistics, the data is normalized by subtracting the mean and dividing by the standard deviation. Finally, the data is scaled and shifted with the learned scale and shift parameters. The batch normalization forward pass $f_{BN}$ is depicted in **FIG. 12.**

In **FIG. 12,** $\mu_\beta$ is the batch mean and $\sigma_\beta^2$ is the batch variance, respectively. The learned scale and shift parameters are denoted by $\gamma$ and $\beta$, respectively. For clarity, the batch normalization procedure is described herein per activation and omit the corresponding indices.

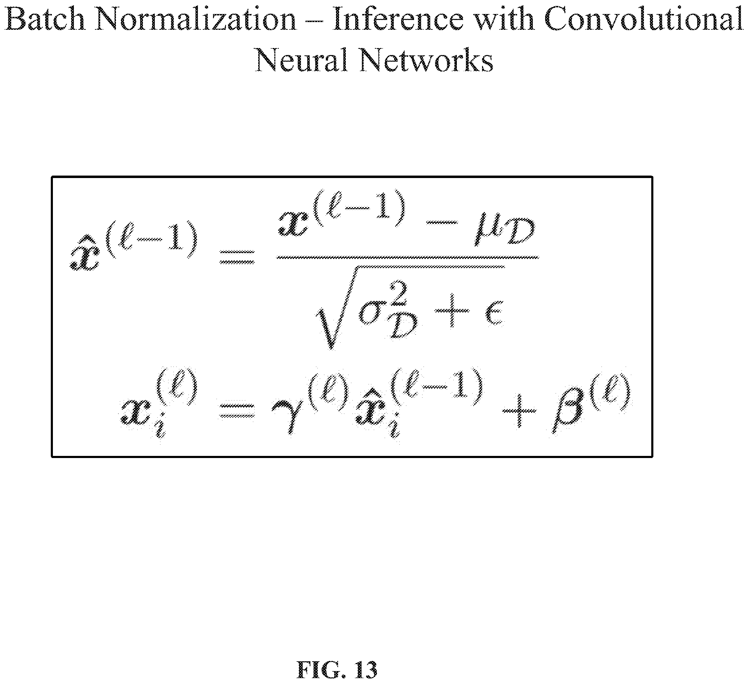

Since normalization is a differentiable transform, the errors are propagated into these learned parameters and are thus able to restore the representational power of the network by learning the identity transform. Conversely, by learning scale and shift parameters that are identical to the corresponding batch statistics, the batch normalization transform would have no effect on the network, if that was the optimal operation to perform. At test time, the batch mean and variance are replaced by the respective population statistics since the input does not depend on other samples from a mini-batch. Another method is to keep running averages of the batch statistics during training and to use these to compute the network output at test time. At test time, the batch normalization transform can be expressed as illustrated in **FIG. 13**. In **FIG. 13**, $\mu_D$ and $\sigma_D^2$ denote the population mean and variance, rather than the batch statistics, respectively.

### Backward Pass

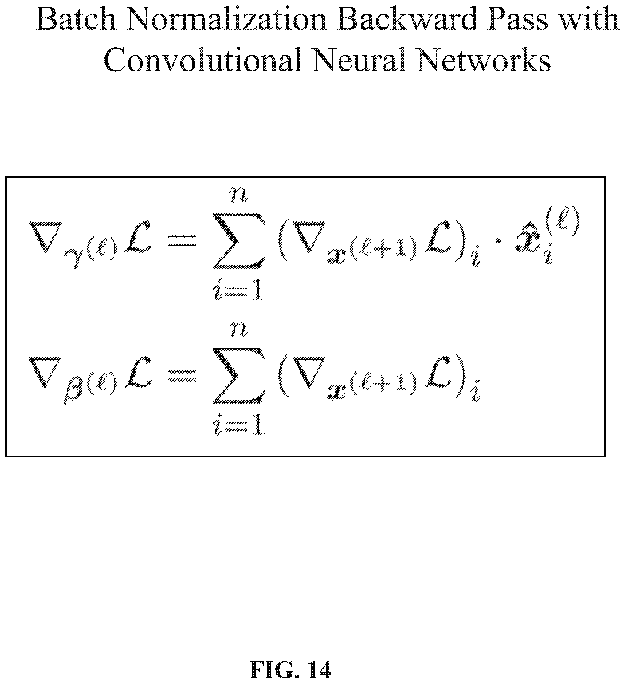

Since normalization is a differentiable operation, the backward pass can be computed as depicted in **FIG. 14.**

### 1D Convolution

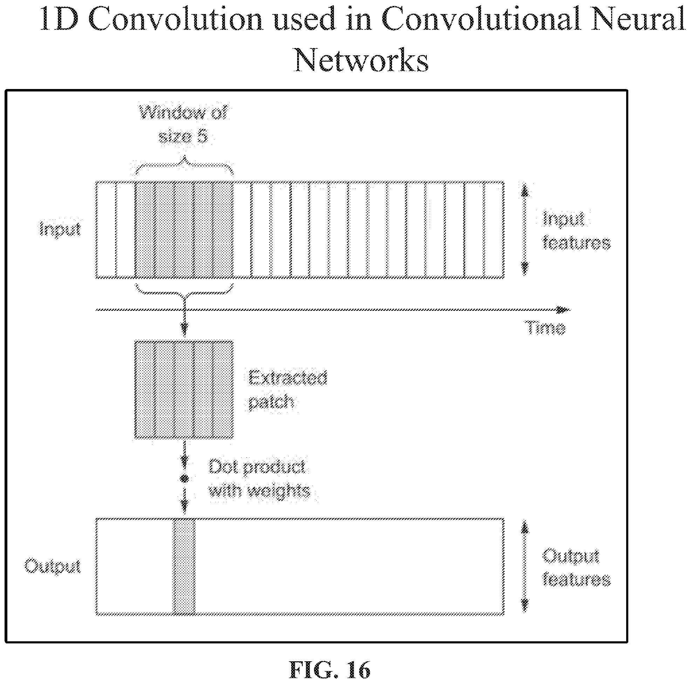

1D convolutions extract local 1D patches or subsequences from sequences, as shown in **FIG. 16**. 1D convolution obtains each output timestep from a temporal patch in the input sequence. 1D convolution layers recognize local patters in a sequence. Because the same input transformation is performed on every patch, a pattern learned at a certain position in the input sequences can be later recognized at a different position, making 1D convolution layers translation invariant for temporal translations. For instance, a 1D convolution layer processing sequences of bases using convolution windows of size 5 should be able to learn bases or base sequences of length 5 or less, and it should be able to recognize the base motifs in any context in an input sequence. A base-level ID convolution is thus able to learn about base morphology.

### Global Average Pooling

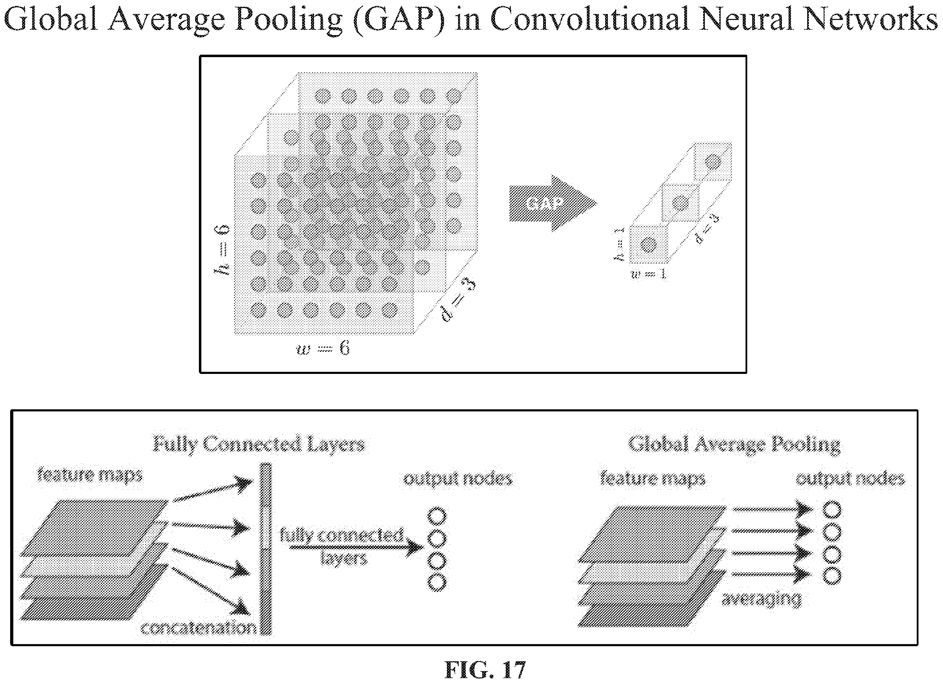

**FIG. 17** illustrates how global average pooling (GAP) works. Global average pooling can be use used to replace fully connected (FC) layers for classification, by taking the spatial average of features in the last layer for scoring. The reduces the training load and bypasses overfitting issues. Global average pooling applies a structural prior to the model and it is equivalent to linear transformation with predefined weights. Global average pooling reduces the number of parameters and eliminates the fully connected layer. Fully connected layers are typically the most parameter and connection intensive layers, and global average pooling provides much lower-cost approach to achieve similar results. The main idea of global average pooling is to generate the average value from each last layer feature map as the confidence factor for scoring, feeding directly into the softmax layer.

Global average pooling have three benefits: (1) there are no extra parameters in global average pooling layers thus overfitting is avoided at global average pooling layers: (2) since the output of global average pooling is the average of the whole feature map, global average pooling will be more robust to spatial translations; and (3) because of the huge number of parameters in fully connected layers which usually take over 50% in all the parameters of the whole network, replacing them by global average pooling layers can significantly reduce the size of the model, and this makes global average pooling very useful in model compression.

Global average pooling makes sense, since stronger features in the last layer are expected to have a higher average value. In some implementations, global average pooling can be used as a proxy for the classification score. The feature maps under global average pooling can be interpreted as confidence maps, and force correspondence between the feature maps and the categories. Global average pooling can be particularly effective if the last layer features are at a sufficient abstraction for direct classification; however, global average pooling alone is not enough if multilevel features should be combined into groups like parts models, which is best performed by adding a simple fully connected layer or other classifier after the global average pooling.

### Terminology

As used herein, the following terms have the meanings indicated.

A base refers to a nucleotide base or nucleotide, A (adenine), C (cytosine), T (thymine), or G (guanine).

This application uses the terms "protein" and "translated sequence" interchangeably.

This application uses the terms "codon" and "base triplet" interchangeably.

This application uses the terms "amino acid" and "translated unit" interchangeably.

This application uses the phrases "variant pathogenicity classifier", "convolutional neural network-based classifier for variant classification", and "deep convolutional neural network-based classifier for variant classification" interchangeably.

The term "chromosome" refers to the heredity-bearing gene carrier of a living cell, which is derived from chromatin strands comprising DNA and protein components (especially histones). The conventional internationally recognized individual human genome chromosome numbering system is employed herein.

The term "site" refers to a unique position (e.g., chromosome ID, chromosome position and orientation) on a reference genome. In some implementations, a site may be a residue, a sequence tag, or a segment's position on a sequence. The term "locus" may be used to refer to the specific location of a nucleic acid sequence or polymorphism on a reference chromosome.

The term "sample" herein refers to a sample, typically derived from a biological fluid, cell, tissue, organ, or organism containing a nucleic acid or a mixture of nucleic acids containing at least one nucleic acid sequence that is to be sequenced and/or phased. Such samples include, but are not limited to sputum/oral fluid, amniotic fluid, blood, a blood fraction, fine needle biopsy samples (e.g., surgical biopsy, fine needle biopsy, etc.), urine, peritoneal fluid, pleural fluid, tissue explant, organ culture and any other tissue or cell preparation. or fraction or derivative thereof or isolated therefrom. Although the sample is often taken from a human subject (e.g., patient), samples can be taken from any organism having chromosomes, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc.

The term "sequence" includes or represents a strand of nucleotides coupled to each other. The nucleotides may be based on DNA or RNA. It should be understood that one sequence may include multiple sub-sequences. For example, a single sequence (e.g., of a PCR amplicon) may have 350 nucleotides. The sample read may include multiple sub-sequences within these 350 nucleotides. For instance, the sample read may include first and second flanking subsequences having, for example, 20-50 nucleotides. The first and second flanking sub-sequences may be located on either side of a repetitive segment having a corresponding sub-sequence (e.g., 40-100 nucleotides). Each of the flanking sub-sequences may include (or include portions of) a primer sub-sequence (e.g., 10-30 nucleotides). For ease of reading, the term "sub-sequence" will be referred to as "sequence," but it is understood that two sequences are not necessarily separate from each other on a common strand. To differentiate the various sequences described herein, the sequences may be given different labels (e.g., target sequence, primer sequence, flanking sequence, reference sequence, and the like). Other terms, such as "allele," may be given different labels to differentiate between like objects.

The term "paired-end sequencing" refers to sequencing methods that sequence both ends of a target fragment. Paired-end sequencing may facilitate detection of genomic rearrangements and repetitive segments, as well as gene fusions and novel transcripts. Methodology for paired-end sequencing are described in PCT publication WO07010252, PCT application Serial No. PCTGB2007/003798 and US patent application publication US 2009/0088327. In one example, a series of operations may be performed as follows; (a) generate clusters of nucleic acids; (b) linearize the nucleic acids; (c) hybridize a first sequencing primer and carry out repeated cycles of extension, scanning and deblocking, as set forth above; (d) "invert" the target nucleic acids on the flow cell surface by synthesizing a complimentary copy; (e) linearize the resynthesized strand; and (f) hybridize a second sequencing primer and carry out repeated cycles of extension, scanning and deblocking, as set forth above. The inversion operation can be carried out be delivering reagents as set forth above for a single cycle of bridge amplification.

The term "reference genome" or "reference sequence" refers to any particular known genome sequence, whether partial or complete, of any organism which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences. A genome includes both the genes and the noncoding sequences of the DNA. The reference sequence may be larger than the reads that are aligned to it. For example, it may be at least about 100 times larger, or at least about 1000 times larger, or at least about 10,000 times larger, or at least about 105 times larger, or at least about 106 times larger, or at least about 107 times larger. In one example, the reference genome sequence is that of a full length human genome. In another example, the reference genome sequence is limited to a specific human chromosome such as chromosome 13. In some implementations, a reference chromosome is a chromosome sequence from human genome version hg19. Such sequences may be referred to as chromosome reference sequences, although the term reference genome is intended to cover such sequences. Other examples of reference sequences include genomes of other species, as well as chromosomes, sub-chromosomal regions (such as strands), etc., of any species. In various implementations, the reference genome is a consensus sequence or other combination derived from multiple individuals. However, in certain applications, the reference sequence may be taken from a particular individual.

The term "read" refers to a collection of sequence data that describes a fragment of a nucleotide sample or reference. The term "read" may refer to a sample read and/or a reference read. Typically, though not necessarily, a read represents a short sequence of contiguous base pairs in the sample or reference. The read may be represented symbolically by the base pair sequence (in ATCG) of the sample or reference fragment. It may be stored in a memory device and processed as appropriate to determine whether the read matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

Next-generation sequencing methods include, for example, sequencing by synthesis technology (Illumina), pyrosequencing (454), ion semiconductor technology (Ion Torrent sequencing), single-molecule real-time sequencing (Pacific Biosciences) and sequencing by ligation (SOLiD sequencing). Depending on the sequencing methods, the length of each read may vary from about 30 bp to more than 10,000 bp. For example, Illumina sequencing method using SOLiD sequencer generates nucleic acid reads of about 50 bp. For another example, Ion Torrent Sequencing generates nucleic acid reads of up to 400 bp and 454 pyrosequencing generates nucleic acid reads of about 700 bp. For yet another example, single-molecule real-time sequencing methods may generate reads of 10,000 bp to 15,000 bp. Therefore, in certain implementations, the nucleic acid sequence reads have a length of 30-100 bp, 50-200 bp, or 50-400 bp.

The terms "sample read", "sample sequence" or "sample fragment" refer to sequence data for a genomic sequence of interest from a sample. For example, the sample read comprises sequence data from a PCR amplicon having a forward and reverse primer sequence. The sequence data can be obtained from any select sequence methodology. The sample read can be, for example, from a sequencing-by-synthesis (SBS) reaction, a sequencing-by-ligation reaction, or any other suitable sequencing methodology for which it is desired to determine the length and/or identity of a repetitive element. The sample read can be a consensus (e.g., averaged or weighted) sequence derived from multiple sample reads. In certain implementations, providing a reference sequence comprises identifying a locus-of-interest based upon the primer sequence of the PCR amplicon.

The term "raw fragment" refers to sequence data for a portion of a genomic sequence of interest that at least partially overlaps a designated position or secondary position of interest within a sample read or sample fragment. Examples of raw fragments include a duplex stitched fragment, a simplex stitched fragment, a duplex un-stitched fragment and a simplex un-stitched fragment. The term "raw" is used to indicate that the raw fragment includes sequence data having some relation to the sequence data in a sample read, regardless of whether the raw fragment exhibits a supporting variant that corresponds to and authenticates or confirms a potential variant in a sample read. The term "raw fragment" does not indicate that the fragment necessarily includes a supporting variant that validates a variant call in a sample read. For example, when a sample read is determined by a variant call application to exhibit a first variant, the variant call application may determine that one or more raw fragments lack a corresponding type of "supporting" variant that may otherwise be expected to occur given the variant in the sample read.

The terms "mapping", "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain implementations, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

The term "indel" refers to the insertion and/or the deletion of bases in the DNA of an organism. A micro-indel represents an indel that results in a net change of 1 to 50 nucleotides. In coding regions of the genome, unless the length of an indel is a multiple of 3, it will produce a frameshi ft mutation. Indels can be contrasted with point mutations. An indel inserts and deletes nucleotides from a sequence, while a point mutation is a form of substitution that replaces one of the nucleotides without changing the overall number in the DNA. Indels can also be contrasted with a Tandem Base Mutation (TBM), which may be defined as substitution at adjacent nucleotides (primarily substitutions at two adjacent nucleotides, but substitutions at three adjacent nucleotides have been observed).

The term "variant" refers to a nucleic acid sequence that is different from a nucleic acid reference. Typical nucleic acid sequence variant includes without limitation single nucleotide polymorphism (SNP), short deletion and insertion polymorphisms (Indel), copy number variation (CNV), microsatellite markers or short tandem repeats and structural variation. Somatic variant calling is the effort to identify variants present at low frequency in the DNA sample. Somatic variant calling is of interest in the context of cancer treatment. Cancer is caused by an accumulation of mutations in DNA. A DNA sample from a tumor is generally heterogeneous, including some normal cells, some cells at an early stage of cancer progression (with fewer mutations), and some late-stage cells (with more mutations). Because of this heterogeneity, when sequencing a tumor (e.g., from an FFPE sample), somatic mutations will often appear at a low frequency. For example, a SNV might be seen in only 10% of the reads covering a given base. A variant that is to be classified as somatic or germline by the variant classifier is also referred to herein as the "variant under test".

The term "noise" refers to a mistaken variant call resulting from one or more errors in the sequencing process and/or in the variant call application.

The term "variant frequency" represents the relative frequency of an allele (variant of a gene) at a particular locus in a population, expressed as a fraction or percentage. For example, the fraction or percentage may be the fraction of all chromosomes in the population that carry that allele. By way of example, sample variant frequency represents the relative frequency of an allele/variant at a particular locus/position along a genomic sequence of interest over a "population" corresponding to the number of reads and/or samples obtained for the genomic sequence of interest from an individual. As another example, a baseline variant frequency represents the relative frequency of an allele/variant at a particular locus/position along one or more baseline genomic sequences where the "population" corresponding to the number of reads and/or samples obtained for the one or more baseline genomic sequences from a population of normal individuals.

The term "variant allele frequency (VAF)" refers to the percentage of sequenced reads observed matching the variant divided by the overall coverage at the target position. VAF is a measure of the proportion of sequenced reads carrying the variant.

The terms "position", "designated position", and "locus" refer to a location or coordinate of one or more nucleotides within a sequence of nucleotides. The terms "position", "designated position", and "locus" also refer to a location or coordinate of one or more base pairs in a sequence of nucleotides.

The term "haplotype" refers to a combination of alleles at adjacent sites on a chromosome that are inherited together. A haplotype may be one locus, several loci, or an entire chromosome depending on the number of recombination events that have occurred between a given set of loci, if any occurred.

The term "threshold" herein refers to a numeric or non-numeric value that is used as a cutoff to characterize a sample, a nucleic acid, or portion thereof (e.g., a read). A threshold may be varied based upon empirical analysis. The threshold may be compared to a measured or calculated value to determine whether the source giving rise to such value suggests should be classified in a particular manner. Threshold values can be identified empirically or analytically. The choice of a threshold is dependent on the level of confidence that the user wishes to have to make the classification. The threshold may be chosen for a particular purpose (e.g., to balance sensitivity and selectivity). As used herein, the term "threshold" indicates a point at which a course of analysis may be changed and/or a point at which an action may be triggered. A threshold is not required to be a predetermined number. Instead, the threshold may be, for instance, a function that is based on a plurality of factors. The threshold may be adaptive to the circumstances. Moreover, a threshold may indicate an upper limit, a lower limit, or a range between limits.

In some implementations, a metric or score that is based on sequencing data may be compared to the threshold. As used herein, the terms "metric" or "score" may include values or results that were determined from the sequencing data or may include functions that are based on the values or results that were determined from the sequencing data. Like a threshold, the metric or score may be adaptive to the circumstances. For instance, the metric or score may be a normalized value. As an example of a score or metric, one or more implementations may use count scores when analyzing the data. A count score may be based on the number of sample reads. The sample reads may have undergone one or more filtering stages such that the sample reads have at least one common characteristic or quality. For example, each of the sample reads that are used to determine a count score may have been aligned with a reference sequence or may be assigned as a potential allele. The number of sample reads having a common characteristic may be counted to determine a read count. Count scores may be based on the read count. In some implementations, the count score may be a value that is equal to the read count. In other implementations, the count score may be based on the read count and other information. For example, a count score may be based on the read count for a particular allele of a genetic locus and a total number of reads for the genetic locus. In some implementations, the count score may be based on the read count and previously-obtained data for the genetic locus. In some implementations, the count scores may be normalized scores between predetermined values. The count score may also be a function of read counts from other loci of a sample or a function of read counts from other samples that were concurrently run with the sample-of-interest. For instance, the count score may be a function of the read count of a particular allele and the read counts of other loci in the sample and/or the read counts from other samples. As one example, the read counts from other loci and/or the read counts from other samples may be used to normalize the count score for the particular allele.

The terms "coverage" or "fragment coverage" refer to a count or other measure of a number of sample reads for the same fragment of a sequence. A read count may represent a count of the number of reads that cover a corresponding fragment. Alternatively, the coverage may be determined by multiplying the read count by a designated factor that is based on historical knowledge, knowledge of the sample, knowledge of the locus, etc.

The term "read depth" (conventionally a number followed by "×") refers to the number of sequenced reads with overlapping alignment at the target position. This is often expressed as an average or percentage exceeding a cutoff over a set of intervals (such as exons, genes, or panels). For example, a clinical report might say that a panel average coverage is 1,105× with 98% of targeted bases covered >100×.

The terms "base call quality score" or "Q score" refer to a PHRED-scaled probability ranging from 0-20 inversely proportional to the probability that a single sequenced base is correct. For example, a T base call with Q of 20 is considered likely correct with a confidence P-value of 0.01. Any base call with Q<20 should be considered low quality, and any variant identified where a substantial proportion of sequenced reads supporting the variant are of low quality should be considered potentially false positive.

The terms "variant reads" or "variant read number" refer to the number of sequenced reads supporting the presence of the variant.

## Sequencing Process

Implementations set forth herein may be applicable to analyzing nucleic acid sequences to identify sequence variations. Implementations may be used to analyze potential variants/alleles of a genetic position/locus and determine a genotype of the genetic locus or, in other words, provide a genotype call for the locus. By way of example, nucleic acid sequences may be analyzed in accordance with the methods and systems described in US Patent Application Publication No. 2016/0085910 and US Patent Application Publication No. 2013/0296175.

In one implementation, a sequencing process includes receiving a sample that includes or is suspected of including nucleic acids, such as DNA. The sample may be from a known or unknown source, such as an animal (e.g., human), plant, bacteria, or fungus. The sample may be taken directly from the source. For instance, blood or saliva may be taken directly from an individual. Alternatively, the sample may not be obtained directly from the source. Then, one or more processors direct the system to prepare the sample for sequencing. The preparation may include removing extraneous material and/or isolating certain material (e.g., DNA). The biological sample may be prepared to include features for a particular assay. For example, the biological sample may be prepared for sequencing-by-synthesis (SBS). In certain implementations, the preparing may include amplification of certain regions of a genome. For instance, the preparing may include amplifying predetermined genetic loci that are known to include STRs and/or SNPs. The genetic loci may be amplified using predetermined primer sequences.

Next, the one or more processors direct the system to sequence the sample. The sequencing may be performed through a variety of known sequencing protocols. In particular implementations, the sequencing includes SBS. In SBS, a plurality of fluorescently-labeled nucleotides are used to sequence a plurality of clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders.

The nucleic acids can be prepared such that they comprise a known primer sequence that is adjacent to an unknown target sequence. To initiate the first SBS sequencing cycle, one or more differently labeled nucleotides, and DNA polymerase, etc., can be flowed into/through the flow cell by a fluid flow subsystem. Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g., A, C, T, G). The nucleotides can include detectable label moieties such as fluorophores. Where the four nucleotides are mixed together, the polymerase is able to select the correct base to incorporate and each sequence is extended by a single base. Non-incorporated nucleotides can be washed away by flowing a wash solution through the flow cell. One or more lasers may excite the nucleic acids and induce fluorescence. The fluorescence emitted from the nucleic acids is based upon the fluorophores of the incorporated base, and different fluorophores may emit different wavelengths of emission light. A deblocking reagent can be added to the flow cell to remove reversible terminator groups from the DNA strands that were extended and detected. The deblocking reagent can then be washed away by flowing a wash solution through the flow cell. The flow cell is then ready for a further cycle of sequencing starting with introduction of a labeled nucleotide as set forth above. The fluidic and detection operations can be repeated several times to complete a sequencing run. Example sequencing methods are described, for example, in Bentley et al., Nature 456:53-59 (2008), International Publication No. WO 04/018497; U.S. Pat. No. 7,057,026; International Publication No. WO 91/06678; International Publication No. WO 07/123744; U.S. Pat. No. 7,329,492; U.S. Patent No. 7,211,414; U.S. Patent No. 7,315,019; U.S. Patent No. 7,405,281.

In some implementations, nucleic acids can be attached to a surface and amplified prior to or during sequencing. For example, amplification can be carried out using bridge amplification to form nucleic acid clusters on a surface. Useful bridge amplification methods are described for example, in U.S. Patent No. 5,641,658; U.S. Patent Application Publication No. 2002/0055100; U.S. Patent No. 7,115,400; U.S. Patent Application Publication No. 2004/0096853; U.S. Patent Application Publication No. 2004/0002090; U.S. Patent Application Publication No. 2007/0128624; and U.S. Patent Application Publication No. 2008/0009420. Another useful method for amplifying nucleic acids on a surface is rolling circle amplification (RCA), for example, as described in Lizardi et al., Nat. Genet. 19:225-232 (1998) and U.S. Patent Application Publication No. 2007/0099208 A1.

One example SBS protocol exploits modified nucleotides having removable 3' blocks, for example, as described in International Publication No. WO 04/018497, U.S. Patent Application Publication No. 2007/0166705A1. and U.S. Patent No. 7,057,026. For example, repeated cycles of SBS reagents can be delivered to a flow cell having target nucleic acids attached thereto, for example, as a result of the bridge amplification protocol. The nucleic acid clusters can be converted to single stranded form using a linearization solution. The linearization solution can contain, for example, a restriction endonuclease capable of cleaving one strand of each cluster. Other methods of cleavage can be used as an alternative to restriction enzymes or nicking enzymes, including inter alia chemical cleavage (e.g., cleavage of a diol linkage with periodate), cleavage of abasic sites by cleavage with endonuclease (for example 'USER', as supplied by NEB, Ipswich, Mass., USA, part number M5505S), by exposure to heat or alkali, cleavage of ribonucleotides incorporated into amplification products otherwise comprised of deoxyribonucleotides, photochemical cleavage or cleavage of a peptide linker. After the linearization operation a sequencing primer can be delivered to the flow cell under conditions for hybridization of the sequencing primer to the target nucleic acids that are to be sequenced.

A flow cell can then be contacted with an SBS extension reagent having modified nucleotides with removable 3' blocks and fluorescent labels under conditions to extend a primer hybridized to each target nucleic acid by a single nucleotide addition. Only a single nucleotide is added to each primer because once the modified nucleotide has been incorporated into the growing polynucleotide chain complementary to the region of the template being sequenced there is no free 3'-OH group available to direct further sequence extension and therefore the polymerase cannot add further nucleotides. The SBS extension reagent can be removed and replaced with scan reagent containing components that protect the sample under excitation with radiation. Example components for scan reagent are described in U.S. Patent Application Publication No. 2008/0280773 A1 and U.S. Patent Application No. 13/018,255. The extended nucleic acids can then be fluorescently detected in the presence of scan reagent. Once the fluorescence has been detected, the 3' block may be removed using a deblock reagent that is appropriate to the blocking group used. Example deblock reagents that are useful for respective blocking groups are described in WO004018497, US 2007/0166705A1 and U.S. Patent No. 7,057,026. The deblock reagent can be washed away leaving target nucleic acids hybridized to extended primers having 3'-OH groups that are now competent for addition of a further nucleotide. Accordingly the cycles of adding extension reagent, scan reagent, and deblock reagent, with optional washes between one or more of the operations, can be repeated until a desired sequence is obtained. The above cycles can be carried out using a single extension reagent delivery operation per cycle when each of the modified nucleotides has a different label attached thereto, known to correspond to the particular base. The different labels facilitate discrimination between the nucleotides added during each incorporation operation. Alternatively, each cycle can include separate operations of extension reagent delivery followed by separate operations of scan reagent delivery and detection, in which case two or more of the nucleotides can have the same label and can be distinguished based on the known order of delivery.

Although the sequencing operation has been discussed above with respect to a particular SBS protocol, it will be understood that other protocols for sequencing any of a variety of other molecular analyses can be carried out as desired.

Then, the one or more processors of the system receive the sequencing data for subsequent analysis. The sequencing data may be formatted in various manners, such as in a .BAM file. The sequencing data may include, for example, a number of sample reads. The sequencing data may include a plurality of sample reads that have corresponding sample sequences of the nucleotides. Although only one sample read is discussed, it should be understood that the sequencing data may include, for example, hundreds, thousands, hundreds of thousands, or millions of sample reads. Different sample reads may have different numbers of nucleotides. For example, a sample read may range between 10 nucleotides to about 500 nucleotides or more. The sample reads may span the entire genome of the source(s). As one example, the sample reads are directed toward predetermined genetic loci, such as those genetic loci having suspected STRs or suspected SNPs.

Each sample read may include a sequence of nucleotides, which may be referred to as a sample sequence, sample fragment or a target sequence. The sample sequence may include, for example, primer sequences, flanking sequences, and a target sequence. The number of nucleotides within the sample sequence may include 30, 40, 50, 60, 70, 80, 90, 100 or more. In some implementations, one or more of the sample reads (or sample sequences) includes at least 150 nucleotides, 200 nucleotides, 300 nucleotides, 400 nucleotides, 500 nucleotides, or more. In some implementations, the sample reads may include more than 1000 nucleotides, 2000 nucleotides, or more. The sample reads (or the sample sequences) may include primer sequences at one or both ends.

Next, the one or more processors analyze the sequencing data to obtain potential variant call(s) and a sample variant frequency of the sample variant call(s). The operation may also be referred to as a variant call application or variant caller. Thus, the variant caller identifies or detects variants and the variant classifier classifies the detected variants as somatic or germline. Alternative variant callers may be utilized in accordance with implementations herein, wherein different variant callers may be used based on the type of sequencing operation being performed, based on features of the sample that are of interest and the like. One example of a variant call application, such as the Pisces™ application by Illumina Inc. (San Diego, CA) hosted at https://github.com/Illumina/Pisces and described in the article Dunn, Tamsen & Berry, Gwenn & Emig-Agius, Dorothea & Jiang, Yu & Iyer, Anita & Udar, Nitin & Strömberg, Michael. (2017). Pisces: An Accurate and Versatile Single Sample Somatic and Germline Variant Caller. 595-595.10.1145/3107411.3108203.

Such a variant call application can comprise four sequentially executed modules:

(1) Pisces Read Stitcher: Reduces noise by stitching paired reads in a BAM (read one and read two of the same molecule) into consensus reads. The output is a stitched BAM.

(2) Pisces Variant Caller: Calls small SNVs, insertions and deletions. Pisces includes a variant-collapsing algorithm to coalesce variants broken up by read boundaries, basic filtering algorithms, and a simple Poisson-based variant confidence-scoring algorithm. The output is a VCF.

(3) Pisces Variant Quality Recalibrator (VQR): In the event that the variant calls overwhelmingly follow a pattern associated with thermal damage or FFPE deamination, the VQR step will downgrade the variant Q score of the suspect variant calls. The output is an adjusted VCF.

(4) Pisces Variant Phaser (Scylla): Uses a read-backed greedy clustering method to assemble small variants into complex alleles from clonal subpopulations. This allows for the more accurate determination of functional consequence by downstream tools. The output is an adjusted VCF.

Additionally or alternatively, the operation may utilize the variant call application Strelka™ application by Illumina Inc. hosted at https://github.com/Illumina/strelka and described in the article T Saunders, Christopher & Wong, Wendy & Swamy, Sajani & Becq, Jennifer & J Murray, Lisa & Cheetham, Keira. (2012). Strelka: Accurate somatic small-variant calling from sequenced tumor-normal sample pairs. Bioinformatics (Oxford, England). 28.1811-7.10.1093/bioinformatics/bts271. Furthermore, additionally or alternatively, the operation may utilize the variant call application Strelka2™ application by Illumina Inc. hosted at https://github.com/Illumina/strelka and described in the article Kim, S., Scheffler, K., Halpern, A.L., Bekritsky, M.A., Noh, E., Källberg, M., Chen, X., Beyter, D., Krusche, P., and Saunders, C.T. (2017). Strelka2: Fast and accurate variant calling for clinical sequencing applications. Moreover, additionally or alternatively, the operation may utilize a variant annotation/call tool, such as the Nirvana™ application by Illumina Inc. hosted at https://github.com/Illumina/Nirvana/wiki and described in the article Stromberg, Michael & Roy, Rajat & Lajugie, Julien & Jiang, Yu & Li, Haochen & Margulies, Elliott. (2017). Nirvana: Clinical Grade Variant Annotator. 596-596.10.1145/3107411.3108204.

Such a variant annotation/call tool can apply different algorithmic techniques such as those disclosed in Nirvana:

a. Identifying all overlapping transcripts with Interval Array: For functional annotation, we can identify all transcripts overlapping a variant and an interval tree can be used. However, since a set of intervals can be static, we were able to further optimize it to an Interval Array. An interval tree returns all overlapping transcripts in $O(min(n,k \lg n))$ time, where n is the number of intervals in the tree and k is the number of overlapping intervals. In practice, since k is really small compared to n for most variants, the effective runtime on interval tree would be $O(k \lg n)$. We improved to $O(\lg n + k)$ by creating an interval array where all intervals are stored in a sorted array so that we only need to find the first overlapping interval and then enumerate through the remaining (k-1).

b. CNVs/SVs (Yu): annotations for Copy Number Variation and Structural Variants can be provided. Similar to the annotation of small variants, transcripts overlapping with the SV and also previously reported structural variants can be annotated in online databases. Unlike the small variants, not all overlapping transcripts need be annotated, since too many transcripts will be overlapped with a large SVs. Instead, all overlapping transcripts can be annotated that belong to a partial overlapping gene. Specifically, for these transcripts, the impacted introns, exons and the consequences caused by the structural variants can be reported. An option to allow output all overlapping transcripts is available, but the basic information for these transcripts can be reported, such as gene symbol, flag whether it is canonical overlap or partial overlapped with the transcripts. For each SV/CNV, it is also of interest to know if these variants have been studied and their frequencies in different populations. Hence, we reported overlapping SVs in external databases, such as 1000 genomes, DGV and ClinGen. To avoid using an arbitrary cutoff to determine which SV is overlapped, instead all overlapping transcripts can be used and the reciprocal overlap can be calculated, i.e. the overlapping length divided by the minimum of the length of these two SVs.

c. Reporting supplementary annotations : Supplementary annotations are of two types: small and structural variants (SVs). SVs can be modeled as intervals and use the interval array discussed above to identify overlapping SVs. Small variants are modeled as points and matched by position and (optionally) allele. As such, they are searched using a binary-search-like algorithm. Since the supplementary annotation database can be quite large, a much smaller index is created to map chromosome positions to file locations where the supplementary annotation resides. The index is a sorted array of objects (made up of chromosome position and file location) that can be binary searched using position. To keep the index size small, multiple positions (up to a certain max count) are compressed to one object that stores the values for the first position and only deltas for subsequent positions. Since we use Binary search, the runtime is O(lg n), where n is the number of items in the database.

d. VEP cache files

e. Transcript Database : The Transcript Cache (cache) and Supplementary database (SAdb) files are serialized dump of data objects such as transcripts and supplementary annotations. We use Ensembl VEP cache as our data source for cache. To create the cache, all transcripts are inserted in an interval array and the final state of the array is stored in the cache files. Thus, during annotation, we only need to load a pre-computed interval array and perform searches on it. Since the cache is loaded up in memory and searching is very fast (described above), finding overlapping transcripts is extremely quick in Nirvana (profiled to less than 1% of total runtime?).

f. Supplementary Database : The data sources for SAdb are listed under supplementary material. The SAdb for small variants is produced by a k -way merge of all data sources such that each object in the database (identified by reference name and position) holds all relevant supplementary annotations. Issues encountered during parsing data source files have been documented in detail in Nirvana's home page. To limit memory usage, only the SA index is loaded up in memory. This index allows a quick lookup of the file location for a supplementary annotation. However, since the data has to be fetched from disk, adding supplementary annotation has been identified as Nirvana's largest bottleneck (profiled at -30% of total runtime.)

g. Consequence and Sequence Ontology: Nirvana's functional annotation (when provided) follows the Sequence Ontology (SO) (http://www.sequenceontology.org/) guidelines. On occasions, we had the opportunity to identify issues in the current SO and collaborate with the SO team to improve the state of annotation.

Such a variant annotation tool can include pre-processing. For example, Nirvana included a large number of annotations from External data sources, like ExAC, EVS, 1000 Genomes project, dbSNP, ClinVar, Cosmic, DGV and ClinGen. To make full use of these databases, we have to sanitize the information from them. We implemented different strategy to deal with different conflicts that exist from different data sources. For example, in case of multiple dbSNP entries for the same position and alternate allele, we join all ids into a comma separated list of ids; if there are multiple entries with different CAF values for the same allele, we use the first CAF value. For conflicting ExAC and EVS entries, we consider the number of sample counts and the entry with higher sample count is used. In 1000 Genome Projects, we removed the allele frequency of the conflicting allele. Another issue is inaccurate information. We mainly extracted the allele frequencies information from 1000 Genome Projects, however, we noticed that for GRCh38, the allele frequency reported in the info field did not exclude samples with genotype not available, leading to deflated frequencies for variants which are not available for all samples. To guarantee the accuracy of our annotation, we use all of the individual level genotype to compute the true allele frequencies. As we know, the same variants can have different representations based on different alignments. To make sure we can accurately report the information for already identified variants, we have to preprocess the variants from different resources to make them have consistent representation. For all external data sources, we trimmed alleles to remove duplicated nucleotides in both reference allele and alternative allele. For ClinVar, we directly parsed the xml file we performed a five-prime alignment for all variants, which is often used in vcf file. Different databases can contain the same set of information. To avoid unnecessary duplicates, we removed some duplicated information. For example, we removed variants in DGV which has data source as 1000 genome projects, since we already reported these variants in 1000 genomes with more detailed information.

In accordance with at least some implementations, the variant call application provides calls for low frequency variants, germline calling and the like. As an example, the variant call application may run on tumor-only samples and/or tumor-normal paired samples. The variant call application may search for single nucleotide variations (SNV), multiple nucleotide variations (MNV), indels and the like. The variant call application identifies variants, while filtering for mismatches due to sequencing or sample preparation errors. For each variant, the variant caller identifies the reference sequence, a position of the variant, and the potential variant sequence(s) (e.g., A to C SNV, or AG to A deletion). The variant call application identifies the sample sequence (or sample fragment), a reference sequence/fragment, and a variant call as an indication that a variant is present. The variant call application may identify raw fragments, and output a designation of the raw fragments, a count of the number of raw fragments that verify the potential variant call, the position within the raw fragment at which a supporting variant occurred and other relevant information. Examples of raw fragments include a duplex stitched fragment, a simplex stitched fragment, a duplex un-stitched fragment and a simplex un- stitched fragment.

The variant call application may output the calls in various formats, such as in a .VCF or .GVCF file. By way of example only, the variant call application may be included in a MiSeqReporter pipeline (e.g., when implemented on the MiSeq® sequencer instrument). Optionally, the application may be implemented with various workflows. The analysis may include a single protocol or a combination of protocols that analyze the sample reads in a designated manner to obtain desired information.

Then, the one or more processors perform a validation operation in connection with the potential variant call.

The validation operation may be based on a quality score, and/or a hierarchy of tiered tests, as explained hereafter. When the validation operation authenticates or verifies that the potential variant call, the validation operation passes the variant call information (from the variant call application) to the sample report generator. Alternatively, when the validation operation invalidates or disqualifies the potential variant call, the validation operation passes a corresponding indication (e.g., a negative indicator, a no call indicator, an in-valid call indicator) to the sample report generator. The validation operation also may pass a confidence score related to a degree of confidence that the variant call is correct or the in-valid call designation is correct.

Next, the one or more processors generate and store a sample report. The sample report may include, for example, information regarding a plurality of genetic loci with respect to the sample. For example, for each genetic locus of a predetermined set of genetic loci, the sample report may at least one of provide a genotype call; indicate that a genotype call cannot be made; provide a confidence score on a certainty of the genotype call; or indicate potential problems with an assay regarding one or more genetic loci. The sample report may also indicate a gender of an individual that provided a sample and/or indicate that the sample include multiple sources. As used herein, a "sample report" may include digital data (e.g., a data file) of a genetic locus or predetermined set of genetic locus and/or a printed report of the genetic locus or the set of genetic loci. Thus, generating or providing may include creating a data file and/or printing the sample report, or displaying the sample report.

The sample report may indicate that a variant call was determined, but was not validated. When a variant call is determined invalid, the sample report may indicate additional information regarding the basis for the determination to not validate the variant call. For example, the additional information in the report may include a description of the raw fragments and an extent (e.g., a count) to which the raw fragments support or contradict the variant call. Additionally or alternatively, the additional information in the report may include the quality score obtained in accordance with implementations described herein.

## Variant Call Application

Implementations disclosed herein include analyzing sequencing data to identify potential variant calls. Variant calling may be performed upon stored data for a previously performed sequencing operation. Additionally or alternatively, it may be performed in real time while a sequencing operation is being performed. Each of the sample reads is assigned to corresponding genetic loci. The sample reads may be assigned to corresponding genetic loci based on the sequence of the nucleotides of the sample read or, in other words, the order of nucleotides within the sample read (e.g., A, C, G, T). Based on this analysis, the sample read may be designated as including a possible variant/allele of a particular genetic locus. The sample read may be collected (or aggregated or binned) with other sample reads that have been designated as including possible variants/alleles of the genetic locus. The assigning operation may also be referred to as a calling operation in which the sample read is identified as being possibly associated with a particular genetic position/locus. The sample reads may be analyzed to locate one or more identifying sequences (e.g., primer sequences) of nucleotides that differentiate the sample read from other sample reads. More specifically, the identifying sequence(s) may identify the sample read from other sample reads as being associated with a particular genetic locus.

The assigning operation may include analyzing the series of n nucleotides of the identifying sequence to determine if the series of n nucleotides of the identifying sequence effectively matches with one or more of the select sequences. In particular implementations, the assigning operation may include analyzing the first n nucleotides of the sample sequence to determine if the first n nucleotides of the sample sequence effectively matches with one or more of the select sequences. The number n may have a variety of values, which may be programmed into the protocol or entered by a user. For example, the number n may be defined as the number of nucleotides of the shortest select sequence within the database. The number n may be a predetermined number. The predetermined number may be, for example, 10, 11, 12, 13, 14, 15, 16, 17, 18, 19, 20, 21, 22, 23, 24, 25, 26, 27, 28, 29, or 30 nucleotides. However, fewer or more nucleotides may be used in other implementations. The number n may also be selected by an individual, such as a user of the system. The number n may be based on one or more conditions. For instance, the number n may be defined as the number of nucleotides of the shortest primer sequence within the database or a designated number, whichever is the smaller number. In some implementations, a minimum value for n may be used, such as 15, such that any primer sequence that is less than 15 nucleotides may be designated as an exception.

In some cases, the series of n nucleotides of an identifying sequence may not precisely match the nucleotides of the select sequence. Nonetheless, the identifying sequence may effectively match the select sequence if the identifying sequence is nearly identical to the select sequence. For example, the sample read may be called for a genetic locus if the series of n nucleotides (e.g., the first n nucleotides) of the identifying sequence match a select sequence with no more than a designated number of mismatches (e.g., 3) and/or a designated number of shifts (e.g., 2). Rules may be established such that each mismatch or shift may count as a difference between the sample read and the primer sequence. If the number of differences is less than a designated number, then the sample read may be called for the corresponding genetic locus (i.e., assigned to the corresponding genetic locus). In some implementations, a matching score may be determined that is based on the number of differences between the identifying sequence of the sample read and the select sequence associated with a genetic locus. If the matching score passes a designated matching threshold, then the genetic locus that corresponds to the select sequence may be designated as a potential locus for the sample read. In some implementations, subsequent analysis may be performed to determine whether the sample read is called for the genetic locus.

If the sample read effectively matches one of the select sequences in the database (i.e., exactly matches or nearly matches as described above), then the sample read is assigned or designated to the genetic locus that correlates to the select sequence. This may be referred to as locus calling or provisional-locus calling, wherein the sample read is called for the genetic locus that correlates to the select sequence. However, as discussed above, a sample read may be called for more than one genetic locus. In such implementations, further analysis may be performed to call or assign the sample read for only one of the potential genetic loci. In some implementations, the sample read that is compared to the database of reference sequences is the first read from paired-end sequencing. When performing paired-end sequencing, a second read (representing a raw fragment) is obtained that correlates to the sample read. After assigning, the subsequent analysis that is performed with the assigned reads may be based on the type of genetic locus that has been called for the assigned read.

Next, the sample reads are analyzed to identify potential variant calls. Among other things, the results of the analysis identify the potential variant call, a sample variant frequency, a reference sequence and a position within the genomic sequence of interest at which the variant occurred. For example, if a genetic locus is known for including SNPs, then the assigned reads that have been called for the genetic locus may undergo analysis to identify the SNPs of the assigned reads. If the genetic locus is known for including polymorphic repetitive DNA elements, then the assigned reads may be analyzed to identify or characterize the polymorphic repetitive DNA elements within the sample reads. In some implementations, if an assigned read effectively matches with a STR locus and a SNP locus, a warning or flag may be assigned to the sample read. The sample read may be designated as both a STR locus and an SNP locus. The analyzing may include aligning the assigned reads in accordance with an alignment protocol to determine sequences and/or lengths of the assigned reads. The alignment protocol may include the method described in International Patent Application No. PCT/US2013/030867 (Publication No. WO 2014/142831), filed on March 15, 2013.

Then, the one or more processors analyze raw fragments to determine whether supporting variants exist at corresponding positions within the raw fragments. Various types of raw fragments may be identified. For example, the variant caller may identify a type of raw fragment that exhibits a variant that validates the original variant call. For example, the type of raw fragment may represent a duplex stitched fragment, a simplex stitched fragment, a duplex un-stitched fragment or a simplex un-stitched fragment. Optionally other raw fragments may be identified instead of or in addition to the foregoing examples. In connection with identifying each type of raw fragment, the variant caller also identifies the position, within the raw fragment, at which the supporting variant occurred, as well as a count of the number of raw fragments that exhibited the supporting variant. For example, the variant caller may output an indication that 10 reads of raw fragments were identified to represent duplex stitched fragments having a supporting variant at a particular position X. The variant caller may also output indication that five reads of raw fragments were identified to represent simplex un-stitched fragments having a supporting variant at a particular position Y. The variant caller may also output a number of raw fragments that corresponded to reference sequences and thus did not include a supporting variant that would otherwise provide evidence validating the potential variant call at the genomic sequence of interest.

Next, a count is maintained of the raw fragments that include supporting variants, as well as the position at which the supporting variant occurred. Additionally or alternatively, a count may be maintained of the raw fragments that did not include supporting variants at the position of interest (relative to the position of the potential variant call in the sample read or sample fragment). Additionally or alternatively, a count may be maintained of raw fragments that correspond to a reference sequence and do not authenticate or confirm the potential variant call. The information determined is output to the variant call validation application, including a count and type of the raw fragments that support the potential variant call, positions of the supporting variance in the raw fragments, a count of the raw fragments that do not support the potential variant call and the like.

When a potential variant call is identified, the process outputs an indicating of the potential variant call, the variant sequence, the variant position and a reference sequence associated therewith. The variant call is designated to represent a "potential" variant as errors may cause the call process to identify a false variant. In accordance with implementations herein, the potential variant call is analyzed to reduce and eliminate false variants or false positives. Additionally or alternatively, the process analyzes one or more raw fragments associated with a sample read and outputs a corresponding variant call associated with the raw fragments.

## Deep Learning in Genomics

Genetic variations can help explain many diseases. Every human being has a unique genetic code and there are lots of genetic variants within a group of individuals. Most of the deleterious genetic variants have been depleted from genomes by natural selection. It is important to identify which genetics variations are likely to be pathogenic or deleterious. This will help researchers focus on the likely pathogenic genetic variants and accelerate the pace of diagnosis and cure of many diseases.

Modeling the properties and functional effects (e.g., pathogenicity) of variants is an important but challenging task in the field of genomics. Despite the rapid advancement of functional genomic sequencing technologies, interpretation of the functional consequences of variants remains a great challenge due to the complexity of cell type-specific transcription regulation systems.

Regarding pathogenicity classifiers, deep neural networks are a type of artificial neural networks that use multiple nonlinear and complex transforming layers to successively model high-level features. Deep neural networks provide feedback via backpropagation which carries the difference between observed and predicted output to adjust parameters. Deep neural networks have evolved with the availability of large training datasets, the power of parallel and distributed computing, and sophisticated training algorithms. Deep neural networks have facilitated major advances in numerous domains such as computer vision, speech recognition, and natural language processing.

Convolutional neural networks (CNNs) and recurrent neural networks (RNNs) are components of deep neural networks. Convolutional neural networks have succeeded particularly in image recognition with an architecture that comprises convolution layers, nonlinear layers, and pooling layers. Recurrent neural networks are designed to utilize sequential information of input data with cyclic connections among building blocks like perceptrons, long short-term memory units, and gated recurrent units. In addition, many other emergent deep neural networks have been proposed for limited contexts, such as deep spatio-temporal neural networks, multi-dimensional recurrent neural networks, and convolutional auto-encoders.

The goal of training deep neural networks is optimization of the weight parameters in each layer, which gradually combines simpler features into complex features so that the most suitable hierarchical representations can be learned from data. A single cycle of the optimization process is organized as follows. First, given a training dataset, the forward pass sequentially computes the output in each layer and propagates the function signals forward through the network. In the final output layer, an objective loss function measures error between the inferenced outputs and the given labels. To minimize the training error, the backward pass uses the chain rule to backpropagate error signals and compute gradients with respect to all weights throughout the neural network. Finally, the weight parameters are updated using optimization algorithms based on stochastic gradient descent. Whereas batch gradient descent performs parameter updates for each complete dataset, stochastic gradient descent provides stochastic approximations by performing the updates for each small set of data examples. Several optimization algorithms stem from stochastic gradient descent. For example, the Adagrad and Adam training algorithms perform stochastic gradient descent while adaptively modifying learning rates based on update frequency and moments of the gradients for each parameter, respectively.

Another core element in the training of deep neural networks is regularization, which refers to strategies intended to avoid overfitting and thus achieve good generalization performance. For example, weight decay adds a penalty term to the objective loss function so that weight parameters converge to smaller absolute values. Dropout randomly removes hidden units from neural networks during training and can be considered an ensemble of possible subnetworks. To enhance the capabilities of dropout, a new activation function, maxout, and a variant of dropout for recurrent neural networks called rnnDrop have been proposed. Furthermore, batch normalization provides a new regularization method through normalization of scalar features for each activation within a mini-batch and learning each mean and variance as parameters.

Given that sequenced data are multi- and high-dimensional, deep neural networks have great promise for bioinformatics research because of their broad applicability and enhanced prediction power. Convolutional neural networks have been adapted to solve sequence-based problems in genomics such as motif discovery, pathogenic variant identification, and gene expression inference. Convolutional neural networks use a weight-sharing strategy that is especially useful for studying DNA because it can capture sequence motifs, which are short, recurring local patterns in DNA that are presumed to have significant biological functions. A hallmark of convolutional neural networks is the use of convolution filters. Unlike traditional classification approaches that are based on elaborately-designed and manually-crafted features, convolution filters perform adaptive learning of features, analogous to a process of mapping raw input data to the informative representation of knowledge. In this sense, the convolution filters serve as a series of motif scanners, since a set of such filters is capable of recognizing relevant patterns in the input and updating themselves during the training procedure. Recurrent neural networks can capture long-range dependencies in sequential data of varying lengths, such as protein or DNA sequences.

Therefore, a powerful computational model for predicting the pathogenicity of variants can have enormous benefits for both basic science and translational research.

At present, only 25-30% of rare disease patients receive a molecular diagnosis from examination of protein-coding sequence, suggesting that the remaining diagnostic yield may reside in the 99% of the genome that is noncoding. Here we describe a novel deep learning network that accurately predicts splice junctions from arbitrary pre-mRNA transcript sequence, enabling precise prediction of the splice-altering effects of noncoding variants. Synonymous and intronic mutations with predicted splice-altering consequence validate at a high rate on RNA-seq and are strongly deleterious in the human population. De novo mutations with predicted splice-altering consequence are significantly enriched in patients with autism and intellectual disability compared to healthy controls and validate against RNA-seq data in 21 out of 28 of these patients. We estimate that 9-11% of pathogenic mutations in patients with rare genetic disorders are caused by this previously underappreciated class of disease variation.

Exome sequencing has transformed the clinical diagnosis of patients and families with rare genetic disorders, and when employed as a first-line test, significantly reduces the time and costs of the diagnostic odyssey (Monroe et al., 2016; Stark et al., 2016; Tan et al., 2017). However, the diagnostic yield of exome sequencing is -25-30% in rare genetic disease cohorts, leaving the majority of patients without a diagnosis even after combined exome and microarray testing (Lee et al., 2014; Trujillano et al., 2017; Yang et al., 2014). Noncoding regions play a significant role in gene regulation and account for 90% of causal disease loci discovered in unbiased genome-wide association studies of human complex diseases (Ernst et al., 2011; Farb et al., 2015; Maurano et al., 2012), suggesting that penetrant noncoding variants may also account for a significant burden of causal mutations in rare genetic diseases. Indeed, penetrant noncoding variants that disrupt the normal pattern of mRNA splicing despite lying outside the essential GT and AG splice dinucleotides, often referred to as cryptic splice variants, have long been recognized to play a significant role in rare genetic diseases (Cooper et al., 2009; Padgett, 2012; Scotti and Swanson, 2016; Wang and Cooper, 2007). However, cryptic splice mutations are often overlooked in clinical practice, due to our incomplete understanding of the splicing code and the resulting difficulty in accurately identifying splice-altering variants outside the essential GT and AG dinucleotides (Wang and Burge, 2008).

Recently, RNA-seq has emerged as a promising assay for detecting splicing abnormalities in Mendelian disorders (Cummings et al., 2017; Kremer et al., 2017), but thus far its utility in a clinical setting remains limited to a minority of cases where the relevant cell type is known and accessible to biopsy. High-throughput screening assays of potential splice-altering variants (Soemedi et al., 2017) have expanded the characterization of splicing variation, but are less practical for evaluating random *de novo* mutations in genetic diseases, since the genomic space where splice-altering mutations may occur is extremely large. General prediction of splicing from arbitrary pre-mRNA sequence would potentially allow precise prediction of the splice-altering consequences of noncoding variants, substantially improving diagnosis in patients with genetic diseases. To date. a general predictive model of splicing from raw sequence that approaches the specificity of the spliceosome remains elusive, despite progress in specific applications, such as modeling the sequence characteristics of the core splicing motifs (Yeo and Burge, 2004), characterizing exonic splice enhancers and silencers (Fairbrother et al., 2002; Wang et al., 2004), and predicting cassette exon inclusion (Barash et al., 2010; Jha et al., 2017; Xiong et al., 2015).

The splicing of long pre-mRNAs into mature transcripts is remarkable for its precision, and the clinical severity of splice-altering mutations, yet the mechanisms by which the cellular machinery determines its specificity remain incompletely understood. Here we train a deep learning network that approaches the accuracy of the spliceosome *in silico*, identifying exon-intron boundaries from pre-mRNA sequence with 95% accuracy, and predicting functional cryptic splice mutations with a validation rate of over 80% on RNA-seq. Noncoding variants predicted to alter splicing are strongly deleterious in the human population, with 80% of newly created cryptic splice mutations experiencing negative selection, similar to the impact of other classes of protein truncating variation. *De novo* cryptic splice mutations in patients with autism and intellectual disability hit the same genes that are recurrently mutated by protein truncating mutations, enabling the discovery of additional candidate disease genes. We estimate that up to 24% of penetrant causal mutations in patients with rare genetic disorders are due to this previously underappreciated class of disease variation, highlighting the need to improve interpretation of the 99% of the genome that is noncoding for clinical sequencing applications.

Clinical exome sequencing has revolutionized diagnosis for patients and families with rare genetic disorders, and when employed as a first line test, significantly reduces the time and costs of diagnostic odyssey. However, the diagnostic yield for exome sequencing has been reported at 25-30% in multiple large cohorts of rare disease patients and their parents, leaving the majority of patients without a diagnosis even after combined exome and microarray testing. The noncoding genome is highly active in gene regulation, and noncoding variants account for -90% of GWAS hits for common diseases, suggesting that rare variants in the noncoding genome may also account for a significant fraction of causal mutations in penetrant diseases such as rare genetic disorders and oncology. However, the difficulty of interpreting variants in the noncoding genome means that outside of large structural variants, the noncoding genome presently offers little additional diagnostic benefit with respect to the rare penetrant variants that have the greatest impact on clinical management.

The role of splice-altering mutations outside the canonical GT and AG splice dinucleotides has long been appreciated in rare disease. Indeed, these cryptic splice variants are the most common mutations for some rare genetic disorders, such as glycogen storage disease XI (Pompe disease) and erythropoetic protoporphyria. The extended splice motifs at the 5' and 3' ends of introns are highly degenerate and equally good motifs occur frequently in the genome, making accurate prediction of which noncoding variants might cause cryptic splicing impractical with existing methods.

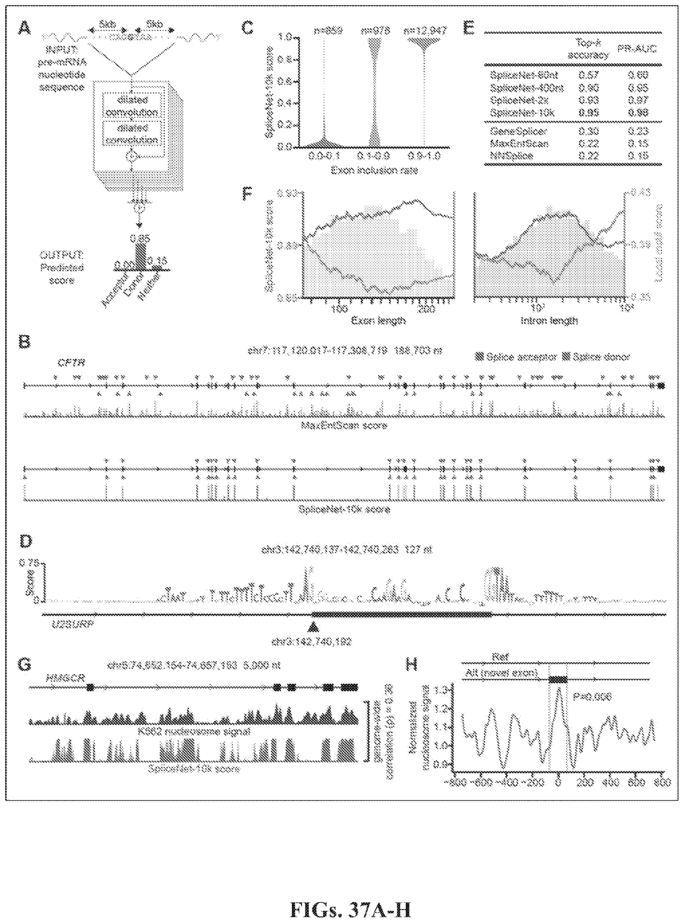

To better understand how the spliceosome achieves its specificity, we trained a deep learning neural network to predict for each nucleotide in a pre-mRNA transcript, whether it was a splice acceptor, splice donor, or neither, using only the transcript sequence as its input **(FIG. 37A)**. Using canonical transcripts on the even chromosomes as a training set and transcripts on the odd chromosomes for testing (with paralogs excluded), the deep learning network calls exon-intron boundaries with 95% accuracy, and even transcripts in excess of 100KB such as CFTR are often reconstructed perfectly to nucleotide precision **(FIG. 37B)**.

We next sought to understand the specificity determinants used by the network to recognize exon-intron boundaries with such remarkable precision. In contrast with previous classifiers which operate on statistical or human-engineered features, deep learning directly learns features from sequence in a hierarchical manner, enabling additional specificity to be imparted from long range sequence context. Indeed, we find that the accuracy of the network is highly dependent on the length of the sequence context flanking the nucleotide under prediction that is provided as input into the network (Table 1), and when we train a deep learning model that only uses 40-nt of sequence, performance only moderately exceeds existing statistical methods. This indicates that deep learning adds little over existing statistical methods for recognizing individual 9-23nt splicing motifs, but wider sequence context is the key to distinguishing functional splice sites from non-functional sites with equally strong motifs. Asking the network to predict on exons where the sequence is perturbed shows that disrupting the donor motif typically also causes the acceptor signal to disappear **(FIG. 37C)**, as is frequently observed with exon-skipping events in vivo, indicating that a significant degree of specificity is imparted simply by requiring pairing between strong acceptor and donor motifs at an acceptable distance.

Although a large body of evidence has shown that experimental perturbation of the length of exons has strong effects on exon inclusion versus exon skipping, it does not explain why the accuracy of the deep learning network continues to increase beyond 1000-nt of context. To better differentiate between local spice motif-driven specificity and long distance specificity determinants, we trained a local network that only takes as input 100-nt of context. Using the local network to score known junctions, we find that both exons and introns have optimal lengths (~115nt for exons, ~1000nt for introns) at which motif strength is minimal **(FIG. 37D)**. This relationship is not present in the 10000-nt deep learning network **(FIG. 37E)**, indicating that variability in intron and exon length are already fully factored into the wide-context deep learning network. Notably, the boundaries of introns and exons were never given to the wide-context deep learning model, indicating that it was able to derive these distances by inferring positions of exons and introns from the sequence alone.

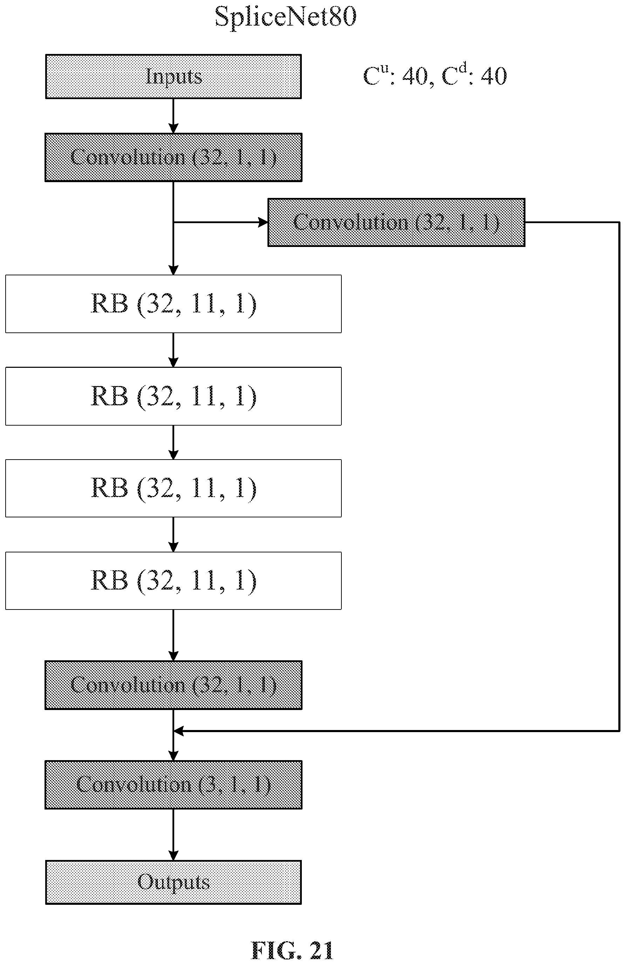

A systematic search of hexamer space also indicated that the deep learning network utilizes motifs in exon-intron definition, particularly the branch point motif TACTAAC from positions -34 to -14, the well-characterized exonic splice enhancer GAAGAA near the ends of exons, and poly-U motifs which are typically part of the polypyrimidine tract, but also appear to act as exonic splice silencers **(FIGs. 21, 22, 23, and 24)**.

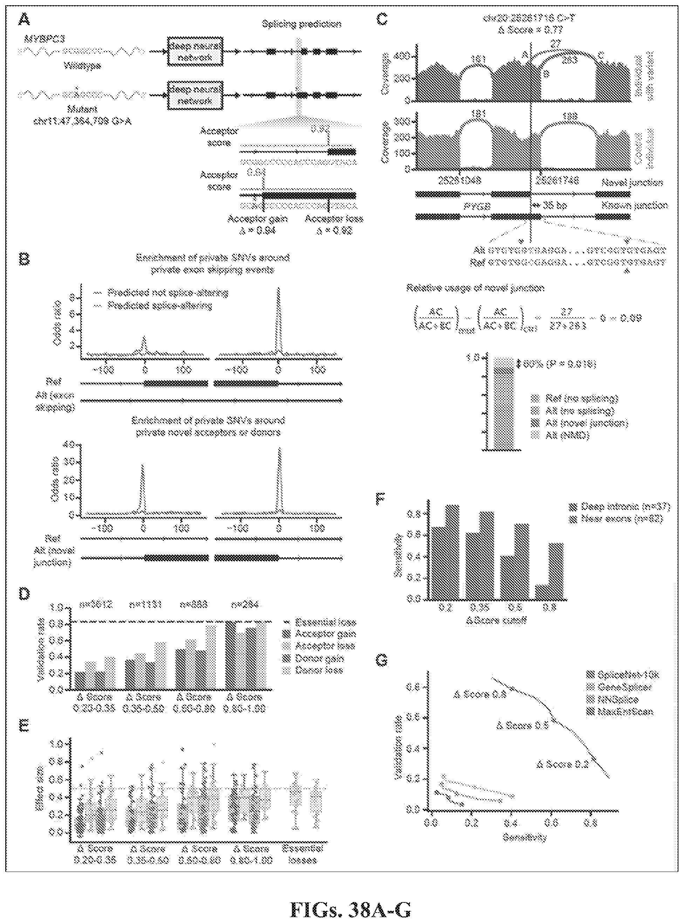

We extend the deep learning network to the evaluation of genetic variants for splice-altering function by predicting exon-intron boundaries on both the reference transcript sequence and on the alternate transcript sequence containing the variant, and looking for any changes in exon-intron boundaries. The recent availability of aggregated exome data from 60,706 humans enables us to evaluate the effects of negative selection on variants predicted to alter splice function by examining their distribution in the allele frequency spectrum. We find that predicted cryptic splice variants are strongly under negative selection **(FIG. 38A)**, as evidenced by their relative depletion at high allele frequencies compared to expected counts, and their magnitude of depletion is comparable with AG or GT splice-disrupting variants and stop-gain variants. The impact of negative selection greater when considering cryptic splice variants that would cause frameshifts over those that cause in-frame changes **(FIG. 38B)**. Based on the depletion of frameshifting cryptic splice variants compared to other classes of protein truncating variation, we estimate that 88% of confidently-predicted cryptic splice mutations are functional.

Although not as much aggregate whole genome data is available as exome data, limiting the power to detect the impact of natural selection in deep intronic regions, we were also able to calculate the observed vs expected counts of cryptic splice mutations far from exonic regions. Overall, we observe a 60% depletion in cryptic splice mutations at a distance > 50nt from an exon-intron boundary **(FIG. 38C)**. The attenuated signal is likely a combination of the smaller sample size with whole genome data compared to exome, and the greater difficulty of predicting the impact of deep intronic variants.

We can also use the observed versus expected number of cryptic splice variants to estimate the number of cryptic splice variants under selection, and how this compares with other classes of protein truncating variation. As cryptic splice variants may only partially abrogate splice function, we also evaluated the number of observed versus expected cryptic splice variants at more relaxed thresholds, and estimate that there are approximately 3 times as many deleterious rare cryptic splice variants compared to rare AG or GT splice-disrupting variants in the ExAC dataset **(FIG. 38D)**. Each individual carries approximately ~20 rare cryptic splice mutations, approximately equal to the number of protein truncating variants **(FIG. 38E)**, although not all of these variants fully abrogate splice function.

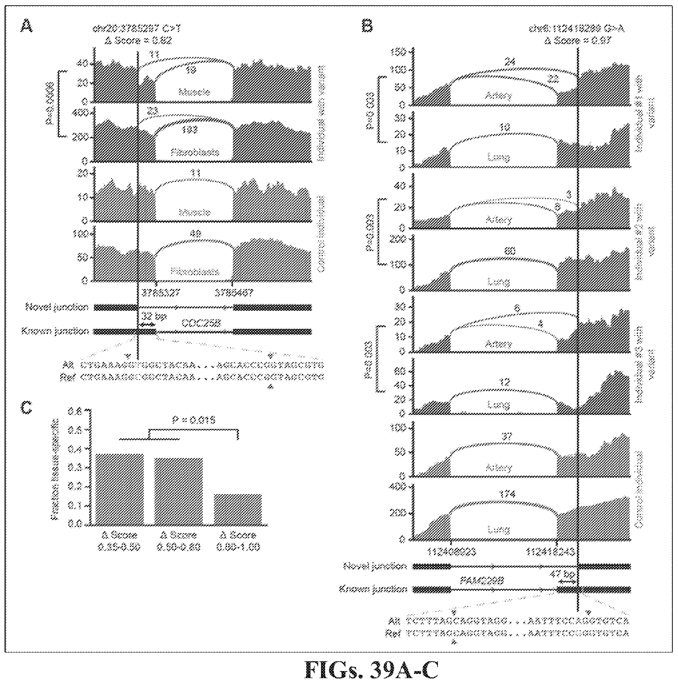

The recent release of GTEx data, comprising 148 individuals with both whole genome sequencing and RNA-seq from multiple tissue sites, enables us to look for the effects of rare cryptic splice variants directly in the RNA-seq data. To approximate the scenario encountered in rare disease sequencing, we only considered rare variants (singleton in the GTEx cohort, and < 1% allele frequency on 1000 Genomes), and paired these with splicing events that were unique to the individual with the variant. Although differences in gene expression and tissue expression and the complexity of splice abnormalities make it challenging to evaluate sensitivity and specificity of the deep learning predictions, we find that at stringent specificity thresholds, over 90% of rare cryptic splice mutations validate on RNA-seq **(FIG. 39A).** A large number of aberrant splicing events present in RNA-seq appear to be associated with variants that are predicted to have modest effects according to the deep learning classifier, suggesting that they only partially affect splice function. At these more sensitive thresholds, approximately 75% of novel junctions are predicted to cause aberrations in splicing function **(FIG. 38B).**

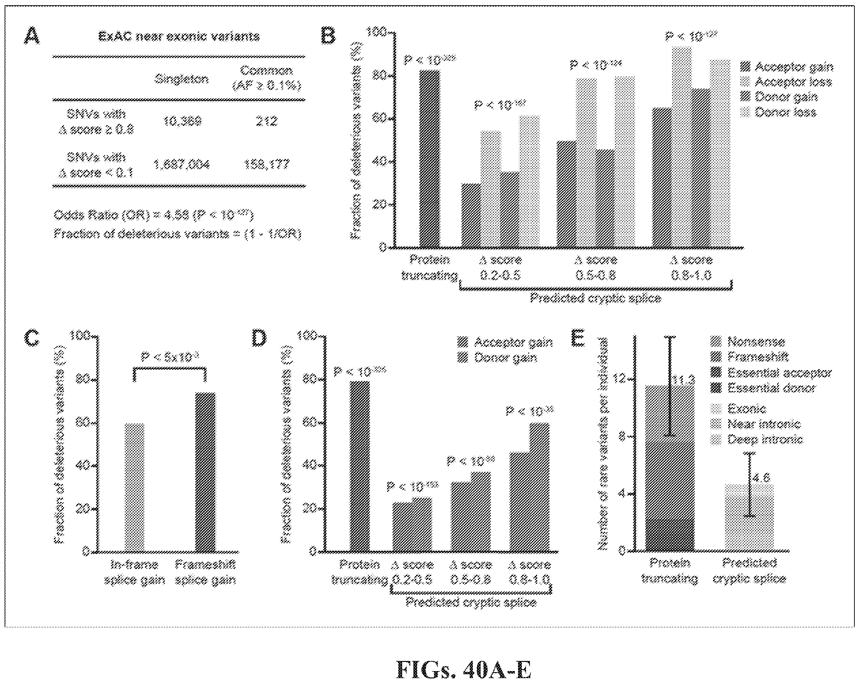

The success of the deep learning network at predicting cryptic splice variants that are strongly deleterious on population sequencing data, and validate at a high rate on RNA-seq, suggests that the method could be used to identify additional diagnoses in rare disease sequencing studies. To test this hypothesis, we examined *de novo* variants in exome sequencing studies for autism and neurodevelopmental disorders, and demonstrate that cryptic splice mutations are significantly enriched in affected individuals versus their healthy siblings **(FIG. 40A).** Moreover, the enrichment of cryptic splice mutations is slightly lower than that for protein truncating variants, indicating that approximately 90% of our predicted cryptic splice variants are functional. Based on these values, approximately ~20% of disease-causing protein truncating variants can be attributed to cryptic splice mutations in exons and the nucleotides immediately adjacent to exons **(FIG. 40B).** Extrapolating this figure out to whole genome studies, which are able to interrogate the entire intronic sequence, we estimate that 24% of causal mutations in rare genetic disorders are due to cryptic splice mutations.

We estimated the probability of calling a *de novo* cryptic splice mutation for each individual gene, enabling us to estimate the enrichment of cryptic splice mutations in candidate disease genes compared to chance. *De novo* cryptic splice mutations were strongly enriched within genes that were previously hit by protein-truncating variation but not missense variation **(FIG. 40C),** indicating that they mostly cause disease through haploinsufficiency rather than other modes of action. Adding predicted cryptic splice mutations to the list of protein truncating variants allows us to identify 3 additional disease genes in autism and 11 additional disease genes in intellectual disability, compared to using only protein-truncating variation alone **(FIG. 40D).**

To evaluate the feasibility of validating cryptic splice mutations in patients for whom the likely tissue of disease was not available (brain, in this case), we performed deep RNA-seq on 37 individuals with predicted *de novo* cryptic splice mutations from the Simon's Simplex Collection, and looked for aberrant splicing events that were present in the individual, and absent in all other individuals in the experiment and in the 149 individuals from the GTEx cohort. We find that NN out of 37 patients showed unique, aberrant splicing on RNA-seq **(FIG. 40E)** explained by the predicted cryptic splice variant.

In summary, we demonstrate a deep learning model that accurately predict cryptic splice variants with sufficient precision to be useful for identifying causal disease mutations in rare genetic disorders. We estimate that a substantial fraction of rare disease diagnoses caused by cryptic splicing are currently missed by considering only the protein coding regions, and stress the need to develop methods for interpreting the effects of penetrant rare variation in the noncoding genome.

### Results

### Accurate prediction of splicing from primary sequence using deep learning

We constructed a deep residual neural network (He et al., 2016a) that predicts whether each position in a pre-mRNA transcript is a splice donor, splice acceptor, or neither **(FIG. 37A** and **FIGs. 21**, 22, 23, and 24), using as input only the genomic sequence of the pre-mRNA transcript. Because splice donors and splice acceptors may be separated by tens of thousands of nucleotides, we employed a novel network architecture consisting of 32 dilated convolutional layers (Yu and Koltun, 2016) that can recognize sequence determinants spanning very large genomic distances. In contrast to previous methods that have only considered short nucleotide windows adjoining exon-intron boundaries (Yeo and Burge, 2004), or relied on human-engineered features (Xing et al, 2015), or experimental data, such as expression or splice factor binding (Jha et al., 2017), our neural network learns splicing determinants directly from the primary sequence by evaluating 10,000 nucleotides of flanking context sequence to predict the splice function of each position in the pre-mRNA transcript.

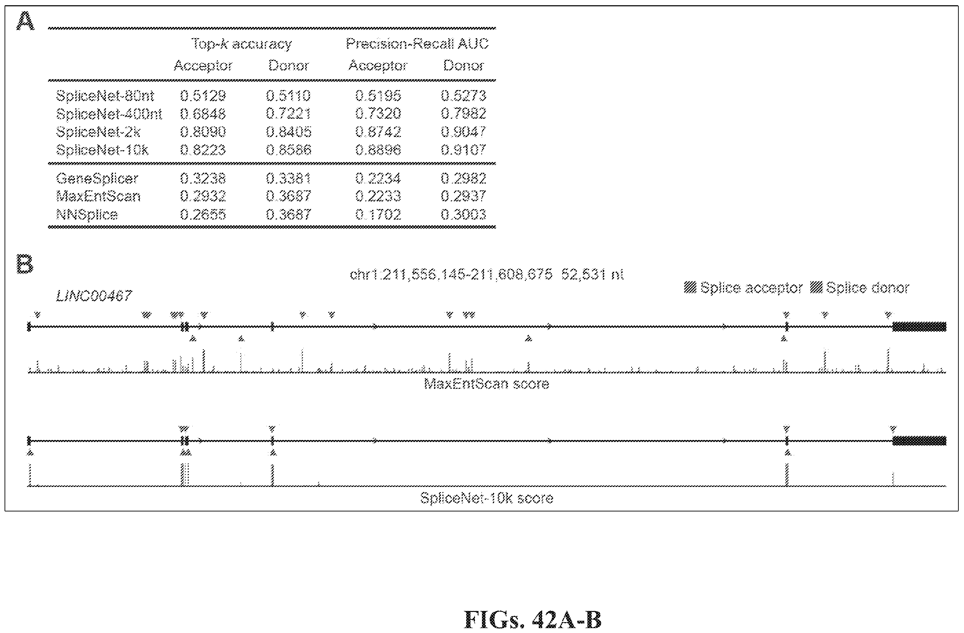

We used GENCODE-annotated pre-mRNA transcript sequences (Harrow et al, 2012) on a subset of the human chromosomes to train the parameters of the neural network, and transcripts on the remaining chromosomes, with paralogs excluded, to test the network's predictions. For pre-mRNA transcripts in the test dataset, the network predicts splice junctions with 95% top-k accuracy, which is the fraction of correctly predicted splice sites at the threshold where the number of predicted sites is equal to the actual number of splice sites present in the test dataset (Boyd et al., 2012; Yeo and Burge, 2004). Even genes in excess of 100 kb such as CFTR are often reconstructed perfectly to nucleotide precision **(FIG. 37B).** To confirm that the network is not simply relying on exonic sequence biases, we also tested the network on long noncoding RNAs. Despite the incompleteness of noncoding transcript annotations, which is expected to reduce our accuracy, the network predicts known splice junctions in lincRNAs with 84% top-k accuracy (**FIGs. 42A** and **42B**), indicating that it can approximate the behavior of the spliceosome on arbitrary sequences that are free from protein-coding selective pressures.

For each GENCODE-annotated exon in the test dataset (excluding the first and last exons of each gene), we also examined whether the network's prediction scores correlate with the fraction of reads supporting exon inclusion versus exon skipping, based on RNA-seq data from the Gene and Tissue Expression atlas (GTEx) (The GTEx Consortium et al., 2015) (**FIG. 37C**). Exons that were constitutively spliced in or spliced out across GTEx tissues had prediction scores that were close to 1 or 0, respectively, whereas exons that underwent a substantial degree of alternative splicing (between 10-90% exon inclusion averaged across samples) tended towards intermediate scores (Pearson correlation = 0.78, $P \approx 0$).

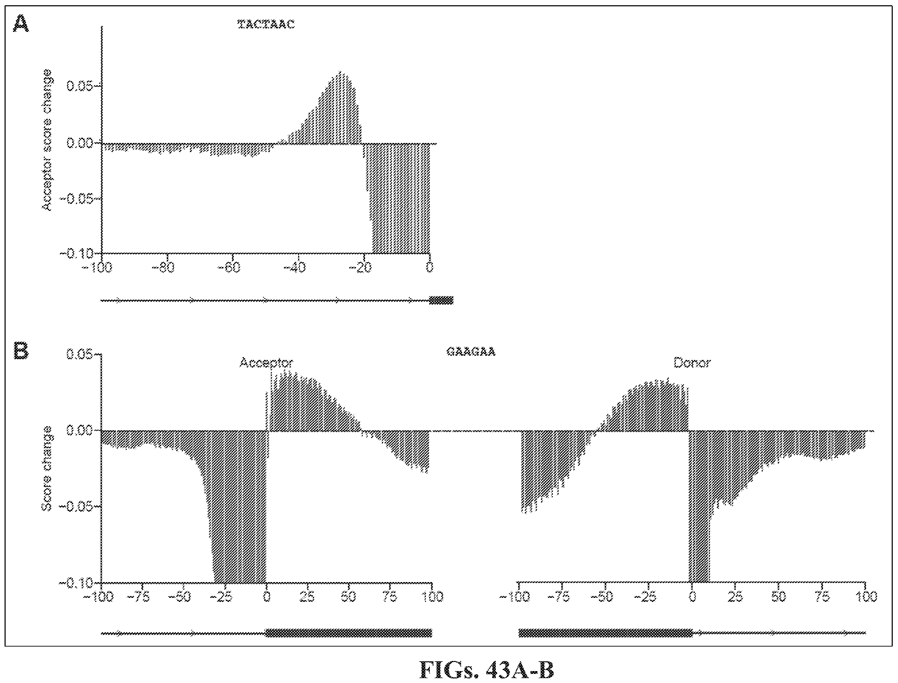

We next sought to understand the sequence determinants utilized by the network to achieve its remarkable accuracy. We performed systematic in silico substitutions of each nucleotide near annotated exons, measuring the effects on the network's prediction scores at the adjoining splice sites (**FIG. 37E**). We found that disrupting the sequence of a splice donor motif frequently caused the network to predict that the upstream splice acceptor site will also be lost, as is observed with exon-skipping events in vivo, indicating that a significant degree of specificity is imparted by exon definition between a paired upstream acceptor motif and a downstream donor motif set at an optimal distance (Berget, 1995). Additional motifs that contribute to the splicing signal include the well-characterized binding motifs of the SR-protein family and the branch point (**FIGs. 43A** and **43B**) (Fairbrother et al., 2002; Reed and Maniatis, 1988). The effects of these motifs are highly dependent on their position in the exon, suggesting that their roles include specifying the precise positioning of intron-exon boundaries by differentiating between competing acceptor and donor sites.

Training the network with varying input sequence context markedly impacts the accuracy of the splice predictions (**FIG. 37E**), indicating that long-range sequence determinants up to 10,000 nt away from the splice site are essential for discerning functional splice junctions from the large number of nonfunctional sites with near-optimal motifs. To examine long-range and short-range specificity determinants, we compared the scores assigned to annotated junctions by a model trained on 80 nt of sequence context (SpliceNet-80nt) versus the full model that is trained on 10,000 nt of context (SpliceNet-1 0k). The network trained on 80 nt of sequence context assigns lower scores to junctions adjoining exons or introns of typical length (150 nt for exons, ~1000 nt for introns) (**FIG. 37F**), in agreement with earlier observations that such sites tend to have weaker splice motifs compared to the splice sites of exons and introns which are unusually long or short (Amit et al., 2012; Gelfman et al., 2012; Li et al., 2015). In contrast, the network trained on 10,000 nt of sequence context shows preference for introns and exons of average length, despite their weaker splice motifs, because it can account for long-range specificity conferred by exon or intron length. The skipping of weaker motifs in long uninterrupted introns is consistent with the faster RNA polymerase II elongation experimentally observed in the absence of exon pausing, which may allow the spliceosome less time to recognize suboptimal motifs (Close et al., 2012; Jonkers et al., 2014; Veloso et al., 2014). Our findings suggest that the average splice junction possesses favorable long-range sequence determinants that confer substantial specificity, explaining the high degree of sequence degeneracy tolerated at most splice motifs.

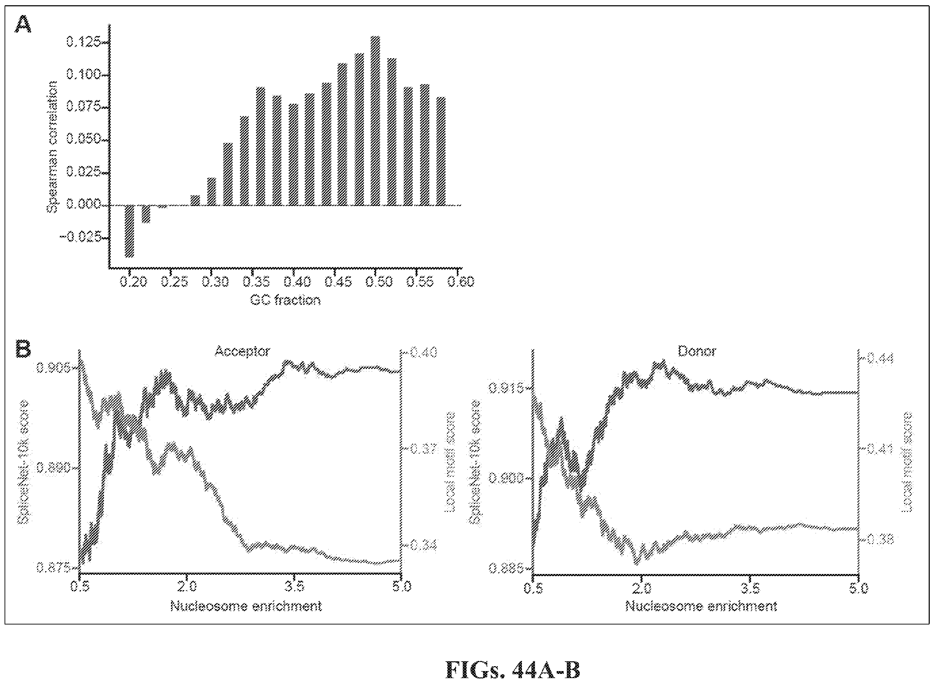

Because splicing occurs co-transcriptionally (Cramer et al., 1997; Tilgner et al., 2012), interactions between chromatin state and co-transcriptional splicing might also guide exon definition (Luco et al., 2011), and have the potential to be utilized by the network to the extent that chromatin state is predictable from primary sequence. In particular, genome-wide studies of nucleosome positioning have shown that nucleosome occupancy is higher in exons (Andersson et al., 2009; Schwartz et al., 2009; Spies et al., 2009; Tilgner et al., 2009). To test whether the network uses sequence determinants of nucleosome positioning for splice site prediction, we walked a pair of optimal acceptor and donor motifs separated by 150 nt (roughly the size of the average exon) across the genome and asked the network to predict whether the pair of motifs would result in exon inclusion at that locus (**FIG. 37G**). We find that positions predicted to be favorable for exon inclusion correlated with positions of high nucleosome occupancy, even in intergenic regions (Spearman correlation = 0.36, $P \approx 0$), and this effect persists after controlling for GC content (**FIG. 44A**). These results suggest that the network has implicitly learned to predict nucleosome positioning from primary sequence and utilizes it as a specificity determinant in exon definition. Similar to exons and introns of average length, exons positioned over nucleosomes have weaker local splice motifs (**FIG. 44B**), consistent with greater tolerance for degenerate motifs in the presence of compensatory factors (Spies et al., 2009).

Although multiple studies have reported a correlation between exons and nucleosome occupancy, a causal role for nucleosome positioning in exon definition has not been firmly established. Using data from 149 individuals with both RNA-seq and whole genome sequencing from the Genotype-Tissue Expression (GTEx) cohort (The GTEx Consortium et al., 2015), we identified novel exons that were private to a single individual, and corresponded to a private splice site-creating genetic mutation. These private exon-creation events were significantly associated with existing nucleosome positioning in K562 and GM 12878 cells (P = 0.006 by permutation test, **FIG. 37H**), even though these cell lines most likely lack the corresponding private genetic mutations. Our results indicate that genetic variants are more likely to trigger creation of a novel exon if the resulting novel exon would overlay a region of existing nucleosome occupancy, supporting a causal role for nucleosome positioning in promoting exon definition.

**Verification of predicted cryptic splice mutations in RNA-seq data**

We extended the deep learning network to the evaluation of genetic variants for splice-altering function by predicting exon-intron boundaries for both the reference pre-mRNA transcript sequence and the alternate transcript sequence containing the variant, and taking the difference between the scores ($\Delta$ Score) (**FIG**. **38A**). Importantly, the network was only trained on reference transcript sequences and splice junction annotations, and never saw variant data during training, making prediction of variant effects a challenging test of the network's ability to accurately model the sequence determinants of splicing.

We looked for the effects of cryptic splice variants in RNA-seq data in the GTEx cohort (The GTEx Consortium et al., 2015), comprising 149 individuals with both whole genome sequencing and RNA-seq from multiple tissues. To approximate the scenario encountered in rare disease sequencing, we first focused on rare, private mutations (present in only one individual in the GTEx cohort). We find that private mutations predicted to have functional consequences by the neural network are strongly enriched at private novel splice junctions and at the boundaries of skipped-over exons in private exon-skipping events (**FIG. 38B**), suggesting that a large fraction of these predictions are functional.

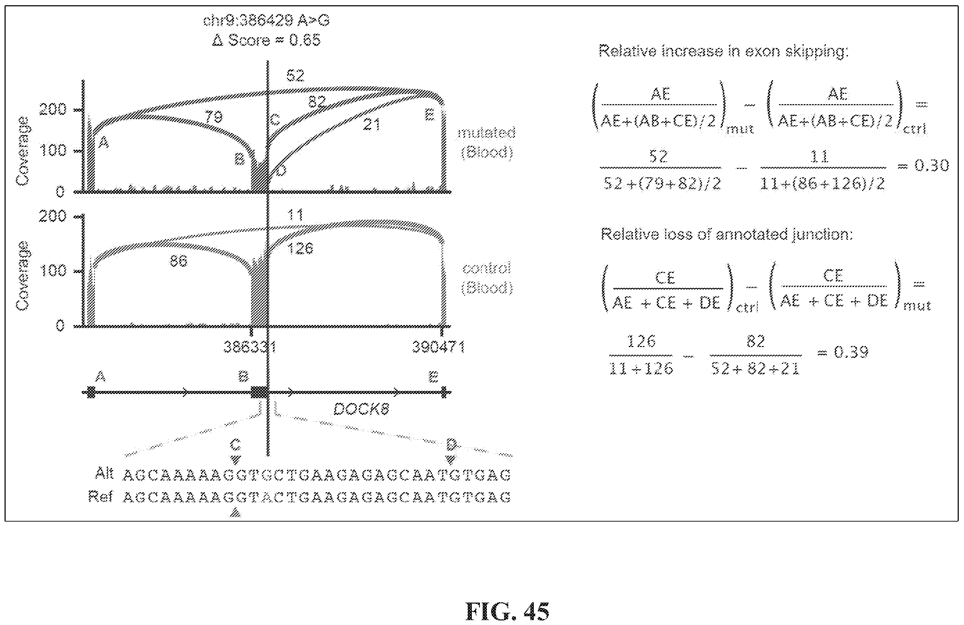

To quantify the effects of splice-site creating variants on the relative production of normal and aberrant splice isoforms, we measured the number of reads supporting the novel splice event as a fraction of the total number of reads covering the site (**FIG. 38C**) (Cummings et al., 2017). For splice-site disrupting variants, we observed that many exons had a low baseline rate of exon-skipping, and the effect of the variant was to increase the fraction of exon-skipping reads. Hence, we calculated both the decrease in the fraction of reads that spliced at the disrupted junction and the increase in the fraction of reads that skipped the exon, taking the larger of the two effects (**FIG. 45** and STAR Methods).

Confidently predicted cryptic splice variants ($\Delta$ Score $\geq$ 0.5) validate on RNA-seq at three-quarters the rate of essential GT or AG splice disruptions (**FIG. 38D**). Both the validation rate and effect size of cryptic splice variants closely track their $\Delta$ Scores (**FIGs. 38D** and **38E**), demonstrating that the model's prediction score is a good proxy for the splice-altering potential of a variant. Validated variants, especially those with lower scores ($\Delta$ Score < 0.5), are often incompletely penetrant, and result in alternative splicing with production of a mixture of both aberrant and normal transcripts in the RNA-seq data (**FIG. 38E**). Our estimates of validation rates and effect sizes are conservative and likely underestimate the true values, due to both unaccounted-for splice isoform changes and nonsense-mediated decay, which preferentially degrades aberrantly-spliced transcripts because they frequently introduce premature stop codons (**FIG. 38C** and **FIG. 45**). This is evidenced by the average effect sizes of variants that disrupt essential GT and AG splice dinucleotides being less than the 50% expected for fully penetrant heterozygous variants.

For cryptic splice variants that produce aberrant splice isoforms in at least three-tenths of the observed copies of the mRNA transcript, the network has a sensitivity of 71% when the variant is near exons, and 41% when the variant is in deep intronic sequence ($\Delta$ Score $\geq$ 0.5, **FIG. 38F**). These findings indicate that deep intronic variants are more challenging to predict, possibly because deep intronic regions contain fewer of the specificity determinants that have been selected to be present near exons.

To benchmark the performance of our network against existing methods, we selected three popular classifiers that have been referenced in the literature for rare genetic disease diagnosis, GeneSplicer (Pertea et al., 2001), Max-EntScan (Yeo and Burge, 2004), and NNSplice (Reese et al., 1997), and plotted the RNA-seq validation rate and sensitivity at varying thresholds (**FIG. 38G**). As has been the experience of others in the field (Cummings et al., 2017), we find that existing classifiers have insufficient specificity given the very large number of noncoding variants genome-wide that can possibly affect splicing, presumably because they focus on local motifs and largely do not account for long-range specificity determinants.

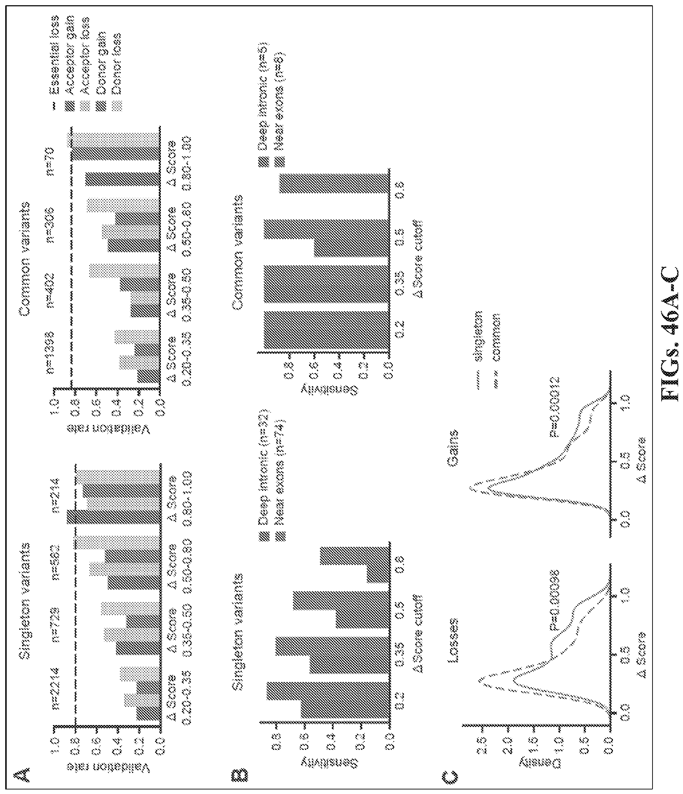

Given the large gap in performance compared with existing methods, we performed additional controls to exclude the possibility that our results in the RNA-seq data could be confounded by overfitting. First, we repeated the validation and sensitivity analyses separately for private variants and variants present in more than one individual in the GTEx cohort (**FIGs. 46A, 46B,** and **46C**). Because neither the splicing machinery nor the deep learning model have access to allele frequency information, verifying that the network has similar performance across the allele frequency spectrum is an important control. We find that at the same $\Delta$ Score thresholds, private and common cryptic splice variants show no significant differences in their validation rate in RNA-seq (P > 0.05, Fisher Exact test), indicating that the network's predictions are robust to allele frequency.

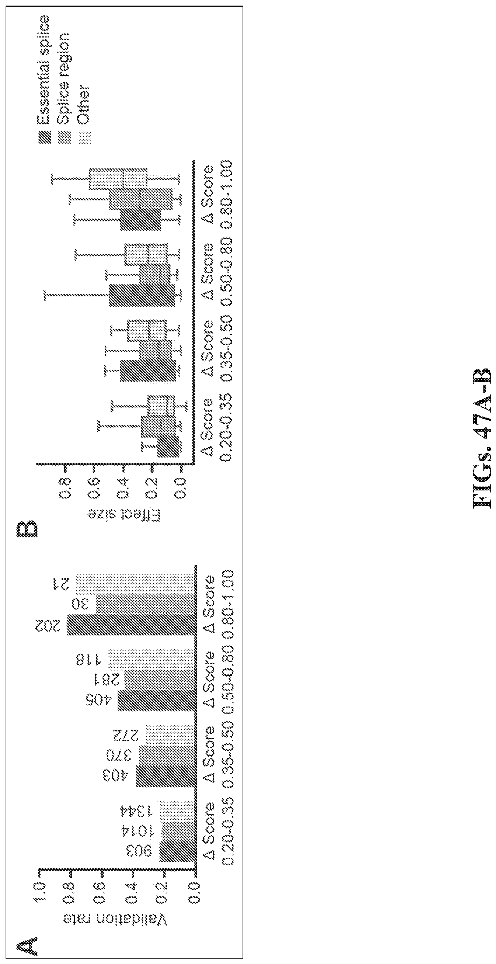

Second, to validate the model's predictions across the different types of cryptic splice variants that can create novel splice junctions, we separately evaluated variants that generate novel GT or AG dinucleotides, those that affect the extended acceptor or donor motif, and variants that occur in more distal regions. We find that cryptic splice variants are distributed roughly equally between the three groups, and that at the same $\Delta$ Score thresholds, there are no significant differences in the validation rate or effect sizes between the groups (P > 0.3 $\chi^2$ test of uniformity and P > 0.3 Mann Whitney U test respectively, **FIGs. 47A** and **47B**).

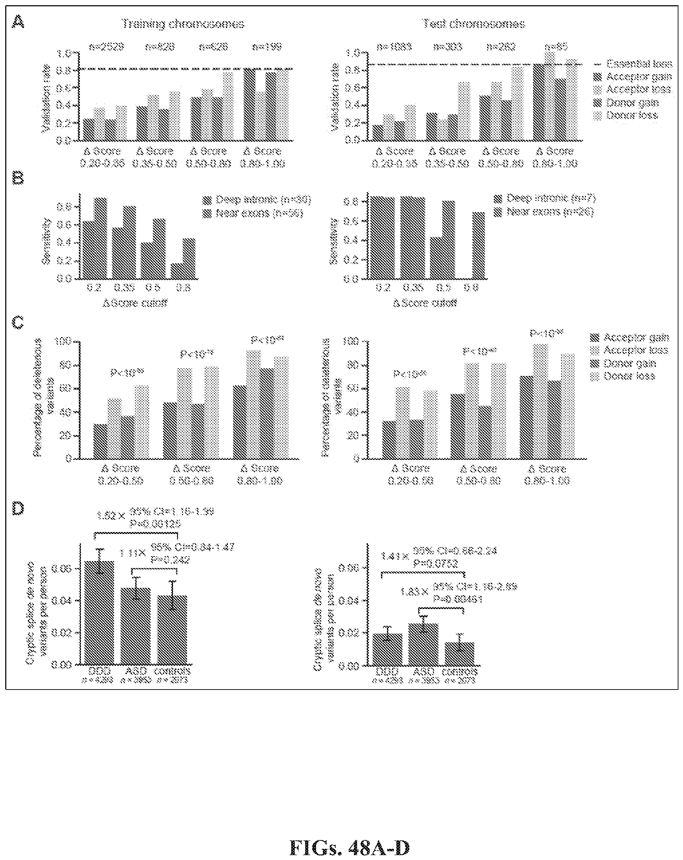

Third, we performed the RNA-seq validation and sensitivity analyses separately for variants on the chromosomes used for training and variants on the rest of the chromosomes (**FIGs. 48A** and **48B).** Although the network was only trained on reference genomic sequence and splice annotations, and was not exposed to variant data during training, we wanted to rule out the possibility of biases in variant predictions arising from the fact that the network has seen the reference sequence in the training chromosomes. We find that the network performs equally well on variants from the training and testing chromosomes, with no significant difference in validation rate or sensitivity (P > 0.05, Fisher Exact test), indicating that the network's variant predictions are unlikely to be explained by overfitting the training sequences.

Predicting cryptic splice variants is a more difficult problem than predicting annotated splice junctions, as reflected by the results of our model as well as other splice prediction algorithms (compare **FIG. 37E** and **FIG**. 38G). An important reason is the difference in the underlying distribution of exon inclusion rates between the two types of analyses. The vast majority of GENCODE-annotated exons have strong specificity determinants, resulting in constitutive splicing and prediction scores near 1 (**FIG. 37C**). In contrast, most cryptic splice variants are only partially penetrant (**FIGs. 38D** and **38E**), have low to intermediate prediction scores, and frequently lead to alternative splicing with the production of a mixture of both normal and aberrant transcripts. This makes the latter problem of predicting the effects of cryptic splice variants an intrinsically harder one than identifying annotated splice sites. Additional factors such as nonsense mediated decay, unaccounted for isoform changes, and limitations of the RNA-seq assay further contribute to lowering the RNA-seq validation rate (**FIGs. 38C** and **FIG. 45**).

## Tissue-specific alternative splicing frequently arises from weak cryptic splice variants

Alternative splicing is a major mode of gene regulation that serves to increase the diversity of transcripts in different tissues and developmental stages, and its dysregulation is associated with disease processes (Blencowe, 2006; Irimia et al., 2014; Keren et al., 2010; Licatalosi and Damell, 2006; Wang et al., 2008). Unexpectedly, we find that the relative usage of novel splice junctions created by cryptic splice mutations can vary substantially across tissues (**FIG. 39A**). Moreover, variants that cause tissue-specific differences in splicing are reproducible across multiple individuals (**FIG. 39B**), indicating that tissue-specific biology likely underlies these differences, rather than stochastic effects. We find that 35% of cryptic splice variants with weak and intermediate predicted scores (Δ Score 0.35-0.8) exhibit significant differences in the fraction of normal and aberrant transcripts produced across tissues (Bonferroni-corrected P < 0.01 for a $\chi^2$ test, **FIG. 39C**). This contrasted with variants with high predicted scores (Δ Score > 0.8), which were significantly less likely to produce tissue-specific effects (P = 0.015). Our findings align with the earlier observation that alternatively spliced exons tend to have intermediate prediction scores (**FIG. 37C**), compared to exons that are constitutively spliced in or spliced out, which have scores that are close to 1 or 0, respectively.

These results support a model where tissue-specific factors, such as the chromatin context and binding of RNA-binding proteins, may swing the contest between two splice junctions that are close in favorability (Gelfman et al., 2013; Luco et al., 2010; Shukla et al., 201 1; Ule et al., 2003). Strong cryptic splice variants are likely to fully shift splicing from the normal to the aberrant isoform irrespective of the epigenetic context, whereas weaker variants bring splice junction selection closer to the decision boundary, resulting in alternative junction usage in different tissue types and cell contexts. This highlights the unexpected role played by cryptic splice mutations in generating novel alternative splicing diversity, as natural selection would then have the opportunity to preserve mutations that create useful tissue-specific alternative splicing.

## Predicted cryptic splice variants are strongly deleterious in human populations

Although predicted cryptic splice variants validate at a high rate in RNA-seq, in many cases the effects are not fully penetrant and a mixture of both normal and aberrant splice isoforms are produced, raising the possibility that a fraction of these cryptic splice-altering variants may not be functionally significant. To explore the signature of natural selection on predicted cryptic splice variants, we scored each variant present in 60,706 human exomes from the Exome Aggregation Consortium (ExAC) database (Lek et al., 2016), and identified variants that were predicted to alter exon-intron boundaries.

To measure the extent of negative selection acting on predicted splice-altering variants, we counted the number of predicted splice-altering variants found at common allele frequencies (20.1% in the human population) and compared it to the number of predicted splice-altering variants at singleton allele frequencies in ExAC (i.e. in 1 out of 60,706 individuals). Because of the recent exponential expansion in human population size, singleton variants represent recently created mutations that have been minimally filtered by purifying selection (Tennessen et al., 2012). In contrast, common variants represent a subset of neutral mutations that have passed through the sieve of purifying selection. Hence, depletion of predicted splice-altering variants in the common allele frequency spectrum relative to singleton variants provides an estimate of the fraction of predicted splice-altering variants that are deleterious, and therefore functional.

To avoid confounding effects on protein-coding sequence, we restricted our analysis to synonymous variants and intronic variants lying outside the essential GT or AG dinucleotides, excluding missense mutations that are also predicted to have splice-altering effects.

At common allele frequencies, confidently predicted cryptic splice variants ($\Delta$ Score $\geq 0.8$) are under strong negative selection, as evidenced by their relative depletion compared to expectation (**FIG. 40A**). At this threshold, where most variants are expected to be close to fully penetrant in the RNA-seq data (**FIG. 38D**), predicted synonymous and intronic cryptic splice mutations are depleted by 78% at common allele frequencies, which is comparable with the 82% depletion of frameshift, stop-gain, and essential GT or AG splice-disrupting variants (**FIG. 40B**). The impact of negative selection is larger when considering cryptic splice variants that would cause frameshifts over those that cause in-frame changes (**FIG. 40C**). The depletion of cryptic splice variants with frameshift consequence is nearly identical to that of other classes of protein-truncating variation, indicating that the vast majority of confidently-predicted cryptic splice mutations in the near-intronic region ($\leq 50$ nt from known exon-intron boundaries) are functional and have strongly deleterious effects in the human population.

To extend this analysis into deep intronic regions >50 nt from known exon-intron boundaries, we used aggregated whole genome sequencing data from 15,496 humans from the Genome Aggregation Database (gnomAD) cohort (Lek et al., 2016) to calculate the observed and expected counts of cryptic splice mutations at common allele frequencies. Overall, we observe a 56% depletion of common cryptic splice mutations ($\Delta$ Score $\geq 0.8$) at a distance >50 nt from an exon-intron boundary (**FIG. 40D**), consistent with greater difficulty in predicting the impact of deep intronic variants, as we had observed in the RNA-seq data.

We next sought to estimate the potential for cryptic splice mutations to contribute to penetrant genetic disease, relative to other types of protein-coding variation, by measuring the number of rare cryptic splice mutations per individual in the gnomAD cohort. Based on the fraction of predicted cryptic splice mutations that are under negative selection (**FIG. 40A**), the average human carries ~5 rare functional cryptic splice mutations (allele frequency <0.1%), compared to ~11 rare protein-truncating variants (**FIG. 40E**). Cryptic splice variants outnumber essential GT or AG splice-disrupting variants roughly 2:1. We caution that a significant fraction of these cryptic splice variants may not fully abrogate gene function, either because they produce in-frame alterations, or because they do not completely shift splicing to the aberrant isoform.

### *De novo* cryptic splice mutations are a major cause of rare genetic disorders

Large-scale sequencing studies of patients with autism spectrum disorders and severe intellectual disability have demonstrated the central role of de novo protein-coding mutations (missense, nonsense, frameshift, and essential splice dinucleotide) that disrupt genes in neurodevelopmental pathways (Fitzgerald et al., 2015; Iossifov et al., 2014; McRae et al., 2017; Neale et al., 2012; De Rubeis et al., 2014; Sanders et al., 2012). To assess the clinical impact of noncoding mutations that act through altered splicing, we applied the neural network to predict the effects of de novo mutations in 4,293 individuals with intellectual disability from the Deciphering Developmental Disorders cohort (DDD) (McRae et al., 2017), 3,953 individuals with autism spectrum disorders (ASD) from the Simons Simplex Collection (De Rubeis et al., 2014; Sanders et al., 2012; Turner et al., 2016) and the Autism Sequencing Consortium, and 2,073 unaffected sibling controls from the Simons Simplex Collection. To control for differences in de novo variant ascertainment across studies, we normalized the expected number of de novo variants such that the number of synonymous mutations per individual was the same across cohorts.

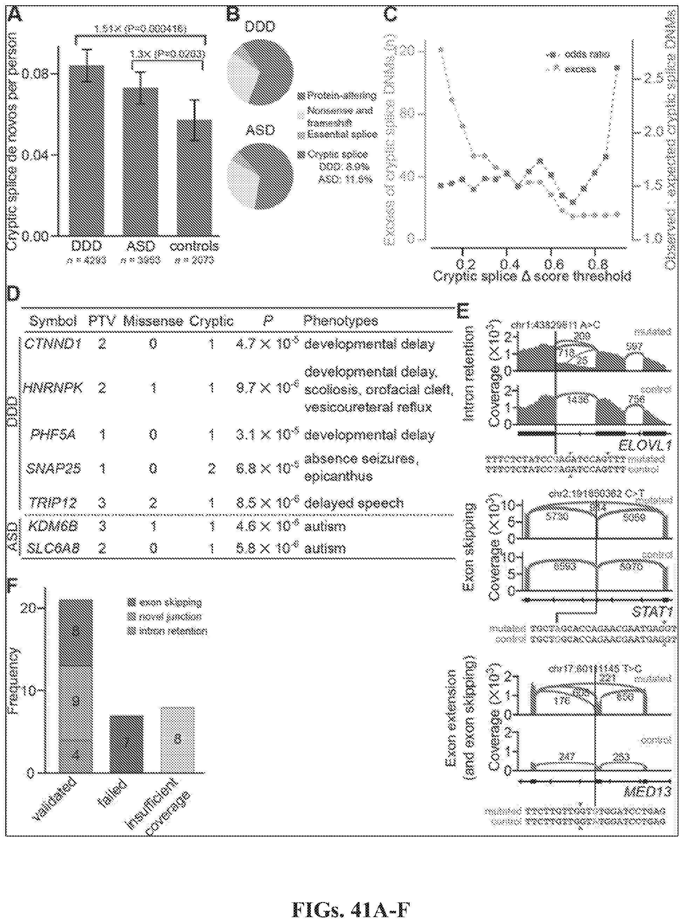

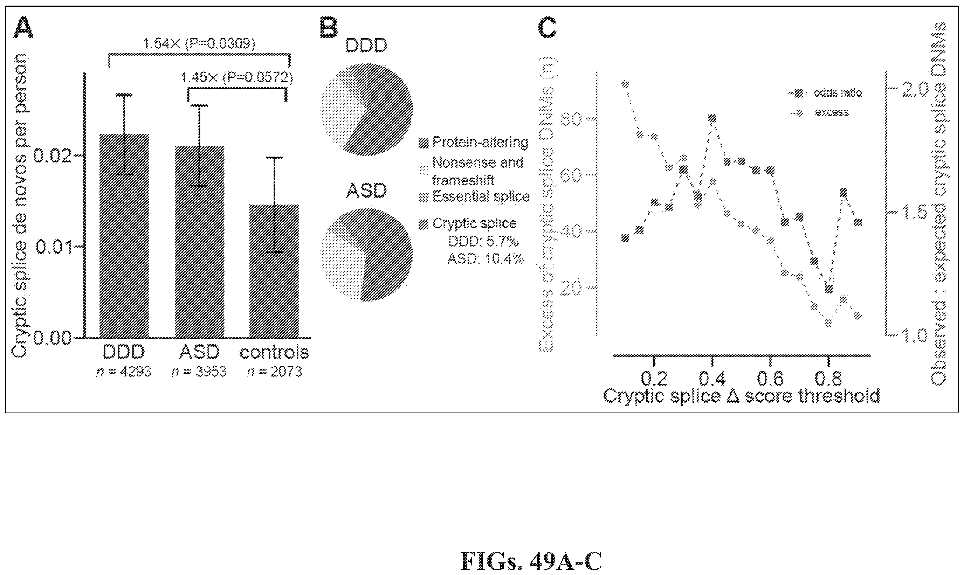

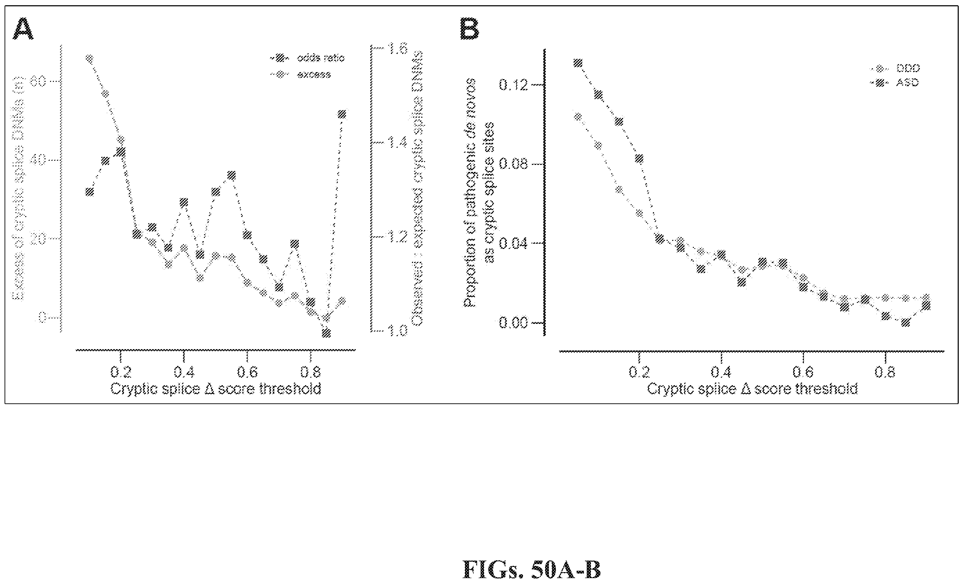

De novo mutations that are predicted to disrupt splicing are enriched 1.51-fold in intellectual disability (P = 0.000416) and 1.30-fold in autism spectrum disorder (P = 0.0203) compared to healthy controls ($\Delta$ Score $\geq 0.1$, **FIG. 41A, FIG. 43A,** and **FIG. 43B**). Splice-disrupting mutations are also significantly enriched in cases versus controls when considering only synonymous and intronic mutations (**FIG. 49A, FIG. 49B,** and **FIG. 49C**), excluding the possibility that the enrichment could be explained solely by mutations with dual protein-coding and splicing effects. Based on the excess of de novo mutations in affected versus unaffected individuals, cryptic splice mutations are estimated to comprise about 11% of pathogenic mutations in autism spectrum disorder, and 9% in intellectual disability (**FIG. 41B**), after adjusting for the expected fraction of mutations in regions that lacked sequencing coverage or variant ascertainment in each study. Most de novo predicted cryptic splice mutations in affected individuals had $\Delta$ Scores < 0.5 (**FIG. 41C, FIG. 50A,** and **FIG. 50B**), and would be expected to produce a mixture of normal and aberrant transcripts based on variants with similar scores in the GTEx RNA-seq dataset.

To estimate the enrichment of cryptic splice mutations in candidate disease genes compared to chance, we calculated the probability of calling a de novo cryptic splice mutation for each individual gene using trinucleotide context to adjust for mutation rate (Samocha et al., 2014) (Table S4). Combining both cryptic splice mutations and protein-coding mutations in novel gene discovery yields 5 additional candidate genes associated with intellectual disability and 2 additional genes associated with autism spectrum disorder (**FIG. 41D** and **FIG. 45**) that would have been below the discovery threshold (FOR < 0.01) when considering only protein-coding mutations (Kosmicki et al., 2017; Sanders et al., 2015).

## Experimental validation of de novo cryptic splice mutations in autism patients

We obtained peripheral blood-derived lymphoblastoid cell lines (LCLs) from 36 individuals from the Simons Simplex Collection, which harbored predicted de novo cryptic splice mutations in genes with at least a minimal level of LCL expression (De Rubeis et al., 2014; Sanders et al., 2012); each individual represented the only case of autism within their immediate family. As is the case for most rare genetic diseases, the tissue and cell type of relevance (presumably developing brain) was not accessible. Hence, we performed high-depth mRNA sequencing (~350 million $\times$ 150 bp single reads per sample, roughly 10 times the coverage of GTEx) to compensate for the weak expression of many of these transcripts in LCLs. To ensure that we were validating a representative set of predicted cryptic splice variants, rather than simply the top predictions, we applied relatively permissive thresholds ($\Delta$ Score > 0.1 for splice loss variants and $\Delta$ Score > 0.5 for splice gain variants. STAR methods) and performed experimental validation on all de novo variants meeting these criteria.

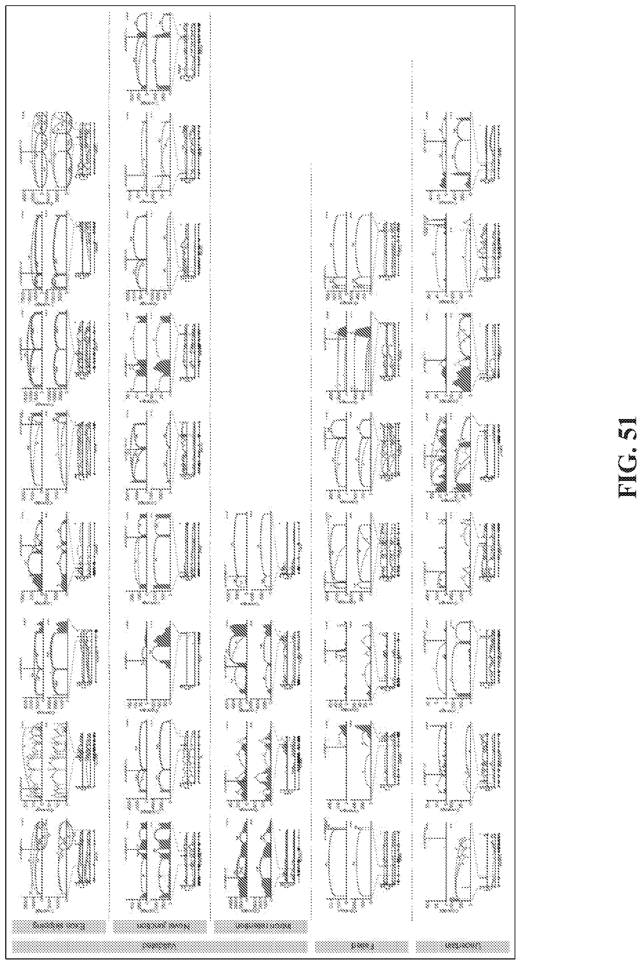

After excluding 8 individuals who had insufficient RNA-seq coverage at the gene of interest, we identified unique, aberrant splicing events associated with the predicted de novo cryptic splice mutation in 21 out of 28 patients (**FIG. 41E** and **FIG. 51**). These aberrant splicing events were absent from the other 35 individuals for whom deep LCL RNA-seq was obtained, as well as the 149 individuals from the GTEx cohort. Among the 21 confirmed de novo cryptic splice mutations, we observed 9 cases of novel junction creation, 8 cases of exon skipping, and 4 cases of intron retention, as well as more complex splicing aberrations (**FIG. 41F, FIG. 46A, FIG. 46B,** and **FIG. 46C**). Seven cases did not show aberrant splicing in LCLs, despite adequate expression of the transcript. Although a subset of these may represent false positive predictions, some cryptic splice mutations may result in tissue-specific alternative splicing that is not observable in LCLs under these experimental conditions.

The high validation rate of predicted cryptic splice mutations in patients with autism spectrum disorder (75%), despite the limitations of the RNA-seq assay, indicates that most predictions are functional. However, the enrichment of de novo cryptic splice variants in cases compared to controls (1.5-fold in DDD and 1.3-fold in ASD, **FIG. 41A)** is only 38% of the effect size observed for de novo protein-truncating variants (2.5-fold in DDD, and 1.7-fold in ASD) (Iossifov et al., 2014; McRae et al., 2017; De Rubeis et al., 2014). This allows us to quantify that functional cryptic splice mutations have roughly 50% of the clinical penetrance of classic forms of protein-truncating mutation (stop-gain, frameshift, and essential splice dinucleotide), on account of many of them only partially disrupting production of the normal transcript. Indeed, some of the most well-characterized cryptic splice mutations in Mendelian diseases, such as c.315-48T>C in FECH (Gouya et al., 2002) and c.-32-13T>G in GAA (Boerkoel et al., 1995), are hypomorphic alleles associated with milder phenotype or later age of onset. The estimate of clinical penetrance is calculated for all de novo variants meeting a relatively permissive threshold ($\Delta$ Score $\geq$ 0.1), and variants with stronger prediction scores would be expected to have correspondingly higher penetrance.

Based on the excess of de novo mutations in cases versus controls across the ASD and DDD cohorts, 250 cases can be explained by de novo cryptic splice mutations compared to 909 cases that can be explained by de novo protein-truncating variants (**FIG. 41B**). This is consistent with our earlier estimate of the average number of rare cryptic splice mutations (~5) compared to rare protein-truncating variants (~11) per person in the general population (**FIG. 38A**), once the reduced penetrance of cryptic splice mutations is factored in. The widespread distribution of cryptic splice mutations across the genome suggests that the fraction of cases explained by cryptic splice mutations in neurodevelopmental disorders (9-11%, **FIG. 41B**) is likely to generalize to other rare genetic disorders where the primary disease mechanism is loss of the functional protein. To facilitate the interpretation of splice-altering mutations, we precomputed the $\Delta$ Score predictions for all possible single nucleotide substitutions genome-wide, and provide them as a resource to the scientific community. We believe that this resource will promote understanding of this previously under-appreciated source of genetic variation.

## Particular Implementations

We describe systems, methods, and articles of manufacture for using a trained atrous convolutional neural network to detect splice sites in a genomic sequence (e.g., a nucleotide sequence or an amino acid sequence). One or more features of an implementation can be combined with the base implementation. Implementations that are not mutually exclusive are taught to be combinable. One or more features of an implementation can be combined with other implementations. This disclosure periodically reminds the user of these options.

This section uses the terms module(s) and stage(s) interchangeably.

A system implementation of the technology disclosed includes one or more processors coupled to the memory. The memory is loaded with computer instructions to train a splice site detector that identifies splice sites in genomic sequences (e.g., nucleotide sequences).

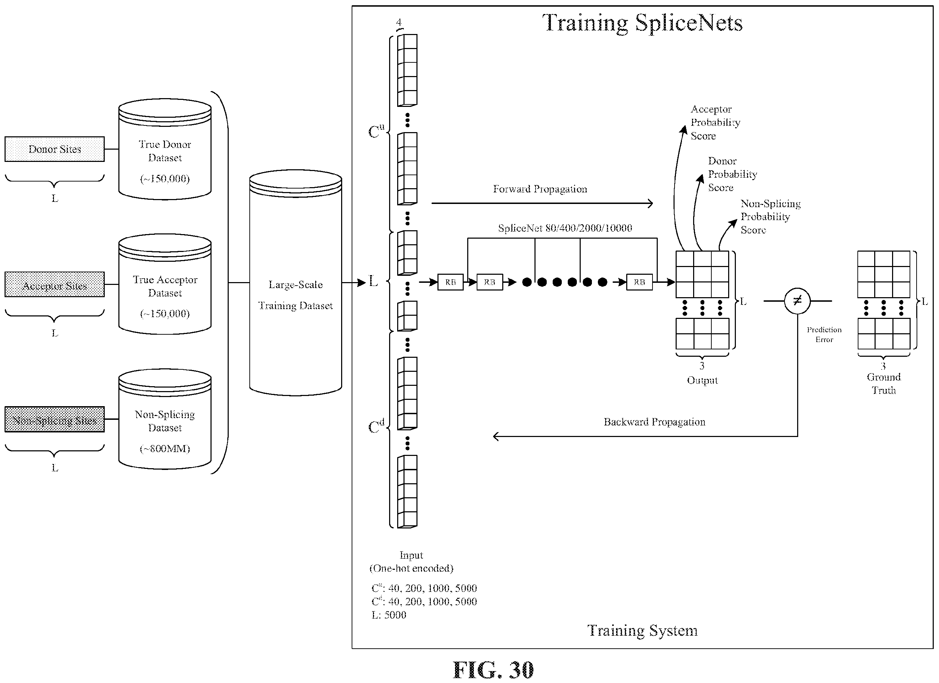

As shown in **FIG. 30,** the system trains an atrous convolutional neural network (abbreviated ACNN) on at least 50000 training examples of donor splice sites, at least 50000 training examples of acceptor splice sites, and at least 100000 training examples of non-splicing sites. Each training example is a target nucleotide sequence having at least one target nucleotide flanked by at least 20 nucleotides on each side.

An ACNN is a convolutional neural network that uses atrous/ditated convolutions which allow for large receptive fields with few trainable parameters. An atrous/dilated convolution is a convolution where the kernel is applied over an area larger than its length by skipping input values with a certain step, also called atrous convolution rate or dilation factor. Atrous/dilated convolutions add spacing between the elements of a convolution filter/kernel so that neighboring input entries (e.g., nucleotides, amino acids) at larger intervals are considered when a convolution operation is performed. This enables incorporation of long-range contextual dependencies in the input. The atrous convolutions conserve partial convolution calculations for reuse as adjacent nucleotides are processed.

As shown in **FIG. 30**, for evaluating a training example using the ACNN, the system provides, as input to the ACNN, a target nucleotide sequence further flanked by at least 40 upstream context nucleotides and at least 40 downstream context nucleotides.

As shown in **FIG. 30,** based on the evaluation, the ACNN then produces, as output, triplet scores for likelihood that each nucleotide in the target nucleotide sequence is a donor splice site, an acceptor splice site, or a non-splicing site.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

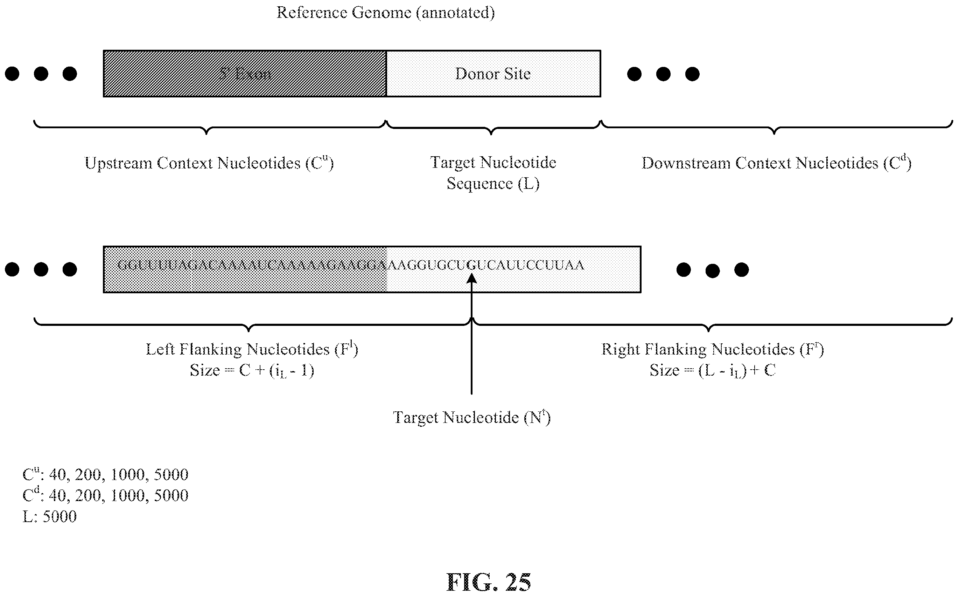

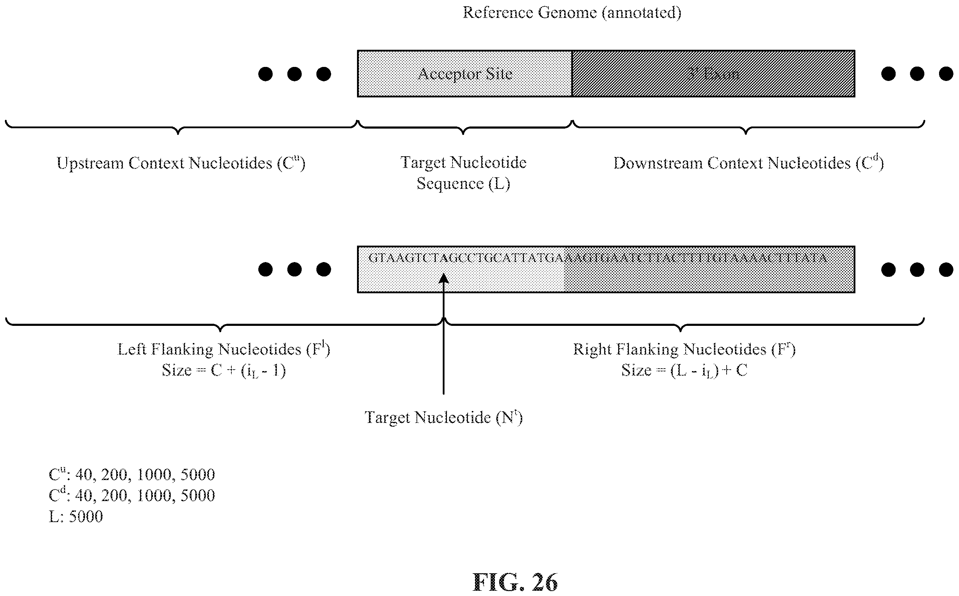

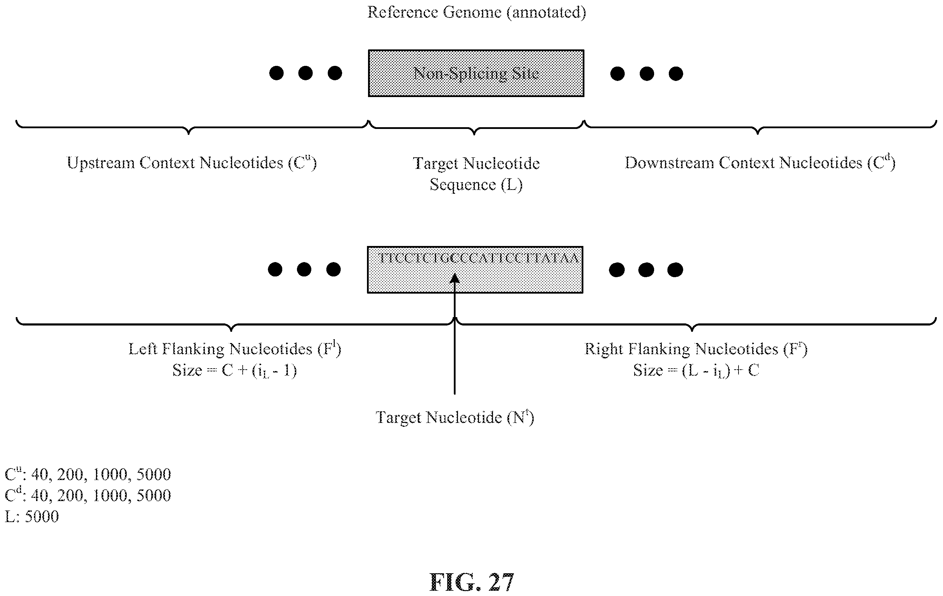

As shown in **FIGs. 25, 26,** and **27,** the input can comprise a target nucleotide sequence that has a target nucleotide flanked by 2500 nucleotides on each side. In such an implementation, the target nucleotide sequence is further flanked by 5000 upstream context nucleotides and 5000 downstream context nucleotides.

The input can comprise a target nucleotide sequence that has a target nucleotide flanked by 100 nucleotides on each side. In such an implementation, the target nucleotide sequence is further flanked by 200 upstream context nucleotides and 200 downstream context nucleotides.

The input can comprise a target nucleotide sequence that has a target nucleotide flanked by 500 nucleotides on each side. In such an implementation, the target nucleotide sequence is further flanked by 1000 upstream context nucleotides and 1000 downstream context nucleotides.

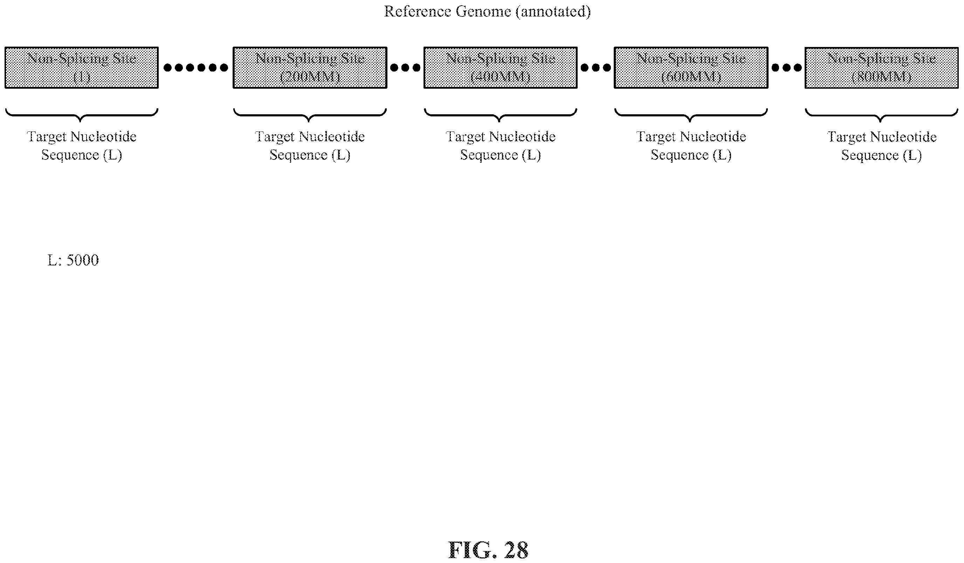

As shown in **FIG. 28,** the system can train the ACNN on 150000 training examples of donor splice sites, 150000 training examples of acceptor splice sites, and 800000000 training examples of non-splicing sites.

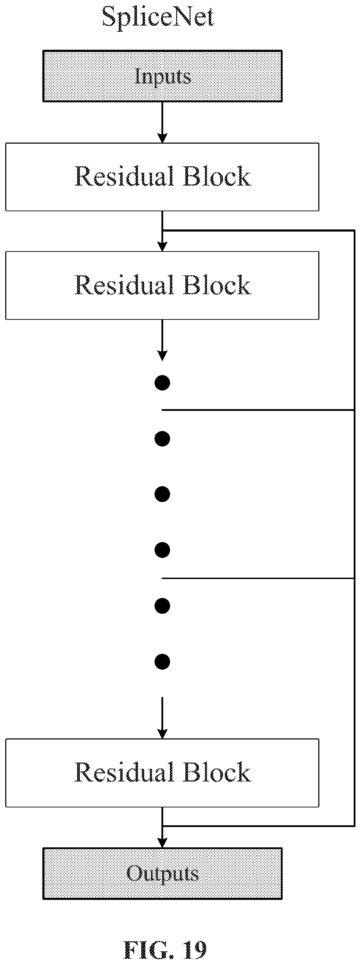

As shown in **FIG. 19,** the ACNN can comprise groups of residual blocks arranged in a sequence from lowest to highest Each group of residual blocks is parameterized by a number of convolution filters in the residual blocks, a convolution window size of the residual blocks, and an atrous convolution rate of the residual blocks.

As shown in **FIGs. 21, 22, 23,** and **24,** in the ACNN, the atrous convolution rate progresses non-exponentially from a lower residual block group to a higher residual block group.

As shown in **FIGs. 21, 22, 23,** and **24**, in the ACNN, the convolution window size varies between groups of residual blocks.

The ACNN can be configured to evaluate an input that comprises a target nucleotide sequence further flanked by 40 upstream context nucleotides and 40 downstream context nucleotides. In such an implementation, the ACNN includes one group of four residual blocks and at least one skip connection. Each residual block has 32 convolution filters, 11 convolution window size, and 1 atrous convolution rate. This implementation of the ACNN is referred to herein as "SpliceNet80" and is shown in **FIG. 21**.

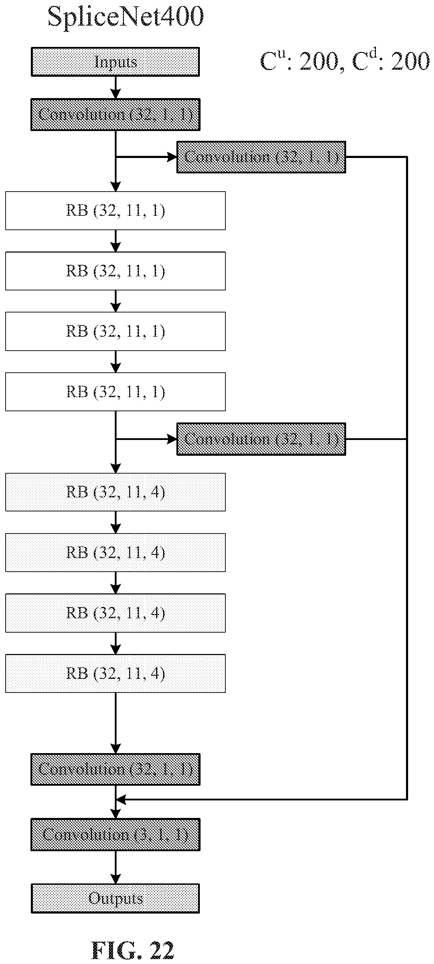

The ACNN can be configured to evaluate an input that comprises the target nucleotide sequence further flanked by 200 upstream context nucleotides and 200 downstream context nucleotides. In such an implementation, the ACNN includes at least two groups of four residual blocks and at least two skip connections. Each residual block in a first group has 32 convolution filters, 11 convolution window size, and I atrous convolution rate. Each residual block in a second group has 32 convolution filters, 11 convolution window size, and 4 atrous convolution rate. This implementation of the ACNN is referred to herein as "SpliceNet400" and is shown in **FIG. 22.**

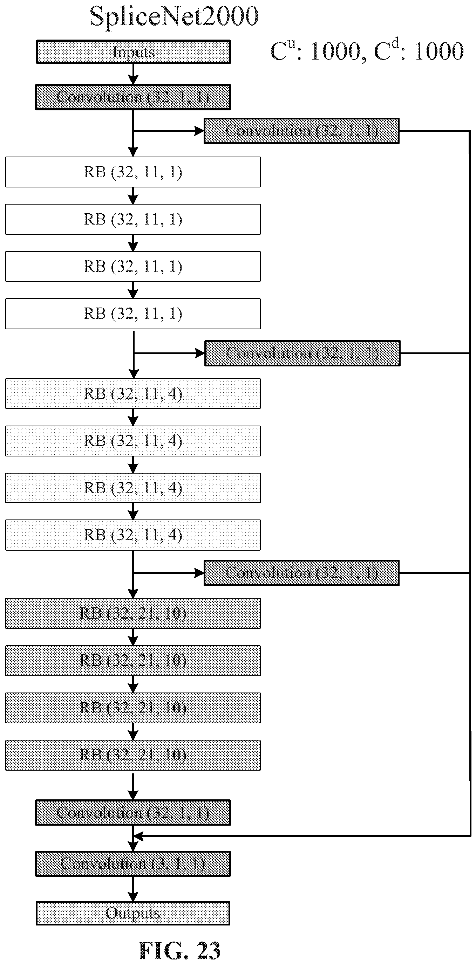

The ACNN can be configured to evaluate an input that comprises a target nucleotide sequence further flanked by 1000 upstream context nucleotides and 1000 downstream context nucleotides. In such an implementation, the ACNN includes at least three groups of four residual blocks and at least three skip connections. Each residual block in a first group has 32 convolution filters, 11 convolution window size, and 1 atrous convolution rate. Each residual block in a second group has 32 convolution filters, 11 convolution window size, and 4 atrous convolution rate. Each residual block in a third group has 32 convolution filters, 21 convolution window size, and 19 atrous convolution rate. This implementation of the ACNN is referred to herein as "SpliceNet2000" and is shown in **FIG. 23.**

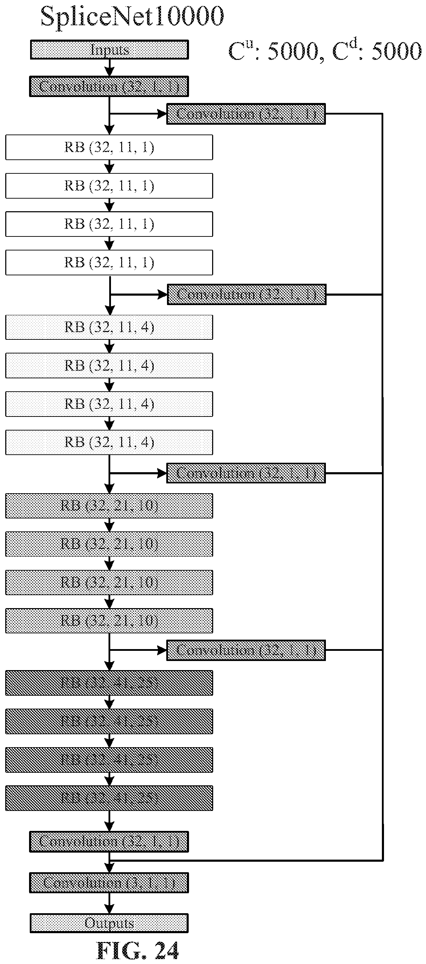

The ACNN can be configured to evaluate an input that comprises a target nucleotide sequence further flanked by 5000 upstream context nucleotides and 5000 downstream context nucleotides. In such an implementation, the ACNN includes at least four groups of four residual blocks and at least four skip connections. Each residual block in a first group has 32 convolution filters, 11 convolution window size, and 1 atrous convolution rate. Each residual block in a second group has 32 convolution filters, 11 convolution window size, and 4 atrous convolution rate. Each residual block in a third group has 32 convolution filters, 21 convolution window size, and 19 atrous convolution rate. Each residual block in a fourth group has 32 convolution filters, 41 convolution window size, and 25 atrous convolution rate. This implementation of the ACNN is referred to herein as "SpliceNet10000" and is shown in **FIG. 24.**

The triplet scores for each nucleotide in the target nucleotide sequence can be exponentially normalized to sum to unity. In such an implementation, the system classifies each nucleotide in the target nucleotide as the donor splice site, the acceptor splice site, or the non-splicing site based on a highest score in the respective triplet scores.

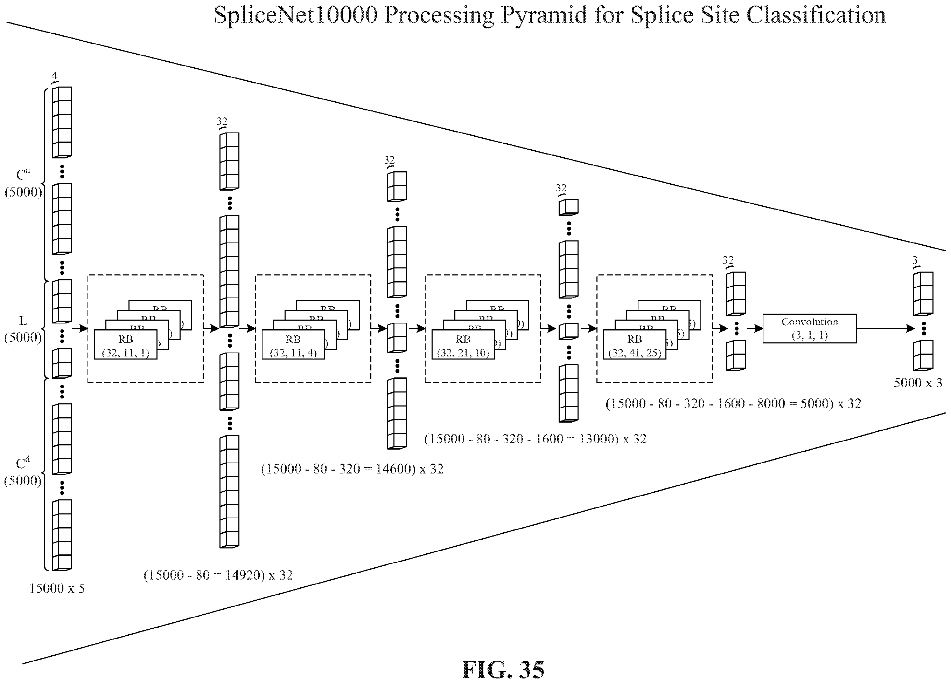

As shown in **FIG. 35,** dimensionality of the ACNN's input can be defined as $(C^u + L + C^d) \times 4$, where $C^u$ is a number of upstream context nucleotides, $C^d$ is a number of downstream context nucleotides, and L is a number of nucleotides in the target nucleotide sequence. In one implementation, the dimensionality of the input is $(5000 + 5000 + 5000) \times 4$.

As shown in **FIG. 35,** dimensionality of the ACNN's output can be defined as $L \times 3$. In one implementation, the dimensionality of the output is $5000 \times 3$.

As shown in **FIG. 35,** each group of residual blocks can produce an intermediate output by processing a preceding input. Dimensionality of the intermediate output can be defined as $(I-[\{(W-1) * D\} * A]) \times N$, where I is dimensionality of the preceding input, W is convolution window size of the residual blocks, D is atrous convolution rate of the residual blocks, A is a number of atrous convolution layers in the group, and N is a number of convolution filters in the residual blocks.

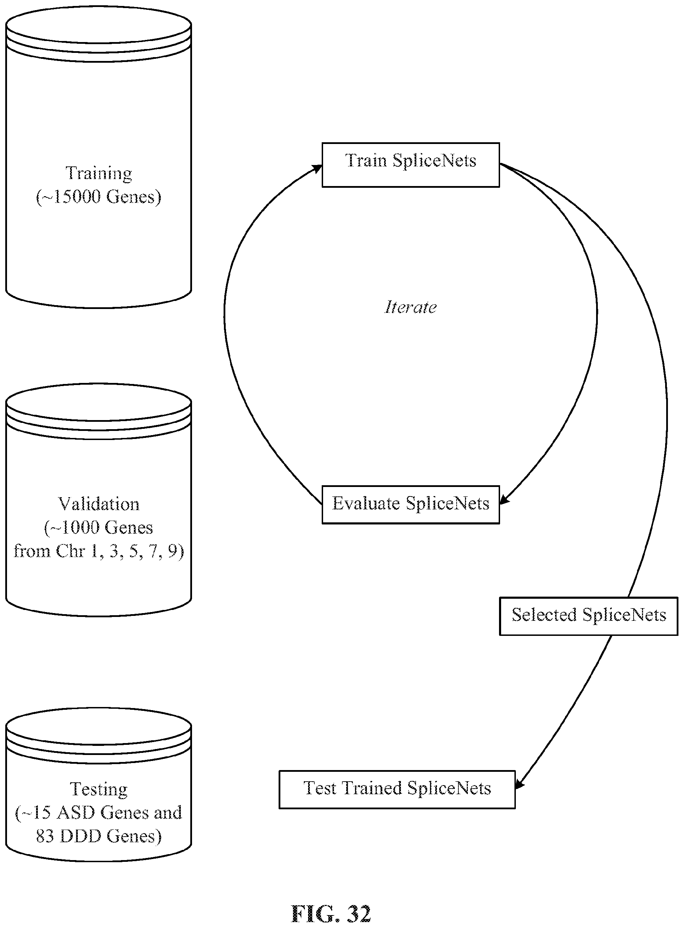

As shown in **FIG. 32,** ACNN batch-wise evaluates the training examples during an epoch. The training examples are randomly sampled into batches. Each batch has a predetermined batch size. The ACNN iterates evaluation of the training examples over a plurality of epochs (e.g., 1-10).

The input can comprise a target nucleotide sequence that has two adjacent target nucleotides. The two adjacent target nucleotides can be adenine (abbreviated A) and guanine (abbreviated G). The two adjacent target nucleotides can be guanine (abbreviated G) and uracil (abbreviated U).

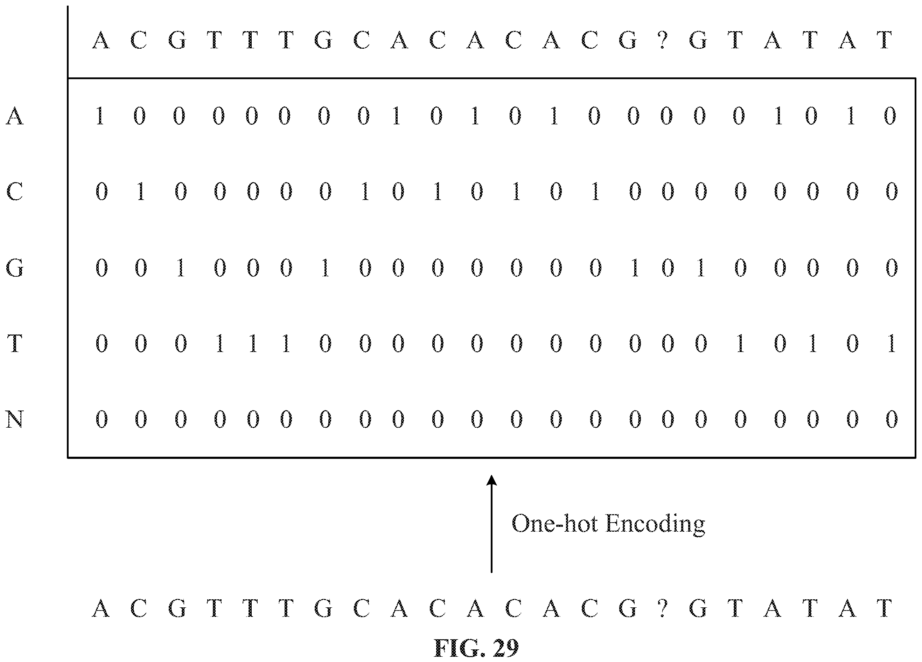

The system includes a one-hot encoder (shown in **FIG. 29**) that sparsely encodes the training examples and provides one-hot encodings as input.

The ACNN can be parameterized by a number of residual blocks, a number of skip connections, and a number of residual connections.

The ACNN can comprise dimensionality altering convolution layers that reshape spatial and feature dimensions of a preceding input.

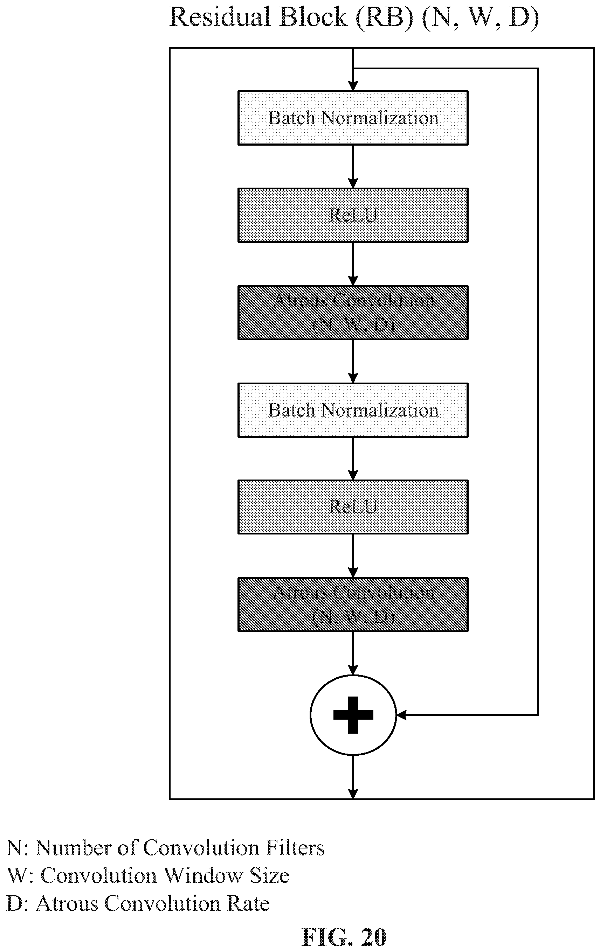

As shown in **FIG. 20,** each residual block can comprise at least one batch normalization layer, at least one rectified linear unit (abbreviated ReLU) layer, at least one atrous convolution layer, and at least one residual connection. In such an implementation, each residual block comprises two batch normalization layers, two ReLU non-linearity layers, two atrous convolution layers, and one residual connection.

Other implementations may include a non-transitory computer readable storage medium storing instructions executable by a processor to perform actions of the system described above. Yet another implementation may include a method performing actions of the system described above.

Another system implementation of the of the technology disclosed includes a trained splice site predictor that runs on numerous processors operating in parallel and coupled to memory. The system trains an atrous convolutional neural network (abbreviated ACNN), running on the numerous processors, on at least 50000 training examples of donor splice sites, at least 50000 training examples of acceptor splice sites, and at least 100000 training examples of non-splicing sites. Each of the training examples used in the training is a nucleotide sequence that includes a target nucleotide flanked by at least 400 nucleotides on each side.

The system includes an input stage of the ACNN which runs on at least one of the numerous processors and feeds an input sequence of at least 801 nucleotides for evaluation of target nucleotides. Each target nucleotide is flanked by at least 400 nucleotides on each side. In other implementations, the system includes an input module of the ACNN which runs on at least one of the numerous processors and feeds an input sequence of at least 801 nucleotides for evaluation of target nucleotides.

The system includes an output stage of the ACNN which runs on at least one of the numerous processors and translates analysis by the ACNN into classification scores for likelihood that each of the target nucleotides is a donor splice site, an acceptor splice site, or a non-splicing site. In other implementations, the system includes an output module of the ACNN which runs on at least one of the numerous processors and translates analysis by the ACNN into classification scores for likelihood that each of the target nucleotides is a donor splice site, an acceptor splice site, or a non-splicing site.

Each of the features discussed in this particular implementation section for the first system implementation apply equally to this system implementation. As indicated above, all the system features are not repeated here and should be considered repeated by reference.

The ACNN can be trained on 150000 training examples of donor splice sites, 150000 training examples of acceptor splice sites, and 800000000 training examples of non-splicing sites. In another implementation of the system, the ACNN comprises groups of residual blocks arranged in a sequence from lowest to highest. In yet another implementation of the system, each group of residual blocks is parameterized by a number of convolution filters in the residual blocks, a convolution window size of the residual blocks, and an atrous convolution rate of the residual blocks.

The ACNN can comprise groups of residual blocks arranged in a sequence from lowest to highest. Each group of residual blocks is parameterized by a number of convolution filters in the residual blocks, a convolution window size of the residual blocks, and an atrous convolution rate of the residual blocks.

In the ACNN, the atrous convolution rate progresses non-exponentially from a lower residual block group to a higher residual block group. Also in the ACNN, the convolution window size varies between groups of residual blocks.

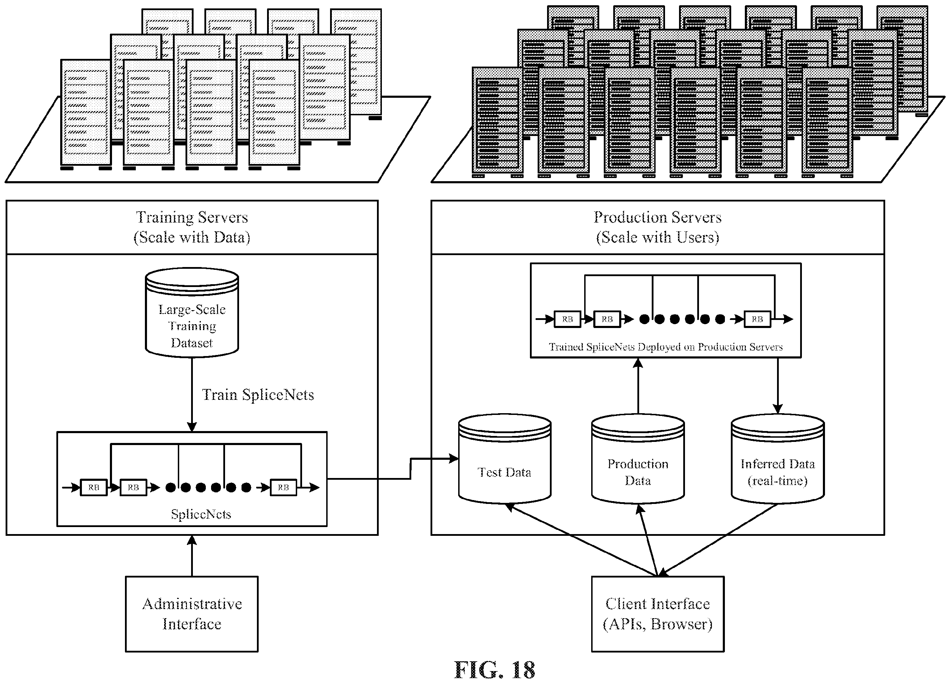

The ACNN can be trained on one or more training servers, as shown in **FIG. 18.**

The trained ACNN can be deployed on one or more production servers that receive input sequences from requesting clients, as shown in **FIG. 18.** In such an implementation, the production servers process the input sequences through the input and output stages of the ACNN to produce outputs that are transmitted to the clients, as shown in **FIG. 18.** In other implementations, the production servers process the input sequences through the input and output modules of the ACNN to produce outputs that are transmitted to the clients, as shown in **FIG. 18.**

Other implementations may include a non-transitory computer readable storage medium storing instructions executable by a processor to perform actions of the system described above. Yet another implementation may include a method performing actions of the system described above.

A method implementation of the technology disclosed includes training a splice site detector that identifies splice sites in genomic sequences (e.g., nucleotide sequences).

The method includes feeding, an atrous convolutional neural network (abbreviated ACNN), an input sequence of at least 801 nucleotides for evaluation of target nucleotides that are each flanked by at least 400 nucleotides on each side.

The ACNN is trained on at least 50000 training examples of donor splice sites, at least 50000 training examples of acceptor splice sites, and at least 100000 training examples of non-splicing sites. Each of the training examples used in the training is a nucleotide sequence that includes a target nucleotide flanked by at least 400 nucleotides on each side.

The method further includes translating analysis by the ACNN into classification scores for likelihood that each of the target nucleotides is a donor splice site, an acceptor splice site, or a non-splicing site.

Each of the features discussed in this particular implementation section for the first system implementation apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

We describe systems, methods, and articles of manufacture for using a trained atrous convolutional neural network to detect aberrant splicing in genomic sequences (e.g., nucleotide sequences). One or more features of an implementation can be combined with the base implementation. Implementations that are not mutually exclusive are taught to be combinable. One or more features of an implementation can be combined with other implementations. This disclosure periodically reminds the user of these options.

A system implementation of the technology disclosed includes one or more processors coupled to the memory. The memory is loaded with computer instructions to implement an aberrant splicing detector running on numerous processors operating in parallel and coupled to memory.

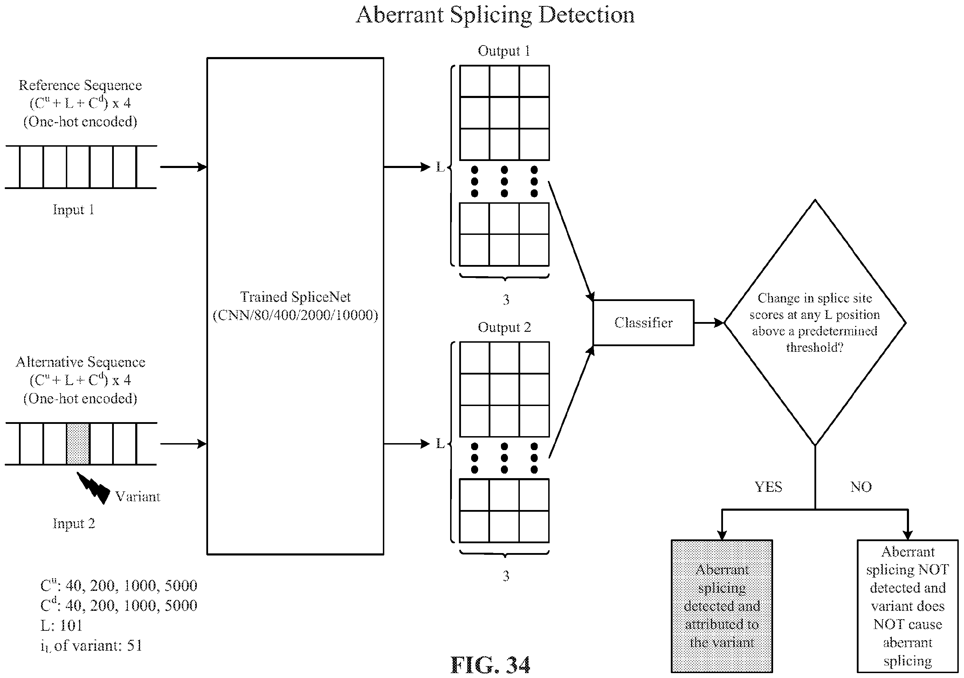

As shown in **FIG. 34,** the system includes a trained atrous convolutional neural network (abbreviated ACNN) running on the numerous processors. An ACNN is a convolutional neural network that uses atrous/dilated convolutions which allow for large receptive fields with few trainable parameters. An atrous/dilated convolution is a convolution where the kernel is applied over an area larger than its length by skipping input values with a certain step, also called atrous convolution rate or dilation factor. Atrous/dilated convolutions add spacing between the elements of a convolution filter/kernel so that neighboring input entries (e.g., nucleotides, amino acids) at larger intervals are considered when a convolution operation is performed. This enables incorporation of long-range contextual dependencies in the input. The atrous convolutions conserve partial convolution calculations for reuse as adjacent nucleotides are processed.

As shown in **FIG. 34,** the ACNN classifies target nucleotides in an input sequence and assigns splice site scores for likelihood that each of the target nucleotides is a donor splice site, an acceptor splice site, or a non-splicing site. The input sequence comprises at least 801 nucleotides and each target nucleotide is flanked by at least 400 nucleotides on each side.

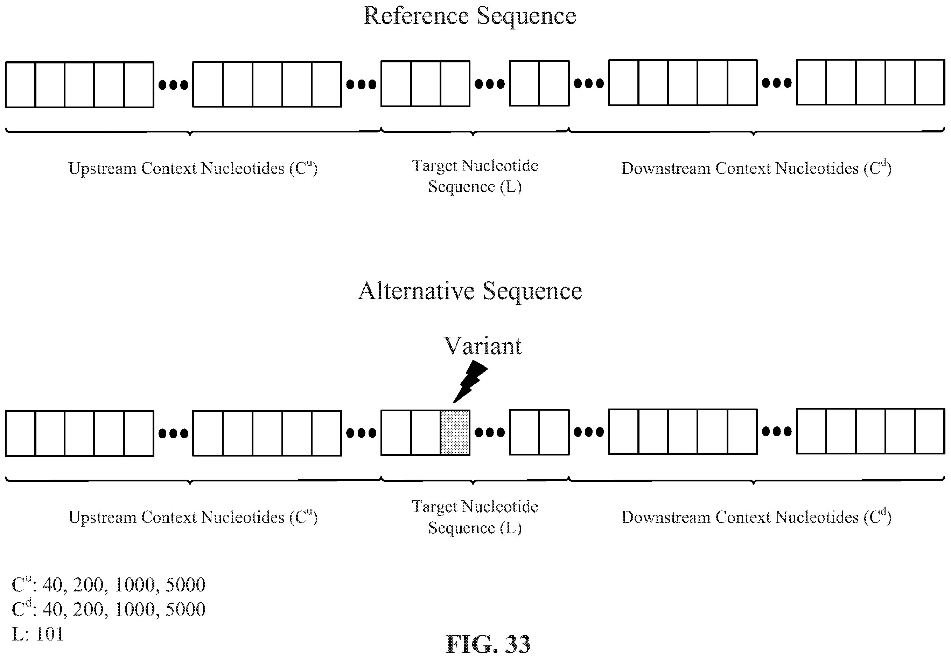

As shown in **FIG. 34,** the system also includes a classifier, running on at least one of the numerous processors, that processes a reference sequence and a variant sequence through the ACNN to produce splice site scores for a likelihood that each target nucleotide in the reference sequence and in the variant sequence is a donor splice site, an acceptor splice site, or a non-splicing site. The reference sequence and the variant sequence each have at least 101 target nucleotides and each target nucleotide is flanked by at least 400 nucleotides on each side. **FIG. 33** depicts a reference sequence and an alternative/variant sequence.

As shown in **FIG. 34,** the system then determines, from differences in the splice site scores of the target nucleotides in the reference sequence and in the variant sequence, whether a variant that generated the variant sequence causes aberrant splicing and is therefore pathogenic.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

As shown in **FIG. 34,** the differences in the splice site scores can be determined position-wise between the target nucleotides in the reference sequence and in the variant sequence.

As shown in **FIG. 34,** for at least one target nucleotide position, when a global maximum difference in the splice site scores is above a predetermined threshold, the ACNN classifies the variant as causing aberrant splicing and therefore pathogenic.

As shown in **FIG. 17,** for at least one target nucleotide position, when a global maximum difference in the splice site scores is below a predetermined threshold, the ACNN classifies the variant as not causing aberrant splicing and therefore benign.

The threshold can be determined from for a plurality of candidate thresholds. This includes processing a first set of reference and variant sequence pairs generated by benign common variants to produce a first set of aberrant splicing detections, processing a second set of reference and variant sequence pairs generated by pathogenic rare variants to produce a second set of aberrant splicing detections, and selecting at least one threshold, for use by the classifier, that maximizes a count of aberrant splicing detections in the second set and minimizes a count of aberrant splicing detections in the first set.

In one implementation, the ACNN identifies variants that cause autism spectrum disorder (abbreviated ASD). In another implementation, the ACNN identifies variants that cause developmental delay disorder (abbreviated DDD).

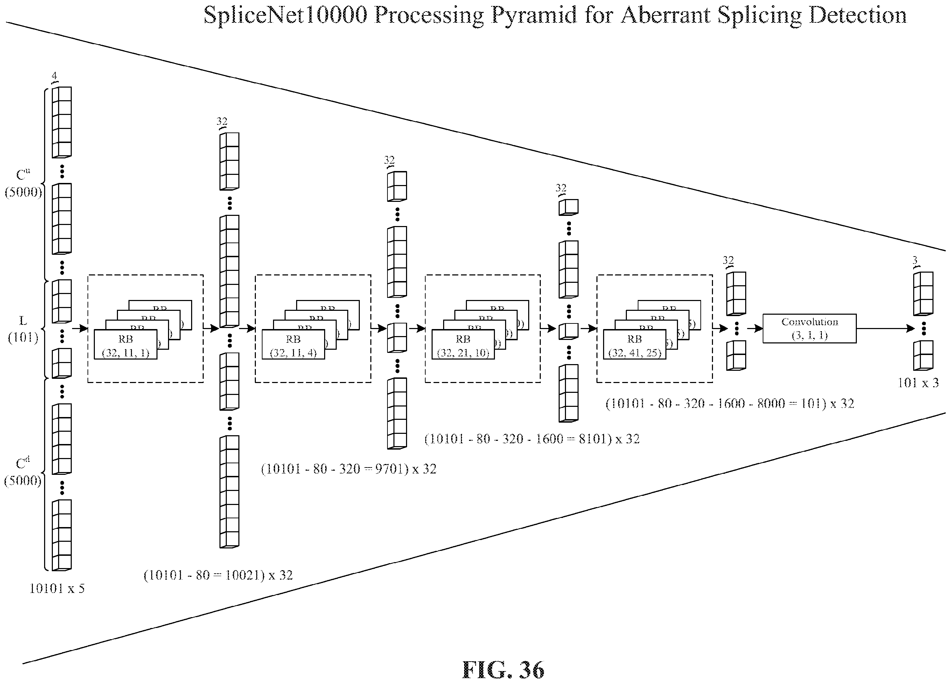

As shown in **FIG. 36,** the reference sequence and the variant sequence can each have at least 101 target nucleotides and each target nucleotide can be flanked by at least 5000 nucleotides on each side.

As shown in **FIG. 36,** the splice site scores of the target nucleotides in the reference sequence can be encoded in a first output of the ACNN and the splice site scores of the target nucleotides in the variant sequence can be encoded in a second output of the ACNN. In one implementation, the first output is encoded as a first $101 \times 3$ matrix and the second output is encoded as a second $101 \times 3$ matrix.

As shown in **FIG. 36,** in such an implementation, each row in the first $101 \times 3$ matrix uniquely represents splice site scores for a likelihood that a target nucleotide in the reference sequence is a donor splice site, an acceptor splice site, or a non-splicing site.

As shown in **FIG. 36,** also in such an implementation, each row in the second $101 \times 3$ matrix uniquely represents splice site scores for a likelihood that a target nucleotide in the variant sequence is a donor splice site, an acceptor splice site, or a non-splicing site.

As shown in **FIG. 36,** in some implementations, splice site scores in each row of the first $101 \times 3$ matrix and the second $101 \times 3$ matrix can be exponentially normalized to sum to unity.

As shown in **FIG. 36,** the classifier can perform a row-to-row comparison of the first $101 \times 3$ matrix and the second $101 \times 3$ matrix and determine, on a row-wise basis, changes in distribution of splice site scores. For at least one instance of the row-to-row comparison, when the change in distribution is above a predetermined threshold, the ACNN classifies the variant as causing aberrant splicing and therefore pathogenic.

The system includes a one-hot encoder (shown in **FIG. 29**) that sparsely encodes the reference sequence and the variant sequence.

Each of the features discussed in this particular implementation section for other system and method implementations apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a method performing actions of the system described above.

A method implementation of the technology disclosed includes detecting genomic variants that cause aberrant splicing.

The method includes processing a reference sequence through an atrous convolutional neural network (abbreviated ACNN) trained to detect differential splicing patterns in a target sub-sequence of an input sequence by classifying each nucleotide in the target sub-sequence as a donor splice site, an acceptor splice site, or a non-splicing site.

The method includes, based on the processing, detecting a first differential splicing pattern in a reference target sub-sequence by classifying each nucleotide in the reference target sub-sequence as a donor splice site, an acceptor splice site, or a non-splicing site.

The method includes processing a variant sequence through the ACNN. The variant sequence and the reference sequence differ by at least one variant nucleotide located in a variant target sub-sequence.

The method includes, based on the processing, detecting a second differential splicing pattern in the variant target sub-sequence by classifying each nucleotide in the variant target sub-sequence as a donor splice site, an acceptor splice site, or a non-splicing site.

The method includes determining a difference between the first differential splicing pattern and the second differential splicing pattern by comparing, on a nucleotide-by-nucleotide basis, splice site classifications of the reference target sub-sequence and the variant target sub-sequence.

When the difference is above a predetermined threshold, the method includes classifying the variant as causing aberrant splicing and therefore pathogenic and storing the classification in memory.

Each of the features discussed in this particular implementation section for other system and method implementations apply equally to this method implementation. As indicated above, all the system features are not repeated here and should be considered repeated by reference.

A differential splicing pattern can identify positional distribution of occurrence of splicing events in a target sub-sequence. Examples of splicing events include at least one of cryptic splicing, exon skipping, mutually exclusive exons, alternative donor site, alternative acceptor site, and intron retention.

The reference target sub-sequence and the variant target sub-sequence can be aligned with respect to nucleotide positions and can differ by the at least one variant nucleotide.

The reference target sub-sequence and the variant target sub-sequence can each have at least 40 nucleotides and can each be flanked by at least 40 nucleotides on each side.

The reference target sub-sequence and the variant target sub-sequence can each have at least 101 nucleotides and can each be flanked by at least 5000 nucleotides on each side.

The variant target sub-sequence can include two variants.

Other implementations may include a non-transitory computer readable storage medium storing instructions executable by a processor to perform the method described above. Yet another implementation may include a system including memory and one or more processors operable to execute instructions, stored in the memory, to perform the method described above.

We describe systems, methods, and articles of manufacture for using a trained convolutional neural network to detect splice sites and aberrant splicing in genomic sequences (e.g., nucleotide sequences). One or more features of an implementation can be combined with the base implementation. Implementations that are not mutually exclusive are taught to be combinable. One or more features of an implementation can be combined with other implementations. This disclosure periodically reminds the user of these options.

A system implementation of the technology disclosed includes one or more processors coupled to the memory. The memory is loaded with computer instructions to train a splice site detector that identifies splice sites in genomic sequences (e.g., nucleotide sequences).

The system trains a convolutional neural network (abbreviated CNN) on at least 50000 training examples of donor splice sites, at least 50000 training examples of acceptor splice sites, and at least 100000 training examples of non-splicing sites. Each training example is a target nucleotide sequence having at least one target nucleotide flanked by at least 20 nucleotides on each side.

For evaluating a training example using the CNN, the system provides, as input to the CNN, a target nucleotide sequence further flanked by at least 40 upstream context nucleotides and at least 40 downstream context nucleotides.

Based on the evaluation, the CNN then produces, as output, triplet scores for likelihood that each nucleotide in the target nucleotide sequence is a donor splice site, an acceptor splice site, or a non-splicing site.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

The input can comprise a target nucleotide sequence that has a target nucleotide flanked by 100 nucleotides on each side. In such an implementation, the target nucleotide sequence is further flanked by 200 upstream context nucleotides and 200 downstream context nucleotides.

As shown in **FIG. 28,** the system can train the CNN on 150000 training examples of donor splice sites, 150000 training examples of acceptor splice sites, and 1000000 training examples of non-splicing sites.

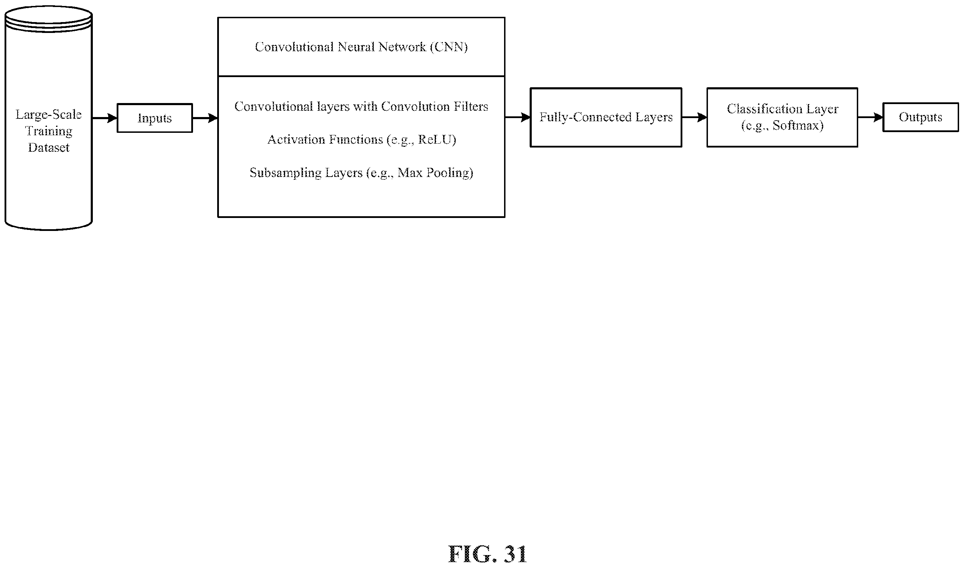

As shown in **FIG. 31,** the CNN can be parameterized by a number of convolution layers, a number of convolution filters, and a number of subsampling layers (e.g., max pooling and average pooling).

As shown in **FIG. 31,** the CNN can include one or more fully-connected layers and a terminal classification layer.

The CNN can comprise dimensionality altering convolution layers that reshape spatial and feature dimensions of a preceding input.

The triplet scores for each nucleotide in the target nucleotide sequence can be exponentially normalized to sum to unity. In such an implementation, the system classifies each nucleotide in the target nucleotide as the donor splice site, the acceptor splice site, or the non-splicing site based on a highest score in the respective triplet scores.

As shown in **FIG. 32,** CNN batch-wise evaluates the training examples during an epoch. The training examples are randomly sampled into batches. Each batch has a predetermined batch size. The CNN iterates evaluation of the training examples over a plurality of epochs (e.g., 1-10).

The input can comprise a target nucleotide sequence that has two adjacent target nucleotides. The two adjacent target nucleotides can be adenine (abbreviated A) and guanine (abbreviated G). The two adjacent target nucleotides can be guanine (abbreviated G) and uracil (abbreviated U).

The system includes a one-hot encoder (shown in **FIG. 32**) that sparsely encodes the training examples and provides one-hot encodings as input.

The CNN can be parameterized by a number of residual blocks, a number of skip connections, and a number of residual connections.

Each residual block can comprise at least one batch normalization layer, at least one rectified linear unit (abbreviated ReLU) layer, at least one dimensionality altering layer, and at least one residual connection. Each residual block can comprise two batch normalization layers, two ReLU non-linearity layers, two dimensionality altering layers, and one residual connection.

Each of the features discussed in this particular implementation section for other system and method implementations apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a method performing actions of the system described above.

Another system implementation of the of the technology disclosed includes a trained splice site predictor that runs on numerous processors operating in parallel and coupled to memory. The system trains a convolutional neural network (abbreviated CNN), running on the numerous processors, on at least 50000 training examples of donor splice sites, at least 50000 training examples of acceptor splice sites, and at least 100000 training examples of non-splicing sites. Each of the training examples used in the training is a nucleotide sequence that includes a target nucleotide flanked by at least 400 nucleotides on each side.

The system includes an input stage of the CNN which runs on at least one of the numerous processors and feeds an input sequence of at least 801 nucleotides for evaluation of target nucleotides. Each target nucleotide is flanked by at least 400 nucleotides on each side. In other implementations, the system includes an input module of the CNN which runs on at least one of the numerous processors and feeds an input sequence of at least 801 nucleotides for evaluation of target nucleotides.

The system includes an output stage of the CNN which runs on at least one of the numerous processors and translates analysis by the CNN into classification scores for likelihood that each of the target nucleotides is a donor splice site, an acceptor splice site, or a non-splicing site. In other implementations, the system includes an output module of the CNN which runs on at least one of the numerous processors and translates analysis by the CNN into classification scores for likelihood that each of the target nucleotides is a donor splice site, an acceptor splice site, or a non-splicing site.

Each of the features discussed in this particular implementation section for other system and method implementations apply equally to this system implementation. As indicated above, all the system features are not repeated here and should be considered repeated by reference.

The CNN can be trained on 150000 training examples of donor splice sites, 150000 training examples of acceptor splice sites, and 800000000 training examples of non-splicing sites.

The CNN can be trained on one or more training servers.

The trained CNN can be deployed on one or more production servers that receive input sequences from requesting clients. In such an implementation, the production servers process the input sequences through the input and output stages of the CNN to produce outputs that are transmitted to the clients. In other implementations, the production servers process the input sequences through the input and output outputs of the CNN to produce outputs that are transmitted to the clients.

Other implementations may include a non-transitory computer readable storage medium storing instructions executable by a processor to perform actions of the system described above. Yet another implementation may include a method performing actions of the system described above.

A method implementation of the technology disclosed includes training a splice site detector that identifies splice sites in genomic sequences (e.g., nucleotide sequences). The method includes feeding, a convolutional neural network (abbreviated CNN), an input sequence of at least 801 nucleotides for evaluation of target nucleotides that are each flanked by at least 400 nucleotides on each side.

The CNN is trained on at least 50000 training examples of donor splice sites, at least 50000 training examples of acceptor splice sites, and at least 100000 training examples of non-splicing sites. Each of the training examples used in the training is a nucleotide sequence that includes a target nucleotide flanked by at least 400 nucleotides on each side.

The method further includes translating analysis by the CNN into classification scores for likelihood that each of the target nucleotides is a donor splice site, an acceptor splice site, or a non-splicing site.

Each of the features discussed in this particular implementation section for other system and method implementations apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

Yet another system implementation of the technology disclosed includes one or more processors coupled to the memory. The memory is loaded with computer instructions to implement an aberrant splicing detector running on numerous processors operating in parallel and coupled to memory.

The system includes a trained convolutional neural network (abbreviated CNN) running on the numerous processors.

As shown in **FIG. 34,** the CNN classifies target nucleotides in an input sequence and assigns splice site scores for likelihood that each of the target nucleotides is a donor splice site, an acceptor splice site, or a non-splicing site. The input sequence comprises at least 801 nucleotides and each target nucleotide is flanked by at least 400 nucleotides on each side.

As shown in **FIG. 34,** the system also includes a classifier, running on at least one of the numerous processors, that processes a reference sequence and a variant sequence through the CNN to produce splice site scores for a likelihood that each target nucleotide in the reference sequence and in the variant sequence is a donor splice site, an acceptor splice site, or a non-splicing site. The reference sequence and the variant sequence each have at least 101 target nucleotides and each target nucleotide is flanked by at least 400 nucleotides on each side.

As shown in **FIG. 34,** the system then determines, from differences in the splice site scores of the target nucleotides in the reference sequence and in the variant sequence, whether a variant that generated the variant sequence causes aberrant splicing and is therefore pathogenic.

Each of the features discussed in this particular implementation section for other system and method implementations apply equally to this system implementation. As indicated above, all the system features are not repeated here and should be considered repeated by reference.

The differences in the splice site scores can be determined position-wise between the target nucleotides in the reference sequence and in the variant sequence.

For at least one target nucleotide position, when a global maximum difference in the splice site scores is above a predetermined threshold, the CNN classifies the variant as causing aberrant splicing and therefore pathogenic.

For at least one target nucleotide position, when a global maximum difference in the splice site scores is below a predetermined threshold, the CNN classifies the variant as not causing aberrant splicing and therefore benign.

The threshold can be determined from for a plurality of candidate thresholds. This includes processing a first set of reference and variant sequence pairs generated by benign common variants to produce a first set of aberrant splicing detections, processing a second set of reference and variant sequence pairs generated by pathogenic rare variants to produce a second set of aberrant splicing detections, and selecting at least one threshold, for use by the classifier, that maximizes a count of aberrant splicing detections in the second set and minimizes a count of aberrant splicing detections in the first set.

In one implementation, the CNN identifies variants that cause autism spectrum disorder (abbreviated ASD). In another implementation, the CNN identifies variants that cause developmental delay disorder (abbreviated DDD).

The reference sequence and the variant sequence can each have at least 101 target nucleotides and each target nucleotide can be flanked by at least 1000 nucleotides on each side.

The splice site scores of the target nucleotides in the reference sequence can be encoded in a first output of the CNN and the splice site scores of the target nucleotides in the variant sequence can be encoded in a second output of the CNN. In one implementation, the first output is encoded as a first $101 \times 3$ matrix and the second output is encoded as a second $101 \times 3$ matrix.

In such an implementation, each row in the first $101 \times 3$ matrix uniquely represents splice site scores for a likelihood that a target nucleotide in the reference sequence is a donor splice site, an acceptor splice site, or a non-splicing site.

Also in such an implementation, each row in the second $101 \times 3$ matrix uniquely represents splice site scores for a likelihood that a target nucleotide in the variant sequence is a donor splice site, an acceptor splice site, or a non-splicing site.

In some implementations, splice site scores in each row of the first $101 \times 3$ matrix and the second $101 \times 3$ matrix can be exponentially normalized to sum to unity.

The classifier can perform a row-to-row comparison of the first $101 \times 3$ matrix and the second $101 \times 3$ matrix and determine, on a row-wise basis, changes in distribution of splice site scores. For at least one instance of the row-to-row comparison, when the change in distribution is above a predetermined threshold, the CNN classifies the variant as causing aberrant splicing and therefore pathogenic.

The system includes a one-hot encoder (shown in **FIG. 29**) that sparsely encodes the reference sequence and the variant sequence.

Other implementations may include a non-transitory computer readable storage medium storing instructions executable by a processor to perform actions of the system described above. Yet another implementation may include a method performing actions of the system described above.

A method implementation of the technology disclosed includes detecting genomic variants that cause aberrant splicing.

The method includes processing a reference sequence through an atrous convolutional neural network (abbreviated CNN) trained to detect differential splicing patterns in a target sub-sequence of an input sequence by classifying each nucleotide in the target sub-sequence as a donor splice site, an acceptor splice site, or a non-splicing site.

The method includes, based on the processing, detecting a first differential splicing pattern in a reference target sub-sequence by classifying each nucleotide in the reference target sub-sequence as a donor splice site, an acceptor splice site, or a non-splicing site.

The method includes processing a variant sequence through the CNN. The variant sequence and the reference sequence differ by at least one variant nucleotide located in a variant target sub-sequence.

The method includes, based on the processing, detecting a second differential splicing pattern in the variant target sub-sequence by classifying each nucleotide in the variant target sub-sequence as a donor splice site, an acceptor splice site, or a non-splicing site.

The method includes determining a difference between the first differential splicing pattern and the second differential splicing pattern by comparing, on a nucleotide-by-nucleotide basis, splice site classifications of the reference target sub-sequence and the variant target sub-sequence.

When the difference is above a predetermined threshold, the method includes classifying the variant as causing aberrant splicing and therefore pathogenic and storing the classification in memory.

Each of the features discussed in this particular implementation section for other system and method implementations apply equally to this method implementation. As indicated above, all the system features are not repeated here and should be considered repeated by reference.

A differential splicing pattern can identify positional distribution of occurrence of splicing events in a target sub-sequence. Examples of splicing events include at least one of cryptic splicing, exon skipping, mutually exclusive exons, alternative donor site, alternative acceptor site, and intron retention.

The reference target sub-sequence and the variant target sub-sequence can be aligned with respect to nucleotide positions and can differ by the at least one variant nucleotide.

The reference target sub-sequence and the variant target sub-sequence can each have at least 40 nucleotides and can each be flanked by at least 40 nucleotides on each side.

The reference target sub-sequence and the variant target sub-sequence can each have at least 101 nucleotides and can each be flanked by at least 1000 nucleotides on each side.

The variant target sub-sequence can include two variants.

Other implementations may include a non-transitory computer readable storage medium storing instructions executable by a processor to perform the method described above. Yet another implementation may include a system including memory and one or more processors operable to execute instructions, stored in the memory, to perform the method described above.

## Per-Gene Enrichment Analysis

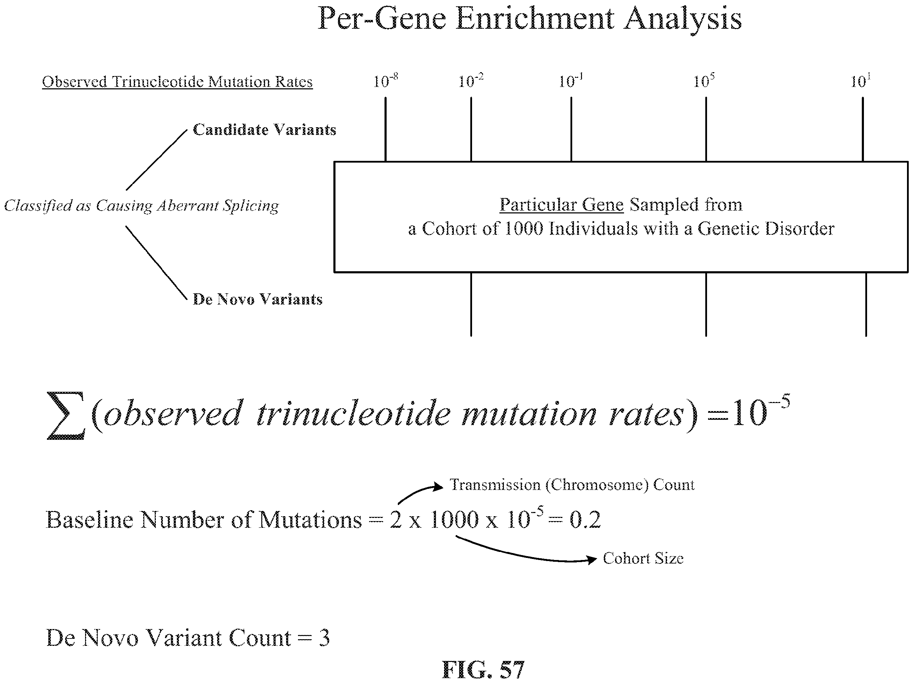

**FIG. 57** shows one implementation of per-gene enrichment analysis. In one implementation, the aberrant splicing detector is further configured to implement a per-gene enrichment analysis which determines pathogenicity of variants that have been determined to cause aberrant splicing. For a particular gene sampled from a cohort of individuals with a genetic disorder, the per-gene enrichment analysis includes applying the trained ACNN to identify candidate variants in the particular gene that cause aberrant splicing, determining a baseline number of mutations for the particular gene based on summing observed trinucleotide mutation rates of the candidate variants and multiplying the sum with a transmission count and a size of the cohort, applying the trained ACNN to identify de novo variants in the particular gene that cause aberrant splicing, and comparing the baseline number of mutations with a count of the de novo variants. Based on an output of the comparison, the per-gene enrichment analysis determines that the particular gene is associated with the genetic disorder and that the de novo variants are pathogenic. In some implementations, the genetic disorder is autism spectrum disorder (abbreviated ASD). In other implementations, the genetic disorder is developmental delay disorder (abbreviated DDD).

In the example shown in **FIG. 57,** five candidate variants in a particular gene have been classified as causing aberrant splicing by the aberrant splicing detector. These five candidate variants have respective observed trinucleotide mutation rates of $10^{-8}$, $10^{-2}$, $10^{-1}$, $10^{5}$, and $10^{1}$. The baseline number of mutations for the particular gene is determined to be $10^{-5}$ based on summing the respective observed trinucleotide mutation rates of the five candidate variants and multiplying the sum with a transmission/chromosome count (2) and a size of the cohort (1000). This is then compared with the *de novo* variant count (3).

In some implementations, the aberrant splicing detector is further configured to perform the comparison using a statistical test that produces a p-value as the output.

In other implementations, the aberrant splicing detector is further configured to compare the baseline number of mutations with the count of the de novo variants, and based on the output of the comparison determine that the particular gene is not associated with the genetic disorder and that the de novo variants are benign.

In one implementation, at least some of the candidate variants are protein-truncating variants.

In another implementation, at least some of the candidate variants are missense variants.

## Genome-Wide Enrichment Analysis

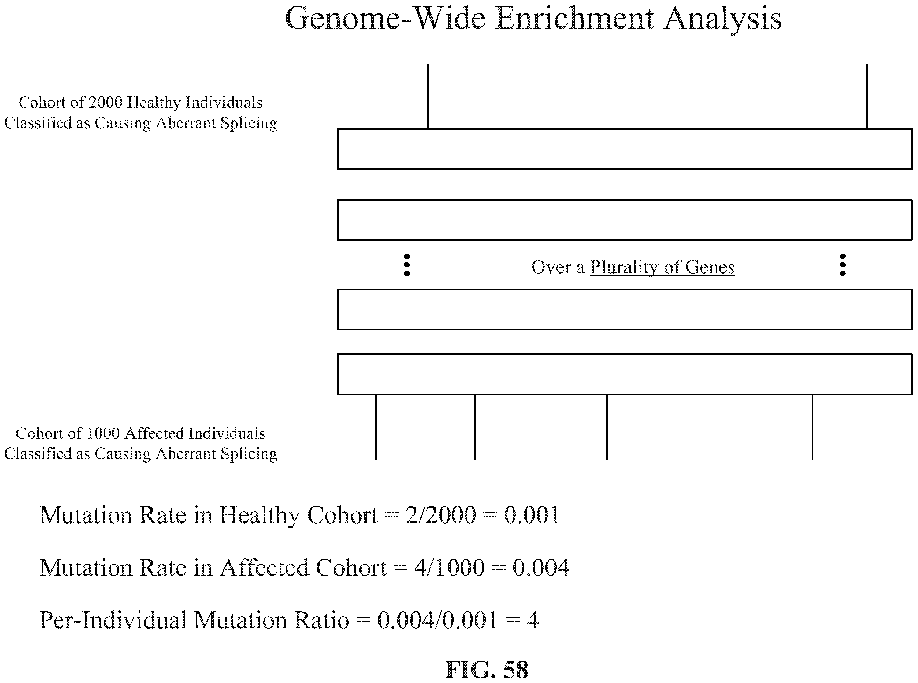

**FIG. 58** shows one implementation of genome-wide enrichment analysis. In another implementation, the aberrant splicing detector is further configured to implement a genome-wide enrichment analysis which determines pathogenicity of variants that have been determined to cause aberrant splicing. The genome-wide enrichment analysis includes applying the trained ACNN to identify a first set of de novo variants that cause aberrant splicing in a plurality of genes sampled from a cohort of healthy individuals, applying the trained ACNN to identify a second set of de novo variants that cause aberrant splicing in the plurality of genes sampled from a cohort of individuals with a genetic disorder, and comparing respective counts of the first and second sets, and based on an output of the comparison determining that the second set of de novo variants are enriched in the cohort of individuals with the genetic disorder and therefore pathogenic. In some implementations, the genetic disorder is autism spectrum disorder (abbreviated ASD). In other implementations, the genetic disorder is developmental delay disorder (abbreviated DDD).

In some implementations, the aberrant splicing detector is further configured to perform the comparison using a statistical test that produces a p-value as the output. In one implementation, the comparison can be further parametrized by respective cohort sizes.

In some implementations, the aberrant splicing detector is further configured to compare the respective counts of the first and second sets, and based on the output of the comparison determining that the second set of de novo variants are not enriched in the cohort of individuals with the genetic disorder and therefore benign.

In the example shown in **FIG. 58,** mutation rate in the healthy cohort (0.001) and mutation rate in affected cohort (0.004) is illustrated, along with per-individual mutation ration (4).

## Discussion

Despite the limited diagnostic yield of exome sequencing in patients with severe genetic disorders, clinical sequencing has focused on rare coding mutations, largely disregarding variation in the noncoding genome due to the difficulty of interpretation. Here we introduce a deep learning network that accurately predicts splicing from primary nucleotide sequence, thereby identifying noncoding mutations that disrupt the normal patterning of exons and introns with severe consequences on the resulting protein. We show that predicted cryptic splice mutations validate at a high rate by RNA-seq, are strongly deleterious in the human population, and are a major cause of rare genetic disease.

By using the deep learning network as an *in silico* model of the spliceosome, we were able to reconstruct the specificity determinants that enable the spliceosome to achieve its remarkable precision *in vivo.* We reaffirm many of the discoveries that were made over the past four decades of research into splicing mechanisms and show that the spliceosome integrates a large number of short- and long-range specificity determinants in its decisions. In particular, we find that the perceived degeneracy of most splice motifs is explained by the presence of long-range determinants such as exon/intron lengths and nucleosome positioning, which more than compensate and render additional specificity at the motif level unnecessary. Our findings demonstrate the promise of deep learning models for providing biological insights, rather than merely serving as black box classifiers.

Deep learning is a relatively new technique in biology, and is not without potential trade-offs. By learning to automatically extract features from sequence, deep learning models can utilize novel sequence determinants not well-described by human experts, but there is also the risk that the model may incorporate features that do not reflect the true behavior of the spliceosome. These irrelevant features could increase the apparent accuracy of predicting annotated exon-intron boundaries, but would reduce the accuracy of predicting the splice-altering effects of arbitrary sequence changes induced by genetic variation. Because accurate prediction of variants provides the strongest evidence that the model can generalize to true biology, we provide validation of predicted splice-altering variants using three fully orthogonal methods: RNA-seq, natural selection in human populations, and enrichment of *de novo* variants in case vs control cohorts. While this does not fully preclude the incorporation of irrelevant features into the model, the resulting model appears faithful enough to the true biology of splicing to be of significant value for practical applications such as identifying cryptic splice mutations in patients with genetic diseases.

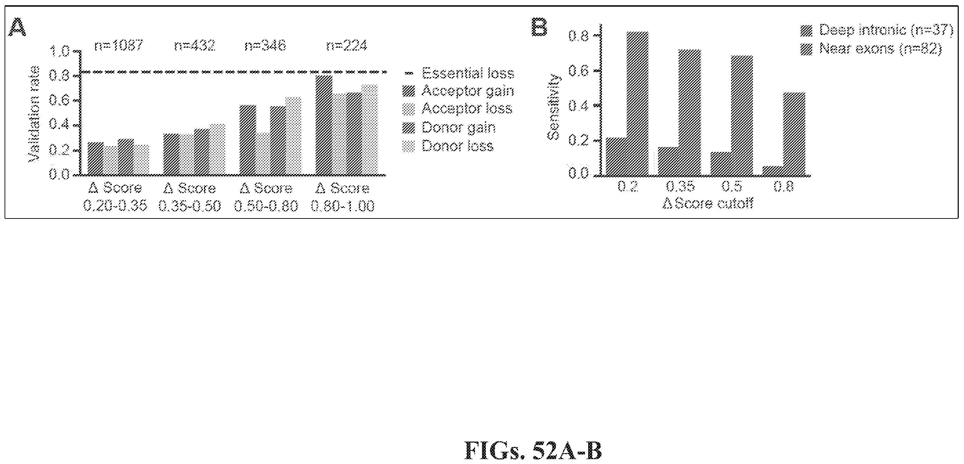

Compared to other classes of protein-truncating mutations, a particularly interesting aspect of cryptic splice mutations is the widespread phenomenon of alternative splicing due to incompletely penetrant splice-altering variants, which tend to weaken canonical splice sites relative to alternative splice sites, resulting in the production of a mixture of both aberrant and normal transcripts in the RNA-seq data. The observation that these variants frequently drive tissue-specific alternative splicing highlights the unexpected role played by cryptic splice mutations in generating novel alternative splicing diversity. A potential future direction would be to train deep learning models on splice junction annotations from RNA-seq of the relevant tissue, thereby obtaining tissue-specific models of alternative splicing. Training the network on annotations derived directly from RNA-seq data also helps to fill gaps in the GENCODE annotations, which improves the performance of the model on variant prediction (**FIGs. 52A** and **52B).**

Our understanding of how mutations in the noncoding genome lead to human disease remains far from complete. The discovery of penetrant *de novo* cryptic splice mutations in childhood neurodevelopmental disorders demonstrates that improved interpretation of the noncoding genome can directly benefit patients with severe genetic disorders. Cryptic splice mutations also play major roles in cancer (Jung et al., 2015; Sanz et al., 2010; Supek et al., 2014), and recurrent somatic mutations in splice factors have been shown to produce widespread alterations in splicing specificity (Graubert et al., 2012; Shirai et al., 2015; Yoshida et al., 2011). Much work remains to be done to understand regulation of splicing in different tissues and cellular contexts, particularly in the event of mutations that directly impact proteins in the spliceosome. In light of recent advances in oligonucleotide therapy that could potentially target splicing defects in a sequence-specific manner (Finkel et al., 2017), greater understanding of the regulatory mechanisms that govern this remarkable process could pave the way for novel candidates for therapeutic intervention.

**FIGs. 37A, 37B, 37C, 37D, 37E, 37F, 37G,** and **37H** illustrate one implementation of predicting splicing from primary sequence with deep learning.

Regarding **FIG. 37A,** for each position in the pre-mRNA transcript, SpliceNet-10k uses 10,000 nucleotides of flanking sequence as input and predicts whether that position is a splice acceptor, donor, or neither.

Regarding **FIG. 37B,** the full pre-mRNA transcript for the *CFTR* gene scored using MaxEntScan (top) and SpliceNet-10k (bottom) is shown, along with predicted acceptor (red arrows) and donor (green arrows) sites and the actual positions of the exons (black boxes). For each method, we applied the threshold that made the number of predicted sites equal to the total number of actual sites.

Regarding **FIG. 37C,** for each exon, we measured the inclusion rate of the exon on RNA-seq and show the SpliceNet-10k score distribution for exons at different inclusion rates. Shown are the maximum of the exon's acceptor and donor scores.

Regarding **FIG. 37D,** impact of *in silico* mutating each nucleotide around exon 9 in the *U2SURP* gene. The vertical size of each nucleotide shows the decrease in the predicted strength of the acceptor site (black arrow) when that nucleotide is mutated (Δ Score).

Regarding **FIG. 37E,** effect of the size of the input sequence context on the accuracy of the network. Top-k accuracy is the fraction of correctly predicted splice sites at the threshold where the number of predicted sites is equal to the actual number of sites present. PR-AUC is the area under the precision-recall curve. We also show the top-k accuracy and PR-AUC for three other algorithms for splice-site detection.

Regarding **FIG. 37F,** relationship between exon/intron length and the strength of the adjoining splice sites, as predicted by SpliceNet-80nt (local motif score) and SpliceNet-10k. The genome-wide distributions of exon length (yellow) and intron length (pink) are shown in the background. The x-axis is in log-scale.

Regarding **FIG. 37G,** a pair of splice acceptor and donor motifs, placed 150 nt apart, are walked along the *HMGCR* gene. Shown are, at each position, K562 nucleosome signal and the likelihood of the pair forming an exon at that position, as predicted by SpliceNet-10k.

Regarding **FIG. 37H,** average K562 and GM 12878 nucleosome signal near private mutations that are predicted by the SpliceNet-10k model to create novel exons in the GTEx cohort. The p-value by permutation test is shown.

**FIGs. 38A, 38B, 38C, 38D, 38E, 38F,** and **38G** depict one implementation of validation of rare cryptic splice mutations in RNA-seq data.

Regarding **FIG. 38A,** to assess the splice-altering impact of a mutation, SpliceNet-10k predicts acceptor and donor scores at each position in the pre-mRNA sequence of the gene with and without the mutation, as shown here for rs397515893, a pathogenic cryptic splice variant in the *MYBPC3* intron associated with cardiomyopathy. The Δ Score value for the mutation is the largest change in splice prediction scores within 50 nt from the variant.

Regarding **FIG. 38B,** we scored private genetic variants (observed in only one out of 1 49 individuals in the GTEx cohort) with the SpliceNet-10k model. Shown are the enrichment of private variants predicted to alter splicing (Δ Score > 0.2, blue) or to have no effect on splicing (Δ Score < 0.01, red) in the vicinity of private exon skipping junctions (top) or private acceptor and donor sites (bottom). The y-axis shows the number of times a private splice event and a nearby private genetic variant co-occur in the same individual, compared to expected numbers obtained through permutations.

Regarding **FIG. 38C,** example of a heterozygous synonymous variant in *PYGB* that creates a novel donor site with incomplete penetrance. RNA-seq coverage, junction read counts, and the positions of junctions (blue and grey arrows) are shown for the individual with the variant and a control individual. The effect size is computed as the difference in the usage of the novel junction (AC) between individuals with the variant and individuals without the variant. In the stacked bar graph below, we show the number of reads with the reference or alternate allele that used the annotated or the novel junction ("no splicing" and "novel junction" respectively). The total number of reference reads differed significantly from the total number of alternate reads (P = 0.018, Binomial test), suggesting that 60% of transcripts splicing at the novel junction are missing in the RNA-seq data, presumably due to nonsense mediated decay (NMD).

Regarding **FIG. 38D,** fraction of cryptic splice mutations predicted by the SpliceNet-10k model that validated against the GTEx RNA-seq data. The validation rate of disruptions of essential acceptor or donor dinucleotides (dashed line) is less than 100% due to coverage and nonsense mediated decay.

Regarding **FIG. 38E,** distribution of effect sizes for validated cryptic splice predictions. The dashed line (50%) corresponds to the expected effect size of fully penetrant heterozygous variants. The measured effect size of essential acceptor or donor dinucleotide disruptions is less than 50% due to nonsense-mediated decay or unaccounted-for isoform changes.

Regarding **FIG. 38F,** sensitivity of SpliceNet- 1 0k at detecting splice-altering private variants in the GTEx cohort at different Δ Score cutoffs. Variants are split into deep intronic variants (>50 nt from exons) and variants near exons (overlapping exons or ≤50 nt from exon-intron boundaries).

Regarding **FIG. 38G,** validation rate and sensitivity of SpliceNct-10k and three other methods for splice site prediction at different confidence cutoffs. The three dots on the SpliceNet-10k curve show the performance of SpliceNet-10k at Δ Score cutoffs of 0.2, 0.5, and 0.8. For the other three algorithms, the three dots on the curve indicate their performance at the thresholds where they predict the same number of cryptic splice variants as SpliceNet-10k at Δ Score cutoffs of 0.2, 0.5, and 0.8.

**FIGs. 39A, 39B,** and **39C** show one implementation of cryptic splice variants frequently create tissue-specific alternative splicing.

Regarding **FIG. 39A,** example of a heterozygous exonic variant in *CDC25B* which creates a novel donor site. The variant is private to a single individual in the GTEx cohort and exhibits tissue-specific alternative splicing that favors a greater fraction of the novel splice isoform in muscle compared to fibroblasts (P = 0.006 by Fisher's exact test). RNA-seq coverage, junction read counts, and the positions of junctions (blue and grey arrows) are shown for the individual with the variant and a control individual, in both muscle and fibroblasts.

Regarding **FIG. 39B,** example of a heterozygous exonic acceptor-creating variant in *FAM229B* which exhibits consistent tissue-specific effects across all three individuals in the GTEx cohort who harbor the variant. RNA-seq for artery and lung are shown for the three individuals with the variant and a control individual.

Regarding **FIG. 39C,** fraction of splice site-creating variants in the GTEx cohort that are associated with significantly non-uniform usage of the novel junction across expressing tissues, evaluated by the chi-square test for homogeneity. Validated cryptic splice variants with low to intermediate Δ Score values were more likely to result in tissue-specific alternative splicing (P = 0.015, Fisher Exact test).

**FIGs. 40A, 40B, 40C, 40D,** and 40F depict one implementation of predicted cryptic splice variants are strongly deleterious in human populations.

Regarding **FIG. 40A,** synonymous and intronic variants (≤50 nt from known exon-intron boundaries and excluding the essential GT or AG dinucleotides) with confidently predicted splice-altering effects (Δ Score ≥ 0.8) are strongly depleted at common allele frequencies (≥0.1%) in the human population relative to rare variants observed only once in 60,706 individuals. The 4.58 odds ratio (P < $10^{-127}$ by chi-square test) indicates that 78% of recently arising predicted cryptic splice variants are sufficiently deleterious to be removed by natural selection.

Regarding **FIG. 40B,** fraction of protein-truncating variants and predicted synonymous and intronic cryptic splice variants in the ExAC dataset that are deleterious, calculated as in (A).

Regarding **FIG. 40C,** fraction of synonymous and intronic cryptic splice gain variants in the ExAC dataset that are deleterious (Δ Score ≥ 0.8), split based on whether the variant is expected to cause a frameshift or not.

Regarding **FIG. 40D,** fraction of protein-truncating variants and predicted deep intronic (>50 nt from known exon-intron boundaries) cryptic splice variants in the gnomAD dataset that are deleterious.

Regarding **FIG. 40E,** average number of rare (allele frequency < 0.1%) protein-truncating variants and rare functional cryptic splice variants per individual human genome. The number of cryptic splice mutations that are expected to be functional is estimated based on the fraction of predictions that are deleterious. The total number of predictions is higher.

**FIGs. 41A, 41B, 41C, 41D, 41E,** and **41F** show one implementation of de novo cryptic splice mutations in patients with rare genetic disease.

Regarding **FIG. 41A,** predicted cryptic splice *de novo* mutations per person for patients from the Deciphering Developmental Disorders cohort (DDD), individuals with autism spectrum disorders (ASD) from the Simons Simplex Collection and the Autism Sequencing Consortium, as well as healthy controls. Enrichment in the DDD and ASD cohorts above healthy controls is shown, adjusting for variant ascertainment between cohorts. Error bars show 95% confidence intervals.

Regarding **FIG. 41B,** estimated proportion of pathogenic *de novo* mutations by functional category for the DDD and ASD cohorts, based on the enrichment of each category compared to healthy controls.

Regarding **FIG. 41C,** enrichment and excess of cryptic splice *de novo* mutations in the DDD and ASD cohorts compared to healthy controls at different Δ Score thresholds.

Regarding **FIG. 41D,** list of novel candidate disease genes enriched for *de novo* mutations in the DDD and ASD cohorts (FOR < 0.01), when predicted cryptic splice mutations were included together with protein-coding mutations in the enrichment analysis. Phenotypes that were present in multiple individuals are shown.

Regarding **FIG. 41E,** three examples of predicted *de novo* cryptic splice mutations in autism patients that validate on RNA-seq, resulting in intron retention, exon skipping, and exon extension, respectively. For each example, RNA-seq coverage and junction counts for the affected individual are shown at the top, and a control individual without the mutation is shown at the bottom. Sequences are shown on the sense strand with respect to the transcription of the gene. Blue and grey arrows demarcate the positions of the junctions in the individual with the variant and the control individual respectively.

Regarding **FIG. 41F,** validation status for 36 predicted cryptic splice sites selected for experimental validation by RNA-seq.

## EXPERIMENTAL MODEL AND SUBJECT DETAILS

Subject details for the 36 autism patients were previously released by Iossifov et al, Nature 2014 (Table S1), and can be cross-referenced using the anonymized identifiers in Column 1 of Table S4 in our paper.

## METHOD DETAILS

### I. Deep learning for splice prediction

### SoliceNet architecture

We trained several ultra-deep convolutional neural network-based models to computationally predict splicing from pre-mRNA nucleotide sequence. We designed four architectures, namely, SpliceNet-80nt, SpliceNet-400nt, SpliceNet-2k and SpliceNet-10k, which use 40, 200, 1,000 and 5,000 nucleotides on each side of a position of interest as input respectively, and output the probability of the position being a splice acceptor and donor. More precisely, the input to the models is a sequence of one-hot encoded nucleotides, where A, C, G and T (or equivalently U) are encoded as [1, 0, 0, 0], [0, 1,0,0], [0, 0, 1,0] and [0, 0, 0, 1] respectively and the output of the models consists of three scores which sum to one, corresponding to the probability of the position of interest being a splice acceptor, splice donor and neither.

The basic unit of the SpliceNet architectures is a residual block (He et al., 2016b), which consists of batch-normalization layers (Ioffe and Szegedy, 2015), rectified linear units (ReLU), and convolutional units organized in a specific manner (**FIGs. 21, 22, 23,** and **24**). Residual blocks are commonly used when designing deep neural networks. Prior to the development of residual blocks, deep neural networks consisting of many convolutional units stacked one after the other were very difficult to train due to the problem of exploding/vanishing gradients (Glorot and Bengio, 2010), and increasing the depth of such neural networks often resulted in a higher training error (He et al., 2016a). Through a comprehensive set of computational experiments, architectures consisting of many residual blocks stacked one after the other were shown to overcome these issues (He et al., 2016a).

The complete SpliceNet architectures are provided in **FIGs. 21, 22, 23,** and **24.** The architectures consist of $K$ stacked residual blocks connecting the input layer to the penultimate layer, and a convolutional unit with softmax activation connecting the penultimate layer to the output layer. The residual blocks are stacked such that the output of the $i^{th}$ residual block is connected to the input of the $i + 1^{th}$ residual block. Further, the output of every fourth residual block is added to the input of the penultimate layer. Such "skip connections" are commonly used in deep neural networks to increase convergence speed during training (Cord et al., 2016).

Each residual block has three hyper-parameters $N$, $W$ and $D$, where $N$ denotes the number of convolutional kernels, $W$ denotes the window size and $D$ denotes the dilation rate (Yu and Koltun, 2016) of each convolutional kernel. Since a convolutional kernel of window size $W$ and dilation rate $D$ extracts features spanning $(W - 1)D$ neighboring positions, a residual block with hyper-parameters $N$, $W$ and $D$ extracts features spanning $2(W - 1)D$ neighboring positions.

Hence, the total neighbor span of the SpliceNet architectures is given by $S = \sum_{i=1}^{K} 2(W_i - 1)D_i$, where $N_i$, $W_i$ and $D_i$ are the hyper-parameters of the $i^{th}$ residual block. For SpliceNet-80nt, SpliceNet-400nt, SpliceNet-2k and SpliceNet-10k architectures, the number of residual blocks and the hyper-parameters for each residual block were chosen so that $S$ is equal to 80, 400, 2,000 and 10,000 respectively.

The SpliceNet architectures only have normalization and non-linear activation units in addition to convolutional units. Consequently, the models can be used in a sequence-to-sequence mode with variable sequence length (Oord et al., 2016). For example, the input to the SpliceNet-10k model ($S = 10,000$) is a one-hot encoded nucleotide sequence of length $S/2 + l + S/2$, and the output is an $l \times 3$ matrix, corresponding to the three scores of the $l$ central positions in the input, i.e., the positions remaining after excluding the first and last $S/2$ nucleotides. This feature can be leveraged to obtain a tremendous amount of computational saving during training as well as testing. This is due to the fact that most of the computations for positions which are close to each other are common, and the shared computations need to be done only once by the models when they are used in a sequence-to-sequence mode.

Our models adopted the architecture of residual blocks, which has become widely adopted due to its success in image classification. The residual blocks comprise repeating units of convolution, interspersed with skip connections that allow information from earlier layers to skip over residual blocks. In each residual block, the input layer is first batch normalized, followed by an activation layer using rectified linear units (ReLU). The activation is then passed through a 10 convolution layer. This intermediate output from the 1D convolution layer is again batch normalized and ReLU activated, followed by another 1D convolution layer. At the end of the second 1D convolution, we summed its output with the original input into the residual block, which acts as a skip connection by allowing the original input information to bypass the residual block. In such an architecture, termed a deep residual learning network by its authors, the input is preserved in its original state and the residual connections are kept free of nonlinear activations from the model, allowing effective training of deeper networks.

Following the residual blocks, the softmax layer computes probabilities of the three states for each amino acid, among which the largest softmax probability determines the state of the amino acid. The model is trained with accumulated categorical cross entropy loss function for the whole protein sequence using the ADAM optimizer.

Atrous/dilated convolutions allow for large receptive fields with few trainable parameters. An atrous/dilated convolution is a convolution where the kernel is applied over an area larger than its length by skipping input values with a certain step, also called atrous convolution rate or dilation factor. Atrous/dilated convolutions add spacing between the elements of a convolution filter/kernel so that neighboring input entries (e.g., nucleotides, amino acids) at larger intervals are considered when a convolution operation is performed. This enables incorporation of long-range contextual dependencies in the input. The atrous convolutions conserve partial convolution calculations for reuse as adjacent nucleotides are processed.

The illustrated example uses 1D convolutions. In other implementations, the model can use different types of convolutions such as 2D convolutions, 3D convolutions, dilated or atrous convolutions, transposed convolutions, separable convolutions, and depthwise separable convolutions. Some layers also use ReLU activation function which greatly accelerates the convergence of stochastic gradient descent compared to saturating nonlinearities such as sigmoid or hyperbolic tangent. Other examples of activation functions that can be used by the technology disclosed include parametric ReLU, leaky ReLU, and exponential linear unit (ELU).

Some layers also use batch normalization (Ioffe and Szegedy 2015). Regarding batch normalization, distribution of each layer in a convolution neural network (CNN) changes during training and it varies from one layer to another. This reduces the convergence speed of the optimization algorithm. Batch normalization is a technique to overcome this problem. Denoting the input of a batch normalization layer with x and its output using z, batch normalization applies the following transformation on x:

$$ z = \frac{x - \mu}{\sqrt{\sigma^2 + \varepsilon}} \gamma + \beta $$

Batch normalization applies mean-variance normalization on the input x using $\mu$ and $\sigma$ and linearly scales and shifts it using $\gamma$ and $\beta$. The normalization parameters $\mu$ and $\sigma$ are computed for the current layer over the training set using a method called exponential moving average. In other words, they are not trainable parameters. In contrast, $\gamma$ and $\beta$ are trainable parameters. The values for $\mu$ and $\sigma$ calculated during training are used in forward pass during inference.

**Model training and testing**

We downloaded the GENCODE (Harrow et al., 2012) V24lift37 gene annotation table from the UCSC table browser and extracted 20,287 protein-coding gene annotations, selecting the principal transcript when multiple isoforms were available. We removed genes which did not have any splice junctions and split the remaining into training and test set genes as follows: The genes which belonged to chromosomes 2, 4, 6, 8, 10-22, X and Y were used for training the models (13,384 genes, 130,796 donor-acceptor pairs). We randomly chose 10% of the training genes and used them for determining the point for early-stopping during training, and the rest were used for training the models. For testing the models, we used genes from chromosomes 1, 3, 5, 7 and 9 which did not have any paralogs (1,652 genes, 14,289 donor-acceptor pairs). To this end, we referred to the human gene paralog list from http://grch37.ensembl .org/biomart/martview.

We used the following procedure to train and test the models in a sequence-to-sequence mode with chunks of size $l$ = 5,000. For each gene, the mRNA transcript sequence between the canonical transcription start and end sites was extracted from the hg19/GRCh37 assembly. The input mRNA transcript sequence was one-hot encoded as follows: A, C, G, T/U mapped to [1, 0, 0, 0], [0, 1, 0, 0], [0, 0, 1, 0], [0, 0, 0, 1] respectively. The one-hot encoded nucleotide sequence was zero-padded until the length became a multiple of 5,000, and then further zero-padded at the start and the end with a flanking sequence of length $S$12, where $S$ is equal to 80, 400, 2,000 and 10,000 for SpliceNet-80nt, SpliceNet-400nt, SpliceNet-2k and SpliceNet-10k models respectively. The padded nucleotide sequence was then split into blocks of length S12 + 5,000 + $S$/2 in such a way that the $i^{th}$ block consisted of the nucleotide positions from 5,000($i$ - 1) - S12 + 1 to 5,0001 + S12. Similarly, the splice output label sequence was one-hot encoded as follows: not a splice site, splice acceptor (first nucleotide of the corresponding exon), and splice donor (last nucleotide of the corresponding exon) were mapped to [1, 0, 0], [0, 1, 0], and [0, 0, 1] respectively. The one-hot encoded splice output label sequence was zero-padded until the length became a multiple of 5,000, and then split into blocks of length 5,000 in such a way that the $i^{th}$ block consisted of the positions from 5,000($i$ - 1) + 1 to 5,000$i$. The one-hot encoded nucleotide sequence and the corresponding one-hot encoded label sequence were used as inputs to the model and target outputs of the model respectively.

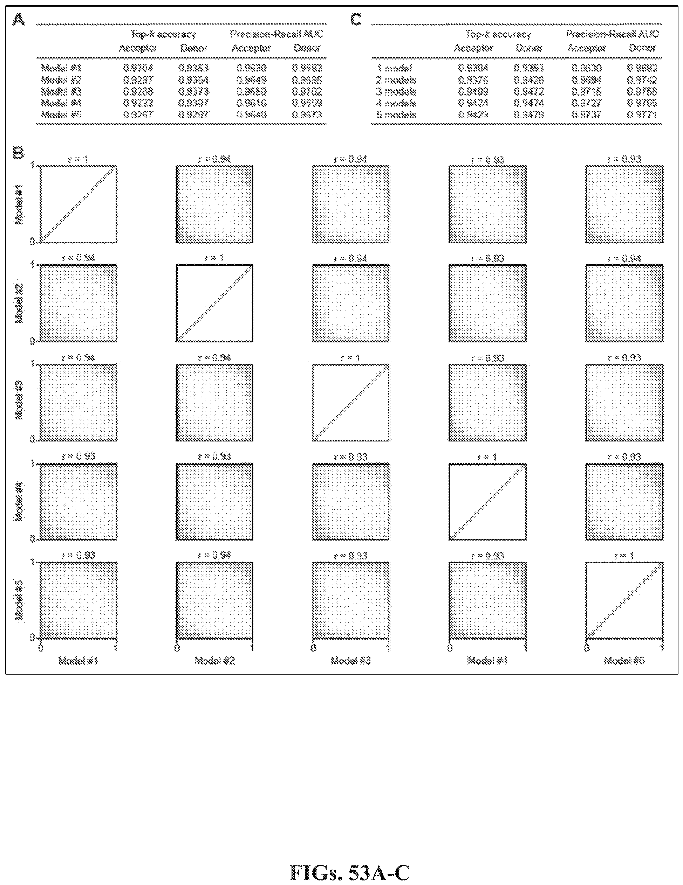

The models were trained for 10 epochs with a batch size of 12 on two NVIDIA GeForce GTX 1080 Ti GPUs. Categorical cross-entropy loss between the target and predicted outputs was minimized using Adam optimizer (Kingma and Ba, 2015) during training. The learning rate of the optimizer was set to 0.001 for the first 6 epochs, and then reduced by a factor of 2 in every subsequent epoch. For each architecture, we repeated the training procedure 5 times and obtained 5 trained models (**FIGs. 53A** and **53B**). During testing, each input was evaluated using all 5 trained models and the average of their outputs was used as the predicted output. We used these models for the analyses in **FIGs. 37A** and other related figures.

For the analyses in FIGs. 38A-G, 39A-C, 40A-E, and **41A-F** involving identification of splice-altering variants, we augmented the training set of GENCODE annotations to also include novel splice junctions commonly observed in the GTEx cohort on chromosomes 2, 4, 6, 8, 10-22, X, Y (67,012 splice donors and 62,911 splice acceptors). This increased the number of splice junction annotations in the training set by ~50%. Training the network on the combined dataset improved the sensitivity of detecting splice-altering variants in the RNA-seq data compared to the network trained on GENCODE annotations alone (**FIGs. 52A** and **52B**), particularly for predicting deep intronic splice-altering variants, and we used this network for the analyses involving evaluation of variants (FIGs. 38A-G, 39A-C, 40A-E, and 41A-F and related figures). To ensure that the GTEx RNA-seq dataset did not contain overlap between training and evaluation, we only included junctions that were present in 5 or more individuals in the training dataset, and only evaluated the performance of the network on variants that were present in 4 or fewer. Details of novel splice junction identification are described in "Detection of splice junctions" under the GTEx analysis section of the methods.

**Too-k accuracy**

An accuracy metric like the percentage of positions classified correctly is largely ineffective due to the fact that most of the positions are not splice sites. We instead evaluated the models using two metrics that are effective in such settings, namely, top-$k$ accuracy and area under the precision-recall curve. The top-$k$ accuracy for a particular class is defined as follows: Suppose the test set has $k$ positions that belong to the class. We choose the threshold so that exactly $k$ test set positions are predicted as belonging to the class. The fraction of these $k$ predicted positions that truly belong to the class is reported as the top-$k$ accuracy. Indeed, this is equal to the precision when the threshold is chosen so that precision and recall have the same value.

**Model evaluation on lincRNAs**

We obtained a list of all lincRNA transcripts based on the GENCODE V24lift37 annotations. Unlike protein-coding genes, lincRNAs are not assigned a principal transcript in the GENCODE annotations. To minimize redundancy in the validation set, we identified the transcript with the longest total exonic sequence per lincRNA gene, and called this the canonical transcript for the gene. Since lincRNA annotations are expected to be less reliable than annotations for protein-coding genes, and such misannotations would affect our estimates of top-$k$ accuracy, we used the GTEx data to eliminate lincRNAs with potential annotation issues (see section "Analyses on the GTEx dataset" below for details on these data). For each lincRNA, we counted all split reads that mapped across the length of the lincRNA across all GTEx samples (see "Detection of splice junctions" below for details). This was an estimate of the total junction-spanning reads of the lincRNA that used either annotated or novel junctions. We also counted the number of reads that spanned junctions of the canonical transcript. We only considered lincRNAs for which at least 95% of junction-spanning reads across all GTEx samples corresponded to the canonical transcript. We also required all junctions of the canonical transcript to be observed at least once in the GTEx cohort (excluding junctions that spanned introns of length < 10 nt). For computing top-$k$ accuracy, we only considered the junctions of the canonical transcripts of the lincRNAs that passed the filters above (781 transcripts, 1047 junctions).

**Identifying splice junctions from pre-mRNA sequence**

In **FIG. 37B,** we compare the performance of MaxEntScan and SpliceNet-10k with respect to identifying the canonical exon boundaries of a gene from its sequence. We used the CFTR gene, which is in our test set and has 26 canonical splice acceptors and donors, as a case study and obtained an acceptor and donor score for each of the 188,703 positions from the canonical transcription start site (chr7:117,120,017) to the canonical transcription end site (chr7: 1 17,308,719) using MaxEntScan and SpliceNet-10k. A position was classified as a splice acceptor or donor if its corresponding score was greater than the threshold chosen while evaluating the top-k accuracy. MaxEntScan predicted 49 splice acceptors and 22 splice donors, out of which 9 and 5 are true splice acceptors and donors respectively. For the sake of better visualization, we show the pre-log scores of MaxEntScan (clipped to a maximum of 2,500). SpliceNet-10k predicted 26 splice acceptors and 26 splice donors, all of which are correct. For **FIG. 42B,** we repeated the analysis using the LINC00467 gene.

**Estimation of exon inclusion at GENCODE-annotated splice junctions**

We computed the inclusion rate of all GENCODE-annotated exons from the GTEx RNA-seq data (**FIG. 37C**). For each exon, excluding the first and last exon of each gene, we computed the inclusion rate as:

$$\frac{(L+R)/2}{S+(L+R)/2}$$

where $L$ is the total read count of the junction from the previous canonical exon to the exon under consideration across all GTEx samples, $R$ is the total read count of the junction from the exon under consideration to the next canonical exon, and 5 is the total read count of the skipping junction from the previous to the next canonical exon.

**Significance of various nucleotides towards splice site recognition**

In **FIG. 37D,** we identify the nucleotides that are considered important by SpliccNet-10k towards the classification of a position as a splice acceptor. To this end, we considered the splice acceptor at chr3:142,740,192 in the U2SURP gene, which is in our test set. The "importance score" of a nucleotide with respect to a splice acceptor is defined as follows: Let $s_{ref}$ denote the acceptor score of the splice acceptor under consideration. The acceptor score is recalculated by replacing the nucleotide under consideration with A, C, G and T. Let these scores be denoted by $s_A$, $s_c$, $s_G$ and $s_T$ respectively. The importance score of the nucleotide is estimated as

$$S_{ref} - \frac{s_A + s_C + s_G + s_T}{4}$$

This procedure is often referred to as in-silico mutagenesis (Zhou and Troyanskaya, 2015). We plot 127 nucleotides from chr3: 142,740,137 to chr3:142,740,263 in such a way that the height of each nucleotide is its importance score with respect to the splice acceptor at chr3: 142,740,192. The plotting function was adapted from DeepLIFT (Shrikumar et al., 2017) software.

**Effect of TACTAAC and GAAGAA motifs on splicing**

In order to study the impact of the position of the branch point sequence on acceptor strength, we first obtained the acceptor scores of the 14,289 test set splice acceptors using SpliceNet-10k. Let $y_{ref}$ denote the vector containing these scores. For each value of $i$ ranging from 0 to 100, we did the following: For each test set splice acceptor, we replaced the nucleotides from $i$ to $i$ - 6 positions before the splice acceptor by TACTAAC and recomputed the acceptor score using SpliceNet-10k. The vector containing these scores is denoted by $y_{alt,i}$. We plot the following quantity as a function of $i$ in **FIG. 43A**:

$$mean\left(y_{alt,i} - y_{ref}\right)$$

**For FIG. 43B,** we repeated the same procedure using the SR-protein motif GAAGAA. In this case, we also studied the impact of the motif when present after the splice acceptor as well as the impact on donor strength. GAAGAA and TACTAAC were the motifs with the greatest impact on acceptor and donor strength, based on a comprehensive search in the k-mer space.

**Role of exon and intron lengths in splicing**

To study the effect of exon length on splicing, we filtered out the test set exons which were either the first or last exon. This filtering step removed 1,652 out of the 14,289 exons. We sorted the remaining 12,637 exons in the order of increasing length. For each of them, we calculated a splicing score by averaging the acceptor score at the splice acceptor site and the donor score at the splice donor site using SpliceNet-80nt. We plot the splicing scores as a function of exon length in **FIG. 37F.** Before plotting, we applied the following smoothing procedure: Let $x$ denote the vector containing the lengths of the exons, and $y$ denote the vector containing their corresponding splicing scores. We smoothed both $x$ and $y$ using an averaging window of size 2,500.

We repeated this analysis by calculating the splicing scores using SpliceNet-10k. In the background, we show the histogram of the lengths of the 12,637 exons considered for this analysis. We applied a similar analysis to study the effect of intron length on splicing, with the main difference being that it was not necessary to exclude the first and last exons.

**Role of nucleosomes in splicing**

We downloaded nucleosome data for the K562 cell line from the UCSC genome browser. We used the HMGR gene, which is in our test set, as an anecdotal example to demonstrate the impact of nucleosome positioning on SpliceNct-10k score. For each position p in the gene, we calculated its "planted splicing score" as follows:

- The 8 nucleotides from positions p+74 to p+81 were replaced by a donor motif AGGTAAGG.
- The 4 nucleotides from positions p-78 to p-75 were replaced by an acceptor motif TAGG.
- The 20 nucleotides from positions p-98 to p-79 were replaced by a poly-pyrimidine tract CCTCCTTTTTCCTCGCCCTC.
- The 7 nucleotides from positions p-105 to p-99 were replaced by a branch point sequence CACTAAC.
- The average of the acceptor score at p-75 and donor score at p+75 predicted by SpliceNet-10k is used as the planted splicing score.

The K562 nucleosome signal as well as the planted splicing score for the 5,000 positions from chr5:74,652,154 to chr5:74,657,153 is shown in **FIG**. 37G.

To calculate the genome-wide Spearman correlation between these two tracks, we randomly chose one million intergenic positions which were at least 100,000 nt away from all canonical genes. For each of these positions, we calculated its planted splicing score as well as its average K562 nucleosome signal (window size of 50 was used for averaging). The correlation between these two values across the one million positions is shown in **FIG. 37G.** We further sub-classified these positions based on their GC content (estimated using the nucleotides in between the planted acceptor and donor motifs) with a bin size of 0.02. We show the genome-wide Spearman correlation for each bin in **FIG. 44A.**

For each of the 14,289 test set splice acceptors, we extracted nucleosome data within 50 nucleotides on each side and calculated its nucleosome enrichment as the average signal on the exon side divided by the average signal on the intron side. We sorted the splice acceptors in the increasing order of their nucleosome enrichment and calculated their acceptor scores using SpliceNet-80nt. The acceptor scores are plotted as a function of nucleosome enrichment in **FIG. 44B.** Before plotting, the smoothing procedure used in **FIG. 37F** was applied. We repeated this analysis using SpliceNet-10k and also for the 14,289 test set splice donors.

**Enrichment of nucleosome signal at novel exons**

For **FIG. 37H,** we wanted to look at the nucleosome signal around predicted novel exons. To ensure that we were looking at highly confident novel exons, we only selected singleton variants (variants present in a single GTEx individual) where the predicted gained junction was entirely private to the individual with the variant. Additionally, to remove confounding effects from nearby exons, we only looked at intronic variants at least 750 nt away from annotated exons. We downloaded nucleosome signals for the GM12878 and K562 cell lines from the UCSC browser and extracted the nucleosome signal within 750 nt from each of the predicted novel acceptor or donor sites. We averaged the nucleosome signal between the two cell lines and flipped the signal vectors for variants that overlapped genes on the negative strand. We shifted the signal from acceptor sites by 70 nt to the right and the signal from donor sites by 70 nt to the left. After shifting, the nucleosome signal for both acceptor and donor sites was centered at the middle of an idealized exon of length 140 nt, which is the median length of exons in the GENCODE v19 annotations. We finally averaged all shifted signals and smoothed the resulting signal by computing the mean within an 11 nt window centered at each position.

To test for an association, we selected random singleton SNVs, that were at least 750 nt away from annotated exons and were predicted by the model to have no effect on splicing ($\Delta$ Score < 0.01). We created 1000 random samples of such SNVs, each sample having as many SNVs as the set of splice-site gain sites that were used for **FIG. 37H** (128 sites). For each random sample, we computed a smoothed average signal as described above. Since the random SNVs were not predicted to create novel exons, we centered the nucleosome signal from each SNV at the SNV itself and randomly shifted either 70 nt to the left or 70 nt to the right. We then compared the nucleosome signal at the middle base of **FIG. 37H** to the signals obtained from the 1000 simulations at that base. An empirical p-value was computed as the fraction of simulated sets that had a middle value greater or equal to that observed for the splice-site gain variants.

**Robustness of the network to differences in exon density**

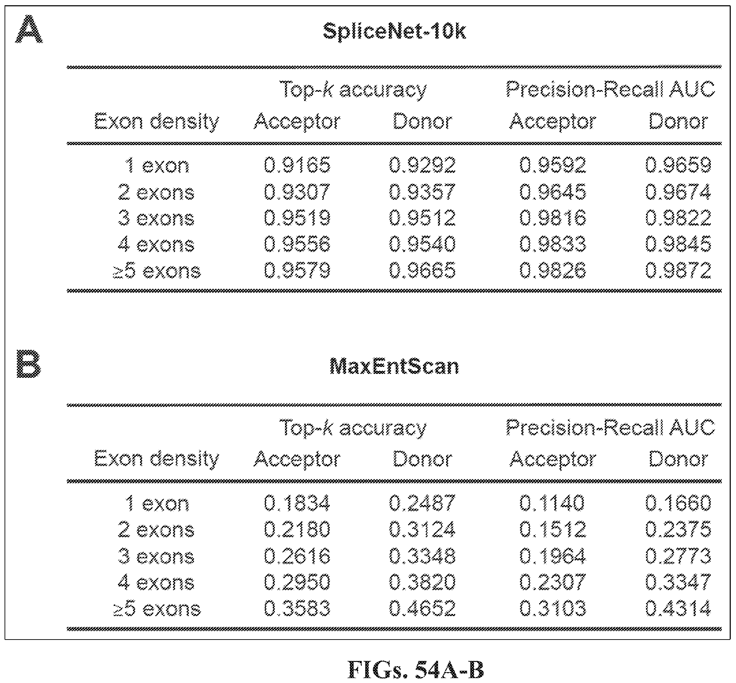

To investigate the generalizability of the network's predictions, we evaluated SpliceNet-10k in regions of varying exon density. We first separated the test set positions into 5 categories depending on the number of canonical exons present in a 10,000 nucleotide window (5,000 nucleotides on each side) (**FIG. 54**). To ensure that the exon count is an integral value for each position, we used the number of exon starts present in the window as a surrogate. For each category, we calculated the top-k accuracy and the area under the precision-recall curve. The number of positions and the value of $k$ is different for different categories (detailed in the table below).

| Exon count | # Positions | # Splice acceptors | # Splice donors |
|---|---|---|---|
| 1 exon | 15,870,045 | 1,712 | 1,878 |
| 2 exons | 10,030,710 | 2,294 | 2,209 |
| 3 exons | 6,927,885 | 2,351 | 2,273 |
| 4 exons | 4,621,341 | 2,095 | 2,042 |
| ≥ 5 exons | 7,247,582 | 5,679 | 5,582 |

**Robustness of the network for each of the five models in the ensemble**

Training multiple models and using the average of their predictions as the output is a common strategy in machine learning to obtain better predictive performance, referred to as ensemble learning. In **FIG. 53A,** we show the top-$k$ accuracies and the area under the precision-recall curves of the 5 SpliceNet-10k models we trained to construct the ensemble. The results clearly demonstrate the stability of the training process.

We also calculated the Pearson correlation between their predictions. Since most positions in the genome are not splice sites, the correlation between the predictions of most models would be close to 1, rendering the analysis meaningless. To overcome this issue, we only considered those positions in the test set which were assigned an acceptor or donor score greater than or equal to 0.01 by at least one model. This criterion was satisfied by 53,272 positions (approximately equal number of splice sites and non-splice sites). The results are summarized in **FIG. 53B.** The very high Pearson correlation between the predictions of the models further illustrate their robustness.

We show the effect of the number of models used to construct the ensemble on the performance in **FIG. 53C.** The results show that the performance improves as the number of models increases, with diminishing returns.

## II. Analyses on the GTEx RNA-seqdataset

### Δ Score of a single nucleotide variant

We quantified the splicing change due to a single nucleotide variant as follows: We first used the reference nucleotide and calculated the acceptor and donor scores for 101 positions around the variant (50 positions on each side). Suppose these scores are denoted by the vectors $a_{ref}$ and $d_{ref}$ respectively. We then used the alternate nucleotide and recalculated the acceptor and donor scores. Let these scores be denoted by the vectors $a_{alt}$ and $d_{alt}$ respectively. We evaluated the following four quantities:

$$\Delta \text{ Score (acceptor gain)} = \max(a_{alt} - a_{ref})$$

$$\Delta \text{ Score (acceptor loss)} = \max(a_{ref} - a_{alt})$$

$$\Delta \text{ Score (donor gain)} = \max(d_{alt} - d_{ref})$$

$$\Delta \text{ Score (donor loss)} = \max(d_{ref} - d_{alt})$$

The maximum of these four scores is referred to as the Δ Score of the variant.

### Criteria for quality control and filtering of variants

We downloaded the GTEx VCF and RNA-seq data from dbGaP (study accession phs000424.v6.p1; https://www.ncbi.ntm.nih.gov/projects/gap/cgi-bin/study.cgi?study_id=phs000424.v6.p1).

We evaluated the performance of Splice Net on autosomal SNVs that appeared in at most 4 individuals in the GTEx cohort. In particular, a variant was considered if it satisfied the following criteria in at least one individual A:

1. The variant was not filtered (the FILTER field of the VCF was PASS).
2. The variant was not marked as MULTI_ALLELIC in the INFO field of individual A's VCF and the VCF contained a single allele in the ALT field.
3. Individual A was heterozygous for the variant.
4. The ratio alt_depth / (alt_depth + ret_depth) was between 0.25 and 0.75, where alt_depth and ref_depth are the number of reads supporting the alternative and reference allele in individual A respectively.
5. The total depth, alt_depth + ref_ depth, was between 20 and 300 in individual A's VCF.
6. The variant overlapped a gene body region. Gene bodies were defined as the regions between the transcription starts and ends of canonical transcripts from GENCODE (V24lift37).

For variants satisfying these criteria in at least one individual, we considered all individuals where the variant appeared (even if it did not satisfy the above criteria) as having the variant. We refer to variants appearing in a single individual as singleton and variants appearing in 2-4 individuals as common. We did not evaluate variants appearing in 5 or more individuals, in order to prevent overlap with the training dataset.

### RNA-seq read alignment

We used OLego (Wu et al., 2013) to map the reads of the GTEx samples against the hg19 reference, allowing an edit distance of at most 4 between the query read and the reference (parameter -M 4). Note that OLego can operate completely de novo and does not require any gene annotations. Since OLego looks for the presence of splicing motifs at the ends of split reads, its alignments can be biased towards or against the reference around SNVs that disrupt or create splice sites respectively. To eliminate such biases, we further created an alternative reference sequence for each GTEx individual, by inserting into the hg19 reference all the SNVs of the individual with a PASS filter. We used OLego with the same parameters to map all samples from each individual against that individual's alternative reference sequence. For each sample, we then combined the two sets of alignments (against the hg19 reference and against the individual's alternative reference), by picking the best alignment for each read pair. To choose the best alignment for a read pair P, we used the following procedure:

1. If both reads of P were unmapped in both sets of alignments, we chose either the hgl9 or the alternative alignments of P at random.

2. If P had more unmapped ends in one set of alignments than in the other (e.g. both ends of P were mapped against the alternative reference but only one end was mapped against hg19), we chose the alignment with both ends of P mapped.

3. If both ends of P were mapped in both sets of alignments, we chose the alignment with the fewest total mismatches, or a random one, if the number of mismatches was the same.

## Detection of splice junctions in aligned RNA-sea data

We used leafcutter_cluster, a utility in the leaf cutter package (Li et al., 2018), to detect and count splice junctions in each sample. We required a single split read to support a junction and assumed a maximum intron length of 500Kb (parameters -m 1 -1 500000). To get a high-confidence set of junctions for training the deep learning model, we compiled the union of all leafcutter junctions across all samples and then removed from consideration junctions that met any of the following criteria:

1. Either end of the junction overlapped an ENCODE blacklist region (table wgEncodeDacMapabilityConsensusExcludable in hgl9 from the UCSC genome browser) or a simple repeat (Simple Repeats track in hgl9 from the UCSC genome browser).
2. Both ends of the junction were on non-canonical exons (based on the canonical transcripts from GENCODE version V24lift37).
3. The two ends of the junction were on different genes or either end was in a non-genic region.
4. Either end lacked the essential GT/AG dinucleotides.

Junctions that were present in 5 or more individuals were used to augment the list of GENCODE annotated splice junctions for the analyses on variant prediction **(**FIGs. 38A-G, 39A-C, 40A-E, and 41A-F). Links to the files containing the list of splice junctions used to train the model are provided in the Key Resources table.

Although we used junctions detected by leaf cutter for augmenting the training dataset, we noticed that, despite the use of relaxed parameters, leafcutter was filtering many junctions with good support in the RNA-seq data. This artificially lowered our validation rates. Thus, for the GTEx RNA-seq validation analyses (FIGs. 38A-G and 39A-C), we recomputed the set of junctions and junction counts directly from the RNA-seq read data. We counted all non-duplicate split-mapped reads with MAPQ at least 10 and with at least 5 nt aligned on each side of the junction. A read was allowed to span more than two exons, in which case the read was counted towards each junction with at least 5 nt of mapped sequence on both sides.

## Definition of private junctions

A junction was considered private in individual A if it satisfied at least one of the following criteria:

*1. The junction had at least 3 reads in at least one sample from A and was never observed in any other individual.*
2. There were at least two tissues that satisfied both of the following two criteria:

a. The average read count of the junction in samples from individual A in the tissue was at least 10.
b. Individual A had at least twice as many normalized reads on average than any other individual in that tissue. Here, the normalized read count of a junction in a sample was defined as the number of reads of the junction normalized by the total number of reads across all junctions for the corresponding gene.

Tissues with fewer than 5 samples from other individuals (not A) were ignored for this test.

## Enrichment of singleton SNVs around private junctions

If a private junction had exactly one end annotated, based on the GENCODE annotations, we considered it a candidate for an acceptor or donor gain and searched for singleton SNVs (SNVs appearing in a single GTEx individual) that were private in the same individual within 150 nt from the unannotated end. If a private junction had both ends annotated, we considered it a candidate for a private exon skipping event if it skipped at least one but no more than 3 exons of the same gene based on the GENCODE annotations. We then searched for singleton SNVs within 150 nt from the ends of each of the skipped exons. Private junctions with both ends absent from the GENCODE exon annotations were ignored, as a substantial fraction of these were alignment errors.

To compute the enrichment of singleton SNVs around novel private acceptors or donors (**FIG. 38B,** bottom), we aggregated the counts of singleton SNVs at each position relative to the private junction. If the overlapping gene was on the negative strand, relative positions were flipped. We split SNVs into two groups: SNVs that were private in the individual with the private junction and SNVs that were private in a different individual. To smooth the resulting signals, we averaged counts in a 7 nt window centered at each position. We then computed the ratio of smoothed counts from the first group (private in the same individual) to the smoothed counts of the second group (private in a different individual). For novel private exon skips (**FIG. 38B,** top), we followed a similar procedure, aggregating the counts of singleton SNVs around the ends of skipped exons.

**Validation of model predictions in GTEx RNA-sea data**

For either private variants (appearing in one individual in the GTEx cohort) or common variants (appearing in two to four individuals in the GTEx cohort), we obtained the predictions of the deep learning model for both the reference and the alternate alleles and computed the Δ Score. We also obtained the location where the model predicted the aberrant (novel or disrupted) junction to be. We then sought to determine whether there was evidence in the RNA-seq data supporting a splicing *aberration* in the individuals with the variant at the predicted location. In many cases, the model can predict multiple effects for the same variant, e.g. a variant disrupting an annotated splice donor could also increase usage of a suboptimal donor as in **FIG. 45**, in which case the model might predict both a donor loss at the annotated splice site and a donor gain at the suboptimal site. However, for validation purposes, we only considered the effect with the highest predicted Δ Score for each variant. Therefore, for each variant, we considered predicted splice site-creating and splice site-disrupting effects separately. Note that junctions appearing in less than five individuals were excluded during model training, to avoid evaluating the model on novel junctions it was trained on.

**Validation of predicted cryptic splice mutations based on private splice junctions**

For each private variant predicted to cause novel junction formation, we used the network to predict the position of a newly created aberrant splice junction, and looked in the RNA-seq data to validate if such a novel junction appeared in only the individual with the SNV and in no other GTEx individuals. Similarly, for a variant predicted to cause a splice-site loss affecting a splice site of exon X, we looked for novel exon skipping events, from the previous canonical exon (the one upstream of X based on GENCODE annotations), to the next canonical exon (the one downstream of X) that appeared in only the individuals with the variant and in no other individuals in GTEx. We excluded predicted losses if the splice site predicted to be lost by the model was not annotated in GENCODE or never observed in GTEx individuals without the variant. We also excluded predicted gains if the splice site predicted to be gained was already annotated in GENCODE. To extend this analysis to common variants (present in two to four individuals), we also validated novel junctions that were present in at least half the individuals with the variant, and absent in all individuals without the variant.

Using the requirement that the predicted aberrant splice event is private to the individuals with the variant, we could validate 40% of predicted high-scoring (Δ Score ≥ 0.5) acceptor and donor gains, but only 3.4% of predicted high-scoring losses and 5.6% of essential GT or AG disruptions (at a false validation rate of < 0.2% based on permutations - see section "Estimating false validation rates"). The reason for the discrepancy in the validation rates of gains and losses is twofold. First, unlike gains, *exon*-skipping events are rarely entirely private to the individuals with the variant, because exons are often skipped at a low baseline level, which can be observed with sufficiently deep RNA-seq. Second, splice-site losses can have other effects besides increasing exon skipping, such as increasing intron retention or increasing the usage of alternative suboptimal splice sites. For these reasons, we did not rely entirely on private novel junctions for validating the model's predictions, we also validated variants based on quantitative evidence for the increase or decrease of the usage of the junction predicted to be affected in the individuals with the variant.

**Validation of predicted cryptic splice mutations through quantitative criteria**

For a junction $j$ from sample $s$, we obtained a normalized junction count $C_{js}$:

$$c_{js} = \operatorname{asinh}\left(\frac{r_{js}}{\sum_g r_{gs}}\right) \quad (1)$$

Here, $r_{js}$ is the raw junction count for junction $j$ in sample $s$, and the sum in the denominator is taken over all other junctions between annotated acceptors and donors of the same gene as $j$ (using annotations from GENCODE v19). The asinh transformation is defined as $\operatorname{asinh}(x) = \ln(x + \sqrt{x^2 + 1})$. It is similar to the log transformation often used to transform RNA-seq data (Lonsdale et al., 2013), however, it is defined at 0, thus eliminating the need for pseudocounts, which would have distorted values substantially, since many junctions, especially novel ones, have low or zero counts. The asinh transformation behaves like a log transformation for large values but is close to linear for small values. For this reason, it is often used in datasets (such as RNA-seq or ChIP-seq datasets) with a large number of near zero values to prevent a small number of large values from dominating the signal (Azad et al., 2016; Herring et al., 2018; Hoffman et al., 2012; Kasowski et al., 2013; SEQCIMAQC-III Consortium, 2014). As described below, in the section "Consideration criteria for validation", samples where the denominator in equation (1) was below 200 were excluded for all validation analyses, thus avoiding numerical issues.

For each gained or lost junction $j$ predicted to be caused by an SNV appearing in a set of individuals $I$, we computed the following z-score in each tissue $t$ separately:

$$z_{jt} = \frac{mean_{s \in A_t}(c_{js}) - mean_{s' \in U_t}(c_{js'})}{std_{s' \in U_t}(c_{js'})} \quad (2)$$

where $A_t$ is the set of samples from individuals in $I$ in tissue t and $U_t$ is the set of samples from all other individuals in tissue $t$. Note that there might be multiple samples in the GTEx dataset for the same individual and tissue. As before $C_{js}$ is the count for junction $j$ in sample $s$. For predicted losses, we also computed a similar z-score for the junction $k$ skipping the putatively affected exon:

$$z_{kt} = \frac{mean_{s' \in U_t}(c_{ks'}) - mean_{s \in A_t}(c_{ks})}{std_{s' \in U_t}(c_{ks'})} \quad (3)$$

Note that a loss that resulted in skipping would lead to a relative decrease of the lost junction and a relative increase in skipping. This justifies the reversion of the difference in the numerators of $z_{jt}$ and $z_{kt}$, so both of these scores would tend to be negative for a real splice site loss.

Finally, we computed the median z-score across all considered tissues. For losses, we computed the median of each of the z-scores from equations (2) and (3) separately. An acceptor or donor loss prediction was considered validated if any of the following was true:

1. The median of the z-scores from equation (2), quantifying the relative loss of the junction was less than the 5th percentile of the corresponding value in permuted data (-1.46) and the median of the z-scores from equation (3), quantifying the relative change in skipping was non-positive (zero, negative, or missing, which would be the case if the skipping junction was not observed in any individual). In other words, there was strong evidence for a reduction in the usage of the affected junction and no evidence suggesting a decrease in skipping in the affected individual.
2. The median of z-scores from equation (3) was less than the 5th percentile of the corresponding value in permuted data (-0.74) and the median of z-scores from equation (3) was non-positive.
3. The median of z-scores from equation (2) was less than the 1st percentile of the corresponding values in permuted data (-2.54).
4. The median of z-scores from equation (3) was less than the 1st percentile of the corresponding values in permuted data (-4.08).
5. The junction skipping the affected exon was observed in at least half of the individuals with the variant and in no other individuals (as described in the section "Validation of predicted cryptic splice mutations based on private splice junctions" above).

A description of the permutations used to get the above cutoffs is given in the section "Estimating false validation rates".

Empirically, we observed that we needed to apply stricter validation criteria for losses compared to gains, since, as explained in the section "Validation of predicted cryptic splice mutations based on private splice junctions", losses tend to result in more mixed effects than gains. Observing a novel junction near a private SNV is very unlikely to occur by chance, so even minor evidence of the junction should be sufficient for validation. In contrast, most predicted losses resulted in weakening of an existing junction, and such weakening is harder to detect than the on-off change caused by gains and more likely to be attributed to noise in the RNA-seq data.

## Inclusion criteria for validation analysis

To avoid computing z-scores in the presence of low counts or poor coverage, we used the following criteria to filter variants for the validation analysis:

1. Samples were considered for the above z-score calculation only if they expressed the gene $(\sum_g r_{gs} > 200$ in equation (1)).

2. A tissue was not considered for a loss or gain z-score calculation if the average count of the lost or "reference" junction respectively in individuals without the variant was less than 10. The "reference" junction is the canonical junction used prior to the gain of the novel junction, based on GENCODE annotations (see section on effect size calculation for details). The intuition is that we should not try to validate a splice-loss variant that affects a junction not expressed in control individuals. Similarly, we should not try to validate a splice-gain variant if control individuals did not sufficiently express transcripts spanning the affected site.

3. In the case of a predicted splice-site loss, samples from individuals without the variant were only considered if they had at least 10 counts of the lost junction. In the case of a predicted acceptor or donor gain, samples from control individuals were only considered if they had at least 10 counts of the "reference" junction. The intuition is that even in a tissue with large average expression of the affected junction (i.e. passing criterion 2.), different samples could have vastly different sequencing depths, so only the control samples with sufficient expression should be included.

4. A tissue was considered only if there was at least one sample passing the above criteria from individuals with the variant, as well as at least 5 samples passing the above criteria from at least 2 distinct control individuals.

Variants for which there were no tissues satisfying the above criteria for consideration were deemed non-ascertainable and were excluded when calculating the validation rate. For splice-gain variants, we filtered those that occur at already existing GENCODE-annotated splice sites. Similar, for splice-loss variants, we only considered those that decrease the scores of existing GENCODE-annotated splice sites. Overall, 55% and 44% of high-scoring ($\Delta$ Score $\geq$ 0.5) predicted gains and losses respectively were considered ascertainable and used for the validation analysis.

### Estimating false validation rates

To ensure that the above procedure had reasonable true validation rates, we first looked at SNVs that appear in 14 GTEx individuals and disrupt essential GT/AG dinucleotides. We argued that such mutations almost certainly affect splicing so their validation rate should be close to 100%. Among such disruptions, 39% were ascertainable based on the criteria described above, and among the ascertainable ones, the validation rate was 81%. To estimate the false validation rate, we permuted the individual labels of the SNV data. For each SNV that appeared in $k$ GTEx individuals, we picked a random subset of $k$ GTEx individuals and assigned the SNV to them. We created 10 such randomized datasets and repeated the validation process on them. The validation rate in the permuted datasets was 1.7-2.1% for gains and 4.3-6.9% for losses, with a median of 1.8% and 5.7% respectively. The higher false validation rate for losses and the relatively low validation rate of essential disruptions are due to the difficulty in validating splice-site losses as highlighted in the section "Validation of predicted cryptic splice mutations based on private splice junctions".

### Calculating the effect size of cryptic splice variants in RNA-sea data

We defined the "effect size" of a variant as the fraction of transcripts of the affected gene that changed splicing patterns due to the variant (e.g. the fraction that switched to a novel acceptor or donor). As a reference example for a predicted splice-gain variant, consider the variant in **FIG. 38C.** For a predicted gained donor A, we first identified the junction (AC) to the closest annotated acceptor C. We then identified a "reference" junction (BC), where B $\neq$ A is the annotated donor closest to A. In each sample s, we then computed the relative usage of the novel junction (AC) compared to the reference junction (BC):

$$u_{(AB)s} = \frac{r_{(AC)s}}{r_{(AC)s} + r_{(BC)s}} \quad (4)$$

Here, $r_{(AC)s}$ is the raw read count of junction (AC) in sample s. For each tissue, we computed the change in the usage of the junction (AC) between the individuals with the variant and all other individuals:

$$mean_{s \in A_t} u_{(AC)s} - mean_{s' \in U_t} u_{(AC)s'} \quad (5)$$

where $A_t$ is the set of samples from individuals with the variant in tissue $t$ and $U_t$ is the set of samples from other individuals in tissue *t*. The final effect size was computed as the median of the above difference across all considered tissues. The computation was similar in the case of a gained acceptor or in the case where the splice-site creating variant was intronic. A simplified version of the effect size computation (assuming a single sample from individuals with and without the variant) is shown in **FIG. 38C.**

For a predicted loss, we first computed the fraction of transcripts that skipped the affected exon. The computation is demonstrated on **FIG. 45.** For a predicted loss of a donor C, we identified the junction (CE) to the next downstream annotated exon, as well as the junction (AB) from the upstream exon to the putatively affected one. We quantified the fraction of transcripts that skipped the affected exon as follows:

$$k_{(AE)s} = \frac{r_{(AE)s}}{r_{(AE)s} + mean(r_{(AB)s} + r_{(CE)s})} \quad (6)$$

As for gains, we then computed the change in the skipped fraction between samples from individuals with the variant and samples from individuals without the variant:

$$mean_{s \in A_t} k_{(AE)s} - mean_{s' \in U_t} k_{(AE)s'} \quad (7)$$

The fraction of skipped transcripts as computed above does not fully capture the effects of an acceptor or donor loss, as such a disruption could also lead to increased levels of intron retention or usage of suboptimal splice sites. To account for some of these effects, we also computed the usage of the lost junction (CE) relative to the usage of other junctions with the same acceptor E:

$$l_{(CE)s} = \frac{r_{(CE)s}}{\sum r_{(\cdot E)s}} \quad (8)$$

Here, $\sum r_{(\cdot E)s}$ is the sum of all junctions from any (annotated or novel) acceptor to the donor E. This includes the affected junction (CE), the skipping junction (AE), as well as potential junctions from other suboptimal donors that compensated for the loss of C, as illustrated in the example in **FIG. 45.** We then computed the change in the relative usage of the affected junction:

$$mean_{s' \in U_t} l_{(CE)s'} - mean_{s \in A_t} l_{(CE)s} \quad (9)$$

Note that, unlike (5) and (7), which measure the increase in usage of the gained or skipping junction in individuals with the variant, in (9) we want to measure the decrease in usage of the lost junction, hence the reversion of the two parts of the difference. For each tissue the effect size was computed as the maximum of (7) and (9). As for gains, the final effect size for the variant was the median effect size across tissues.

### Inclusion criteria for effect size analysis

A variant was considered for effect size computation only if it was deemed validated based on the criteria described in the previous section. To avoid calculating the fraction of aberrant transcripts on very small numbers, we only considered samples where the counts of the aberrant and reference junctions were both at least 10. Because most cryptic splice variants were in the intron, the effect size could not be computed directly by counting the number of reference and alternate reads overlapping the variant. Hence, the effect size of losses is calculated indirectly from the decrease in the relative usage of the normal splice junction. For the effect size of novel junction gains, the aberrant transcripts can be impacted by nonsense mediated decay, attenuating the observed effect sizes. Despite the limitations of these measurements, we observe a consistent trend towards smaller effect sizes for lower-scoring cryptic splice variants across both gain and loss events.

### Expected effect size of fully penetrant heterozygous private SNVs

For a fully penetrant splice-site creating variant that causes all transcripts from the variant haplotype of the individuals with the variant to switch to the novel junction, and assuming that the novel junction does not occur in control individuals, the expected effect size would be 0.5 by equation (5).

Similarly, if a heterozygous SNV causes a novel exon skipping event, and all transcripts of the affected haplotype switched to the skipping junction, the expected effect size in equation (7) is 0.5. If all transcripts from individuals with the variant switched to a different junction (either the skipping junction, or another compensating one), the ratio in equation (8) would be 0.5 in samples from individuals with the variant and 1 in samples from other individuals, so the difference in equation (9) would be 0.5. This assumes that that there was no skipping or other junctions into acceptor E in individuals without the variant. It also assumes that the splice site disruption does not trigger intron retention. In practice, at least low levels of intron retention are often associated with splice site disruptions. Furthermore, exon skipping is widespread, even in the absence of splice-altering variants. This explains why the measured effect sizes are below 0.5, even for variants disrupting essential GT/AG dinucleotides.

The expectation of effect sizes of 0.5 for fully penetrant heterozygous variants also assumes that the variant did not trigger nonsense-mediated decay (NMD). In the presence of NMD, both the numerator and the denominator of equations (4), (6), and (8) would drop, thus diminishing the observed effect size.

**Fraction of transcripts degraded through nonsense-mediated decay (NMD)**

For **FIG. 38C** since the variant was exonic, we could count the number of reads that spanned the variant and had the reference or the alternate allele ("Ref (no splicing)" and "Alt (no splicing)" respectively). We also counted the number of reads that spliced at the novel splice site, and that presumably carried the alternate allele ("Alt (novel junction)"). In the example of **FIG. 38C** and in many other cases we looked at, we observed that the total number of reads coming from the haplotype with the alternate allele (the sum of "Alt (no splicing)" and "Alt (novel junction)") was less than the number of reads with the reference allele ("Ref (no splicing)"). Since we believe that we have eliminated reference biases during read mapping, by mapping to both the reference and alternate haplotypes, and assuming that the number of reads is proportional to the number of transcripts with each allele, we were expecting that the reference allele would account for half of the reads at the variant locus. We assume that the "missing" alternate allele reads correspond to transcripts from the alternate allele haplotype that spliced at the novel junction and were degraded through nonsense mediated decay (NMD). We called this group "Alt (NMD)".

To determine whether the difference between the observed number of reference and alternate reads was significant we computed the probability of observing Alt (no splicing) + Alt (novel junction) (or fewer) reads under a binomial distribution with success probability 0.5 and a total number of trials of Alt (no splicing) + Alt (novel junction) + Ref (no splicing). This is a conservative p-value since we are underestimating the total number of "trials" by not counting the potentially degraded transcripts. The fraction of NMD transcripts in **FIG. 38C** was computed as the number of "Alt (NMD)" reads over the total number of reads splicing at the novel junction (Alt (NMD) + Alt (novel junction)).

**Sensitivity of the network at detecting cryptic splice junctions**

For evaluating the sensitivity of the SpliceNet model (**FIG. 38F**), we used SNVs that were within 20 nt from the affected splice site (i.e. the novel or disrupted acceptor or donor) and not overlapping the essential GT/AG dinucleotide of an annotated exon, and had an estimated effect size of at least 0.3 (sec section "Effect size calculation"). In all sensitivity plots, SNVs were defined as being "near exons" if they overlapped an annotated exon or were within 50 nt of the boundaries of an annotated exon. All other SNVs were considered "deep intronic". Using this truth dataset of strongly supported cryptic splice sites, we evaluated our model at varying Δ Score thresholds and report the fraction of cryptic splice sites in the truth dataset that are predicted by the model at that cutoff.

**Comparison with existing splicing prediction models**

We performed a head-to-head comparison of SpliceNet-10k, MaxEntScan (Yeo and Burge, 2004), GeneSplicer (Pertea et al., 2001) and NNSplice (Reese et al., 1997) with respect to various metrics. We downloaded the MaxEntScan and GeneSplicer software from http://genes.mit.edu/burgelab/maxent/download/ and http://www.cs;jhu.edu/-genomics/GeneSplicer/ respectively. NNSplice is not available as a downloadable software, so we downloaded the training and testing sets from http://www.fruitfly.org/data/seq_tools/datasets/Human/GENIE_96/splicesets/, and trained models with the best performing architectures described in (Reese et al., 1997). As a sanity check, we reproduced the test set metrics reported in (Reese et al., 1997).

To evaluate the top-k accuracies and the area under the precision-recall curves of these algorithms, we scored all the positions in the test set genes and lincRNAs with each algorithm (**FIG. 37D**).

MaxEntScan and GeneSplicer outputs correspond to log odds ratios, whereas NNSplice and SpliceNet-10k outputs correspond to probabilities. To ensure that we gave MaxEntScan and GeneSplicer the best chance of success, we calculated Δ Scores using them with the default output as well as with a transformed output where we first transform their outputs so that they correspond to probabilities. More precisely, the default output of MaxEntScan corresponds to

$$x = \log_2 \frac{p(splice\ site)}{p(not\ a\ splice\ site)}$$

which, after the transformation $\frac{2^x}{2^x+1}$, corresponds to the desired quantity. We compiled the GeneSplicer software twice, once by setting the RETURN TRUE PROB flag to 0 and once by setting it to 1. We picked the output strategy that led to the best validation rate against RNA-seq data (MaxEntScan: transformed output, GeneSplicer: default output).

To compare the validation rate and sensitivity of the various algorithms (**FIG. 38G**), we found cutoffs at which all algorithms predicted the same number of gains and losses genome-wide. That is, for each cutoff on the SpliceNet-10k $\Delta$ Score values, we found the cutoffs at which each competing algorithm would make the same number of gain predictions and the same number of loss predictions as SpiceNet-10k. The chosen cutoffs are given in Table S2.

**Comparison of variant prediction for singleton versus common variants**

We performed the validation and sensitivity analysis (as described in sections "Sensitivity analysis" and "Validation of model predictions") separately for singleton SNVs and SNVs appearing in 2-4 GTEx individuals **(FIGs. 46A, 46B,** and **46C).** To test whether the validation rate differed significantly between singleton and common variants we performed a Fisher Exact test, comparing the validation rates in each $\Delta$ Score group (0.2 - 0.35, 0.35 - 0.5, 0.5 - 0.8, 0.8 - 1) and for each predicted effect (acceptor or donor gain or loss). After Bonferroni correction to account for 16 tests, all P-values were greater than 0.05. We similarly compared the sensitivity for detecting singleton or common variants. We used a Fisher Exact test to test whether the validation rate differed significantly between the two groups of variants. We considered deep-intronic variants and variants near exons separately and performed Bonferroni correction for two tests. None of the P-values were significant using a 0.05 cutoff. We therefore combined singleton and common GTEx variants and considered them together for the analyses presented in **FIGs. 48A, 48B, 48C, 48D**, 48E, 48F, and 48G and **FIGs. 39A, 39B,** and **39C.**

**Comparison of variant prediction on the training versus testing chromosomes**

We compared the validation rate on RNA-seq and sensitivity of SpliceNet-10k between variants on the chromosomes used during training and variants on the rest of the chromosomes **(FIGs. 48A** and **48B).** All P-values were greater than 0.05 after Bonferroni correction. We also computed the fraction of deleterious variants separately for variants on the training and test chromosomes, as described in the section "Fraction of deleterious variants" below **(FIGs. 48C).** For each A Score group and each type of variant, we used a Fisher Exact test to compare the number of common and rare variants between training and test chromosomes. After Bonferroni correction for 12 tests, all P-values were greater than 0.05. Finally, we computed the number of cryptic splice *de novo* variants on the training and test chromosomes **(FIGs. 48D)** as described in the section "Enrichment *of de novo* mutations per cohort".

**Comparison of variant prediction across different types of cryptic splice variants**

We split predicted splice site-creating variants into three groups: variants creating a novel GT or AG splice dinucleotide, variants overlapping the rest of the splicing motif (positions around the exon-intron boundary up to 3 nt into the exon and 8 nt into the intron), and variants outside the splice motif **(FIGs. 47A** and **47B).** For each $\Delta$ Score group (0.2 - 0.35, 0.35 - 0.5, 0.5 - 0.8, 0.8 - 1), we performed a $X^2$ test to test the hypothesis that the validation rate is uniform across the three types of splice site-creating variants. All tests yielded P-values > 0.3 even before multiple hypothesis correction. To compare the effect size distribution between the three types of variants, we used a Mann-Whitney *U* test and compared all three pairs of variant types for each $\Delta$ Score group (for a total of 4 x 3 = 12 tests). After Bonferroni correction for 12 tests, all P-values were > 0.3.

**Detection of tissue-specific splice-gain variants**

For **FIG. 39C,** we wanted to test whether the usage rate of novel junctions was uniform across tissues expressing the affected gene. We focused on SNVs that created novel private splice sites, that is, SNVs resulting in a gained splice junction which only appeared in at least half of the individuals with the variant and in no other individuals. For each such novel junction j, we computed, in each tissue t, the total counts of the junction across all samples from individuals with the variant in the tissue: $\sum_{s \in A_t} r_{js}$. Here $A_t$ is the set of samples from individuals with the variant in tissue $t$. Similarly, we computed the total counts of all annotated junctions of the gene for the same samples $\sum_{s \in A_t} \sum_g r_{gs}$, where $g$ indexes the annotated junctions of the gene. The relative usage of the novel junction in tissue $t$, normalized against the gene's background counts, can then be measured as:

$$m_t = \frac{\sum_{s \in A_t} r_{js}}{\sum_{s \in A_t} (r_{js} + \sum_g r_{gs})}$$

We also computed the average usage of the junction across tissues:

$$m = \frac{\sum_t \sum_{s \in A_t} r_{js}}{\sum_t \sum_{s \in A_t} (r_{js} + \sum_g r_{gs})}$$

We wanted to test the hypothesis that the relative usage of the junction is uniform across tissues and equal to $m$. We thus performed a $X^2$ test comparing the observed tissue counts $\sum_{s \in A_t} r_{js}$ to the expected counts under the assumption of a uniform rate, $m \sum_{s \in A_t} (r_{js} + \sum_g r_{gs})$. A splice-site creating variant was considered tissue-specific if the Bonferroni-corrected $X^2$ p-value was less than $10^{-2}$. The degrees of freedom for the test are T - 1, where T is the number of considered tissues. Only tissues that satisfied the consideration criteria described in the validation section were used in the test. Further, to avoid cases with low counts, where the uniformity test was underpowered, we only tested for uniformity variants with at least three considered tissues, at least one aberrant read per tissue on average (i.e. $m > 1$), and at least 15 aberrant reads in total across all considered tissues (i.e. $\sum_t \sum_{s \in A_t} r_{js} > 15$). We ignored all variants with $\Delta$ Score less than 0.35, since this class of variants has generally low effect sizes and low junction counts. We observed that the fraction of tissue-specific variants was very low for this class, but we believe that this was due to power issues.

## III. Analyses on the ExAC and gnomAD datasets

### Variant filtering

We downloaded the Sites VCF release 0.3 file (60,706 exomes) from the ExAC browser (Lek et al., 2016) and the Sites VCF release 2.0.1 file (15,496 whole genomes) from the gnomAD browser. We created a filtered list of variants from them in order to evaluate SpliceNet-10k. In particular, variants which satisfied the following criteria were considered:

- The FILTER field was PASS.
- The variant was a single nucleotide variant, and there was only one alternate nucleotide.
- The AN field (total number of alleles in called genotypes) had a value at least 10,000.
- The variant was in between the transcription start and end site of a canonical GENCODE transcript.

A total of 7,615,051 and 73,099,995 variants passed these filters in the ExAC and gnomAD datasets respectively.

### Fraction of deleterious variants

For this analysis, we considered only those variants in the ExAC and gnomAD filtered lists which were singleton or common (allele frequency (AF) $\geq 0.1\%$) in the cohort. We sub-classified these variants based on their genomic position according to the GENCODE canonical annotations:

- Exonic: This group consists of synonymous ExAC variants (676,594 singleton and 66,524 common). Missense variants were not considered here to ensure that most of the deleteriousness of the variants in this group was due to splicing changes.
- Near intronic: This group consists of intronic ExAC variants which are between 3 and 50 nt from a canonical exon boundary. More precisely, for the analysis of acceptor gain/loss and donor gain/loss variants, only those variants which were 3-50 nt from a splice acceptor and donor respectively were considered (575,636 singleton and 48,362 common for acceptor gain/loss, 567,774 singleton and 50,614 common for donor gain/loss).
- Deep intronic: This group consists of intronic gnomAD variants which are more than 50 nt away from a canonical exon boundary (34,150,431 singleton and 8,215,361 common).

For each variant, we calculated its $\Delta$ Scores for the four splice types using SpliceNet-10k. Then, for each splice type, we constructed a 2 x 2 chi-square contingency table where the two rows corresponded to predicted splice-altering variants ($\Delta$ Score in the appropriate range for the splice type) vs predicted not splice-altering variants ($\Delta$ Score < 0.1 for all splice types), and the two columns corresponded to singleton vs common variants. For splice-gain variants, we filtered those that occur at already existing GENCODE-annotated splice sites. Similar, for splice-loss variants, we only considered those that decrease the scores of existing GENCODE-annotated splice sites. The odds ratio was calculated, and the fraction of deleterious variants was estimated as

$$\left(1 - \frac{1}{\text{Odds ratio}}\right) \times 100\%$$

The protein-truncating variants in the ExAC and gnomAD filtered lists were identified as follows:

- Nonsense: VEP (McLaren et al., 2016) consequence was 'stop_gained' (44,046 singleton and 722 common in ExAC, 20,660 singleton and 970 common in gnomAD).
- Frameshift: VEP consequence was 'frameshift_variant'. The single nucleotide variant criterion during variant filtering was relaxed in order to create this group (48,265 singleton and 896 common in ExAC, 30,342 singleton and 1,472 common in gnomAD).
- Essential acceptor/donor loss: The variant was in the first or the last two positions of a canonical intron (29,240 singleton and 481 common in ExAC, 12,387 singleton and 746 common in gnomAD).

The 2 x 2 chi-square contingency table for protein-truncating variants was constructed for the ExAC and gnomAD filtered lists, and used to estimate the fraction of deleterious variants. Here, the two rows corresponded to protein-truncating vs synonymous variants, and the two columns corresponded to singleton vs common variants as before.

The results for the ExAC (exonic and near intronic) and gnomAD (deep intronic) variants are shown in **FIGs. 40B** and **40D** respectively.

### Frameshift vs in-frame splice gain

For this analysis, we focused our attention on the ExAC variants which were exonic (synonymous only) or near intronic, and were singleton or common (AF $\geq$ 0.1%) in the cohort. To classify an acceptor gain variant as in-frame or frameshift, we measured the distance between the canonical splice acceptor and the newly created splice acceptor, and checked whether it was a multiple of 3 or not. We classified donor gain variants similarly by measuring the distance between the canonical splice donor and the newly created splice donor.

The fraction of deleterious in-frame splice gain variants was estimated from a 2 x 2 chi-square contingency table where the two rows corresponded to predicted in-frame splice gain variants ($\Delta$ Score $\geq$ 0.8 for acceptor or donor gain) vs predicted not splice-altering variants ($\Delta$ Score < 0.1 for all splice types), and the two columns corresponded to singleton vs common variants. This procedure was repeated for frameshift splice gain variants by replacing the first row in the contingency table with predicted frameshift splice gain variants.

To calculate the p-value shown in **FIG. 40C,** we constructed a 2 x 2 chi-square contingency table using only the predicted splice gain variants. Here, the two rows corresponded to in-frame vs frameshift splice gain variants, and the two columns corresponded to singleton vs common variants as before.

### Number of cryptic splice variants per individual

To estimate the number of rare functional cryptic splice variants per individual (**FIG. 40E**), we first simulated 100 gnomAD individuals by including each gnomAD variant in each allele with a probability equal to its allele frequency. In other words, each variant was sampled twice independently for each individual to mimic diploidy. We counted the number of rare (AF < 0.1%) exonic (synonymous only), near intronic, and deep intronic variants per person which had a $\Delta$ Score greater than or equal to 0.2, 0.2, and 0.5 respectively. These are relatively permissive $\Delta$ Score thresholds which optimize sensitivity while ensuring that at least 40% of the predicted variants are deleterious. At these cutoffs, we obtained an average of 7.92 synonymous/near intronic and 3.03 deep intronic rare cryptic splice variants per person. Because not all of these variants are functional, we multiplied the counts by the fraction of variants that are deleterious at these cutoffs.

## IV. Analyses on the DDD and ASD datasets

## Cryptic splicing de novo mutations

We obtained published de novo mutations (DNMs). These included 3953 probands with autism spectrum disorder (Dong et al., 2014; Iossifov et al., 2014; De Rubeis et al., 2014), 4293 probands from the Deciphering Developmental Disorders cohort (McRae et al., 2017) and 2073 healthy controls (Iossifov et al., 2014). Low quality DNMs were excluded from analyses (ASD and healthy controls: Confidence = lowConf, DDD: PP(DNM) < 0.00781, (McRae et al., 2017)). The DNMs were evaluated with the network, and we used Δ scores (see methods above) to classify cryptic splice mutations depending on the context. We only considered mutations annotated with VFP consequences of synonymous variant, splice region _variant, intron _variant, 5_prime_UTR_variant, 3_primc_UTR_variant or missense_variant. We used sites with Δ scores > 0.1 for **FIGs. 41A, 41B, 41C, 41D, 41E, and 41F** and **FIGs. 50A** and **50B,** and sites with Δ scores > 0.2 for **FIGs. 49A, 49B, and 49C.**

**FIGs. 20, 21, 22, 23,** and **24** show detailed description of the SpliceNet-80nt, SpliceNet-400nt, SpliceNet-2k and SpliceNet-10k architectures. The four architectures use flanking nucleotide sequence of lengths 40, 200, 1,000 and 5,000 respectively on each side of the position of interest as input, and output the probability of the position being a splice acceptor, splice donor and neither. The architectures mainly consist of convolutional layers Conv(N, W, D), where N, W and D are the number of convolutional kernels, window size and dilation rate of each convolutional kernel in the layer respectively.

**FIGs. 42A and 42B** depict evaluation of various splicing prediction algorithms on lincRNAs. **FIG. 42A** shows the top-k accuracies and the area under the precision-recall curves of various splicing prediction algorithms when evaluated on lincRNAs. **FIG. 42B** shows the full pre-mRNA transcript for the LINC00467 gene scored using MaxEntScan and SpliceNet-10k, along with predicted acceptor (red arrows) and donor (green arrows) sites and the actual positions of the exons.

**FIGs. 43A and 43B** illustrate position-dependent effects of the TACTAAC branch point and GAAGAA exonic-splice enhancer motifs. Regarding **FIG. 43A,** the optimal branch point sequence TACTAAC was introduced at various distances from each of the 14,289 test set splice acceptors and the acceptor scores were calculated using SpliceNet-10k. The average change in the predicted acceptor score is plotted as a function of the distance from the splice acceptor. The predicted scores increase when the distance from the splice acceptor is in between 20 and 45 nt; at less than 20 nt distance, TACTAAC disrupts the polypyrimidine tract due to which the predicted acceptor scores are very low.

Regarding **FIG. 43B,** the SR-protein hexamer motif GAAGAA was similarly introduced at various distances from each of the 14,289 test set splice acceptors and donors. The average change in the predicted SpliceNet-10k acceptor and donor scores are plotted as a function of the distance from the splice acceptor and donor respectively. The predicted scores increase when the motif is on the exonic side and less than ~50 nt from the splice site. At larger distances into the exon, the GAAGAA motif tends to disfavor the usage of the splice acceptor or donor site under consideration, presumably because it now preferentially supports a more proximal acceptor or donor motif. The very low acceptor and donor scores when GAAGAA is placed at positions very close to the intron is due to disruption of the extended acceptor or donor splice motifs.

**FIGs. 44A** and **44B** depict effects of nucleosome positioning on splicing. Regarding **FIG. 44A,** at 1 million randomly chosen intergenic positions, strong acceptor and donor motifs spaced 150 nt apart were introduced and the probability of exon inclusion was calculated using SpliceNet-10k. To show that the correlation between SpliceNet-10k predictions and nucleosome positioning occurs independent of GC composition, the positions were binned based on their GC content (calculated using the 150 nucleotides between the introduced splice sites) and the Spearman correlation between SpliceNet-1 0k predictions and nucleosome signal is plotted for each bin.

Regarding **FIG. 44B,** splice acceptor and donor sites from the test set were scored using SpliceNet-80nt (referred to as local motif score) and SpliccNet-1 0k, and the scores are plotted as a function of nucleosome enrichment. Nucleosome enrichment is calculated as the nucleosome signal averaged across 50 nt on the exonic side of the splice site divided by the nucleosome signal averaged across 50 nt on the intronic side of the splice site. SpliceNet-80nt score, which is a surrogate for motif strength, is negatively correlated with nucleosome enrichment, whereas SpliceNet-10k score is positively correlated with nucleosome enrichment. This suggests that nucleosome positioning is a long range specificity determinant that can compensate for weak local splice motifs.

**FIG. 45** illustrates example of calculating effect size for a splice-disrupting variant with complex effects. The intronic variant chr9:386429 A>G disrupts the normal donor site (C) and activates a previously suppressed intronic downstream donor (D). Shown are the RNA-seq coverage and junction read counts in whole blood from the individual with the variant and a control individual. The donor sites in the individual with the variant and the control individual are marked with blue and grey arrows respectively. Bold red letters correspond to junction endpoints. For visibility, exon lengths have been exaggerated 4-fold compared to intron lengths. To estimate the effect size, we compute both the increase in the usage of the exon skipping junction (AE) and the decrease in the usage of the disrupted junction (CE) relative to all other junctions with the same donor E. The final effect size is the maximum of the two values (0.39). An increased amount of intron retention is also present in the mutated sample. These variable effects are common at exon skipping events and increase the complexity of validating rare variants that are predicted to cause acceptor or donor site loss.

**FIGs. 46A, 46B,** and **46C** show evaluation of the SpliceNet-10k model on singleton and common variants. Regarding **FIG. 46A,** fraction of cryptic splice mutations predicted by SpliceNet-10k that validated against the GTEx RNA-seq data. The model was evaluated on all variants appearing in at most four individuals in the GTEx cohort. Variants with predicted splice-altering effects were validated against RNA-seq data. The validation rate is shown separately for variants appearing in a single GTEx individual (left) and variants appearing in two to four GTEx individuals (right). Predictions are grouped by their $\Delta$ Score. We compared the validation rate between singleton and common variants for each of the four classes of variants (gain or loss of acceptor or donor) in each $\Delta$ Score group. The differences are not significant (P > 0.05, Fisher Exact test with Bonferroni correction for 16 tests).

Regarding **FIG. 46B,** sensitivity of SpliceNet-10k at detecting splice-altering variants in the GTEx cohort at different $\Delta$ Score cutoffs. The model's sensitivity is shown separately for singleton (left) and common (right) variants. The differences in sensitivity between singleton and common variants at a $\Delta$ Score cutoff of 0.2 are not significant for either variants near exons or deep intronic variants (P > 0.05, Fisher Exact test with Bonferroni correction for two tests).

Regarding **FIG. 46C,** distribution of $\Delta$ Score values for validated singleton and common variants. P-values are for Mann-Whitney U tests comparing the scores of singleton and common variants. Common variants have significantly weaker $\Delta$ Score values, due to natural selection filtering out splice-disrupting mutations with large effects.

**FIGs. 47A** and **47B** depict validation rate and effect sizes of splice site-creating variants, split by the location of the variant. Predicted splice site-creating variants were grouped based on whether the variant created a new essential GT or AG splice dinucleotide, whether it overlapped the rest of the splice motif (all positions around the exon-intron boundary up to 3 nt into the exon and 8 nt into the intron, excluding the essential dinucleotide), or whether it was outside the splice motif.

Regarding **FIG. 47A,** validation rate for each of the three categories of splice site-creating variants. The total number of variants in each category is shown above the bars. Within each $\Delta$ Score group, the differences in validation rates between the three groups of variants are not significant (P > 0.3, $\chi^2$ test of uniformity).

Regarding **FIG. 47B,** distribution of effect sizes for each of the three categories of splice site-creating variants. Within each $\Delta$ Score group, the differences in effect sizes between the three groups of variants are not significant (P > 0.3, Mann-Whitney U test with Bonferroni correction).

**FIGs. 48A, 48B, 49C,** and 49D depict evaluation of the SpliceNet-10k model on train and test chromosomes. Regarding **FIG. 48A,** fraction of cryptic splice mutations predicted by the SpliceNet-10k model that validated against the GTEx RNA-seq data. The validation rate is shown separately for variants on chromosomes used during training (all chromosomes except chr1, chr3, chr5, chr7, and chr9; left) and the rest of the chromosomes (right). Predictions are grouped by their $\Delta$ Score. We compared the validation rate between train and test chromosomes for each of the four classes of variants (gain or loss of acceptor or donor) in each $\Delta$ Score group. This accounts for potential differences in the distribution of predicted $\Delta$ Score values between train and test chromosomes. The differences in validation rates are not significant (P > 0.05, Fisher Exact test with Bonferroni correction for 16 tests).

Regarding **FIG. 48B,** sensitivity of SpliceNet-10k at detecting splice-altering variants in the GTEx cohort at different $\Delta$ Score cutoffs. The model's sensitivity is shown separately for variants on the chromosomes used for training (left) and on the rest of the chromosomes (right). We used a Fisher Exact test to compare the model's sensitivity at a $\Delta$ Score cutoff of 0.2 between train and test chromosomes. The differences are not significant for either variants near exons or deep intronic variants (P > 0.05 after Bonferroni correction for two tests).

Regarding **FIG. 48C,** fraction of predicted synonymous and intronic cryptic splice variants in the ExAC dataset that are deleterious, calculated separately for variants on chromosomes used for training (left) and the rest of the chromosomes (right). Fractions and P-values are computed as shown in Figure 4A. We compared the number of common and rare variants between training and test chromosomes for each of the four classes of variants (gain or loss of acceptor or donor) in each $\Delta$ Score group. The differences are not significant (P > 0.05, Fisher Exact test with Bonferroni correction for 12 tests).

Regarding **FIG. 48D,** predicted cryptic splice de novo mutations (DNMs) per person for DDD, ASD, and control cohorts, shown separately for variants on the chromosomes used for training (left) and the rest of the chromosomes (right). Error bars show 95% confidence intervals (CI). The number of cryptic splice de novo variants per person is smaller for the test set because it is roughly half the size of the training set. Numbers are noisy due to small sample size.

**FIG. 49A, 49B,** and **49C** illustrate de novo cryptic splice mutations in patients with rare genetic disease, only from synonymous, intronic or untranslated region sites. Regarding **FIG. 49A,** predicted cryptic splice de novo mutations (DNMs) with cryptic splice $\Delta$ score > 0.2 per person for patients from the Deciphering Developmental Disorders cohort (DDD), individuals with autism spectrum disorders (ASD) from the Simons Simplex Collection and the Autism Sequencing Consortium, as well as healthy controls. Enrichment in the DDD and ASD cohorts above healthy controls is shown, adjusting for variant ascertainment between cohorts. Error bars show 95% confidence intervals.

Regarding **FIG. 49B,** estimated proportion of pathogenic DNMs by functional category for the DDD and ASD cohorts, based on the enrichment of each category compared to healthy controls. The cryptic splice proportion is adjusted for the lack of missense and deeper intronic sites.

Regarding **FIG. 49C,** enrichment and excess of cryptic splice DNMs in the DDD and ASD cohorts compared to healthy controls at different Δ score thresholds. The cryptic splice excess is adjusted for the lack of missense and deeper intronic sites.

**FIGs. 50A** and **50B** show cryptic splice de novo mutations in ASD and as a proportion of pathogenic DNMs. Regarding **FIG. 50A,** enrichment and excess of cryptic splice DNMs within ASD probands at different Δ score thresholds for predicting cryptic splice sites.

Regarding **FIG. 50B,** proportion of pathogenic DNMs attributable to cryptic splice sites as a fraction of all classes of pathogenic DNMs (including proteincoding mutations), using different Δ score thresholds for predicting cryptic splice sites. More permissive Δ score thresholds increase the number of cryptic splice sites identified over background expectation, at the trade-off of having a lower odds ratio.

**FIG. 51** depicts RNA-seq validation of predicted cryptic splice de novo mutations in ASD patients. Coverage and splice junction counts of RNA expression from 36 predicted cryptic splice sites selected for experimental validation by RNA-seq. For each sample, RNA-seq coverage and junction counts for the affected individual are shown at the top, and a control individual without the mutation is shown at the bottom. The plots are grouped by validation status and splice aberration type.

**FIGs. 52A** and **52B** illustrate validation rate and sensitivity on RNA-seq of a model trained on canonical transcripts only. Regarding **FIG. 52A,** we trained the SpliceNet-10k model using only junctions from canonical G ENCODE transcripts and compared the performance of this model to a model trained on both canonical junctions and splice junctions appearing in at least five individuals in the GTEx cohort. We compared the validation rates of the two models for each of the four classes of variants (gain or loss of acceptor or donor) in each Δ Score group. The differences in validation rates between the two models are not significant (P > 0.05, Fisher Exact test with Bonferroni correction for 16 tests).

Regarding **FIG. 52B,** sensitivity of the model that was trained on canonical junctions at detecting splice-altering variants in the GTEx cohort at different Δ Score cutoffs. The sensitivity of this model in deep intronic regions is lower than that of the model on Figure 2 (P < 0.001, Fisher Exact test with Bonferroni correction). The sensitivity near exons is not significantly different.

**FIGs. 53A, 53B,** and **53C** illustrate ensemble modeling improves SpliceNet-10k performance. Regarding **FIG. 53A,** the top-k accuracies and the area under the precision-recall curves of the 5 individual SpliceNet-IOk models are shown. The models have the same architecture and were trained using the same dataset. However, they differ from each other due to the various random aspects involved in the training process, such as parameter initialization, data shuffling, etc.

Regarding **FIG. 53B,** the predictions of the 5 individual SpliceNet-10k models are highly correlated. For this study, we only considered those positions in the test set which were assigned an acceptor or donor score greater than or equal to 0.01 by at least one model. Subplot (i, j) is constructed by plotting the predictions of Model #i against the predictions of Model #j (the corresponding Pearson correlation is displayed above the subplot).

Regarding **FIG. 53C,** the performance improves as the number of models used to construct the SpliceNet-10k ensemble is increased from 1 to 5.

**FIGs. 54A** and **54B** show evaluation of SpliceNet-1 0k in regions of varying exon density. Regarding **FIG. 54A,** the test set positions were categorized into 5 bins depending on the number of canonical exons present in a 10,000 nucleotide window. For each bin, we calculated the top-k accuracy and the area under the precision-recall curve for SpliceNet-10k.

Regarding **FIG. 54B,** we repeated the analysis with MaxEntScan as a comparison. Note that the performance of both models improves at higher exon density, as measured by top-k accuracy and Precision-Recall AUC, because the number of positive test cases increases relative to the number of negative test cases.

## Enrichment of de novo mutations per cohort

Candidate cryptic splice DNMs were counted in each of the three cohorts. The DDD cohort did not report intronic DNMs > 8 nt away from exons and so regions > 8 nt from exons were excluded from all cohorts for the purposes of the enrichment analysis to enable equivalent comparison between the DDD and ASD cohorts (**FIG. 41A**). We also performed a separate analysis which excluded mutations with dual cryptic splicing and proteincoding function consequences to demonstrate the enrichment is not due to the enrichment of mutations with proteincoding effects within the affected cohorts (**FIGs. 49A, 49B,** and **49C**). Counts were scaled for differing ascertainment of DNMs between cohorts by normalizing the rate of synonymous DNMs per individual between cohorts, using the healthy control cohort as the baseline. We compared the rate of cryptic splice DNMs per cohort using an E-test to compare two Poisson rates (Krishnamoorthy and Thomson, 2004).

The plotted rates for enrichment over expectation (**FIG. 41C**) were adjusted for the lack of DNMs > 8 nt from exons by scaling upwards by the proportion of all cryptic splice DNMs expected to occur between 9-50 nt away from exons using a trinucleotide sequence context model (see below, Enrichment of *de novo* mutations per gene). The silent-only diagnostic proportion and excess of cryptic sites (**FIGs. 49B** and **49C**) were also adjusted for the lack of missense sites by scaling the cryptic count by the proportion of cryptic splice sites expected to occur at missense sites versus synonymous sites. The impact of $\Delta$ Score threshold on enrichment was assessed by calculating the enrichment of cryptic splice DNMs within the DDD cohort across a range of cutoffs. For each of these the observed: expected odds ratio was calculated, along with the excess of cryptic splice DNMs.

**Proportion of pathogenic DNMs**

The excess of DNMs compared to baseline mutation rates can be considered the pathogenic yield within a cohort. We estimated the excess of DNMs by functional type within ASD and DDD cohorts, against the background of the healthy control cohort (**FIG. 41B**). The DNM counts were normalized to the rate of synonymous DNMs per individual as described above. The DDD cryptic splice count was adjusted for the lack of DNMs 9-50 nt away from introns as described above. For both ASD and DDD cohorts, we also adjusted for the missing ascertainment of deep intronic variants > 50 nt away from exons, using the ratio of near-intronic (< 50 nt) vs deep intronic (> 50 nt) cryptic splice variants from the negative selection analysis (**FIG. 38G**).

**Enrichment of de novo mutations per gene**

We determined null mutation rates for every variant in the genome using a trinucleotide sequence context model (Samocha et al., 2014). We used the network to predict the $\Delta$ Score for all possible single nucleotide substitutions within exons and up to 8 nt into the intron. Based on the null mutation rate model, we obtained the expected number of *de* novo cryptic splice mutations per gene (using $\Delta$ Score > 0.2 as a cutoff).

As per the DDD study (McRae et al., 2017), genes were assessed for enrichment of DNMs compared to chance under two models, one considering only protein-truncating (PTV) DNMs, and one considering all protein-altering DNMs (PTVs, missense, and in-frame indels). For each gene, we selected the most significant model, and adjusted the P-value for multiple hypothesis testing. These tests were run once where we didn't consider cryptic splice DNMs or cryptic splice rates (the default test, used in the original DDD study), and once where we also counted cryptic splice DNMs and their mutation rates. We report additional candidate genes that were identified as genes with FOR-adjusted P-value < 0.01 when including cryptic splice DNMs, but FOR-adjusted P-value > 0.01 when not including cryptic splice DNMs (the default test). Enrichment tests were performed similarly for the ASD cohort.

**Validation of predicted cryptic splice sites**

We selected high confidence *de novos* from affected probands in the Simons Simplex Collection, with at least RPKM> I RNA-seq expression in lymphoblastoid cell lines. We selected *de novo* cryptic splice variants for validation based on a $\Delta$ Score threshold > 0.1 for splice loss variants and a $\Delta$ Score threshold > 0.5 for splice gain variants. Because the cell lines needed to be procured far in advance, these thresholds reflect an earlier iteration of our methods, compared to the thresholds we adopted elsewhere in the paper (**FIG. 38G** and **FIGs. 41A**, 41B, 41C, and **41D**), and the network did not include GTEx novel splice junctions for model training.

Lymphoblastoid cell lines were obtained from the SSC for these probands. Cells were cultured in Culture Medium (RPMI 1640, 2mM L-glutamine, 15% fetal bovine serum) to a maximum cell density of $1 \times 10^6$ cells/ml. When cells reached maximum density, they were passaged by dissociating the cells by pipetting up and down 4 or 5 times and seeding to a density of 200,000-500,000 viable cells/ml. Cells were grown under 37°C, 5% $CO_2$ conditions for 10 days. Approximately $5 \times 10^5$ cells were then detached and spun down at 300 x g for 5 minutes at 4°C. RNA was extracted using RNeasy® Plus Micro Kit (QIAGEN) following manufacturer's protocol. RNA quality was assessed using Agilent RNA 6000 Nano Kit (Agilent Technologies) and ran on Bioanalyzer 2100 (Agilent Technologies). RNA-seq libraries were generated by TruSeq® Stranded Total RNA Library Prep Kit with Ribo-Zero Gold Set A (Illumina). Libraries were sequenced on NiSeq 4000 instruments at Center for Advanced Technology (UCSF) using 150-nt single-read sequencing at a coverage of 270-388 million reads (median 358 million reads).

Sequencing reads for each patient were aligned with OLego (Wu et al., 2013) against a reference created from hg19 by substituting *de novo* variants of the patient (lossifov et al., 2014) with the corresponding alternative allele. Sequencing coverage, splice j unction usage and transcript locations were plotted with sashimi plot from MISO (Katz et al., 2010). We evaluated the predicted cryptic splice sites as described above in the validation of model predictions section. 13 novel splice sites (9 novel junction, 4 exon skipping) were confirmed as they were only observed in the sample containing the cryptic splice site and not observed in any of the 149 GTEx samples or in the other 35 sequenced samples. For 4 additional exon skipping events, low levels of exon-skipping were often observed in GTEx. In these cases, we computed the fraction of reads that used the skipping junction and verified that this fraction was highest in the cryptic splice site containing sample compared to other samples. 4 additional cases were validated on the basis of prominent intron retention that was absent or much lower in other samples. Modest intron retention in control samples prevented us from resolving events in *DDX11* and *WDR4.* Two events (in *CSAD,* and *GSAP*) were classified as failing validation because the variant was not present in sequencing reads.

## DATA AND SOFTWARE AVAILABILITY

Training and test data, prediction scores for all single nucleotide substitutions in the reference genome, RNA-seq validation results, RNA-seq junctions, and source code are publicly hosted at: https://basespace.illumina.com/s/5u6ThOblecrh

RNA-seq data for the 36 lymphoblastoid cell lines are being deposited in the ArrayExpress database at EMBL-EBI (www.ebi.ac.uk/arrayexpress) under accession number E-MTAB-xxxx.

Prediction scores and source code are publicly released under an open source modified Apache License v2.0 and are free for use for academic and non-commercial software applications. To reduce problems with circularity that have become a concern for the field, the authors explicitly request that the prediction scores from the method not be incorporated as a component of other classifiers, and instead ask that interested parties employ the provided source code and data to directly train and improve upon their own deep learning models.

## KEY RESOURCES TABLE

| REAGENT or RESOURCE | SOURCE | IDENTIFIER |
| --- | --- | --- |
| Deposited Data | | |
| RNA-seq data and variant calls for the GTEx cohort | https://www.ncbi.nlm.ni h.gov/ projects/gap | dbGAP accession: phs000424.v6.p1 |
| De-novo mutations for autism patients and healthy controls | (Iossifov et al., 2014) | N/A |
| De-novo mutations from the Deciphering Developmental Disorders cohort | (McRae et al., 2017) | N/A |
| Splice junctions from GENCODE principal transcripts used to train the canonical SpliceNet model | This study | https:// basespace.illumina.eom/s/ 5u6Th Oblecrh |
| Splice junctions from GTEx used to augment the training dataset | This study | https:// basespace.illumina.eom/s/ 5u6Th Oblecrh |
| Splice junctions from GENCODE principal transcripts used to test the model, with paralogs excluded | This study | https:// basespace.illumina.com/s/ 5u6Th Oblecrh |
| Splice junctions of lincRNAs used to test the model | This study | https:// basespace.illumina.com/s/ 5u6Th Oblecrh |
| Predictions of canonical model | This study | https:// basespace.illumina.com/s/ 5u6Th Oblecrh |
| Predictions of GTEx-supplemented model | This study | https:// basespace.illumina.com/s/ 5u6Th Oblecrh |
| All GTEx junctions in all GTEx v6.p1 samples | This study | https:// basespace.illumina.com/s/ 5u6Th Oblecrh |

(continued)

| REAGENT or RESOURCE | SOURCE | IDENTIFIER |
|---|---|---|
| Deposited Data | | |
| List of validated GTEx private variants with ∆ Score > 0.1 | This study | https://basespace.illumina.com/s/5u6Th Oblecrh |
| Aligned BAM files for RNA-seq in 36 autism patients | This study | ArrayExpress accession: E-MTAB-xxxx |
| Software and Algorithms | | |
| SpliceNet source code | This study | https://basespace.illumina.com/s/5u6Th Oblecrh |

## SUPPLEMENTAL TABLE TITLES

Table S 1 shows GTEx samples used for demonstrating effect size calculations and tissue-specific splicing effects. Related to **FIGs. 38A, 38B, 38C, 38D, 38E, 38F,** and **38G, FIG. 39A, FIG. 39B,** and **FIG. 45**

Table S2 shows matched confidence cutoffs for SpliceNet-10k, GeneSplicer, MaxEntScan, and NNSplice at which all algorithms predict the same number of gains and losses genome-wide. Related to **FIG. 38G.**

Table S3 shows counts of predicted cryptic splice DNMs in each cohort. Related to **FIGs. 41A, 41B, 41C, 41D, 41E,** and **41F** and is produced below:

| cohort | Probands (n) | synonymous de novos per proband | exons + introns up to 8 nt | | introns > 8 nt from exons | |
|---|---|---|---|---|---|---|
| | | | unadjusted | normalized to synonymous | unadjusted | normalized to synonymous |
| DDD | 4293 | 0.28744 | 347 | 298.7 | 14 | 12.1 |
| ASD | 3953 | 0.24462 | 236 | 238.7 | 64 | 64.7 |
| controls | 2073 | 0.24747 | 98 | 98 | 20 | 20 |

Table S4 shows expected de novo mutation rates per gene for each mutational category. Related to **FIG. 41A, 41B, 41C, 41D, 41E,** and **41F.**

Table S5 illustrates p-values for gene enrichment in DDD and ASD. Related to **FIGs. 41A, 41B, 41C, 41D, 41E,** and **41F.**

Table S6 depicts validation results for 36 predicted cryptic splice DNMs in autism patients. Related to **FIG. 41A, 41B, 41C, 41D, 41E,** and **41F.**

## Computer System

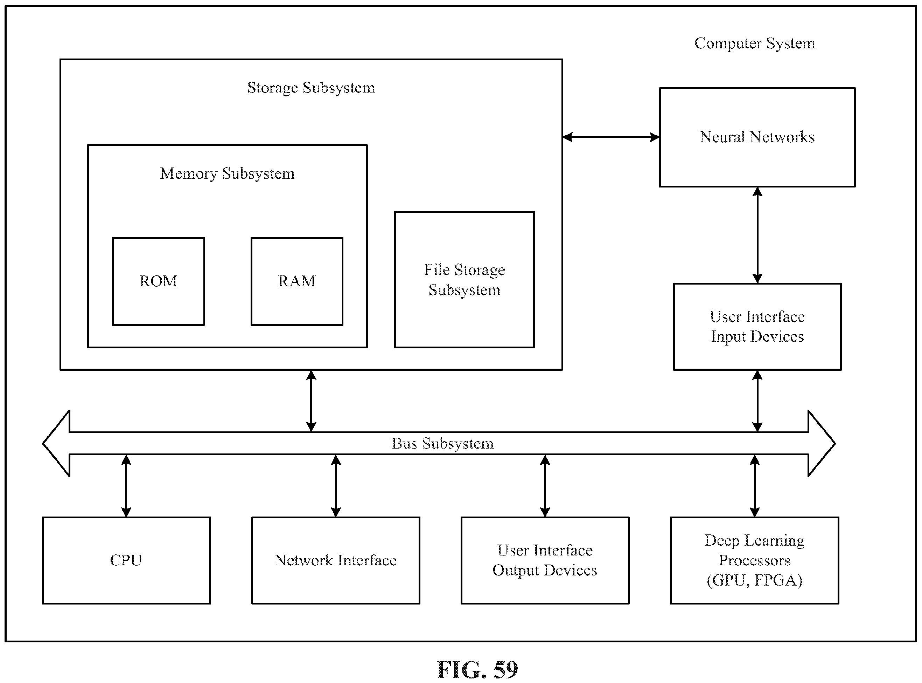

**FIG. 59** is a simplified block diagram of a computer system that can be used to implement the technology disclosed. Computer system typically includes at least one processor that communicates with a number of peripheral devices via bus subsystem. These peripheral devices can include a storage subsystem including, for example, memory devices and a file storage subsystem, user interface input devices, user interface output devices, and a network interface subsystem. The input and output devices allow user interaction with computer system. Network interface subsystem provides an interface to outside networks, including an interface to corresponding interface devices in other computer systems.

In one implementation, the neural networks such as ACNN and CNN are communicably linked to the storage subsystem and user interface input devices.

User interface input devices can include a keyboard; pointing devices such as a mouse, trackball, touchpad, or graphics tablet; a scanner, a touch screen incorporated into the display; audio input devices such as voice recognition systems and microphones; and other types of input devices. In general, use of the term "input device" is intended to include all possible types of devices and ways to input information into computer system.

User interface output devices can include a display subsystem, a printer, a fax machine, or non-visual displays such as audio output devices. The display subsystem can include a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system to the user or to another machine or computer system.

Storage subsystem stores programming and data constructs that provide the functionality of some or all of the modules and methods described herein. These software modules are generally executed by processor alone or in combination with other processors.

Memory used in the storage subsystem can include a number of memories including a main random access memory (RAM) for storage of instructions and data during program execution and a read only memory (ROM) in which fixed instructions are stored. A file storage subsystem can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem in the storage subsystem, or in other machines accessible by the processor.

Bus subsystem provides a mechanism for letting the various components and subsystems of computer system communicate with each other as intended. Although bus subsystem is shown schematically as a single bus, alternative implementations of the bus subsystem can use multiple busses.

Computer system itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system depicted in **FIG. 59** is intended only as a specific example for purposes of illustrating the technology disclosed. Many other configurations of computer system are possible having more or less components than the computer system depicted in **FIG. 59.**

The deep learning processors can be GPUs or FPGAs and can be hosted by a deep learning cloud platforms such as Google Cloud Platform, Xilinx, and Cirrascale. Examples of deep learning processors include Google's Tensor Processing Unit (TPU), rackmount solutions like GX4 Rackmount Series, GX8 Rackmount Series, NVIDIA DGX-1, Microsoft' Stratix V FPGA, Graphcore's Intelligent Processor Unit (IPU), Qualcomm's Zeroth platform with Snapdragon processors, NVIDIA's Volta, NVIDIA's DRIVE PX, NVIDIA's JETSON TX1/TX2 MODULE, Intel's Nirvana, Movidius VPU, Fujitsu DPI, ARM's DynamicIQ, IBM TrueNorth, and others.

**Claims**

1. A system for aberrant splicing determination, including numerous processors operating in parallel, coupled to memory, the memory loaded with computer instructions to determine aberrant splicing, comprising:
   a trained atrous convolutional neural network, abbreviated ACNN, running on the numerous processors, that processes pre-mRNA sequences, including:

     an input module that

       receives a variant sequence of at least 101 target nucleotides and flanking nucleotides, comprising at least 901 nucleotides with the target nucleotide flanked by at least 400 nucleotides on each side; and
       accesses a reference sequence comprising at least 901 nucleotides;

     layers that perform atrous convolutions on the target nucleotides in the variant sequence and the reference sequence, and assign splice site scores for likelihood that each of the target nucleotides is a donor splice site, an acceptor splice site, or a non-splicing site; and
     an output module that determines, from position-wise differences in the splice site scores of the target nucleotides in the variant sequence and the reference sequence, whether a variant in the variant sequence causes aberrant splicing at any of the target nucleotides in the variant sequence and is therefore pathogenic.

2. The system of claim 1, wherein, for at least one target nucleotide position, at least one position-wise difference in the splice site scores is above a predetermined threshold.

3. The system of claim 2, wherein a maximum difference in the splice site scores at a position among the target nucleotides is below a predetermined threshold, further configured to classify the variant sequence as not causing aberrant splicing and therefore benign.

4. The system of any of claims 2-3, wherein the predetermined threshold is determined by:
   for a plurality of candidate thresholds,

processing a first set of reference and variant sequence pairs generated by benign common variants to produce a first set of aberrant splicing detections; and

processing a second set of reference and variant sequence pairs generated by pathogenic rare variants to produce a second set of aberrant splicing detections; and

selecting at least one threshold that maximizes a count of aberrant splicing detections in the second set and minimizes a count of aberrant splicing detections in the first set.

5. The system of any of claims 2-4, wherein the reference sequence and the variant sequence each has at least 101 target nucleotides and each target nucleotide is flanked by at least 5000 nucleotides on each side.

6. The system of any of claims 2-5, wherein the splice site scores of the target nucleotides in the reference sequence are encoded in a first output of the ACNN and the splice site scores of the target nucleotides in the variant sequence are encoded in a second output of the ACNN.

7. The system of claim 6, wherein the first output is encoded as a first $101 \times 3$ matrix and the second output is encoded as a second $101 \times 3$ matrix;

8. The system of claim 7, wherein each row in the first $101 \times 3$ matrix uniquely represents splice site scores for a likelihood that a target nucleotide in the reference sequence is a donor splice site, an acceptor splice site, or a non-splicing site.

9. The system of any of claims 1-8, wherein each row in the second $101 \times 3$ matrix uniquely represents splice site scores for a likelihood that a target nucleotide in the variant sequence is a donor splice site, an acceptor splice site, or a non-splicing site,

preferably wherein splice site scores in each row of the first $101 \times 3$ matrix and the second $101 \times 3$ matrix are exponentially normalized and sum to unity.

10. The system of claim 8 or 9, wherein the classifier performs a row-to-row comparison of the first $101 \times 3$ matrix and the second $101 \times 3$ matrix and determines, on a row-wise basis, changes in distribution of splice site scores.
preferably wherein, for at least one instance of the row-to-row comparison, the change in distribution is above a predetermined threshold, further configured to classify the variant as causing aberrant splicing and therefore pathogenic.

11. The system of any preceding claim, wherein the reference sequence and the variant sequence are one-hot encoded.

12. A computer-implemented method of determining aberrant splicing in a pre-mRNA sequence, comprising:

processing a pre-mRNA sequence using a trained atrous convolutional neural network, abbreviated ACNN, including:

receiving a variant sequence of at least 101 target nucleotides and flanking nucleotides, comprising at least 901 nucleotides with the target nucleotides flanked by at least 400 nucleotides on each side and accessing a reference sequence comprising at least 901 nucleotides;
performing atrous convolutions on the target nucleotides in the variant sequence and the reference sequence and assigning splice site scores for likelihood that each of the target nucleotides is a donor splice site, an acceptor splice site, or a non-splicing site; and

determining position-wise differences in the splice site scores of the target nucleotides in the variant sequence and the reference sequence and classifying, from the position-wise differences, whether the variant sequence causes aberrant splicing at any of the target nucleotides in the variant sequence and is therefore pathogenic.

13. The method of claim 12, applied to per-gene enrichment analysis for a particular gene sampled from a diseased cohort of individuals with a genetic disorder, further including:

applying the trained ACNN system to identify candidate variants from the diseased cohort in the sampled particular gene that cause aberrant splicing;
identifying de novo candidate variants among the candidate variants;
determining a baseline number of mutations at the de novo candidate variants for the particular gene based on summing observed trinucleotide mutation rates of the de novo candidate variants in a population of normal individuals, and multiplying the sum of the trinucleotide mutation rates by with a transmission count and a size of the diseased cohort;
and
comparing the baseline number of mutations with a count of observed variants at the de novo candidate variants in the diseased cohort, and based on the comparison, determining that the particular gene is associated with the genetic disorder.

14. The method of claim 13, further configured to perform the comparing using a statistical test that produces a p-value as the output.

15. The method of claim 13 or 14, wherein the method has one or more of the following properties:

wherein the genetic disorder is autism spectrum disorder, ASD;
wherein the diseased cohort is a developmental delay disorder, DDD, cohort;
wherein at least some of the candidate variants are protein-truncating variants; and
wherein at least some of the candidate variants are missense variants.

16. A non-transitory computer readable storage medium impressed with computer program instructions to detect aberrant splicing, the instructions, when executed on a processor, cause the processor to perform a method according to any one of claims 12 to 15.

**Patentansprüche**

1. System zum Bestimmen von aberranter Spleißung, das mehrere parallel arbeitende Prozessoren einschließt, die mit einem Speicher gekoppelt sind, wobei der Speicher mit Computerbefehlen zur Bestimmung von aberranter Spleißung geladen ist, umfassend:
ein trainiertes atröses neuronales Faltungsnetzwerk, ACNN, das auf den mehreren Prozessoren läuft und prä-mRNA-Sequenzen verarbeitet, Folgendes einschließend:

ein Eingabemodul, das
eine Variantensequenz von mindestens 101 Zielnukleotiden und flankierenden Nukleotiden empfängt, die mindestens 901 Nukleotide umfassen, wobei das Zielnukleotid von mindestens 400 Nukleotiden auf jeder Seite flankiert wird; und
auf eine Referenzsequenz zugreift, die mindestens 901 Nukleotide umfasst;
Schichten, die atröse Faltungen an den Zielnukleotiden in der Variantensequenz und der Referenzsequenz durchführen und Spleißstellenbewertungen für die Wahrscheinlichkeit zuweisen, dass jedes der Zielnukleotide eine Spender-Spleißstelle, eine Akzeptor-Spleißstelle oder eine Nicht-Spleißstelle ist; und
ein Ausgabemodul, das aus positionsweisen Unterschieden in den Spleißstellenbewertungen der Zielnukleotiden in der Variantensequenz und der Referenzsequenz bestimmt, ob eine Variante in der Variantensequenz eine aberrante Spleißung an irgendeinem der Zielnukleotide in der Variantensequenz verursacht und daher pathogen ist.

2. System nach Anspruch 1, wobei für mindestens eine Zielnukleotidposition mindestens ein positionsweiser Unterschied in den Spleißstellenbewertungen über einem vorbestimmten Schwellenwert liegt.

3. System nach Anspruch 2, wobei ein maximaler Unterschied in den Spleißstellenbewertungen an einer Position unter den Zielnukleotiden unter einem vorbestimmten Schwellenwert liegt, das ferner derart konfiguriert ist, dass es die Variantensequenz als nicht eine aberrante Spleißung verursachend und daher gutartig einstuft.

4. System nach einem der Ansprüche 2 bis 3, wobei der vorbestimmte Schwellenwert bestimmt wird durch:

für eine Vielzahl von Kandidatenschwellenwerten,
Verarbeiten eines ersten Satzes von Referenz- und Variantensequenzpaaren, die von gutartigen üblichen Varianten erzeugt wurden, um einen ersten Satz von Erfassungen von aberranter Spleißung zu erzeugen; und
Verarbeiten eines zweiten Satzes von Referenz- und Variantensequenzpaaren, die von pathogenen seltenen Varianten erzeugt wurden, um einen zweiten Satz von Erfassungen von aberranter Spleißung zu erzeugen; und

Auswählen von mindestens einem Schwellenwert, der eine Anzahl der Erfassungen von aberranter Spleißung in dem zweiten Satz maximiert und eine Anzahl der Erfassungen von aberranter Spleißung in dem erstem Satz minimiert.

5. System nach einem der Ansprüche 2 bis 4, wobei die Referenzsequenz und die Variantensequenz jeweils mindestens 101 Zielnukleotide aufweisen und jedes Zielnukleotid von mindestens 5000 Nukleotiden auf jeder Seite flankiert wird.

6. System nach einem der Ansprüche 2 bis 5, wobei die Spleißstellenbewertungen der Zielnukleotide in der Referenzsequenz in einer ersten Ausgabe des ACNN kodiert sind und die Spleißstellenbewertungen der Zielnukleotide in der Variantensequenz in einer zweiten Ausgabe des ACNN kodiert sind.

7. System nach Anspruch 6, wobei die erste Ausgabe als eine erste $101 \times 3$-Matrix und die zweite Ausgabe als eine zweite $101 \times 3$-Matrix kodiert ist;

8. System nach Anspruch 7, wobei jede Reihe in der ersten $101 \times 3$-Matrix einzigartig Spleißstellenbewertungen für eine Wahrscheinlichkeit darstellt, dass ein Zielnukleotid in der Referenzsequenz eine Spender-Spleißstelle, eine Akzeptor-Spleißstelle oder eine Nicht-Spleißstelle ist.

9. System nach einem der Ansprüche 1 bis 8, wobei jede Reihe in der zweiten $101 \times 3$-Matrix einzigartig Spleißstellenbewertungen für eine Wahrscheinlichkeit darstellt, dass ein Zielnukleotid in der Variantensequenz eine Spender-Spleißstelle, eine Akzeptor-Spleißstelle oder eine Nicht-Spleißstelle ist, wobei die Spleißstellenbewertungen in jeder Reihe der ersten $101 \times 3$-Matrix und der zweiten $101 \times 3$-Matrix vorzugsweise exponentiell normalisiert sind und sich zu einer Einheit summieren.

10. System nach Anspruch 8 oder 9, wobei der Klassifikator einen Zeile-zu-Zeile-Vergleich der ersten $101 \times 3$-Matrix und der zweiten $101 \times 3$-Matrix durchführt und reihenweise Änderungen in der Verteilung der Spleißstellenbewertungen bestimmt.
vorzugsweise wobei, für mindestens eine Instanz des reihenweisen Vergleichs, die Änderung in der Verteilung über einem vorbestimmten Schwellenwert liegt, wobei es ferner derart konfiguriert ist, dass es die Variante als eine aberrante Spleißung verursachend und daher als pathogen klassifiziert.

11. System nach einem der vorhergehenden Ansprüche, wobei die Referenzsequenz und die Variantensequenz One-Hot-codiert sind.

12. Computerimplementiertes Verfahren zum Bestimmen von aberranter Spleißung in einer prä-mRNA-Sequenz, umfassend:
Verarbeiten einer prä-mRNA-Sequenz unter Verwendung eines trainierten atrösen neuronalen Faltungsnetzwerks, ACNN, einschließend:

Empfangen einer Variantensequenz von mindestens 101 Zielnukleotiden und flankierenden Nukleotiden, die mindestens 901 Nukleotide umfasst, wobei die Zielnukleotide von mindestens 400 Nukleotiden auf jeder Seite flankiert werden, und Zugreifen auf eine Referenzsequenz, die mindestens 901 Nukleotide umfasst;
Durchführen von atrösen Faltungen an den Zielnukleotiden in der Variantensequenz und der Referenzsequenz und Zuweisen von Spleißstellenbewertungen für die Wahrscheinlichkeit, dass jedes der Zielnukleotide eine Spender-Spleißstelle, eine Akzeptor-Spleißstelle oder eine Nicht-Spleißstelle ist; und
Bestimmen von positionsweisen Unterschieden in den Spleißstellenbewertungen der Zielnukleotide in der Variantensequenz und der Referenzsequenz und Klassifizieren, aus den positionsweisen Unterschieden, ob die abweichende Sequenz eine aberrante Spleißung an einem der Zielnukleotide in der Variantensequenz verursacht und daher pathogen ist.

13. Verfahren nach Anspruch 12, angewandt auf eine Analyse der Anreicherung pro Gen für ein bestimmtes Gen, das aus einer erkrankten Kohorte von Individuen mit einer genetischen Störung entnommen wurde, ferner einschließend:

Anwenden des trainierten ACNN-Systems zur Identifizierung von Kandidatenvarianten aus der erkrankten Kohorte in dem untersuchten bestimmten Gen, die eine aberrante Spleißung verursachen;
Identifizieren von de-novo Kandidatenvarianten unter den Kandidatenvarianten;
Bestimmen einer Basisanzahl von Mutationen an den de-novo-Kandidatenvarianten für das bestimmte Gen auf der Grundlage der Summierung der beobachteten Trinukleotid-Mutationsraten der de-novo-Kandidatenvarianten in einer Population normaler Individuen und Multiplizieren der Summe der Trinukleotid-Mutationsraten mit einer Übertragungszahl und einer Größe der erkrankten Kohorte; und

Vergleichen der Basisanzahl von Mutationen mit einer Anzahl von beobachteten Varianten an den de-novo-Kandidatenvarianten in der erkrankten Kohorte und, auf der Grundlage des Vergleichs, Bestimmen, dass das bestimmte Gen der genetischen Störung zugeordnet ist.

**14.** Verfahren nach Anspruch 13, das ferner derart konfiguriert ist, dass es den Vergleich unter Verwendung eines statistischen Tests durchführt, der einen p-Wert als Ausgabe erzeugt.

**15.** Verfahren nach Anspruch 13 oder 14, wobei das Verfahren eine oder mehrere der folgenden Eigenschaften aufweist:

wobei die genetische Störung eine Autismus-Spektrum-Störung, ASD, ist;
wobei die erkrankte Kohorte eine Kohorte mit Entwicklungsverzögerung, DDD, ist;
wobei mindestens einige der Kandidatenvarianten Protein-abbrechende Varianten sind; und
wobei mindestens einige der Kandidatenvarianten Missense-Varianten sind.

**16.** Nichtflüchtiges computerlesbares Speichermedium, dem Computerprogrammanweisungen zum Erfassen von aberranter Spleißung aufgeprägt sind, wobei die Anweisungen, wenn sie auf einem Prozessor ausgeführt werden, den Prozessor veranlassen, ein Verfahren nach einem der Ansprüche 12 bis 15 durchzuführen.


**Revendications**

**1.** Système de détermination d'épissage aberrant, incluant de nombreux processeurs fonctionnant en parallèle, couplés à une mémoire, la mémoire étant chargée d'instructions-machine pour déterminer l'épissage aberrant, comprenant :
un réseau neuronal convolutif à trous formé, abrégé en ACNN, fonctionnant sur les nombreux processeurs, qui traite des séquences de pré-ARNm, incluant :

un module d'entrée qui
reçoit une séquence de variants d'au moins 101 nucléotides cibles et de nucléotides adjacents, comprenant au moins 901 nucléotides avec le nucléotide cible encadré de chaque côté par au moins 400 nucléotides ; et accède à une séquence de référence comprenant au moins 901 nucléotides ;
des couches qui effectuent des convolutions à trous sur les nucléotides cibles dans la séquence de variants et la séquence de référence, et attribuent des scores de site d'épissage pour la probabilité ue chacun des nucléotides cibles soit un site d'épissage donneur, un site d'épissage accepteur, ou un site de non-épissage ; et
un module de sortie qui détermine, à partir des différences de position dans les scores de site d'épissage des nucléotides cibles de la séquence de variants et la séquence de référence, si un variant de la séquence de variants provoque un épissage aberrant à l'un quelconque des nucléotides cibles de la séquence de variants et est par conséquent pathogène.

**2.** Système selon la revendication 1, dans lequel, pour au moins une position de nucléotide cible, au moins une différence de position dans les scores de site d'épissage est supérieure à un seuil prédéterminé.

**3.** Système selon la revendication 2, dans lequel une différence maximale dans les scores de site d'épissage à une position parmi les nucléotides cibles est inférieure à un seuil prédéterminé, configuré en outre pour classer la séquence de variants comme ne provoquant pas d'épissage aberrant et en conséquence comme bénigne.

**4.** Système selon l'une des revendications 2 à 3, dans lequel le seuil prédéterminé est déterminé par :

pour une pluralité de seuils candidats,
le traitement d'un premier ensemble de paires de séquences de référence et de variants générées par des variants communs bénins pour produire un premier ensemble de détections d'épissage aberrant ; et
le traitement d'un deuxième ensemble de paires de séquences de référence et de variants générées par des variants rares pathogènes pour produire un deuxième ensemble de détections d'épissage aberrant ; et
la sélection d'au moins un seuil qui maximise un compte de détections d'épissage aberrant dans le deuxième ensemble et minimise un compte de détections d'épissage aberrant dans le premier ensemble.

**5.** Système selon l'une quelconque des revendications 2 à 4, dans lequel la séquence de référence et la séquence de variants ont chacune au moins 101 nucléotides cibles et chaque nucléotide cible est encadré de chaque côté par au moins 5000 nucléotides.

**6.** Système selon l'une quelconque des revendications 2 à 5, dans lequel les scores de site d'épissage des nucléotides cibles dans la séquence de référence sont encodés dans une première sortie de l'ACNN et les scores de site d'épissage des nucléotides cibles dans la séquence de variants sont encodés dans une deuxième sortie de l'ACNN.

**7.** Système selon la revendication 6, dans lequel la première sortie est encodée comme première matrice $101 \times 3$ et la deuxième sortie est encodée comme deuxième matrice $101 \times 3$.

**8.** Système selon la revendication 7, dans lequel chaque rang de la première matrice $101 \times 3$ représente de façon unique des scores de site d'épissage pour une probabilité qu'un nucléotide cible de la séquence de référence soit un site d'épissage donneur, un site d'épissage accepteur, ou un site de non-épissage.

**9.** Système selon l'une quelconque des revendications 1 à 8, dans lequel chaque rang de la seconde matrice $101 \times 3$ représente de façon unique des scores de site d'épissage pour une probabilité qu'un nucléotide cible de la séquence de variants soit un site d'épissage donneur, un site d'épissage accepteur, ou un site de non-épissage, dans lequel, de préférence, des scores de site d'épissage dans chaque rang de la première matrice $101 \times 3$ et de la deuxième matrice $101 \times 3$ sont normalisés d'une manière exponentielle et s'additionnent pour donner une unité.

**10.** Système selon la revendication 8 ou 9, dans lequel le classificateur effectue une comparaison rang par rang de la première matrice $101 \times 3$ et de la deuxième matrice $101 \times 3$ et détermine, par rang, des modifications de répartition des scores de site d'épissage,
dans lequel, de préférence, pour au moins un exemple de la comparaison rang par rang, la modification de répartition est supérieure à un seuil prédéterminé configuré en outre pour classer le variant comme provoquant l'épissage aberrant et, par conséquent, comme pathogène.

**11.** Système selon l'une quelconque des revendications précédentes, dans lequel la séquence de référence et la séquence de variants sont encodées one-hot.

**12.** Procédé mis en oeuvre par ordinateur de détermination d'épissage aberrant dans une séquence de pré-ARNm, comprenant :

le traitement d'une séquence de pré-ARNm utilisant un réseau neuronal convolutif à trous formé, abrégé en ACNN, incluant
la réception d'une séquence de variants d'au moins 101 nucléotides cibles et de nucléotides adjacents, comprenant au moins 901 nucléotides avec les nucléotides cibles encadrés de chaque côté par au moins 400 nucléotides et l'accès à une séquence de référence comprenant au moins 901 nucléotides ;
la réalisation de convolutions à trous sur les nucléotides cibles dans la séquence de variants et la séquence de référence et l'attribution de scores de site d'épissage pour la probabilité que chacun des nucléotides cibles soit un site d'épissage donneur, un site d'épissage accepteur, ou un site de non-épissage ; et
la détermination de différences de position dans les scores de site d'épissage des nucléotides cibles de la séquence de variants et la séquence de référence et la classification, à partir des différences de position, si la séquence de variants provoque un épissage aberrant à l'un quelconque des nucléotides cibles de la séquence de variants et est par conséquent pathogène.

**13.** Procédé selon la revendication 12, appliqué à une analyse d'enrichissement par gène pour un gène particulier échantillonné à partir d'une cohorte malade d'individus avec un trouble génétique, incluant, en outre :

l'application du système ACNN formé pour identifier des variants candidats de la cohorte malade dans le gène particulier échantillonné qui provoquent l'épissage aberrant ;
l'identification de variants candidats de novo parmi les variants candidats ;
la détermination d'un nombre de base de mutations au niveau des variants candidats de novo pour le gène particulier sur la base de l'addition des taux de mutation des trinucléotides observés des variants candidats de novo dans une population d'individus normaux, et la multiplication de la somme des taux de mutation des trinucléotides par un nombre de transmissions et une taille de la cohorte malade ;
et

la comparaison du nombre de base de mutations avec un nombre de variants observés au niveau des variants candidats de novo dans la cohorte malade, et sur la base de la comparaison, la détermination que le gène particulier est associé au trouble génétique.

14. Procédé selon la revendication 13, configuré en outre pour effectuer la comparaison en utilisant un test statistique qui produit une valeur p comme sortie.

15. Procédé selon la revendication 13 ou 14, dans lequel le procédé a une ou plusieurs des propriétés suivantes :

dans lequel le trouble génétique est un trouble du spectre autistique, TSA ;
dans lequel la cohorte malade est une cohorte à trouble du retard de développement, TRD ;
dans lequel au moins certains des variants candidats sont des variants à protéine tronquée ; et
dans lequel au moins certains des variants candidats sont des variants faux-sens.

16. Support de stockage lisible par ordinateur non transitoire comportant des instructions de programme d'ordinateur pour détecter un épissage aberrant, les instructions, lorsqu'elles sont exécutées sur un processeur, entraînant le processeur à effectuer un procédé selon l'une quelconque des revendications 12 à 15.

Single Layer Perceptron

FIG. 1

EP 3 622 519 B1

# Feed-Forward Neural Network

**FIG. 2**

EP 3 622 519 B1

## Convolutional Neural Network

**FIG. 3**

## Training Convolutional Neural Network

Adjust Weights

Back Propagation

Input Data →

Convolutional Neural Network (with weights between neurons)

Output Estimate

Σ Compare

Error

Ground Truth +

**FIG. 4**

EP 3 622 519 B1

# Non-Linear Layers of Convolutional Neural Networks

| 100 | -43 | 76 | -21 |
| --- | --- | --- | --- |
| -90 | -10 | 40 | 21 |
| -19 | 44 | 1 | -35 |
| 221 | 64 | 41 | 23 |

0,0

| 100 | 0 | 76 | 0 |
| --- | --- | --- | --- |
| 0 | 0 | 40 | 21 |
| 0 | 44 | 1 | 0 |
| 221 | 64 | 41 | 23 |

ReLU Activation Function

FIG. 5

EP 3 622 519 B1

**Dilated Convolutions**

input

layer 1

output

$x_{t-3}$  $x_{t-2}$  $x_{t-1}$  $x_t$

$x_{t+1}$

EP 3 622 519 B1

**FIG. 6**

# Sub-Sampling Layers of Convolutional Neural Networks

Feature Map

Average Pooling

Max Pooling

**FIG. 7**

EP 3 622 519 B1

# Convolution Layers of Convolutional Neural Networks

Input

2048 Dimensions

| Convolution 1 | Convolution 1 |
|---|---|
| 16 Kernels of size 3 × 3 | 16 Kernels of size 3 × 3 |

16 Feature maps

Convolution 1

ReLU 1

Pool 1 (AVG Pooling)
16 Kernels of size 3 × 3

Pool 1

Convolution 2
16 Kernels of size 3 × 3

ReLU 2

Pool 2 (AVG Pooling)
32 Kernels of size 2 × 2

Feature Vector
512 Dimensions

**FIG. 8**

# Residual Connections used in Convolutional Neural Networks

**FIG. 9**

Residual Block and Skip-Connections used in
Convolutional Neural Networks

FIG. 10

## Stacked Dilated Convolutions

FIG. 11

# Batch Normalization Forward Pass with Convolutional Neural Networks

$$\mu_{\mathcal{B}} = \frac{1}{n} \sum_{i=1}^{n} x_i^{(\ell-1)}$$

$$\sigma_{\mathcal{B}}^2 = \frac{1}{n} \sum_{i=1}^{n} (x_i^{(\ell-1)} - \mu_{\mathcal{B}})^2$$

$$\hat{x}^{(\ell-1)} = \frac{x^{(\ell-1)} - \mu_{\mathcal{B}}}{\sqrt{\sigma_{\mathcal{B}}^2 + \epsilon}}$$

$$x^{(\ell)} = \gamma^{(\ell)} \hat{x}^{(\ell-1)} + \beta^{(\ell)}$$

**FIG. 12**

EP 3 622 519 B1

# Batch Normalization – Inference with Convolutional Neural Networks

$$\hat{x}^{(\ell-1)} = \frac{x^{(\ell-1)} - \mu_{\mathcal{D}}}{\sqrt{\sigma_{\mathcal{D}}^2 + \epsilon}}$$

$$x_i^{(\ell)} = \gamma^{(\ell)} \hat{x}_i^{(\ell-1)} + \beta^{(\ell)}$$

**FIG. 13**

# Batch Normalization Backward Pass with Convolutional Neural Networks

$$\nabla_{\gamma^{(\ell)}} \mathcal{L} = \sum_{i=1}^{n} \left( \nabla_{x^{(\ell+1)}} \mathcal{L} \right)_i \cdot \hat{x}_i^{(\ell)}$$

$$\nabla_{\beta^{(\ell)}} \mathcal{L} = \sum_{i=1}^{n} \left( \nabla_{x^{(\ell+1)}} \mathcal{L} \right)_i$$

**FIG. 14**

EP 3 622 519 B1

# Batch Normalization in Convolution Layers

```
conv_model.add(layers.Conv2D(32, 3, activation='relu'))    <———  After a Conv layer
conv_model.add(layers.BatchNormalization())

dense_model.add(layers.Dense(32, activation='relu'))    <———  After a Dense layer
dense_model.add(layers.BatchNormalization())
```

**FIG. 15**

# 1D Convolution used in Convolutional Neural Networks

FIG. 16

EP 3 622 519 B1

# Global Average Pooling (GAP) in Convolutional Neural Networks

EP 3 622 519 B1

**FIG. 17**

Training Servers
(Scale with Data)

Large-Scale
Training
Dataset

Train SpliceNets

RB → RB → • • • • • → RB

SpliceNets

Administrative
Interface

Production Servers
(Scale with Users)

RB → RB → • • • • • → RB

Trained SpliceNets Deployed on Production Servers

Test Data

Production
Data

Inferred Data
(real-time)

Client Interface
(APIs, Browser)

**FIG. 18**

## SpliceNet

```
┌─────────────────────┐
│       Inputs        │
└─────────────────────┘
           │
           ▼
┌─────────────────────┐
│   Residual Block    │
└─────────────────────┘
           │
           ▼
┌─────────────────────┐
│   Residual Block    │
└─────────────────────┘
           │
           ▼
           ●
           ●
           ●
           ●
           ●
           ●
           │
           ▼
┌─────────────────────┐
│   Residual Block    │
└─────────────────────┘
           │
           ▼
┌─────────────────────┐
│       Outputs       │
└─────────────────────┘
```

**FIG. 19**

## Residual Block (RB) (N, W, D)

N: Number of Convolution Filters
W: Convolution Window Size
D: Atrous Convolution Rate

**FIG. 20**

SpliceNet80

$C^u$: 40, $C^d$: 40

FIG. 21

## SpliceNet400

| Inputs |
| --- |

$C^u$: 200, $C^d$: 200

| Convolution (32, 1, 1) |
| --- |

| Convolution (32, 1, 1) |
| --- |

| RB (32, 11, 1) |
| --- |

| RB (32, 11, 1) |
| --- |

| RB (32, 11, 1) |
| --- |

| RB (32, 11, 1) |
| --- |

| Convolution (32, 1, 1) |
| --- |

| RB (32, 11, 4) |
| --- |

| RB (32, 11, 4) |
| --- |

| RB (32, 11, 4) |
| --- |

| RB (32, 11, 4) |
| --- |

| Convolution (32, 1, 1) |
| --- |

| Convolution (3, 1, 1) |
| --- |

| Outputs |
| --- |

**FIG. 22**

# SpliceNet2000

$C^u$: 1000, $C^d$: 1000

**FIG. 23**

SpliceNet10000

$C^u$: 5000, $C^d$: 5000

| Inputs |

| Convolution (32, 1, 1) |

| Convolution (32, 1, 1) |

| RB (32, 11, 1) |

| RB (32, 11, 1) |

| RB (32, 11, 1) |

| RB (32, 11, 1) |

| Convolution (32, 1, 1) |

| RB (32, 11, 4) |

| RB (32, 11, 4) |

| RB (32, 11, 4) |

| RB (32, 11, 4) |

| Convolution (32, 1, 1) |

| RB (32, 21, 10) |

| RB (32, 21, 10) |

| RB (32, 21, 10) |

| RB (32, 21, 10) |

| Convolution (32, 1, 1) |

| RB (32, 41, 25) |

| RB (32, 41, 25) |

| RB (32, 41, 25) |

| RB (32, 41, 25) |

| Convolution (32, 1, 1) |

| Convolution (3, 1, 1) |

| Outputs |

**FIG. 24**

Reference Genome (annotated)

Donor Site

Upstream Context Nucleotides (C^u)

Target Nucleotide Sequence (L)

Downstream Context Nucleotides (C^d)

Left Flanking Nucleotides (F^l)
Size = C + (i_L - 1)

Target Nucleotide (N^t)

Right Flanking Nucleotides (F^r)
Size = (L - i_L) + C

CGGUUUAUGCAAAAUCAAAUGGAUGAAGGUGCUGUCAUUCCUUAA

C^u: 40, 200, 1000, 5000
C^d: 40, 200, 1000, 5000
L: 5000

**FIG. 25**

Reference Genome (annotated)

● ● ● Acceptor Site Exon ● ● ●

$\underbrace{\hspace{4cm}}$ $\underbrace{\hspace{2cm}}$ $\underbrace{\hspace{4cm}}$

Upstream Context Nucleotides ($C^u$)    Target Nucleotide    Downstream Context Nucleotides ($C^d$)
Sequence (L)

● ● ● GTAAGTCTAGCCTGCATTATGAAAGTGAATCTTACTTTTGTAAAACTTTATA ● ● ●

$\underbrace{\hspace{5cm}}$ $\underbrace{\hspace{5cm}}$

Left Flanking Nucleotides ($F^l$)    Right Flanking Nucleotides ($F^r$)
Size = C + ($i_L$ - 1)    Size = (L - $i_L$) + C

Target Nucleotide ($N^t$)

$C^u$: 40, 200, 1000, 5000
$C^d$: 40, 200, 1000, 5000
L: 5000

FIG. 26

Reference Genome (annotated)

Upstream Context Nucleotides ($C^u$)

Target Nucleotide Sequence (L)

Downstream Context Nucleotides ($C^d$)

Left Flanking Nucleotides ($F^l$)
Size = $C + (i_L - 1)$

Right Flanking Nucleotides ($F^r$)
Size = $(L - i_L) + C$

Target Nucleotide ($N^t$)

$C^u$: 40, 200, 1000, 5000
$C^d$: 40, 200, 1000, 5000
L: 5000

**FIG. 27**

Reference Genome (annotated)

| Non-Splicing Site (1) | ••••••• | Non-Splicing Site (200MM) | ••• | Non-Splicing Site (400MM) | ••• | Non-Splicing Site (600MM) | ••• | Non-Splicing Site (800MM) |

Target Nucleotide Sequence (L)　　Target Nucleotide Sequence (L)　　Target Nucleotide Sequence (L)　　Target Nucleotide Sequence (L)　　Target Nucleotide Sequence (L)

L: 5000

100

**FIG. 28**

A C G T T T G C A C A C A C G ? G T A T A T

| | A | C | G | T | T | T | G | C | A | C | A | C | A | C | G | ? | G | T | A | T | A | T |
|---|---|---|---|---|---|---|---|---|---|---|---|---|---|---|---|---|---|---|---|---|---|---|
| A | 1 | 0 | 0 | 0 | 0 | 0 | 0 | 0 | 1 | 0 | 1 | 0 | 1 | 0 | 0 | 0 | 0 | 0 | 1 | 0 | 1 | 0 |
| C | 0 | 1 | 0 | 0 | 0 | 0 | 1 | 0 | 1 | 0 | 1 | 0 | 1 | 0 | 0 | 0 | 0 | 0 | 0 | 0 | 0 | 0 |
| G | 0 | 0 | 1 | 0 | 0 | 1 | 0 | 0 | 0 | 0 | 0 | 0 | 0 | 1 | 0 | 1 | 0 | 0 | 0 | 0 | 0 | 0 |
| T | 0 | 0 | 0 | 1 | 1 | 1 | 0 | 0 | 0 | 0 | 0 | 0 | 0 | 0 | 0 | 0 | 1 | 0 | 1 | 0 | 1 |
| N | 0 | 0 | 0 | 0 | 0 | 0 | 0 | 0 | 0 | 0 | 0 | 0 | 0 | 0 | 0 | 0 | 0 | 0 | 0 | 0 | 0 | 0 |

One-hot Encoding

A C G T T T G C A C A C A C G ? G T A T A T

**FIG. 29**

FIG. 30

FIG. 31

EP 3 622 519 B1

**FIG. 32**

## Reference Sequence

Upstream Context Nucleotides (C$^u$)    Target Nucleotide
Sequence (L)    Downstream Context Nucleotides (C$^d$)

## Alternative Sequence

Variant

Upstream Context Nucleotides (C$^u$)    Target Nucleotide
Sequence (L)    Downstream Context Nucleotides (C$^d$)

C$^u$: 40, 200, 1000, 5000
C$^d$: 40, 200, 1000, 5000
L: 101

**FIG. 33**

EP 3 622 519 B1

# Aberrant Splicing Detection

Reference Sequence
$(C^u + L + C^d) \times 4$
(One-hot encoded)

Input 1

Alternative Sequence
$(C^u + L + C^d) \times 4$
(One-hot encoded)

Variant

Input 2

$C^u$: 40, 200, 1000, 5000
$C^d$: 40, 200, 1000, 5000
L: 101
$i_L$ of variant: 51

Trained SpliceNet
(CNN/80/400/2000/10000)

Output 1

L

3

Output 2

L

3

Classifier

Change in splice site
scores at any L position
above a predetermined
threshold?

YES          NO

Aberrant
splicing
detected and
attributed to
the variant

Aberrant
splicing NOT
detected and
variant does
NOT cause
aberrant
splicing

**FIG. 34**

SpliceNet10000 Processing Pyramid for Splice Site Classification

RB (32, 11, 1)

RB (32, 11, 4)

RB (32, 21, 10)

RB (32, 41, 25)

Convolution (3, 1, 1)

$C^u$ (5000)

L (5000)

$C^d$ (5000)

15000 x 5

(15000 - 80 = 14920) x 32

(15000 - 80 - 320 = 14600) x 32

(15000 - 80 - 320 - 1600 = 13000) x 32

(15000 - 80 - 320 - 1600 - 8000 = 5000) x 32

5000 x 3

FIG. 35

EP 3 622 519 B1

107

EP 3 622 519 B1

SpliceNet10000 Processing Pyramid for Aberrant Splicing Detection

FIG. 36

**FIGs. 37A-H**

FIGs. 38A-G

FIGs. 39A-C

**FIGs. 40A–E**

**FIGs. 41A-F**

**A**

| | Top-k accuracy | | Precision-Recall AUC | |
|---|---|---|---|---|
| | Acceptor | Donor | Acceptor | Donor |
| SpliceNet-80nt | 0.5129 | 0.5110 | 0.5195 | 0.5273 |
| SpliceNet-400nt | 0.6848 | 0.7221 | 0.7320 | 0.7982 |
| SpliceNet-2k | 0.8090 | 0.8405 | 0.8742 | 0.9047 |
| SpliceNet-10k | 0.8223 | 0.8586 | 0.8896 | 0.9107 |
| GeneSplicer | 0.3238 | 0.3381 | 0.2234 | 0.2982 |
| MaxEntScan | 0.2932 | 0.3687 | 0.2233 | 0.2937 |
| NNSplice | 0.2655 | 0.3687 | 0.1702 | 0.3003 |

**B**

chr1:211,556,145-211,608,675 52,531 nt

LINC00467

Splice acceptor  Splice donor

MaxEntScan score

SpliceNet-10k score

FIGs. 42A-B

EP 3 622 519 B1

EP 3 622 519 B1

**FIGs. 43A-B**

FIGs. 44A-B

FIG. 45

**FIGs. 46A-C**

EP 3 622 519 B1

**FIGs. 47A-B**

FIGs. 48A-D

120

FIGs. 49A-C

FIGs. 50A-B

FIG. 51

FIGs. 52A-B

**A**

| | Top-k accuracy | | Precision-Recall AUC | |
|---|---|---|---|---|
| | Acceptor | Donor | Acceptor | Donor |
| Model #1 | 0.9304 | 0.9353 | 0.9630 | 0.9682 |
| Model #2 | 0.9297 | 0.9354 | 0.9649 | 0.9695 |
| Model #3 | 0.9288 | 0.9373 | 0.9650 | 0.9702 |
| Model #4 | 0.9222 | 0.9397 | 0.9616 | 0.9659 |
| Model #5 | 0.9287 | 0.9297 | 0.9640 | 0.9673 |

**C**

| | Top-k accuracy | | Precision-Recall AUC | |
|---|---|---|---|---|
| | Acceptor | Donor | Acceptor | Donor |
| 1 model | 0.9304 | 0.9353 | 0.9630 | 0.9682 |
| 2 models | 0.9376 | 0.9428 | 0.9694 | 0.9742 |
| 3 models | 0.9409 | 0.9472 | 0.9715 | 0.9758 |
| 4 models | 0.9434 | 0.9474 | 0.9727 | 0.9765 |
| 5 models | 0.9429 | 0.9479 | 0.9737 | 0.9771 |

**B**

FIGs. 53A-C

## A

**SpliceNet-10k**

| | Top-k accuracy | | Precision-Recall AUC | |
|---|---|---|---|---|
| Exon density | Acceptor | Donor | Acceptor | Donor |
| 1 exon | 0.9165 | 0.9292 | 0.9592 | 0.9659 |
| 2 exons | 0.9307 | 0.9357 | 0.9645 | 0.9674 |
| 3 exons | 0.9519 | 0.9512 | 0.9816 | 0.9822 |
| 4 exons | 0.9556 | 0.9540 | 0.9833 | 0.9845 |
| ≥5 exons | 0.9579 | 0.9665 | 0.9826 | 0.9872 |

## B

**MaxEntScan**

| | Top-k accuracy | | Precision-Recall AUC | |
|---|---|---|---|---|
| Exon density | Acceptor | Donor | Acceptor | Donor |
| 1 exon | 0.1834 | 0.2487 | 0.1140 | 0.1660 |
| 2 exons | 0.2180 | 0.3124 | 0.1512 | 0.2375 |
| 3 exons | 0.2616 | 0.3348 | 0.1964 | 0.2773 |
| 4 exons | 0.2950 | 0.3820 | 0.2307 | 0.3347 |
| ≥5 exons | 0.3583 | 0.4652 | 0.3103 | 0.4314 |

**FIGs. 54A-B**

| Tissues | Variant individuals | Control individuals |
|---|---|---|
| Skin - Sun Exposed (Lower leg) | T6MO | OXRK |
| Muscle - Skeletal, Transformed fibroblasts | WHSB | WK11 |
| Artery - Tibial, Lung | XOT4, S341, POMQ | WFON |
| Whole Blood | TMMY | T8EM |

**FIG. 55**

| Gains | | | | Losses | | | |
|---|---|---|---|---|---|---|---|
| SpliceNet-10k | GeneSplicer | NNSplice | MaxEntScan | SpliceNet-10k | GeneSplicer | NNSplice | MaxEntScan |
| 0.1 | 103.5145 | 0.9024 | 0.901 | 0.1 | 1.5183 | 0.1063 | 0 |
| 0.2 | 105.6838 | 0.9288 | 0.9171 | 0.2 | 2.6295 | 0.2315 | 0.0068 |
| 0.3 | 107.1061 | 0.9542 | 0.9419 | 0.3 | 3.4707 | 0.3554 | 0.0312 |
| 0.4 | 108.0655 | 0.967 | 0.9582 | 0.4 | 4.0856 | 0.4625 | 0.0646 |
| 0.5 | 108.9011 | 0.9739 | 0.9694 | 0.5 | 4.6085 | 0.5349 | 0.1003 |
| 0.6 | 109.6138 | 0.9781 | 0.9759 | 0.6 | 5.2064 | 0.6345 | 0.1624 |
| 0.7 | 110.388 | 0.9817 | 0.9826 | 0.7 | 6.4456 | 0.7573 | 0.2838 |
| 0.8 | 111.2775 | 0.9839 | 0.9875 | 0.8 | 8.1053 | 0.8202 | 0.4271 |
| 0.9 | 112.6826 | 0.9858 | 0.9915 | 0.9 | 10.2586 | 0.8529 | 0.641 |

**FIG. 56**

EP 3 622 519 B1

# Per-Gene Enrichment Analysis

Observed Trinucleotide Mutation Rates  $10^{-8}$   $10^{-2}$   $10^{-1}$   $10^{5}$   $10^{1}$

**Candidate Variants**

*Classified as Causing Aberrant Splicing*

Particular Gene Sampled from
a Cohort of 1000 Individuals with a Genetic Disorder

**De Novo Variants**

$$\sum (observed\ trinucleotide\ mutation\ rates) = 10^{-5}$$

Transmission (Chromosome) Count

Baseline Number of Mutations $= 2 \times 1000 \times 10^{-5} = 0.2$

Cohort Size

De Novo Variant Count $= 3$

**FIG. 57**

EP 3 622 519 B1

# Genome-Wide Enrichment Analysis

Cohort of 2000 Healthy Individuals
Classified as Causing Aberrant Splicing

Over a <u>Plurality of Genes</u>

Cohort of 1000 Affected Individuals
Classified as Causing Aberrant Splicing

Mutation Rate in Healthy Cohort = 2/2000 = 0.001

Mutation Rate in Affected Cohort = 4/1000 = 0.004

Per-Individual Mutation Ratio = 0.004/0.001 = 4

**FIG. 58**

EP 3 622 519 B1

Computer System

Storage Subsystem

Memory Subsystem

ROM

RAM

File Storage
Subsystem

Neural Networks

User Interface
Input Devices

Bus Subsystem

CPU

Network Interface

User Interface
Output Devices

Deep Learning
Processors
(GPU, FPGA)

EP 3 622 519 B1

**FIG. 59**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62573125, Kishore Jaganathan, Kai-How Farh, Sofia Kyriazopoulou Panagiotopoulou and Jeremy Francis McRae
- US 62573131, Kishore Jaganathan, Kai-How Farh, Sofia Kyriazopoulou Panagiotopoulou and Jeremy Francis McRae
- US 62573135, Kishore Jaganathan, Kai-How Farh, Sofia Kyriazopoulou Panagiotopoulou and Jeremy Francis McRae
- US 62726158, Kishore Jaganathan, Kai-How Farh, Sofia Kyriazopoulou Panagiotopoulou and Jeremy Francis McRae
- WO 07010252 A
- GB 2007003798 W
- US 20090088327 A
- US 20160085910
- US 20130296175
- WO 04018497 A
- US 7057026 B
- WO 9106678 A
- WO 07123744 A
- US 7329492 B
- US 7211414 B
- US 7315019 B
- US 7405281 B
- US 5641658 A
- US 20020055100
- US 7115400 B
- US 20040096853
- US 20040002090
- US 20070128624
- US 20080009420
- US 20070099208 A1
- US 20070166705 A1
- US 20080280773 A1
- US 018255
- WO 004018497 A
- US 2013030867 W
- WO 2014142831 A

### Non-patent literature cited in the description

- **XUEQIU JIAN et al.** *In silico prediction of splice-altering single nucleotide variants in the human genome*
- **T. CHING et al.** *Opportunities And Obstacles For Deep Learning In Biology And Medicine,* 2017, www.biorxiv.org:142760
- **ANGERMUELLER C ; PÄRNAMAA T ; PARTS L ; STEGLE O.** Deep Learning For Computational Biology. *Mol Syst Biol.,* 2016, vol. 12, 878
- **PARK Y ; KELLIS M.** Deep Learning For Regulatory Genomics. *Nat. Biotechnol.,* 2015, vol. 33, 825-826
- **MIN, S. ; LEE, B. ; YOON, S.** Deep Learning In Bioinformatics. *Brief. Bioinform.,* 2016, bbw068
- **LEUNG MK ; DELONG A ; ALIPANAHI B et al.** *Machine Learning In Genomic Medicine: A Review of Computational Problems and Data Sets,* 2016
- **LIBBRECHT MW ; NOBLE WS.** Machine Learning Applications In Genetics and Genomics. *Nature Reviews Genetics,* 2015, vol. 16 (6), 321-32
- **BENTLEY et al.** *Nature,* 2008, vol. 456, 53-59
- **LIZARDI et al.** *Nat. Genet.,* 1998, vol. 19, 225-232
- **DUNN, TAMSEN ; BERRY, GWENN ; EMIG-AGIUS, DOROTHEA ; JIANG, YU ; IYER, ANITA ; UDAR, NITIN ; STRÖMBERG, MICHAEL.** *Pisces: An Accurate and Versatile Single Sample Somatic and Germline Variant Caller,* 2017, 595-595
- **T SAUNDERS, CHRISTOPHER ; WONG, WENDY ; SWAMY, SAJANI ; BECQ, JENNIFER ; J MURRAY, LISA ; CHEETHAM, KEIRA.** Strelka: Accurate somatic small-variant calling from sequenced tumor-normal sample pairs. *Bioinformatics (Oxford, England),* 2012
- **KIM, S. ; SCHEFFLER, K. ; HALPERN, A.L. ; BEKRITSKY, M.A. ; NOH, E. ; KÄLLBERG, M. ; CHEN, X. ; BEYTER, D. ; KRUSCHE, P. ; SAUNDERS, C.T.** *Strelka2: Fast and accurate variant calling for clinical sequencing applications,* 2017
- **STROMBERG, MICHAEL ; ROY, RAJAT ; LAJUGIE, JULIEN ; JIANG, YU ; LI, HAOCHEN ; MARGULIES, ELLIOTT.** *Nirvana: Clinical Grade Variant Annotator,* 2017
- **IOSSIFOV et al.** *Nature,* 2014